# EUROPEAN PATENT APPLICATION

(11) **EP 2 075 346 A2**
(43) Date of publication of application: **01.07.2009**
(21) Application number: 09156623.2
(22) Date of filing: 08.01.2003
(51) Int. Cl.: C12Q 1/68, A01N 43/04, C07H 21/04, A61K 31/07

(54) **Gene products differentially expressed in cancerous breast cells and their methods of use**

(30) Priority: 08.01.2002 US 345637 P
(62) Divisional of application: 03729383.4
(71) Applicant: Novartis Vaccines and Diagnostics, Inc., Emeryville, CA 94608 (US)
(72) Inventor: Hansen, Rhonda, Richmond, CA 94806 (US)
(74) Representative: UEXKÜLL & STOLBERG

(57) **Abstract**

The present invention provides polynucleotides, as well as polypeptides encoded thereby, that are differentially expressed in breast cancer cells. These polynucleotides are useful in a variety of diagnostic and therapeutic methods. The present invention further provides methods of reducing growth of breast cancer cells. These methods are useful for treating breast cancer.

## Description

### STATEMENT OF RIGHTS OF INVENTION

The United States Government has certain rights in this invention pursuant to a CRADA (BG98-053) between Chiron Corporation and the University of California, which operates the Lawrence Berkeley National Laboratory for the United States Department of Energy under Contract No. DE-AC03-76SF00098.

### CROSS-REFERENCING

This patent application claims the benefit of U.S. provisional application serial number 60/345,637 filed January 8, 2002, which application is incorporated by reference in its entirety.

### FIELD OF THE INVENTION

The present invention relates to polynucleotides of human origin in substantially isolated form and gene products that are differentially expressed in breast cancer cells, and uses thereof.

### BACKGROUND OF THE INVENTION

Cancer, like many diseases, is not the result of a single, well-defined cause, but rather can be viewed as several diseases, each caused by different aberrations in informational pathways, that ultimately result in apparently similar pathologic phenotypes. Identification of polynucleotides that correspond to genes that are differentially expressed in cancerous, pre-cancerous, or low metastatic potential cells relative to normal cells of the same tissue type, provides the basis for diagnostic tools, facilitates drug discovery by providing for targets for candidate agents, and further serves to identify therapeutic targets for cancer therapies that are more tailored for the type of cancer to be treated.

Identification of differentially expressed gene products also furthers the understanding of the progression and nature of complex diseases such as cancer, and is key to identifying the genetic factors that are responsible for the phenotypes associated with development of, for example, the metastatic phenotype. Identification of gene products that are differentially expressed at various stages, and in various types of cancers, can both provide for early diagnostic tests, and further serve as therapeutic targets. Additionally, the product of a differentially expressed gene can be the basis for screening assays to identify chemotherapeutic agents that modulate its activity (e.g. its expression, biological activity, and the like).

Early disease diagnosis is of central importance to halting disease progression, and reducing morbidity. Analysis of a patient's tumor to identify the gene products that are differentially expressed, and administration of therapeutic agent(s) designed to modulate the activity of those differentially expressed gene products, provides the basis for more specific, rational cancer therapy that may result in diminished adverse side effects relative to conventional therapies. Furthermore, confirmation that a tumor poses less risk to the patient (*e.g.*, that the tumor is benign) can avoid unnecessary therapies. In short, identification of genes and the encoded gene products that are differentially expressed in cancerous cells can provide the basis of therapeutics, diagnostics, prognostics, therametrics, and the like.

Breast cancer is a leading cause of death among women. One of the priorities in breast cancer research is the discovery of new biochemical markers that can be used for diagnosis, prognosis and monitoring of breast cancer. The prognostic usefulness of these markers depends on the ability of the marker to distinguish between patients with breast cancer who require aggressive therapeutic treatment and patients who should be monitored.

While the pathogenesis of breast cancer is unclear, transformation of non-tumorigenic breast epithelium to a malignant phenotype may be the result of genetic factors, especially in women under 30 (Miki, et al., Science, 266: 66-71, 1994). However, it is likely that other, non-genetic factors are also significant in the etiology of the disease. Regardless of its origin, breast cancer morbidity increases significantly if a lesion is not detected early in its progression. Thus, considerable effort has focused on the elucidation of early cellular events surrounding transformation in breast tissue. Such effort has led to the identification of several potential breast cancer markers.

Thus, the identification of new markers associated with breast cancer, and the identification of genes involved in transforming cells into the cancerous phenotype, remains a significant goal in the management of this disease. In exemplary aspects, the invention described herein provides breast cancer diagnostics, prognostics, therametrics, and therapeutics based upon polynucleotides and/or their encoded gene products.

### SUMMARY OF THE INVENTION

The present invention provides methods and compositions useful in detection of cancerous cells, identification of agents that modulate the phenotype of cancerous cells, and identification of therapeutic targets for chemotherapy of cancerous cells. Cancerous breast cells are of particular interest in each of these aspects of the invention. More specifically, the invention provides polynucleotides in substantially isolated form, as well as polypeptides encoded thereby, that are differentially expressed in breast cancer cells. Also provided are antibodies that specifically bind the encoded polypeptides. These polynucleotides, polypeptides and antibodies are thus useful in a variety of diagnostic, therapeutic, and drug discovery methods. In some embodiments, a polynucleotide that is differentially expressed in breast cancer cells can be used in diagnostic assays to detect breast cancer cells. In other embodiments, a polynucleotide that is differentially expressed in breast cancer cells, and/or a polypeptide encoded thereby, is itself a target for therapeutic intervention.

Accordingly, in one aspect the invention provides a method for detecting a cancerous breast cell. In general, the method involves contacting a test sample obtained from a cell that is suspected of being a breast cancer cell with a probe for detecting a gene product differentially expressed in breast cancer. Many embodiments of the invention involve a gene identifiable or comprising a sequence selected from the group consisting of SEQ ID NOS: 1-499, contacting the probe and the gene product for a time sufficient for binding of the probe to the gene product; and comparing a level of binding of the probe to the sample with a level of probe binding to a control sample obtained from a control breast cell of known cancerous state. A modulated (i.e. increased or decreased) level of binding of the probe in the test breast cell sample relative to the level of binding in a control sample is indicative of the cancerous state of the test breast cell. In certain embodiments, the level of binding of the probe in the test cell sample, usually in relation to at least one control gene, is similar to binding of the probe to a cancerous cell sample. In certain other embodiments, the level of binding of the probe in the test cell sample, usually in relation to at least one control gene, is different, i.e. opposite, to binding of the probe to a non-cancerous cell sample. In specific embodiments, the probe is a polynucleotide probe and the gene product is nucleic acid. In other specific embodiments, the gene product is a polypeptide. In further embodiments, the gene product or the probe is immobilized on an array.

In another aspect, the invention provides a method for assessing the cancerous phenotype (*e.g.*, metastasis, metastatic potential, aberrant cellular proliferation, and the like) of a breast cell comprising detecting expression of a gene product in a test breast cell sample,
wherein the gene comprises a sequence selected from the group consisting of SEQ ID NOS: 1-499; and comparing a level of expression of the gene product in the test breast cell sample with a level of expression of the gene in a control cell sample. Comparison of the level of expression of the gene in the test cell sample relative to the level of expression in the control cell sample is indicative of the cancerous phenotype of the test cell sample. In specific embodiments, detection of gene expression is by detecting a level of an RNA transcript in the test cell sample. In other specific embodiments detection of expression of the gene is by detecting a level of a polypeptide in a test sample.

In another aspect, the invention provides a method for suppressing or inhibiting a cancerous phenotype of a cancerous cell, the method comprising introducing into a mammalian cell an expression modulatory agent (e.g. an antisense molecule, small molecule, antibody, neutralizing antibody, inhibitory RNA molecule, etc.) to inhibition of expression of a gene identified by a sequence selected from the group consisting of SEQ ID NOS: 1-499. Inhibition of expression of the gene inhibits development of a cancerous phenotype in the cell. In specific embodiments, the cancerous phenotype is metastasis, aberrant cellular proliferation relative to a normal cell, or loss of contact inhibition of cell growth. In the context of this invention "expression" of a gene is intended to encompass the expression of an activity of a gene product, and, as such, inhibiting expression of a gene includes inhibiting the activity of a product of the gene.

In another aspect, the invention provides a method for assessing the tumor burden of a subject, the method comprising detecting a level of a differentially expressed gene product in a test sample from a subject suspected of or having a tumor, the differentially expressed gene product comprising a sequence selected from the group consisting of SEQ ID NOS: 1-499. Detection of the level of the gene product in the test sample is indicative of the tumor burden in the subject.

In another aspect, the invention provides a method for identifying a gene product as a target for a cancer therapeutic, the method comprising contacting a cancerous cell expressing a candidate gene product with an anti-cancer agent, wherein the candidate gene product corresponds to a sequence selected from the group consisting of SEQ ID NOS: 1-499; and analyzing the effect of the anti-cancer agent upon a biological activity of the candidate gene product and/or upon a cancerous phenotype of the cancerous cell. Modulation of the biological activity of the candidate gene product and modulation of the cancerous phenotype of the cancerous cell indicates the candidate gene product is a target for a cancer therapeutic. In specific embodiments, the cancerous cell is a cancerous breast cell. In other specific embodiments, the inhibitor is an antisense oligonucleotide. In further embodiments, the cancerous phenotype is aberrant cellular proliferation relative to a normal cell, or colony formation due to loss of contact inhibition of cell growth.

In another aspect, the invention provides a method for identifying agents that modulate (i.e. increase or decrease) the biological activity of a gene product differentially expressed in a cancerous cell, the method comprising contacting a candidate agent with a differentially expressed gene product, the differentially expressed gene product corresponding to a sequence selected from the group consisting of SEQ ID NOS: 1-499; and detecting a modulation in a biological activity of the gene product relative to a level of biological activity of the gene product in the absence of the candidate agent. In specific embodiments, the detecting is by identifying an increase or decrease in expression of the differentially expressed gene product. In other specific embodiments, the gene product is mRNA or cDNA prepared from the mRNA gene product. In further embodiments, the gene product is a polypeptide.

In another aspect, the invention provides a method of inhibiting growth of a tumor cell by modulating expression of a gene product, where the gene product is encoded by a gene identified by a sequence selected from the group consisting of: SEQ ID NOS:1-499.

The invention provides a method of determining the cancerous state of a cell, comprising detecting a level of a product of a gene in a test cell wherein said gene is defined by a sequence selected from a group consisting of SEQ ID NOS:1-499 wherein the cancerous state of the test cell is indicated by detection of said level and comparison to a control level of said gene product. In certain embodiments of this method, the gene product is a nucleic acid or a polypeptide. In certain embodiments of this method, the gene product is immobilized on an array. In one embodiment of this method, the control level is a level of said gene product associated with a control cell of known cancerous state. In other embodiments of this method, the known cancerous state is a non-cancerous state. In another embodiment of this method, the level differs from the control level by at least two fold, indicating the test cell is not of the same cancerous state as that indicated by the control level.

The invention also provides a method for detecting a cancerous breast cell in a sample. This method involves detecting a level of a product of a gene in a test sample obtained from a subject wherein said gene is defined by a sequence selected from a group consisting of SEQ ID NOS:1-499, wherein the presence of a cancerous breast cell or metastasized breast cancer cell is indicated by detection of said level and comparison to a control level of said gene product. In certain embodiments of this method, the sample is a sample of breast tissue. In certain other embodiments of this method, the sample is selected from the group consisting of a liver, brain, kidney, lung, bone and skin sample. In one embodiment of this method, the cancerous breast cell is a metastasized cancerous breast cell. In certain other embodiments of the method, the control level is a level of said gene product associated with a control sample containing cells of known cancerous state. In another embodiment, the known cancerous state is a non-cancerous state.

The invention also provides a method for suppressing a cancerous phenotype of a cancerous mammalian cell comprising introducing into said cancerous cell an antisense polynucleotide for inhibition of expression of a gene defined by a sequence selected from the group consisting of SEQ ID NOS: 1-499, wherein inhibition of expression of said gene suppresses development of said cancerous phenotype. In many embodiments of the method, the cancerous phenotype is metastatic. In certain embodiments of the method, the cancerous phenotype is aberrant cellular proliferation relative to a normal cell. In other embodiments of the method, the cancerous phenotype is loss of contact inhibition of cell growth. In certain other embodiments of the method, the cancerous phenotype is aberrant cellular proliferation of a metastasized breast cancer cell relative to a normal cell.

The invention further provides a method for assessing the tumor burden of a subject. This method involves detecting a level of a differentially expressed gene product in a test sample from a subject suspected of having a tumor, the differentially expressed gene product comprising a sequence selected from the group consisting of SEQ ID NOS: 1-499; wherein detection of the level of the gene product in the test sample is indicative of the tumor burden in the subject.

The method further provides a method for identifying a gene product as a target for a cancer therapeutic. This method involves contacting a cancerous cell expressing a candidate gene product with an anti-cancer agent, wherein the candidate gene product corresponds to a sequence selected from the group consisting of SEQ ID NOS: 1-499; and analyzing the effect of the anti-cancer agent upon a biological activity of the candidate gene product and upon a cancerous phenotype of the cancerous cell; wherein modulation of the biological activity of the candidate gene product and modulation of the cancerous phenotype of the cancerous cell indicates the candidate gene product is a target for a cancer therapeutic. In certain embodiments of this method, the cancerous cell is a cancerous breast cell. In other embodiments of this method, the inhibitor is an antisense oligonucleotide. In certain other embodiments of this method, the cancerous phenotype is aberrant cellular proliferation relative to a normal cell. In another embodiment of this method, the cancerous phenotype is colony formation due to loss of contact inhibition of growth.

The invention further provides a method for identifying agents that decrease biological activity of a gene product differentially expressed in a cancerous cell, the method comprising: contacting a candidate agent with a differentially expressed gene product, the differentially expressed gene product corresponding to a sequence selected from the group consisting of SEQ ID NOS: 1-499; and detecting a decrease in a biological activity of the gene product relative to a level of biological activity of the gene product in the absence of the candidate agent. In certain embodiments of this method, the detecting step is by detection of a decrease in expression of the differentially expressed gene product. In certain other embodiments of this method, the gene product is mRNA or a cDNA prepared from the mRNA gene product. In other embodiments of the invention, the gene product is a polypeptide.

The invention further provides a method of inhibiting growth of a tumor cell by modulating expression of a gene product, the gene product being encoded by a gene identified by a sequence selected from the group consisting of: SEQ ID NOS:1-499.

These and other objects, advantages, and features of the invention will become apparent to those persons skilled in the art upon reading the details of the invention as more fully described below.

### BRIEF DESCRIPTION OF THE FIGURES

Fig. 1 is three panels of autoradiographs showing expression of GAK polypeptide in different cell lines.
Fig. 2 is a graph of a hydropathy plot and a table showing the hydrophobic regions of DKFZp566I133.
Fig. 3 is six panels of photographs of MDA-231 cells exposed to C180-7, C180-8 and positive control antisense (AS) and control (RC) oligonucleotides.
Fig. 4 is an alignment of spot ID 22793 and spot ID 26883.
Fig. 5 is a figure of three sequence alignments showing the mapping of each of three sequences onto VMP1 (DKFZ).

### DETAILED DESCRIPTION OF THE INVENTION

The present invention provides polynucleotides, as well as polypeptides encoded thereby, that are differentially expressed in breast cancer cells. Methods are provided in which these polynucleotides and polypeptides are used for detecting and reducing the growth of breast cancer cells. Also provided are methods in which the polynucleotides and polypeptides of the invention are used in a variety of diagnostic and therapeutic applications for breast cancer.

Before the present invention is described, it is to be understood that this invention is not limited to particular embodiments described, as such may, of course, vary. It is also to be understood that the terminology used herein is for the purpose of describing particular embodiments only, and is not intended to be limiting.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications and patent applications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a polynucleotide" includes a plurality of such polynucleotides and reference to "the breast cancer cell" includes reference to one or more cells and equivalents thereof known to those skilled in the art, and so forth.

The publications and applications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Definitions

The terms "polynucleotide" and "nucleic acid", used interchangeably herein, refer to polymeric forms of nucleotides of any length, either ribonucleotides or deoxynucleotides. Thus, these terms include, but are not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. These terms further include, but are not limited to, mRNA or cDNA that comprise intronic sequences (see, *e.g.*, Niwa et al. (1999) Cell 99(7):691-702). The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) Nucl. Acids Res. 24:1841-1848; Chaturvedi et al. (1996) Nucl. Acids Res. 24:2318-2323. A polynucleotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support. The term "polynucleotide" also encompasses peptidic nucleic acids (Pooga et al Curr Cancer Drug Targets. (2001) 1:231-9).

A "gene product" is a biopolymeric product that is expressed or produced by a gene. A gene product may be, for example, an unspliced RNA, an mRNA, a splice variant mRNA, a polypeptide, a post-translationally modified polypeptide, a splice variant polypeptide etc. Also encompassed by this term is biopolymeric products that are made using an RNA gene product as a template (i.e. cDNA of the RNA). A gene product may be made enzymatically, recombinantly, chemically, or within a cell to which the gene is native. In many embodiments, if the gene product is proteinaceous, it exhibits a biological activity. In many embodiments, if the gene product is a nucleic acid, it can be translated into a proteinaceous gene product that exhibits a biological activity.

A composition (e.g. a polynucleotide, polypeptide, antibody, or host cell) that is "isolated" or "in substantially isolated form" refers to a composition that is in an environment different from that in which the composition naturally occurs. For example, a polynucleotide that is in substantially isolated form is outside of the host cell in which the polynucleotide naturally occurs, and could be a purified fragment of DNA, could be part of a heterologous vector, or could be contained within a host cell that is not a host cell from which the polynucleotide naturally occurs. The term "isolated" does not refer to a genomic or cDNA library, whole cell total protein or mRNA preparation, genomic DNA preparation, or an isolated human chromosome. A composition which is in substantially isolated form is usually substantially purified.

As used herein, the term "substantially purified" refers to a compound (*e.g.*, a polynucleotide, a polypeptide or an antibody, etc.,) that is removed from its natural environment and is usually at least 60% free, preferably 75% free, and most preferably 90% free from other components with which it is naturally associated. Thus, for example, a composition containing A is "substantially free of" B when at least 85% by weight of the total A+B in the composition is A. Preferably, A comprises at least about 90% by weight of the total of A+B in the composition, more preferably at least about 95% or even 99% by weight. In the case of polynucleotides, "A" and "B" may be two different genes positioned on different chromosomes or adjacently on the same chromosome, or two isolated cDNA species, for example.

The terms "polypeptide" and "protein", interchangeably used herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

"Heterologous" refers to materials that are derived from different sources (*e.g.*, from different genes, different species, etc.).

As used herein, the terms "a gene that is differentially expressed in a breast cancer cell," and "a polynucleotide that is differentially expressed in a breast cancer cell" are used interchangeably herein, and generally refer to a polynucleotide that represents or corresponds to a gene that is differentially expressed in a cancerous breast cell when compared with a cell of the same cell type that is not cancerous, e.g., mRNA is found at levels at least about 25%, at least about 50% to about 75%, at least about 90%, at least about 1.5-fold, at least about 2-fold, at least about 5-fold, at least about 10-fold, or at least about 50-fold or more, different (*e.g.*, higher or lower). The comparison can be made in tissue, for example, if one is using in situ hybridization or another assay method that allows some degree of discrimination among cell types in the tissue. The comparison may also or alternatively be made between cells removed from their tissue source.

"Differentially expressed polynucleotide" as used herein refers to a nucleic acid molecule (RNA or DNA) comprising a sequence that represents a differentially expressed gene, *e.g.*, the differentially expressed polynucleotide comprises a sequence (*e.g.*, an open reading frame encoding a gene product; a non-coding sequence) that uniquely identifies a differentially expressed gene so that detection of the differentially expressed polynucleotide in a sample is correlated with the presence of a differentially expressed gene in a sample. "Differentially expressed polynucleotides" is also meant to encompass fragments of the disclosed polynucleotides, *e.g.*, fragments retaining biological activity, as well as nucleic acids homologous, substantially similar, or substantially identical (*e.g.*, having about 90% sequence identity) to the disclosed polynucleotides.

By "cyclin G associated kinase", or "GAK" is meant any polypeptide composition that exhibits cyclin G associated kinase activity. Examples of cyclin G associated kinase include the polypeptide defined by NCBI accession number XM_003450, M_005255, NP_005246 and NM_031030. Assays for determining whether a polypeptide has cyclin G associated kinase activity are described in Ausubel et al., eds., 1998, Current Protocols in Molecular Biology, John Wiley & Sons, NY. Variants of the human cyclin G associated kinase that retain biological activity may be produced by, *inter alia*, substituting amino acids that are in equivalent positions between two cyclin G associated kinases, such as the cyclin G associated kinases from rat and humans.

With regard to cyclin G associated kinases, further references of interest include:
Kanaoka et al, FEBS Lett. 1997 Jan 27;402(1):73-80; Kimura et al, Genomics. 1997 Sep 1;44(2):179-87; Greener et al, J Biol Chem. 2000 Jan 14;275(2):1365-70; and Korolchuk et al, Traffic. 2002 Jun;3(6):428-39.

"DKFZ_{P}566I133" and "DKFZ" are used interchangeably herein to refer to a polypeptide composition that exhibits DKFZ_{P}566I133 activity. Assays for determining whether a polypeptide has DKFZ_{P}566I133 activity (i.e. for determining whether DKFZ_{P}566I133 may have intracytoplasmatic vacuole promoting activity) are described in Dusetti et al, (Biochem Biophys Res Commun. 2002 Jan 18;290(2):641-9). Variants of the DKFZ_{P}566I133 that retain biological activity may be produced by, *inter alia,* substituting amino acids that are in equivalent positions between two DKFZ_{P}566I133, such as the DKFZp566I133 from rat and humans. DKFZ is also known as VMP1, or vacuole membrane protein 1.

Alternatively, "DKFZ_{P}566I133", or "DKFZ" refers to an amino acid sequence defined by NCBI accession number NP_112200, AAH09758, NM_138839, and NM_030938, polynucleotides encoding the amino acid sequences set forth in these accession numbers (SEQ ID NO:512 and SEQ ID NO:513, respectively).

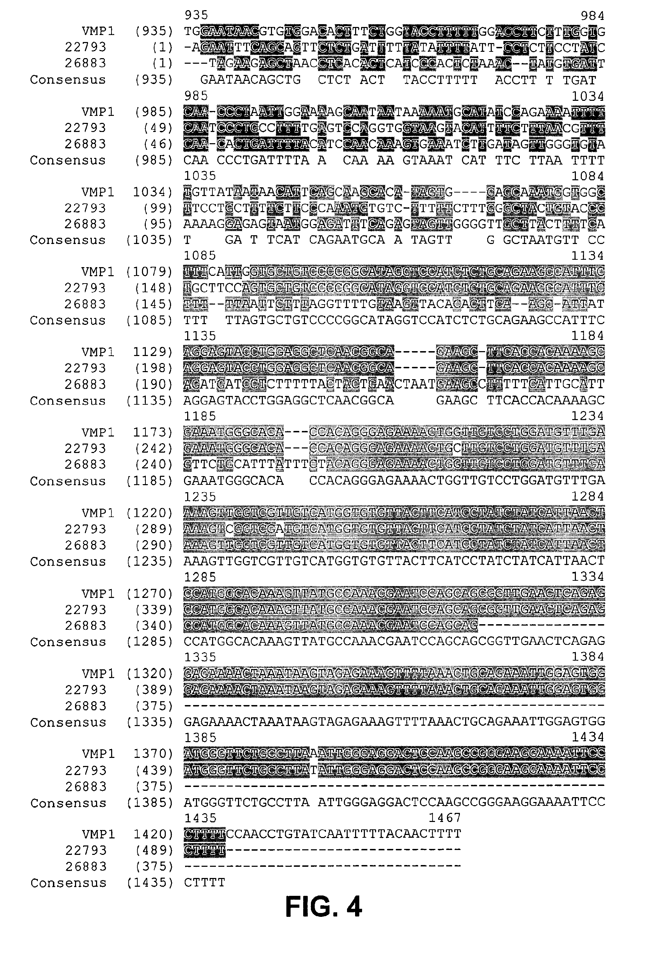

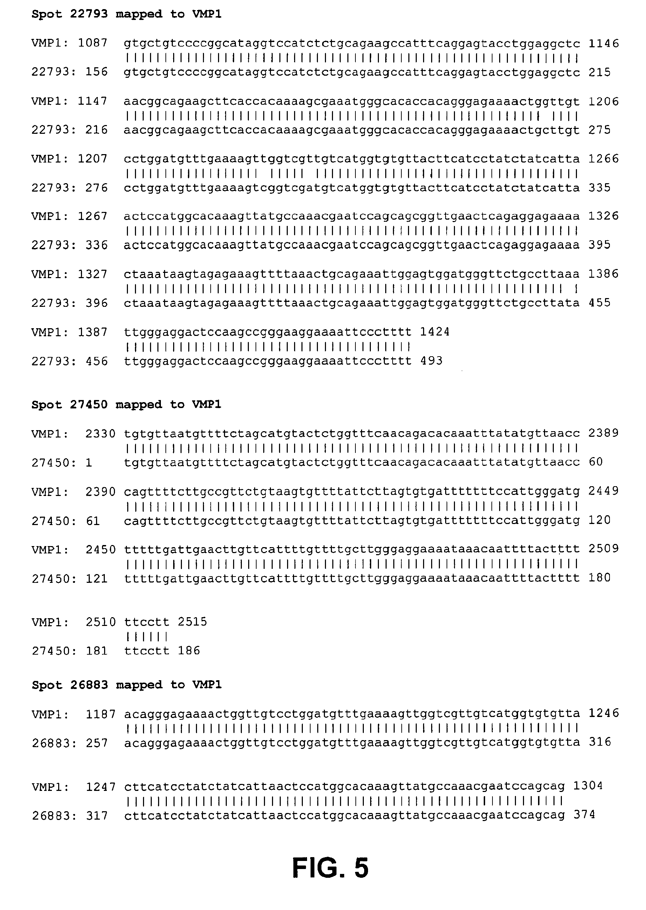

In addition, "DKFZ_{P}566I133", or "DKFZ" refers to the polynucleotide sequences represented by Spot ID NOS 22793, 26883 and 27450 (SEQ ID NOS: 274-275 and SEQ ID NOS: 276-277 and SEQ ID NOS:459-460, respectively). Figure 4 shows an alignment between Spot ID NOS: 22793, 26883 and VMP1 (NM_030938) (i.e. DKFZ), identifying a VMP1 or DKFZ gene product as corresponding to these spot IDs. Figure 5 depicts fragments of Spot ID NOS 22793, 26883, 27450 which align with VMP1 (SEQ ID NOS 514, 515, and 516 respectively). These fragments, or their encoded products, may also be used as a DKFZ identifying sequence.

"Corresponds to" or "represents" when used in the context of, for example, a polynucleotide or sequence that "corresponds to" or "represents" a gene means that at least a portion of a sequence of the polynucleotide is present in the gene or in the nucleic acid gene product (*e.g*., mRNA or cDNA). A subject nucleic acid may also be "identified" by a polynucleotide if the polynucleotide corresponds to or represents the gene. Several genes identified by the polynucleotides of the sequence listing may be found in table 1. Genes identified by a polynucleotide may have all or a portion of the identifying sequence wholly present within an exon of a genomic sequence of the gene, or different portions of the sequence of the polynucleotide may be present in different exons (*e.g.*, such that the contiguous polynucleotide sequence is present in an mRNA, either pre- or post-splicing, that is an expression product of the gene). In some embodiments, the polynucleotide may represent or correspond to a gene that is modified in a cancerous cell relative to a normal cell. The gene in the cancerous cell may contain a deletion, insertion, substitution, or translocation relative to the polynucleotide and may have altered regulatory sequences, or may encode a splice variant gene product, for example. The gene in the cancerous cell may be modified by insertion of an endogenous retrovirus, a transposable element, or other naturally occurring or non-naturally occurring nucleic acid. In most cases, a polynucleotide corresponds to or represents a gene if the sequence of the polynucleotide is most identical to the sequence of a gene or its product (e.g. mRNA or cDNA) as compared to other genes or their products. In most embodiments, the most identical gene is determined using a sequence comparison of a polynucleotide to a database of polynucleotides (e.g. GenBank) using the BLAST program at default settings For example, if the most similar gene in the human genome to an exemplary polynucleotide is the protein kinase C gene, the exemplary polynucleotide corresponds to protein kinase C. In most cases, the sequence of a fragment of an exemplary polynucleotide is at least 95%, 96%, 97%, 98%, 99% or up to 100% identical to a sequence of at least 15, 20, 25, 30, 35, 40, 45, or 50 contiguous nucleotides of a corresponding gene or its product (mRNA or cDNA), when nucleotides that are "N" represent G, A, T or C.

An "identifying sequence" is a minimal fragment of a sequence of contiguous nucleotides that uniquely identifies or defines a polynucleotide sequence or its complement. In many embodiments, a fragment of a polynucleotide uniquely identifies or defines a polynucleotide sequence or its complement. In some embodiments, the entire contiguous sequence of a gene, cDNA, EST, or other provided sequence is an identifying sequence.

"Diagnosis" as used herein generally includes determination of a subject's susceptibility to a disease or disorder, determination as to whether a subject is presently affected by a disease or disorder, prognosis of a subject affected by a disease or disorder (*e.g.*, identification of pre-metastatic or metastatic cancerous states, stages of cancer, or responsiveness of cancer to therapy), and use of therametrics (*e.g.*, monitoring a subject's condition to provide information as to the effect or efficacy of therapy).

As used herein, the term "a polypeptide associated with breast cancer" refers to a polypeptide encoded by a polynucleotide that is differentially expressed in a breast cancer cell. Several examples of polypeptides associated with breast cancer are shown in Table 1.

The term "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

The terms "individual," "subject," "host," and "patient," used interchangeably herein and refer to any mammalian subject for whom diagnosis, treatment, or therapy is desired, particularly humans. Other subjects may include cattle, dogs, cats, guinea pigs, rabbits, rats, mice, horses, and the like.

A "host cell", as used herein, refers to a microorganism or a eukaryotic cell or cell line cultured as a unicellular entity which can be, or has been, used as a recipient for a recombinant vector or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

The terms "cancer", "neoplasm", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection or treatment in the present application include precancerous (*e.g.*, benign), malignant, pre-metastatic, metastatic, and non-metastatic cells. Detection of cancerous cells is of particular interest.

The term "normal" as used in the context of "normal cell," is meant to refer to a cell of an untransformed phenotype or exhibiting a morphology of a non-transformed cell of the tissue type being examined.

"Cancerous phenotype" generally refers to any of a variety of biological phenomena that are characteristic of a cancerous cell, which phenomena can vary with the type of cancer. The cancerous phenotype is generally identified by abnormalities in, for example, cell growth or proliferation (*e*.*g*., uncontrolled growth or proliferation), regulation of the cell cycle, cell mobility, cell-cell interaction, or metastasis, etc. "Therapeutic target" generally refers to a gene or gene product that, upon modulation of its activity (*e*.*g*., by modulation of expression, biological activity, and the like), can provide for modulation of the cancerous phenotype.

As used throughout, "modulation" is meant to refer to an increase or a decrease in the indicated phenomenon (*e*.*g*., modulation of a biological activity refers to an increase in a biological activity or a decrease in a biological activity).

### POLYNUCLEOTIDE COMPOSITIONS

The present invention provides isolated polynucleotides that represent genes that are differentially expressed in breast cancer cells. The polynucleotides, as well as polypeptides encoded thereby, find use in a variety of therapeutic and diagnostic methods.

The scope of the invention with respect to compositions containing the isolated polynucleotides useful in the methods described herein includes, but is not necessarily limited to, polynucleotides having a sequence set forth in any one of the polynucleotide sequences provided herein; polynucleotides obtained from the biological materials described herein or other biological sources (particularly human sources) by hybridization under stringent conditions (particularly conditions of high stringency); genes corresponding to the provided polynucleotides; cDNAs corresponding to the provided polynucleotides; variants of the provided polynucleotides and their corresponding genes, particularly those variants that retain a biological activity of the encoded gene product (*e*.*g*., a biological activity ascribed to a gene product corresponding to the provided polynucleotides as a result of the assignment of the gene product to a protein family(ies) and/or identification of a functional domain present in the gene product). Other nucleic acid compositions contemplated by and within the scope of the present invention will be readily apparent to one of ordinary skill in the art when provided with the disclosure here. "Polynucleotide" and "nucleic acid" as used herein with reference to nucleic acids of the composition is not intended to be limiting as to the length or structure of the nucleic acid unless specifically indicated.

The invention features polynucleotides that represent genes that are expressed in human tissue, specifically human breast tissue, particularly polynucleotides that are differentially expressed in cancerous breast cells. Nucleic acid compositions described herein of particular interest are at least about 15 bp in length, at least about 30 bp in length, at least about 50 bp in length, at least about 100 bp, at least about 200 bp in length, at least about 300 bp in length, at least about 500 bp in length, at least about 800 bp in length, at least about 1 kb in length, at least about 2.0 kb in length, at least about 3.0 kb in length, at least about 5 kb in length, at least about 10 kb in length, at least about 50kb in length and are usually less than about 200 kb in length. These polynucleotides (or polynucleotide fragments) have uses that include, but are not limited to, diagnostic probes and primers as starting materials for probes and primers, as discussed herein.

The subject polynucleotides usually comprise a sequence set forth in any one of the polynucleotide sequences provided herein, for example, in the sequence listing, incorporated by reference in a table (e.g. by an NCBI accession number), a cDNA deposited at the A.T.C.C., or a fragment or variant thereof. A "fragment" or "portion" of a polynucleotide is a contiguous sequence of residues at least about 10 nt to about 12 nt, 15 nt, 16 nt, 18 nt or 20 nt in length, usually at least about 22 nt, 24 nt, 25 nt, 30 nt, 40 nt, 50 nt, 60nt, 70 nt, 80 nt, 90 nt, 100 nt to at least about 150 nt, 200 nt, 250 nt, 300 nt, 350 nt, 400 nt, 500 nt, 800 nt or up to about 1000 nt, 1500 or 2000 nt in length. In some embodiments, a fragment of a polynucleotide is the coding sequence of a polynucleotide. A fragment of a polynucleotide may start at position 1 (i.e. the first nucleotide) of a nucleotide sequence provided herein, or may start at about position 10, 20, 30, 50, 75, 100, 150, 200, 250, 300, 350, 400, 450, 500, 600, 700, 800, 900, 1000, 1500 or 2000, or an ATG translational initiation codon of a nucleotide sequence provided herein. In this context "about" includes the particularly recited value or a value larger or smaller by several (5, 4, 3, 2, or 1) nucleotides. The described polynucleotides and fragments thereof find use as hybridization probes, PCR primers, BLAST probes, or as an identifying sequence, for example.

The subject nucleic acids may be variants or degenerate variants of a sequence provided herein. In general, a variants of a polynucleotide provided herein have a fragment of sequence identity that is greater than at least about 65%, greater than at least about 70%, greater than at least about 75%, greater than at least about 80%, greater than at least about 85%, or greater than at least about 90%, 95%, 96%, 97%, 98%, 99% or more (i.e. 100%) as compared to an identically sized fragment of a provided sequence, as determined by the Smith-Waterman homology search algorithm as implemented in MPSRCH program (Oxford Molecular). For the purposes of this invention, a preferred method of calculating percent identity is the Smith-Waterman algorithm. Global DNA sequence identity should be greater than 65% as determined by the Smith-Waterman homology search algorithm as implemented in MPSRCH program (Oxford Molecular) using an gap search with the following search parameters: gap open penalty, 12; and gap extension penalty, 1.

The subject nucleic acid compositions include full-length cDNAs or mRNAs that encompass an identifying sequence of contiguous nucleotides from any one of the polynucleotide sequences provided herein.

As discussed above, the polynucleotides useful in the methods described herein also include polynucleotide variants having sequence similarity or sequence identity. Nucleic acids having sequence similarity are detected by hybridization under low stringency conditions, for example, at 50°C and 10XSSC (0.9 M saline/0.09 M sodium citrate) and remain bound when subjected to washing at 55°C in 1XSSC. Sequence identity can be determined by hybridization under high stringency conditions, for example, at 50°C or higher and 0.1XSSC (9 mM saline/0.9 mM sodium citrate). Hybridization methods and conditions are well known in the art, see, *e*.*g*., USPN 5,707,829. Nucleic acids that are substantially identical to the provided polynucleotide sequences, *e*.*g*. allelic variants, genetically altered versions of the gene, *etc*., bind to the provided polynucleotide sequences under stringent hybridization conditions. By using probes, particularly labeled probes of DNA sequences, one can isolate homologous or related genes. The source of homologous genes can be any species, *e*.*g*. primate species, particularly human; rodents, such as rats and mice; canines, felines, bovines, ovines, equines, yeast, nematodes, *etc.*

In one embodiment, hybridization is performed using a fragment of at least 15 contiguous nucleotides (nt) of at least one of the polynucleotide sequences provided herein. That is, when at least 15 contiguous nt of one of the disclosed polynucleotide sequences is used as a probe, the probe will preferentially hybridize with a nucleic acid comprising the complementary sequence, allowing the identification and retrieval of the nucleic acids that uniquely hybridize to the selected probe. Probes from more than one polynucleotide sequence provided herein can hybridize with the same nucleic acid if the cDNA from which they were derived corresponds to one mRNA.

Polynucleotides contemplated for use in the invention also include those having a sequence of naturally occurring variants of the nucleotide sequences (*e.g*., degenerate variants (*e.g.*, sequences that encode the same polypeptides but, due to the degenerate nature of the genetic code, different in nucleotide sequence), allelic variants, *etc*.). Variants of the polynucleotides contemplated by the invention are identified by hybridization of putative variants with nucleotide sequences disclosed herein, preferably by hybridization under stringent conditions. For example, by using appropriate wash conditions, variants of the polynucleotides described herein can be identified where the allelic variant exhibits at most about 25-30% base pair (bp) mismatches relative to the selected polynucleotide probe. In general, allelic variants contain 15-25% bp mismatches, and can contain as little as even 5-15%, or 2-5%, or 1-2% bp mismatches, as well as a single bp mismatch.

The invention also encompasses homologs corresponding to any one of the polynucleotide sequences provided herein, where the source of homologous genes can be any mammalian species, *e.g.*, primate species, particularly human; rodents, such as rats; canines, felines, bovines, ovines, equines, yeast, nematodes, etc. Between mammalian species, *e.g*., human and mouse, homologs generally have substantial sequence similarity, *e.g*., at least 75% sequence identity, usually at least 80%%, at least 85, at least 90%, at least 95%, at least 96%, at least 97%, at least 98%, at least 99% or even 100% identity between nucleotide sequences. Sequence similarity is calculated based on a reference sequence, which may be a subset of a larger sequence, such as a conserved motif, coding region, flanking region, *etc*. A reference sequence will usually be at least about a fragment of a polynucleotide sequence and may extend to the complete sequence that is being compared. Algorithms for sequence analysis are known in the art, such as gapped BLAST, described in Altschul, et al. Nucleic Acids Res. (1997) 25:3389-3402, or TeraBLAST available from TimeLogic Corp. (Crystal Bay, Nevada).

Moreover, representative examples of polynucleotide fragments of the invention (useful, for example, as probes), include, for example, fragments comprising, or alternatively consisting of, a sequence from about nucleotide number 1-50, 51-100, 101-150, 151-200, 201-250, 251-300, 301-350, 351-400, 401-450, 451-500, 501-550, 551-600, 651-700,701-750, 751-800, 800-850, 851-900, 901-950,951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951-2000, 2001-2050, 2051-2100, 2101-2150, 2151-2200, 2201-2250, 2251-2300, 2301-2350, 2351-2400, 2401-2450, 2451-2500, 2501-2550, 2551-2600, 2601-2650, 2651-2700, 2701-2750, 2751-2800, 2801-2850, 2851-2900, 2901-2950, 2951-3000, 3001-3050, 3051-3100, 3101-3150, 3151-3200, 3201-3250, 3251-3300, 3301-3350, 3351-3400, 3401-3450, 3451-3500, 3501-3550, 3551-3600, 3601-3650, 3651-3700, 3701-3750, 3751-3800, 3801-3850, 3851-3900, 3901-3950, 3951-4000, 4001-4050, 4051-4100, 4101-4150, 4151-4200, 4201-4250, 4251-4300, 4301-4350, 4351-4400, 4401-4450, 4451-4500, 4501-4550, 4551-4600, 4601-4650, 4651-4700, 4701-4750, 4751-4800, 4801-4850, 4851-4900, 4901-4950, 4951-5000, 5001-5050, 5051-5100, 5101-5150, 5151-5200, 5201-5250, 5251-5300, 5301-5350, 5351-5400, 5401- 5450, 5451-5500, 5501-5550, 5551-5600, 5601-5650, 5651-5700, 5701-5750, 5751-5800, 5801-5850, 5851-5900, 5901-5950, 5951-6000, 6001-6050, 6051-6100, 6101-6150, and 6151 of a subject nucleic acid, or the complementary strand thereto. In this context "about" includes the particularly recited range or a range larger or smaller by several (5, 4, 3, 2, or 1) nucleotides, at either terminus or at both termini. In some embodiments, these fragments encode a polypeptide which has a functional activity (e.g., biological activity) whereas in other embodiments, these fragments are probes, or starting materials for probes. Polynucleotides which hybridize to one or more of these nucleic acid molecules under stringent hybridization conditions or alternatively, under lower stringency conditions, are also encompassed by the invention, as are polypeptides encoded by these polynucleotides or fragments.

The subject nucleic acids can be cDNAs or genomic DNAs, as well as fragments thereof, particularly fragments that encode a biologically active gene product and/or are useful in the methods disclosed herein (e.g., in diagnosis, as a unique identifier of a differentially expressed gene of interest, etc.). The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, being removed by nuclear RNA splicing, to create a continuous open reading frame encoding a polypeptide. mRNA species can also exist with both exons and introns, where the introns may be removed by alternative splicing. Furthermore it should be noted that different species of mRNAs encoded by the same genomic sequence can exist at varying levels in a cell, and detection of these various levels of mRNA species can be indicative of differential expression of the encoded gene product in the cell.

A genomic sequence of interest comprises the nucleic acid present between the initiation codon and the stop codon, as defined in the listed sequences, including all of the introns that are normally present in a native chromosome. It can further include the 3' and 5' untranslated regions found in the mature mRNA. It can further include specific transcriptional and translational regulatory sequences, such as promoters, enhancers, *etc.*, including about 1 kb, but possibly more, of flanking genomic DNA at either the 5' and 3' end of the transcribed region. The genomic DNA can be isolated as a fragment of 100 kbp or smaller; and substantially free of flanking chromosomal sequence. The genomic DNA flanking the coding region, either 3' and 5', or internal regulatory sequences as sometimes found in introns, contains sequences required for proper tissue, stage-specific, or disease-state specific expression.

The nucleic acid compositions of the subject invention can encode all or a part of the naturally-occurring polypeptides. Double or single stranded fragments can be obtained from the DNA sequence by chemically synthesizing oligonucleotides in accordance with conventional methods, by restriction enzyme digestion, by PCR amplification, *etc*.

Probes specific to the polynucleotides described herein can be generated using the polynucleotide sequences disclosed herein. The probes are usually a fragment of a polynucleotide sequences provided herein. The probes can be synthesized chemically or can be generated from longer polynucleotides using restriction enzymes. The probes can be labeled, for example, with a radioactive, biotinylated, or fluorescent tag. Preferably, probes are designed based upon an identifying sequence of any one of the polynucleotide sequences provided herein. More preferably, probes are designed based on a contiguous sequence of one of the subject polynucleotides that remain unmasked following application of a masking program for masking low complexity (*e*.*g*., XBLAST, RepeatMasker, etc.) to the sequence., *i*.*e*., one would select an unmasked region, as indicated by the polynucleotides outside the poly-n stretches of the masked sequence produced by the masking program.

The polynucleotides of interest in the subject invention are isolated and obtained in substantial purity, generally as other than an intact chromosome. Usually, the polynucleotides, either as DNA or RNA, will be obtained substantially free of other naturally-occurring nucleic acid sequences that they are usually associated with, generally being at least about 50%, usually at least about 90% pure and are typically "recombinant", *e.g.*, flanked by one or more nucleotides with which it is not normally associated on a naturally occurring chromosome.

The polynucleotides described herein can be provided as a linear molecule or within a circular molecule, and can be provided within autonomously replicating molecules (vectors) or within molecules without replication sequences. Expression of the polynucleotides can be regulated by their own or by other regulatory sequences known in the art. The polynucleotides can be introduced into suitable host cells using a variety of techniques available in the art, such as transferrin polycation-mediated DNA transfer, transfection with naked or encapsulated nucleic acids, liposome-mediated DNA transfer, intracellular transportation of DNA-coated latex beads, protoplast fusion, viral infection, electroporation, gene gun, calcium phosphate-mediated transfection, and the like.

The nucleic acid compositions described herein can be used to, for example, produce polypeptides, as probes for the detection of mRNA in biological samples (*e.g.*, extracts of human cells) or cDNA produced from such samples, to generate additional copies of the polynucleotides, to generate ribozymes or antisense oligonucleotides, and as single stranded DNA probes or as triple-strand forming oligonucleotides. The probes described herein can be used to, for example, determine the presence or absence of any one of the polynucleotide provided herein or variants thereof in a sample. These and other uses are described in more detail below.

### POLYPEPTIDES AND VARIANTS THEREOF

The present invention further provides polypeptides encoded by polynucleotides that represent genes that are differentially expressed in breast cancer cells. Such polypeptides are referred to herein as "polypeptides associated with breast cancer." The polypeptides can be used to generate antibodies specific for a polypeptide associated with breast cancer, which antibodies are in turn useful in diagnostic methods, prognostics methods, therametric methods, and the like as discussed in more detail herein. Polypeptides are also useful as targets for therapeutic intervention, as discussed in more detail herein.

The polypeptides contemplated by the invention include those encoded by the disclosed polynucleotides and the genes to which these polynucleotides correspond, as well as nucleic acids that, by virtue of the degeneracy of the genetic code, are not identical in sequence to the disclosed polynucleotides. Further polypeptides contemplated by the invention include polypeptides that are encoded by polynucleotides that hybridize to polynucleotide of the sequence listing. Thus, the invention includes within its scope a polypeptide encoded by a polynucleotide having the sequence of any one of the polynucleotide sequences provided herein, or a variant thereof.

In general, the term "polypeptide" as used herein refers to both the full length polypeptide encoded by the recited polynucleotide, the polypeptide encoded by the gene represented by the recited polynucleotide, as well as portions or fragments thereof. "Polypeptides" also includes variants of the naturally occurring proteins, where such variants are homologous or substantially similar to the naturally occurring protein, and can be of an origin of the same or different species as the naturally occurring protein (*e.g.*, human, murine, or some other species that naturally expresses the recited polypeptide, usually a mammalian species). In general, variant polypeptides have a sequence that has at least about 80%, usually at least about 90%, and more usually at least about 98% sequence identity with a differentially expressed polypeptide described herein, as measured by BLAST 2.0 using the parameters described above. The variant polypeptides can be naturally or non-naturally glycosylated, i. e., the polypeptide has a glycosylation pattern that differs from the glycosylation pattern found in the corresponding naturally occurring protein.

The invention also encompasses homologs of the disclosed polypeptides (or fragments thereof) where the homologs are isolated from other species, *i*.*e*. other animal or plant species, where such homologs, usually mammalian species, *e.g*. rodents, such as mice, rats; domestic animals, *e.g.*, horse, cow, dog, cat; and humans. By "homolog" is meant a polypeptide having at least about 35%, usually at least about 40% and more usually at least about 60% amino acid sequence identity to a particular differentially expressed protein as identified above, where sequence identity is determined using the BLAST 2.0 algorithm, with the parameters described *supra*.

In general, the polypeptides of interest in the subject invention are provided in a non-naturally occurring environment, *e.g.* are separated from their naturally occurring environment. In certain embodiments, the subject protein is present in a composition that is enriched for the protein as compared to a cell or extract of a cell that naturally produces the protein. As such, isolated polypeptide is provided, where by "isolated" or "in substantially isolated form" is meant that the protein is present in a composition that is substantially free of other polypeptides, where by substantially free is meant that less than 90%, usually less than 60% and more usually less than 50% of the composition is made up of other polypeptides of a cell that the protein is naturally found.

Also within the scope of the invention are variants; variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted.

Variants can be designed so as to retain or have enhanced biological activity of a particular region of the protein (*e*.*g*., a functional domain and/or, where the polypeptide is a member of a protein family, a region associated with a consensus sequence). For example, muteins can be made which are optimized for increased antigenicity, i.e. amino acid variants of a polypeptide may be made that increase the antigenicity of the polypeptide. Selection of amino acid alterations for production of variants can be based upon the accessibility (interior vs. exterior) of the amino acid (*see, e.g.,* Go et al, Int. J. Peptide Protein Res. (1980) *15*:211), the thermostability of the variant polypeptide *(see, e.g.,* Querol et al., Prot. Eng. (1996) 9:265), desired glycosylation sites (see, e.g., Olsen and Thomsen, J. Gen. Microbiol. (1991) 137:579), desired disulfide bridges *(see, e.g.,* Clarke et al., Biochemistry (1993) 32:4322; and Wakarchuk et al., Protein Eng. (1994) 7:1379), desired metal binding sites *(see, e.g.,* Toma et al., Biochemistry (1991) 30:97, and Haezerbrouck et al., Protein Eng. (1993) 6:643), and desired substitutions with in proline loops *(see, e.g*., Masul et al., Appl. Env. Microbiol. (1994) 60:3579). Cysteine-depleted muteins can be produced as disclosed in USPN 4,959,314.Variants also include fragments of the polypeptides disclosed herein, particularly biologically active fragments and/or fragments corresponding to functional domains. Fragments of interest will typically be at least about 10 aa to at least about 15 aa in length, usually at least about 50 aa in length, and can be as long as 300 aa in length or longer, but will usually not exceed about 1000 aa in length, where the fragment will have a stretch of amino acids that is identical to a polypeptide encoded by a polynucleotide having a sequence of any one of the polynucleotide sequences provided herein, or a homolog thereof. The protein variants described herein are encoded by polynucleotides that are within the scope of the invention. The genetic code can be used to select the appropriate codons to construct the corresponding variants.

A fragment of a subject polypeptide is, for example, a polypeptide having an amino acid sequence which is a portion of a subject polypeptide e.g. a polypeptide encoded by a subject polynucleotide that is identified by any one of the sequence of SEQ ID NOS 1 - 499 or its complement. The polypeptide fragments of the invention are preferably at least about 9 aa, at least about 15 aa, and more preferably at least about 20 aa, still more preferably at least about 30 aa, and even more preferably, at least about 40 aa, at least about 50 aa, at least about 75 aa, at least about 100 aa, at least about 125 aa or at least about 150 aa in length. A fragment "at least 20 aa in length," for example, is intended to include 20 or more contiguous amino acids from, for example, the polypeptide encoded by a cDNA, in a cDNA clone contained in a deposited library, or a nucleotide sequence shown in SEQ ID NOS: 1-499 or the complementary stand thereof. In this context "about" includes the particularly recited value or a value larger or smaller by several (5, 4, 3, 2, or 1) amino acids. These polypeptide fragments have uses that include, but are not limited to, production of antibodies as discussed herein. Of course, larger fragments (e.g., at least 150, 175, 200, 250, 500, 600, 1000, or 2000 amino acids in length) are also encompassed by the invention.

Moreover, representative examples of polypeptides fragments of the invention (useful in, for example, as antigens for antibody production), include, for example, fragments comprising, or alternatively consisting of, a sequence from about amino acid number 1-10, 5-10, 10-20, 21-31, 31-40, 41-61, 61-81, 91-120, 121-140, 141-162, 162-200, 201-240, 241-280, 281- 320, 321-360, 360-400, 400-450, 451-500, 500-600, 600-700, 700-800, 800-900 and the like. In this context "about" includes the particularly recited range or a range larger or smaller by several (5, 4, 3, 2, or 1) amino acids, at either terminus or at both termini. In some embodiments, these fragments has a functional activity (e.g., biological activity) whereas in other embodiments, these fragments may be used to make an antibody.

Further polypeptide variants may are described in PCT publications WO/00-55173, WO/01-07611 and WO/02-16429

### VECTORS, HOST CELLS AND PROTEIN PRODUCTION

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides of the invention may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria.

Representative examples of appropriate hosts include,but are not limited to, bacterial cells, such as E. coli, Streptomyces and Salmonella typhimurium cells; fungal cells, such as yeast cells (e.g., Saccharomyces cerevisiae or Pichia pastoris (ATCC Accession No. 201178)); insect cells such as Drosophila S2 and Spodoptera Sf9 cells; animal cells such as CHO, COS, 293, and Bowes melanoma cells; and plant cells. 5 Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNHSA, pNH16a, pNH18A, pNH46A, available from Stratagene Cloning Systems, Inc.; and ptrc99a, pKK223-3, pKK233-3, pDR540, pRITS available from Pharmacia Biotech, Inc. Among preferred eukaryotic vectors are pWLNEO, pSV2CAT, pOG44, pXTl and pSG available from Stratagene; and pSVK3, pBPV, pMSG and pSVL available from Pharmacia. Preferred expression vectors for use in yeast systems include, but are not limited to pYES2, pYDl, pTEFl/Zeo, pYES2/GS, pPICZ, pGAPZ, pGAPZalph, pPIC9, pPIC3.5, pHIL-D2, pHIL-Sl, pPIC3.5K, pPIC9K, and PAO815 (all available from Invitrogen, Carload, CA). Other suitable vectors will be readily apparent to the skilled artisan.

Nucleic acids of interest may be cloned into a suitable vector by route methods. Suitable vectors include plasmids, cosmids, recombinant viral vectors e.g. retroviral vectors, YACs, BACs and the like, phage vectors.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

Suitable methods and compositions for polypeptide expression may be found in PCT publications WO/00-55173, WO/01-07611 and WO/02-16429.

In addition, polypeptides of the invention can be chemically synthesized using techniques known in the art (e.g., see Creighton, 1983, Proteins: Structures and Molecular Principles, W.H. Freeman & Co., N.Y., and Hunkapiller et al., Nature, 310:105-111 (1984)). For example, a polypeptide corresponding to a fragment of a polypeptide can be synthesized by use of a peptide synthesizer. Furthermore, if desired, nonclassical amino acids or chemical amino acid analogs can be introduced as a substitution or addition into the polypeptide sequence. Non-classical amino acids include, but are not limited to, to the D-isomers of the common amino acids, 2,4-diaminobutyric acid, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, 5 hydroxyproline, sarcosine, citrulline, homocitrulline, cysteic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be D (dextrorotary) or L (levorotary).

Non-naturally occurring variants may be produced using art-known mutagenesis techniques, which include, but are not limited to oligonucleotide mediated mutagenesis, alanine scanning, PCR mutagenesis, site directed mutagenesis (see, e.g., Carter et al., Nucl. Acids Res. 73:4331 (1986); and Zoller et al., Nucl. Acids Res. 70:6487 (1982)), cassette mutagenesis (see, e.g., Wells et al., Gene 34:315 (1985)), restriction selection mutagenesis (see, e.g., Wells et al., Philos. Trans. R. Soc. London SerA 377:415 (1986)).

Scanning amino acid analysis can also be employed to identify one or more amino acids along a contiguous sequence. Among the preferred scanning amino acids are relatively small neutral amino acids. Such amino acids include alanine, glycine, serine and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. If alanine substituting does not yield adequate amounts of variant, an isoteric amino acid can be used.

Any cyseine reside not involved in maintaining the proper conformation of a polypeptide may also be substituted, generally with serine, to improve the oxidative stability of the molecule and prevent aberrant crosslinking. Conversely, cysteine bonds may be added to the polypeptide to improve its stability.

The invention additionally, encompasses polypeptides of the present invention which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, NaBH4; acetylation, formylation, oxidation, reduction; metabolic synthesis in the presence of tunicamycin; etc.

Additional post-translational modifications encompassed by the invention include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptides may also be modified with a detectable label, such as an enzymatic, fluorescent, isotopic or affinity label to allow for detection and isolation of the protein.

Also provided by the invention are chemically modified derivatives of the polypeptides of the invention which may provide additional advantages such as increased solubility, stability and circulating time of the polypeptide, or decreased immunogenicity (see U.S. Patent No. 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The 5 polypeptides may be modified at random positions within the molecule, or at predetermined positions within the molecule and may include one, two, three or more attached chemical moieties.

Suitable methods and compositions for production of modified polypeptides may be found in PCT publications WO/00-55173, WO/01-07611 and WO/02-16429.

### ANTIBODIES AND OTHER POLYPEPTIDE OR POLYNUCLEOTIDE BINDING MOLECULES

The present invention further provides antibodies, which may be isolated antibodies, that are specific for a polypeptide encoded by a polynucleotide described herein and/or a polypeptide of a gene that corresponds to a polynucleotide described herein. Antibodies can be provided in a composition comprising the antibody and a buffer and/or a pharmaceutically acceptable excipient. Antibodies specific for a polypeptide associated with breast cancer are useful in a variety of diagnostic and therapeutic methods, as discussed in detail herein.

Gene products, including polypeptides, mRNA (particularly mRNAs having distinct secondary and/or tertiary structures), cDNA, or complete gene, can be prepared and used for raising antibodies for experimental, diagnostic, and therapeutic purposes. Antibodies may be used to identify a gene corresponding to a polynucleotide. The polynucleotide or related cDNA is expressed as described above, and antibodies are prepared. These antibodies are specific to an epitope on the polypeptide encoded by the polynucleotide, and can precipitate or bind to the corresponding native protein in a cell or tissue preparation or in a cell-free extract of an in vitro expression system.

### Antibodies

Further polypeptides of the invention relate to antibodies and T-cell antigen receptors (TCR) which immunospecifically bind a subject polypeptide, subject polypeptide fragment, or variant thereof, and/or an epitope thereof (as determined by immunoassays well known in the art for assaying specific antibody-antigen binding). Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class (e.g., IgG1, IgG2, lgG3, IgG4, IgAl and IgA2) or subclass of immunoglobulin molecule.

Most preferably the antibodies are human antigen-binding antibody fragments of the present invention and include, but are not limited to, Fab. Fab' and F(ab')2, Fd, single-chain Fvs (scFv), single-chain antibodies, disulfide-linked Fvs (sdFv) and fragments comprising either a V_{L} or V_{H} domain. Antigen-binding antibody fragments, including single-chain antibodies, may comprise the variable region(s) alone or in combination with the entirety or a portion of the following: hinge region, C_{H}1, C_{H}2, and C_{H}3 domains. Also included in the invention are antigen-binding fragments also comprising any combination of variable region(s) with a hinge region, C_{H}1, C_{H}2, and C_{H}3 domains. The antibodies of the invention may be from any animal origin including birds and mammals. Preferably, the antibodies are human, murine (e.g., mouse and rat), donkey, ship rabbit, goat, guinea pig, camel, horse, or chicken. As used herein, "human" antibodies include antibodies having the amino acid sequence of a human immunoglobulin and include antibodies isolated from, human immunoglobulin libraries or from animals transgenic for one or more human immunoglobulin and that do not express endogenous immunoglobulins, as described infra and, for example in, U.S. Patent No. 5,939,598 by Kucherlapati et al.

The antibodies of the present invention may be monospecific, bispecific, trispecific or of greater multispecificity. Multispecific antibodies may be specific for different epitopes of a polypeptide of the present invention or may be specific for both a polypeptide of the present invention as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4,474,893; 4,714,681; 4,925,648; 5,573,920; 5,601,819; Kostelny et al., J. Immunol. 148:1547-1553 (1992).

Antibodies of the present invention may be described or specified in terms of the epitope(s) or portion(s) of a polypeptide of the present invention which they recognize or specifically bind. The epitope(s) or polypeptide portion(s) may be specified as described herein, e.g., by N-terminal and C-terminal positions, or by size in contiguous amino acid residues. Antibodies which specifically bind any epitope or polypeptide of the present invention may also be excluded. Therefore, the present invention includes antibodies that specifically bind polypeptides of the present invention, and allows for the exclusion of the same.

Antibodies of the present invention may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide of the present invention are also included in the present invention. In specific embodiments, antibodies of the present invention cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. In a specific embodiment, the above-described cross-reactivity is with respect to any single specific antigenic or immunogenic polypeptide, or combination(s) of 2, 3, 4, 5, or more of the specific antigenic and/or immunogenic polypeptides disclosed herein. Further included in the present invention are antibodies which bind polypeptides encoded by polynucleotides which hybridize to a polynucleotide of the present invention under stringent hybridization conditions (as described herein). Antibodies of the present invention may also be described or specified in terms of their binding affinity to a polypeptide of the invention. Preferred binding affinities include those with a dissociation constant or Kd less than 5 X 10⁻² M, 10⁻² M, 5 X 10⁻³ M, 10⁻³ M, 5 X 10⁻⁴ M, 10⁻⁴ M, 5 X 10⁻⁵ M, 10⁻⁵ M, 5 X 10⁻⁶ M, 10 ⁻⁶M, 5 X 10⁻⁷ M, 10⁻⁷ M, 5 X ID⁻⁸ M, 10⁻⁸ M,5 X 10⁻⁹ M, 10⁻⁹ M, 5 X 10⁻¹⁰ M, 5 X 10⁻¹¹ M, 10⁻¹¹ M, 5 X 10⁻¹² M, 5 X 10⁻¹³ M, 10⁻¹³ M, 5 X 10⁻¹⁴ M, 10⁻¹⁴ M, 5 X 10⁻¹⁵ M, or 10⁻¹⁵ M.

The invention also provides antibodies that competitively inhibit binding of an antibody to an epitope of the invention as determined by any method known in the art for determining competitive binding, for example, the immunoassays described herein. In preferred embodiments, the antibody competitively inhibits binding to the epitope by at least 95%, at least 90%, at least 85 %, at least 80%, at least 75%, at least 70%, at least 60%, or at least 50%.

Methods for making screening, humanizing, and modifying different types of antibody are well known in the art and may be found in PCT publications WO/00-55173, WO/01-07611 and WO/02-16429.

Antibodies of the present invention may act as agonists or antagonists of the polypeptides of the present invention. For example, the present invention includes antibodies which disrupt the receptor/ligand interactions with the polypeptides of the invention either partially or fully. Preferably, antibodies of the present invention bind an antigenic epitope disclosed herein, or a portion thereof. The invention features both receptor-specific antibodies and ligand-specific antibodies. The invention also features receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or its substrate by immunoprecipitation followed by western blot analysis (for example, as described supra). In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

The invention also features receptor-specific antibodies which both prevent ligand binding and receptor activation as well as antibodies that recognize the receptor-ligand complex, and, preferably, do not specifically recognize the unbound receptor or the unbound ligand. Likewise, included in the invention are neutralizing antibodies which bind the ligand and prevent binding of the ligand to the receptor, as well as antibodies which bind the ligand, thereby preventing receptor activation, but do not prevent the ligand from binding the receptor. Further included in the invention are antibodies which activate the receptor. These antibodies may act as receptor agonists, i.e., potentiate or activate either all or a subset of the biological activities of the ligand-mediated receptor activation, for example, by inducing dimerization of the receptor. The antibodies may be specified as agonists, antagonists or inverse agonists for biological activities comprising the specific biological activities of the peptides of the invention disclosed herein. The above antibody agonists can be made using methods known in the art. See, e.g., PCT publication WO 96/40281; U.S. Patent No. 5,811,097; Deng et al., Blood 92(6): 1981-1988 (1998); Chen et al., Cancer Res. 58(16):3668-4998 (1998); Harrop et al., J. Immunol. 161(4): 1786-1794 (1998); Zhu et al., Cancer Res. 58(15):3209-3214 (1998): Yoon et al., J. Immunol. 160(7):3 170-3179 (1998); Prat et al., J. Cell. Sci. 11 1(Pt2):237-247 (1998); Pitard et al., J. Immunol. Methods 205(2): 177-190 (1997); Liautard et al., Cytokine 9(4):233-241 (1997); Carlson et al., J. Biol. Chem. 272(17): 11295-1 1301 (1997); Taryman et al., Neuron 14(4):755-762 (1995); Muller et al., Structure 6(9): 1153-1167 (1998); Bartunek et al., Cytokine 8(1):14-20 (1996) (which are all incorporated by reference herein in their entireties).

Antibodies of the present invention may be used, for example, but not limited to, to purify, detect, and target the polypeptides of the present invention, including both in vitro and in vivo diagnostic and therapeutic methods. For example, the antibodies have use in immunoassays for qualitatively and quantitatively measuring levels of the polypeptides of the present invention in biological samples. See, e.g., Harlow et al., Antibodies: A Laboratory Manual, (Cold Spring Harbor Laboratory Press, 2nd ed. 1988) (incorporated by reference herein in its entirety).

Further methods and compositions involving antibodies may be found in PCT publications WO/00-55173, WO/01-07611 and WO/02-16429.

### Polynucleotides Encoding Antibodies

The invention further provides polynucleotides comprising a nucleotide sequence encoding an antibody of the invention and fragments thereof. The invention also encompasses polynucleotides that hybridize under stringent or alternatively, under lower stringency hybridization conditions, e.g., as defined supra, to polynucleotides that encode an antibody, preferably, that specifically binds to a polypeptide of the invention, preferably, an antibody that binds to a subject polypeptide.

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)). which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably poly A+ RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by' PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties ), to generate antibodies having a different amino acid sequence, for example to create amino acid substitutions, deletions, and/or insertions.

### Methods of Producing Antibodies

The antibodies of the invention can be produced by any method known in the art for the synthesis of antibodies, in particular, by chemical synthesis or preferably, by recombinant expression techniques. Recombinant expression of an antibody of the invention, or fragment, derivative or analog thereof, (e.g., a heavy or light chain of an antibody of the invention or a single chain antibody of the invention), requires construction of an expression vector containing a polynucleotide that encodes the antibody. Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding an antibody molecule of the invention, or a heavy or light chain thereof, or a heavy or light chain variable domain, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody of the invention. Thus, the invention includes host cells containing a polynucleotide encoding an antibody of the invention, or a heavy or light chain thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention. Such host-expression systems represent vehicles by which the coding sequences of interest may be produced and subsequently purified, but also represent cells which may, when transformed or transfected with the appropriate nucleotide coding sequences, express an antibody molecule of the invention in situ. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as Escherichia coli, and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett etal., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2:1791 (1983)), in which the antibody coding sequence may be ligated individually into the vector in frame with the lac Z coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera fnigiperda* ceils. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non- essential region of the viral genome (e.g., region E1 or E3) will result in a recombinant virus that is viable and capable of expressing the antibody molecule in infected hosts, (e.g., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., Methods in Enzymol. 153:51-544 (1987)).

Antibodies production is well known in the art. Exemplary methods and compositions for making antibodies may be found in PCT publications WO/00-55173, WO/01-07611 and WO/02-16429.

### Immunophenotyping

The antibodies of the invention may be utilized for immunophenotyping of cell lines and biological samples. The translation product of the gene of the present invention may be useful as a cell specific marker, or more specifically as a cellular marker that is differentially expressed at various stages of differentiation and/or maturation of particular cell types. Monoclonal antibodies directed against a specific epitope, or combination of epitopes, will allow for the screening of cellular populations expressing the marker. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (See, e.g., U.S. Patent 5,985,660; and Morrison et al. Cell, 96:737-49 (1999)).

These techniques allow for the screening of particular populations of cells, such as might be found with hematological malignancies (i.e. minimal residual disease (MRD) in acute leukemic patients) and "non-self cells in transplantations to prevent Graft-versus-Host Disease (GVHD). Alternatively, these techniques allow for the screening of hematopoietic stem and progenitor cells capable of undergoing proliferation and/or differentiation, as might be found in human umbilical cord blood.

### Assays For Antibody Binding

The antibodies of the invention may be assayed for immunospecific binding by any method known in the art. The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al, eds, 1994, Current Protocols in Molecular Biology, Vol. 1, John Wiley & Sons, Inc., New York, which is incorporated by reference herein in its entirety). Exemplary immunoassays are described briefly below (but are not intended by way of limitation).

Immunoprecipitation protocols generally comprise lysing a population of cells in a lysis buffer such as R1PA buffer (1% NP-40 or Triton X- 100, 1 % sodium deoxycholate, 0.1% SDS, 0.15 M NaCl, 0.01 M sodium phosphate at pH 7.2, 1 % Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4° C, adding protein A and/or protein G sepharose beads to the cell lysate, incubating for about an hour or more at 4° C, washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8%- 20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., 3H or 1251) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e.g., 3H or 1251) in the presence of increasing amounts of an unlabeled second antibody.

### Therapeutic Uses

The present invention is further directed to antibody-based therapies which involve administering antibodies of the invention to an animal, preferably a mammal, and most preferably a human, patient for treating one or more of the disclosed diseases, disorders, or conditions. Therapeutic compounds of the invention include, but are not limited to, antibodies of the invention (including fragments, analogs and derivatives thereof as described herein) and nucleic acids encoding antibodies of the invention (including fragments, analogs and derivatives thereof and anti-idiotypic antibodies as described herein). The antibodies of the invention can be used to treat, inhibit or prevent diseases, disorders or conditions associated with aberrant expression and/or activity of a polypeptide of the invention, including, but not limited to, any one or more of the diseases, disorders, or conditions described herein. The treatment and/or prevention of diseases, disorders, or conditions associated with aberrant expression and/or activity of a polypeptide of the invention includes, but is not limited to, alleviating symptoms associated with those diseases, disorders or conditions. Antibodies of the invention may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

The antibodies of this invention may be advantageously utilized in combination with other monoclonal or chimeric antibodies, or with lymphokines or hematopoietic growth factors (such as, e.g., IL-2, IL-3 and IL-7), for example, which serve to increase the number or activity of effector cells which interact with the antibodies.

The antibodies of the invention may be administered alone or in combination with other types of treatments (e.g., radiation therapy, chemotherapy, hormonal therapy, immunotherapy and anti-tumor agents). Generally, administration of products of a species origin or species reactivity (in the case of antibodies) that is the same species as that of the patient is preferred. Thus, in a preferred embodiment, human antibodies, fragments derivatives, analogs, or nucleic acids, are administered to a human patient for therapy or prophylaxis.

It is preferred to use high affinity and/or potent in vivo inhibiting and/or neutralizing antibodies against polypeptides or polynucleotides of the present invention, fragments or regions thereof, for both immunoassays directed to and therapy of disorders related to polynucleotides or polypeptides, including fragments thereof, of the present invention. Such antibodies, fragments, or regions, will preferably have an affinity for polynucleotides or polypeptides of the invention, including fragments thereof. Preferred binding affinities include those with a dissociation constant or Kd less than 5 X 10¹² M, 10⁻² M, 5 X 10⁻³ M, 10⁻³ M, 5 X 10⁻⁴ M, 10⁻⁴ M, 5 X 10⁻⁵ M, 10⁻⁵ M, 5 X 10⁻⁶ M, 10⁻⁶ M, 5 X 10⁻⁷ M, 10⁻⁷ M, 5 X ID⁻⁸ M, 10⁻⁸ M, 5 X 10⁻⁹ M, 10⁻⁹ M, 5 X 10⁻¹⁰ M, 10⁻¹⁰ M, 5 X 10⁻ⁿ M, 10⁻ⁿ M, 5 X 10⁻¹² M, 10⁻¹² M, 5 X 10⁻¹³ M, 10⁻¹³ M, 5 X 10⁻¹⁴ M, 10⁻¹⁴ M, 5 X 10⁻¹⁵ M, or 10⁻¹⁵ M.

### KITS

Also provided by the subject invention are kits for practicing the subject methods, as described above. The subject kits include at least one or more of: a subject nucleic acid, isolated polypeptide or an antibody thereto. Other optional components of the kit include:
restriction enzymes, control primers and plasmids; buffers, cells, carriers adjuvents etc. The nucleic acids of the kit may also have restrictions sites, multiple cloning sites, primer sites, etc to facilitate their ligation other plasmids. The various components of the kit may be present in separate containers or certain compatible components may be precombined into a single container, as desired. In many embodiments, kits with unit doses of the active agent, e.g. in oral or injectable doses, are provided. In certain embodiments, controls, such as samples from a cancerous or non-cancerous cell are provided by the invention. Further embodiments of the kit include an antibody for a subject polypeptide and a chemotherapeutic agent to be used in combination with the polypeptide as a treatment.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### COMPUTER-RELATED EMBODIMENTS

In general, a library of polynucleotides is a collection of sequence information, which information is provided in either biochemical form (*e.g*., as a collection of polynucleotide molecules), or in electronic form (*e.g*., as a collection of polynucleotide sequences stored in a computer-readable form, as in a computer system and/or as part of a computer program). The sequence information of the polynucleotides can be used in a variety of ways, *e.g.*, as a resource for gene discovery, as a representation of sequences expressed in a selected cell type (*e.g.*, cell type markers), and/or as markers of a given disease or disease state. For example, in the instant case, the sequences of polynucleotides and polypeptides corresponding to genes differentially expressed in cancer, particular in breast cancer, as well as the nucleic acid and amino acid sequences of the genes themselves, can be provided in electronic form in a computer database.

In general, a disease marker is a representation of a gene product that is present in all cells affected by disease either at an increased or decreased level relative to a normal cell (*e*.*g*., a cell of the same or similar type that is not substantially affected by disease). For example, a polynucleotide sequence in a library can be a polynucleotide that represents an mRNA, polypeptide, or other gene product encoded by the polynucleotide, that is either overexpressed or underexpressed in a cancerous breast cell affected by cancer relative to a normal (*i*.*e*., substantially disease-free) breast cell.

The nucleotide sequence information of the library can be embodied in any suitable form, *e*.*g*., electronic or biochemical forms. For example, a library of sequence information embodied in electronic form comprises an accessible computer data file (or, in biochemical form, a collection of nucleic acid molecules) that contains the representative nucleotide sequences of genes that are differentially expressed (*e*.*g*., overexpressed or underexpressed) as between, for example, i) a cancerous cell and a normal cell; ii) a cancerous cell and a dysplastic cell; iii) a cancerous cell and a cell affected by a disease or condition other than cancer; iv) a metastatic cancerous cell and a normal cell and/or non-metastatic cancerous cell; v) a malignant cancerous cell and a non-malignant cancerous cell (or a normal cell) and/or vi) a dysplastic cell relative to a normal cell. Other combinations and comparisons of cells affected by various diseases or stages of disease will be readily apparent to the ordinarily skilled artisan. Biochemical embodiments of the library include a collection of nucleic acids that have the sequences of the genes in the library, where the nucleic acids can correspond to the entire gene in the library or to a fragment thereof, as described in greater detail below.

The polynucleotide libraries of the subject invention generally comprise sequence information of a plurality of polynucleotide sequences, where at least one of the polynucleotides has a sequence of any of sequence described herein. By plurality is meant at least 2, usually at least 3 and can include up to all of the sequences described herein. The length and number of polynucleotides in the library will vary with the nature of the library, *e.g*., if the library is an oligonucleotide array, a cDNA array, a computer database of the sequence information, etc.

Where the library is an electronic library, the nucleic acid sequence information can be present in a variety of media. "Media" refers to a manufacture, other than an isolated nucleic acid molecule, that contains the sequence information of the present invention. Such a manufacture provides the genome sequence or a subset thereof in a form that can be examined by means not directly applicable to the sequence as it exists in a nucleic acid. For example, the nucleotide sequence of the present invention, *e.g*. the nucleic acid sequences of any of the polynucleotides of the sequences described herein, can be recorded on computer readable media, *e*.*g*. any medium that can be read and accessed directly by a computer. Such media include, but are not limited to: magnetic storage media, such as a floppy disc, a hard disc storage medium, and a magnetic tape; optical storage media such as CD-ROM; electrical storage media such as RAM and ROM; and hybrids of these categories such as magnetic/optical storage media.

One of skill in the art can readily appreciate how any of the presently known computer readable mediums can be used to create a manufacture comprising a recording of the present sequence information. "Recorded" refers to a process for storing information on computer readable medium, using any such methods as known in the art. Any convenient data storage structure can be chosen, based on the means used to access the stored information. A variety of data processor programs and formats can be used for storage, *e*.*g*. word processing text file, database format, *etc.* In addition to the sequence information, electronic versions of libraries comprising one or more sequence described herein can be provided in conjunction or connection with other computer-readable information and/or other types of computer-readable files (*e.g.*, searchable files, executable files, *etc,* including, but not limited to, for example, search program software, *etc.*).

By providing the nucleotide sequence in computer readable form, the information can be accessed for a variety of purposes. Computer software to access sequence information (e.g. the NCBI sequence database) is publicly available. For example, the gapped BLAST (Altschul et al., Nucleic Acids Res. (1997) 25:3389-3402) and BLAZE (Brutlag et al., Comp. Chem. (1993) 17:203) search algorithms on a Sybase system, or the TeraBLAST (TimeLogic, Crystal Bay, Nevada) program optionally running on a specialized computer platform available from TimeLogic, can be used to identify open reading frames (ORFs) within the genome that contain homology to ORFs from other organisms.

As used herein, "a computer-based system" refers to the hardware means, software means, and data storage means used to analyze the nucleotide sequence information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means can comprise any manufacture comprising a recording of the present sequence information as described above, or a memory access means that can access such a manufacture. "Search means" refers to one or more programs implemented on the computer-based system, to compare a target sequence or target structural motif, or expression levels of a polynucleotide in a sample, with the stored sequence information. Search means can be used to identify fragments or regions of the genome that match a particular target sequence or target motif. A variety of known algorithms are publicly known and commercially available, *e*.*g*. MacPattern (EMBL), TeraBLAST (TimeLogic), BLASTN and BLASTX (NCBI). A "target sequence" can be any polynucleotide or amino acid sequence of six or more contiguous nucleotides or two or more amino acids, preferably from about 10 to 100 amino acids or from about 30 to 300 nt. A variety of means for comparing nucleic acids or polypeptides may be used to compare accomplish a sequence comparison (e.g., to analyze target sequences, target motifs, or relative expression levels) with the data storage means. A skilled artisan can readily recognize that any one of the publicly available homology search programs can be used to search the computer based systems of the present invention to compare of target sequences and motifs. Computer programs to analyze expression levels in a sample and in controls are also known in the art.

A "target structural motif," or "target motif," refers to any rationally selected sequence or combination of sequences in which the sequence(s) are chosen based on a three-dimensional configuration that is formed upon the folding of the target motif, or on consensus sequences of regulatory or active sites. There are a variety of target motifs known in the art. Protein target motifs include, but are not limited to, enzyme active sites and signal sequences, kinase domains, receptor binding domains, SH2 domains, SH3 domains, phosphorylation sites, protein interaction domains, transmembrane domains, etc. Nucleic acid target motifs include, but are not limited to, hairpin structures, promoter sequences and other expression elements such as binding sites for transcription factors.

A variety of structural formats for the input and output means can be used to input and output the information in the computer-based systems of the present invention. One format for an output means ranks the relative expression levels of different polynucleotides. Such presentation provides a skilled artisan with a ranking of relative expression levels to determine a gene expression profile. A gene expression profile can be generated from, for example, a cDNA library prepared from mRNA isolated from a test cell suspected of being cancerous or pre-cancerous, comparing the sequences or partial sequences of the clones against the sequences in an electronic database, where the sequences of the electronic database represent genes differentially expressed in a cancerous cell, *e.g.*, a cancerous breast cell. The number of clones having a sequence that has substantial similarity to a sequence that represents a gene differentially expressed in a cancerous cell is then determined, and the number of clones corresponding to each of such genes is determined. An increased number of clones that correspond to differentially expressed gene is present in the cDNA library of the test cell (relative to, for example, the number of clones expected in a cDNA of a normal cell) indicates that the test cell is cancerous.

As discussed above, the "library" as used herein also encompasses biochemical libraries of the polynucleotides of the sequences described herein, *e.g.*, collections of nucleic acids representing the provided polynucleotides. The biochemical libraries can take a variety of forms, *e*.*g*., a solution of cDNAs, a pattern of probe nucleic acids stably associated with a surface of a solid support (*i*.*e*., an array) and the like. Of particular interest are nucleic acid arrays in which one or more of the genes described herein is represented by a sequence on the array. By array is meant an article of manufacture that has at least a substrate with at least two distinct nucleic acid targets on one of its surfaces, where the number of distinct nucleic acids can be considerably higher, typically being at least 10 nt, usually at least 20 nt and often at least 25 nt. A variety of different array formats have been developed and are known to those of skill in the art. The arrays of the subject invention find use in a variety of applications, including gene expression analysis, drug screening, mutation analysis and the like, as disclosed in the above-listed exemplary patent documents.

In addition to the above nucleic acid libraries, analogous libraries of polypeptides are also provided, where the polypeptides of the library will represent at least a portion of the polypeptides encoded by a gene corresponding to a sequence described herein.

### DIAGNOSTIC AND OTHER METHODS INVOLVING DETECTION OF DIFFERENTIALLY EXPRESSED GENES

The present invention provides methods of using the polynucleotides described herein in, for example, diagnosis of cancer and classification of cancer cells according to expression profiles. In specific non-limiting embodiments, the methods are useful for detecting breast cancer cells, facilitating diagnosis of cancer and the severity of a cancer (*e.g.*, tumor grade, tumor burden, and the like) in a subject, facilitating a determination of the prognosis of a subject, and assessing the responsiveness of the subject to therapy (*e.g.*, by providing a measure of therapeutic effect through, for example, assessing tumor burden during or following a chemotherapeutic regimen). Detection can be based on detection of a polynucleotide that is differentially expressed in a breast cancer cell, and/or detection of a polypeptide encoded by a polynucleotide that is differentially expressed in a breast cancer cell ("a polypeptide associated with breast cancer"). The detection methods of the invention can be conducted *in vitro* or *in vivo*, on isolated cells, or in whole tissues or a bodily fluid, *e*.*g*., blood, plasma, serum, urine, and the like).

In general, methods of the invention involving detection of a gene product (*e.g.*, mRNA, cDNA generated from such mRNA, and polypeptides) involve contacting a sample with a probe specific for the gene product of interest. "Probe" as used herein in such methods is meant to refer to a molecule that specifically binds a gene product of interest (*e.g.*, the probe binds to the target gene product with a specificity sufficient to distinguish binding to target over non-specific binding to non-target (background) molecules). "Probes" include, but are not necessarily limited to, nucleic acid probes (*e.g.*, DNA, RNA, modified nucleic acid, and the like), antibodies (*e.g.*, antibodies, antibody fragments that retain binding to a target epitope, single chain antibodies, and the like), or other polypeptide, peptide, or molecule (*e.g.*, receptor ligand) that specifically binds a target gene product of interest.

The probe and sample suspected of having the gene product of interest are contacted under conditions suitable for binding of the probe to the gene product. For example, contacting is generally for a time sufficient to allow binding of the probe to the gene product (*e.g.*, from several minutes to a few hours), and at a temperature and conditions of osmolarity and the like that provide for binding of the probe to the gene product at a level that is sufficiently distinguishable from background binding of the probe (*e.g.*, under conditions that minimize non-specific binding). Suitable conditions for probe-target gene product binding can be readily determined using controls and other techniques available and known to one of ordinary skill in the art.

In this embodiment, the probe can be an antibody or other polypeptide, peptide, or molecule (*e.g*., receptor ligand) that specifically binds a target polypeptide of interest.

The detection methods can be provided as part of a kit. Thus, the invention further provides kits for detecting the presence and/or a level of a polynucleotide that is differentially expressed in a breast cancer cell (*e.g.*, by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits of the invention for detecting a polypeptide encoded by a polynucleotide that is differentially expressed in a breast cancer cell comprise a moiety that specifically binds the polypeptide, which may be a specific antibody. The kits of the invention for detecting a polynucleotide that is differentially expressed in a breast cancer cell comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information.

### Detecting a polypeptide encoded by a polynucleotide that is differentially expressed in a breast cancer cell

In some embodiments, methods are provided for a detecting breast cancer cell by detecting in a cell, particularly a breast cell, a polypeptide encoded by a gene differentially expressed in a breast cancer cell. Any of a variety of known methods can be used for detection, including, but not limited to, immunoassay, using an antibody specific for the encoded polypeptide, e.g., by enzyme-linked immunosorbent assay (ELISA), radioimmunoassay (RIA), and the like; and functional assays for the encoded polypeptide, e.g., binding activity or enzymatic activity.

For example, an immunofluorescence assay can be easily performed on cells without first isolating the encoded polypeptide. The cells are first fixed onto a solid support, such as a microscope slide or microtiter well. This fixing step can permeabilize the cell membrane. The permeablization of the cell membrane permits the polypeptide-specific probe (e.g, antibody) to bind. Alternatively, where the polypeptide is secreted or membrane-bound, or is otherwise accessible at the cell-surface (*e.g*., receptors, and other molecule stably-associated with the outer cell membrane or otherwise stably associated with the cell membrane, such permeabilization may not be necessary.

Next, the fixed cells are exposed to an antibody specific for the encoded polypeptide. To increase the sensitivity of the assay, the fixed cells may be further exposed to a second antibody, which is labeled and binds to the first antibody, which is specific for the encoded polypeptide. Typically, the secondary antibody is detectably labeled, e.g., with a fluorescent marker. The cells which express the encoded polypeptide will be fluorescently labeled and easily visualized under the microscope. See, for example, Hashido et al. (1992) Biochem. Biophys. Res. Comm. 187:1241-1248.

As will be readily apparent to the ordinarily skilled artisan upon reading the present specification, the detection methods and other methods described herein can be varied. Such variations are within the intended scope of the invention. For example, in the above detection scheme, the probe for use in detection can be immobilized on a solid support, and the test sample contacted with the immobilized probe. Binding of the test sample to the probe can then be detected in a variety of ways, *e*.*g*., by detecting a detectable label bound to the test sample.

The present invention further provides methods for detecting the presence of and/or measuring a level of a polypeptide in a biological sample, which polypeptide is encoded by a polynucleotide that represents a gene differentially expressed in cancer, particularly in a polynucleotide that represents a gene differentially cancer cell, using a probe specific for the encoded polypeptide. In this embodiment, the probe can be a an antibody or other polypeptide, peptide, or molecule (*e.g.*, receptor ligand) that specifically binds a target polypeptide of interest.

The methods generally comprise: a) contacting the sample with an antibody specific for a differentially expressed polypeptide in a test cell; and b) detecting binding between the antibody and molecules of the sample. The level of antibody binding (either qualitative or quantitative) indicates the cancerous state of the cell. For example, where the differentially expressed gene is increased in cancerous cells, detection of an increased level of antibody binding to the test sample relative to antibody binding level associated with a normal cell indicates that the test cell is cancerous.

Suitable controls include a sample known not to contain the encoded polypeptide; and a sample contacted with an antibody not specific for the encoded polypeptide, e.g., an anti-idiotype antibody. A variety of methods to detect specific antibody-antigen interactions are known in the art and can be used in the method, including, but not limited to, standard immunohistological methods, immunoprecipitation, an enzyme immunoassay, and a radioimmunoassay.

In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., ¹⁵²Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like.

The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins. The support may then be washed with suitable buffers, followed by contacting with a detectably-labeled first specific antibody. Detection methods are known in the art and will be chosen as appropriate to the signal emitted by the detectable label. Detection is generally accomplished in comparison to suitable controls, and to appropriate standards.

In some embodiments, the methods are adapted for use *in vivo,* e.g., to locate or identify sites where breast cancer cells are present. In these embodiments, a detectably-labeled moiety, e.g., an antibody, which is specific for a breast cancer-associated polypeptide is administered to an individual (e.g., by injection), and labeled cells are located using standard imaging techniques, including, but not limited to, magnetic resonance imaging, computed tomography scanning, and the like. In this manner, breast cancer cells are differentially labeled.

### Detecting a polynucleotide that represents a gene differentially expressed in a breast cancer cell

In some embodiments, methods are provided for detecting a breast cancer cell by detecting expression in the cell of a transcript or that is differentially expressed in a breast cancer cell. Any of a variety of known methods can be used for detection, including, but not limited to, detection of a transcript by hybridization with a polynucleotide that hybridizes to a polynucleotide that is differentially expressed in a breast cancer cell; detection of a transcript by a polymerase chain reaction using specific oligonucleotide primers; *in situ* hybridization of a cell using as a probe a polynucleotide that hybridizes to a gene that is differentially expressed in a breast cancer cell and the like.

In many embodiments, the levels of a subject gene product are measured. By measured is meant qualitatively or quantitatively estimating the level of the gene product in a first biological sample either directly (e.g. by determining or estimating absolute levels of gene product) or relatively by comparing the levels to a second control biological sample. In many embodiments the second control biological sample is obtained from an individual not having not having breast cancer. As will be appreciated in the art, once a standard control level of gene expression is known, it can be used repeatedly as a standard for comparison. Other control samples include samples of cancerous breast tissue.

The methods can be used to detect and/or measure mRNA levels of a gene that is differentially expressed in a breast cancer cell. In some embodiments, the methods comprise: a) contacting a sample with a polynucleotide that corresponds to a differentially expressed gene described herein under conditions that allow hybridization; and b) detecting hybridization, if any. Detection of differential hybridization, when compared to a suitable control, is an indication of the presence in the sample of a polynucleotide that is differentially expressed in a breast cancer cell. Appropriate controls include, for example, a sample that is known not to contain a polynucleotide that is differentially expressed in a breast cancer cell. Conditions that allow hybridization are known in the art, and have been described in more detail above.

Detection can also be accomplished by any known method, including, but not limited to, *in situ* hybridization, PCR (polymerase chain reaction), RT-PCR (reverse transcription-PCR), and "Northern" or RNA blotting, arrays, microarrays, etc, or combinations of such techniques, using a suitably labeled polynucleotide. A variety of labels and labeling methods for polynucleotides are known in the art and can be used in the assay methods of the invention. Specific hybridization can be determined by comparison to appropriate controls.

Polynucleotide generally comprising at least 12 contiguous nt of a polynucleotide provided herein, as shown in the Sequence Listing or of the sequences of the genes corresponding to the polynucleotides of the Sequence Listing, are used for a variety of purposes, such as probes for detection of and/or measurement of, transcription levels of a polynucleotide that is differentially expressed in a breast cancer cell. Additional disclosure about preferred regions of the disclosed polynucleotide sequences is found in the Examples. A probe that hybridizes specifically to a polynucleotide disclosed herein should provide a detection signal at least 2-, 5-, 10-, or 20-fold higher than the background hybridization provided with other unrelated sequences. It should be noted that "probe" as used in this context of detection of nucleic acid is meant to refer to a polynucleotide sequence used to detect a differentially expressed gene product in a test sample. As will be readily appreciated by the ordinarily skilled artisan, the probe can be detectably labeled and contacted with, for example, an array comprising immobilized polynucleotides obtained from a test sample (*e.g*., mRNA). Alternatively, the probe can be immobilized on an array and the test sample detectably labeled. These and other variations of the methods of the invention are well within the skill in the art and are within the scope of the invention.

Labeled nucleic acid probes may be used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluorophores, and enzymes. Other examples of nucleotide hybridization assays are described in WO92/02526 and USPN 5,124,246.

PCR is another means for detecting small amounts of target nucleic acids, methods for which may be found in Sambrook, et al. Molecular Cloning: A Laboratory Manual, CSH Press 1989, pp.14.2-14.33.

A detectable label may be included in the amplification reaction. Suitable detectable labels include fluorochromes,(*e.g.* fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)), radioactive labels, (*e*.*g*. ³²P, ³⁵S, ³H, *etc*.), and the like. The label may be a two stage system, where the polynucleotides is conjugated to biotin, haptens, *etc*. having a high affinity binding partner, *e.g*. avidin, specific antibodies, *etc*., where the binding partner is conjugated to a detectable label. The label may be conjugated to one or both of the primers. Alternatively, the pool of nucleotides used in the amplification is labeled, so as to incorporate the label into the amplification product.

### Arrays

Polynucleotide arrays provide a high throughput technique that can assay a large number of polynucleotides or polypeptides in a sample. This technology can be used as a tool to test for differential expression.

A variety of methods of producing arrays, as well as variations of these methods, are known in the art and contemplated for use in the invention. For example, arrays can be created by spotting polynucleotide probes onto a substrate (*e.g.*, glass, nitrocellulose, *etc*.) in a two-dimensional matrix or array having bound probes. The probes can be bound to the substrate by either covalent bonds or by non-specific interactions, such as hydrophobic interactions.

Samples of polynucleotides can be detectably labeled (*e.g.*, using radioactive or fluorescent labels) and then hybridized to the probes. Double stranded polynucleotides, comprising the labeled sample polynucleotides bound to probe polynucleotides, can be detected once the unbound portion of the sample is washed away. Alternatively, the polynucleotides of the test sample can be immobilized on the array, and the probes detectably labeled. Techniques for constructing arrays and methods of using these arrays are described in, for example, Schena et al. (1996) Proc Natl Acad Sci U S A. 93(20):10614-9; Schena et al. (1995) Science 270(5235):467-70; Shalon et al. (1996) Genome Res. 6(7):639-45, USPN 5,807,522, EP 799 897; WO 97/29212; WO 97/27317; EP 785 280; WO 97/02357; USPN 5,593,839; USPN 5,578,832; EP 728 520; USPN 5,599,695; EP 721 016; USPN 5,556,752; WO 95/22058; and USPN 5,631,734. In most embodiments, the "probe" is detectably labeled. In other embodiments, the probe is immobilized on the array and not detectably labeled.

Arrays can be used, for example, to examine differential expression of genes and can be used to determine gene function. For example, arrays can be used to detect differential expression of a gene corresponding to a polynucleotide described herein, where expression is compared between a test cell and control cell (*e.g*., cancer cells and normal cells). For example, high expression of a particular message in a cancer cell, which is not observed in a corresponding normal cell, can indicate a cancer specific gene product. Exemplary uses of arrays are further described in, for example, Pappalarado et al., Sem. Radiation Oncol. (1998) 8:217; and Ramsay, Nature Biotechnol. (1998) 16:40. Furthermore, many variations on methods of detection using arrays are well within the skill in the art and within the scope of the present invention. For example, rather than immobilizing the probe to a solid support, the test sample can be immobilized on a solid support which is then contacted with the probe.

### DIAGNOSIS, PROGNOSIS, ASSESSMENT OF THERAPY (THERAMETRICS), AND MANAGEMENT OF CANCER

The polynucleotides described herein, as well as their gene products and corresponding genes and gene products, are of particular interest as genetic or biochemical markers (e.g., in blood or tissues) that will detect the earliest changes along the carcinogenesis pathway and/or to monitor the efficacy of various therapies and preventive interventions.

For example, the level of expression of certain polynucleotides can be indicative of a poorer prognosis, and therefore warrant more aggressive chemo- or radio-therapy for a patient or vice versa. The correlation of novel surrogate tumor specific features with response to treatment and outcome in patients can define prognostic indicators that allow the design of tailored therapy based on the molecular profile of the tumor. These therapies include antibody targeting, antagonists (*e.g.*, small molecules), and gene therapy.

Determining expression of certain polynucleotides and comparison of a patient's profile with known expression in normal tissue and variants of the disease allows a determination of the best possible treatment for a patient, both in terms of specificity of treatment and in terms of comfort level of the patient. Surrogate tumor markers, such as polynucleotide expression, can also be used to better classify, and thus diagnose and treat, different forms and disease states of cancer. Two classifications widely used in oncology that can benefit from identification of the expression levels of the genes corresponding to the polynucleotides described herein are staging of the cancerous disorder, and grading the nature of the cancerous tissue.

The polynucleotides that correspond to differentially expressed genes, as well as their encoded gene products, can be useful to monitor patients having or susceptible to cancer to detect potentially malignant events at a molecular level before they are detectable at a gross morphological level. In addition, the polynucleotides described herein, as well as the genes corresponding to such polynucleotides, can be useful as therametrics, *e.g*., to assess the effectiveness of therapy by using the polynucleotides or their encoded gene products, to assess, for example, tumor burden in the patient before, during, and after therapy.

Furthermore, a polynucleotide identified as corresponding to a gene that is differentially expressed in, and thus is important for, one type of cancer can also have implications for development or risk of development of other types of cancer, e.g., where a polynucleotide represents a gene differentially expressed across various cancer types. Thus, for example, expression of a polynucleotide corresponding to a gene that has clinical implications for breast cancer can also have clinical implications for metastatic breast cancer, colon cancer, or ovarian cancer.

Staging. Staging is a process used by physicians to describe how advanced the cancerous state is in a patient. Staging assists the physician in determining a prognosis, planning treatment and evaluating the results of such treatment. Staging systems vary with the types of cancer, but generally involve the following "TNM" system: the type of tumor, indicated by T; whether the cancer has metastasized to nearby lymph nodes, indicated by N; and whether the cancer has metastasized to more distant parts of the body, indicated by M. Generally, if a cancer is only detectable in the area of the primary lesion without having spread to any lymph nodes it is called Stage I. If it has spread only to the closest lymph nodes, it is called Stage II. In Stage III, the cancer has generally spread to the lymph nodes in near proximity to the site of the primary lesion. Cancers that have spread to a distant part of the body, such as the liver, bone, brain or other site, are Stage IV, the most advanced stage.

The polynucleotides and corresponding genes and gene products described herein can facilitate fine-tuning of the staging process by identifying markers for the aggressiveness of a cancer, *e.g.* the metastatic potential, as well as the presence in different areas of the body. Thus, a Stage II cancer with a polynucleotide signifying a high metastatic potential cancer can be used to change a borderline Stage II tumor to a Stage III tumor, justifying more aggressive therapy. Conversely, the presence of a polynucleotide signifying a lower metastatic potential allows more conservative staging of a tumor.

One type of breast cancer is ductal carcinoma in situ (DCIS): DCIS is when the breast cancer cells are completely contained within the breast ducts (the channels in the breast that carry milk to the nipple), and have not spread into the surrounding breast tissue. This may also be referred to as non-invasive or intraductal cancer, as the cancer cells have not yet spread into the surrounding breast tissue and so usually have not spread into any other part of the body.

Lobular carcinoma in situ breast cancer (LCIS) means that cell changes are found in the lining of the lobules of the breast. It can be present in both breasts. It is also referred to as non-invasive cancer as it has not spread into the surrounding breast tissue.

Invasive breast cancer can be staged as follows: Stage 1 tumours: these measure less than two centimetres. The lymph glands in the armpit are not affected and there are no signs that the cancer has spread elsewhere in the body; Stage 2 tumours: these measure between two and five centimetres, or the lymph glands in the armpit are affected, or both. However, there are no signs that the cancer has spread further; Stage 3 tumours: these are larger than five centimetres and may be attached to surrounding structures such as the muscle or skin. The lymph glands are usually affected, but there are no signs that the cancer has spread beyond the breast or the lymph glands in the armpit; Stage 4 tumours: these are of any size, but the lymph glands are usually affected and the cancer has spread to other parts of the body. This is secondary breast cancer.

Grading of cancers. Grade is a term used to describe how closely a tumor resembles normal tissue of its same type. The microscopic appearance of a tumor is used to identify tumor grade based on parameters such as cell morphology, cellular organization, and other markers of differentiation. As a general rule, the grade of a tumor corresponds to its rate of growth or aggressiveness, with undifferentiated or high-grade tumors generally being more aggressive than well-differentiated or low-grade tumors.

The polynucleotides of the Sequence Listing, and their corresponding genes and gene products, can be especially valuable in determining the grade of the tumor, as they not only can aid in determining the differentiation status of the cells of a tumor, they can also identify factors other than differentiation that are valuable in determining the aggressiveness of a tumor, such as metastatic potential.

Low grade means that the cancer cells look very like the normal cells of the breast. They are usually slowly growing and are less likely to spread. In high grade tumors the cells look very abnormal. They are likely to grow more quickly and are more likely to spread.

Assessment of proliferation of cells in tumor. The differential expression level of the polynucleotides described herein can facilitate assessment of the rate of proliferation of tumor cells, and thus provide an indicator of the aggressiveness of the rate of tumor growth. For example, assessment of the relative expression levels of genes involved in cell cycle can provide an indication of cellular proliferation, and thus serve as a marker of proliferation.

### Detection of breast cancer.

The polynucleotides corresponding to genes that exhibit the appropriate expression pattern can be used to detect breast cancer in a subject. Breast cancer is one of the most common neoplasms in women, and prevention and early detection are key factors in controlling and curing breast cancer. The expression of appropriate polynucleotides can be used in the diagnosis, prognosis and management of breast cancer. Detection of breast cancer can be determined using expression levels of any of these sequences alone or in combination with the levels of expression of other known cancer genes. Determination of the aggressive nature and/or the metastatic potential of a breast cancer can be determined by comparing levels of one or more gene products of the genes corresponding to the polynucleotides described herein, and comparing total levels of another sequence known to vary in cancerous tissue, *e*.*g*., expression of p53, DCC, ras, FAP (see, e.g., Fearon ER, et al., Cell (1990) 61(5):759; Hamilton SR et al., Cancer (1993) 72:957; Bodmer W, et al., Nat Genet. (1994) 4(3):217; Fearon ER, Ann N YAcad Sci. (1995) 768:101). For example, development of breast cancer can be detected by examining the level of expression of a gene corresponding to a polynucleotides described herein to the levels of oncogenes (*e*.*g*. ras) or tumor suppressor genes (*e*.*g*. FAP or p53). Thus expression of specific marker polynucleotides can be used to discriminate between normal and cancerous breast tissue, to discriminate between breast cancers with different cells of origin, to discriminate between breast cancers with different potential metastatic rates, etc. For a review of other markers of cancer, see, *e*.*g*., Hanahan et al. (2000) Cell 100:57-70.

### Treatment of breast cancer

The invention further provides methods for reducing growth of breast cancer cells. The methods provide for decreasing the expression of a gene that is differentially expressed in a breast cancer cell or decreasing the level of and/or decreasing an activity of a breast cancer-associated polypeptide. In general, the methods comprise contacting a breast cancer cell with a substance that modulates (1) expression of a gene that is differentially expressed in breast cancer; or (2) a level of and/or an activity of a breast cancer-associated polypeptide.

"Reducing growth of breast cancer cells" includes, but is not limited to, reducing proliferation of breast cancer cells, and reducing the incidence of a non-cancerous breast cell becoming a cancerous breast cell. Whether a reduction in breast cancer cell growth has been achieved can be readily determined using any known assay, including, but not limited to, [³H]-thymidine incorporation; counting cell number over a period of time; detecting and/or measuring a marker associated with breast cancer (e.g., PSA).

The present invention provides methods for treating breast cancer, generally comprising administering to an individual in need thereof a substance that reduces breast cancer cell growth, in an amount sufficient to reduce breast cancer cell growth and treat the breast cancer. Whether a substance, or a specific amount of the substance, is effective in treating breast cancer can be assessed using any of a variety of known diagnostic assays for breast cancer, including, but not limited to, proctoscopy, rectal examination, biopsy, contrast radiographic studies, CAT scan, and detection of a tumor marker associated with breast cancer in the blood of the individual (*e*.*g*., PSA (breast-specific antigen)). The substance can be administered systemically or locally. Thus, in some embodiments, the substance is administered locally, and breast cancer growth is decreased at the site of administration. Local administration may be useful in treating, e.g., a solid tumor.

A substance that reduces breast cancer cell growth can be targeted to a breast cancer cell. Thus, in some embodiments, the invention provides a method of delivering a drug to a breast cancer cell, comprising administering a drug-antibody complex to a subject, wherein the antibody is specific for a breast cancer-associated polypeptide, and the drug is one that reduces breast cancer cell growth, a variety of which are known in the art. Targeting can be accomplished by coupling (e.g., linking, directly or via a linker molecule, either covalently or non-covalently, so as to form a drug-antibody complex) a drug to an antibody specific for a breast cancer-associated polypeptide. Methods of coupling a drug to an antibody are well known in the art and need not be elaborated upon herein.

### Tumor classification and patient stratification

The invention further provides for methods of classifying tumors, and thus grouping or "stratifying" patients, according to the expression profile of selected differentially expressed genes in a tumor. Differentially expressed genes can be analyzed for correlation with other differentially expressed genes in a single tumor type or across tumor types. Genes that demonstrate consistent correlation in expression profile in a given cancer cell type (*e*.*g*., in a breast cancer cell or type of breast cancer) can be grouped together, e.g., when one gene is overexpressed in a tumor, a second gene is also usually overexpressed. Tumors can then be classified according to the expression profile of one or more genes selected from one or more groups.

The tumor of each patient in a pool of potential patients can be classified as described above. Patients having similarly classified tumors can then be selected for participation in an investigative or clinical trial of a cancer therapeutic where a homogeneous population is desired. The tumor classification of a patient can also be used in assessing the efficacy of a cancer therapeutic in a heterogeneous patient population. In addition, therapy for a patient having a tumor of a given expression profile can then be selected accordingly.

In another embodiment, differentially expressed gene products (*e*.*g*., polypeptides or polynucleotides encoding such polypeptides) may be effectively used in treatment through vaccination. The growth of cancer cells is naturally limited in part due to immune surveillance. Stimulation of the immune system using a particular tumor-specific antigen enhances the effect towards the tumor expressing the antigen. An active vaccine comprising a polypeptide encoded by the cDNA of this invention would be appropriately administered to subjects having an alteration, e.g., overabundance, of the corresponding RNA, or those predisposed for developing cancer cells with an alteration of the same RNA. Polypeptide antigens are typically combined with an adjuvant as part of a vaccine composition. The vaccine is preferably administered first as a priming dose, and then again as a boosting dose, usually at least four weeks later. Further boosting doses may be given to enhance the effect. The dose and its timing are usually determined by the person responsible for the treatment.

The invention also encompasses the selection of a therapeutic regimen based upon the expression profile of differentially expressed genes in the patient's tumor. For example, a tumor can be analyzed for its expression profile of the genes corresponding to SEQ ID NOS:1-499 as described herein, *e*.*g*., the tumor is analyzed to determine which genes are expressed at elevated levels or at decreased levels relative to normal cells of the same tissue type. The expression patterns of the tumor are then compared to the expression patterns of tumors that respond to a selected therapy. Where the expression profiles of the test tumor cell and the expression profile of a tumor cell of known drug responsivity at least substantially match (*e*.*g*., selected sets of genes at elevated levels in the tumor of known drug responsivity and are also at elevated levels in the test tumor cell), then the therapeutic agent selected for therapy is the drug to which tumors with that expression pattern respond.

### Pattern matching in diagnosis using arrays

In another embodiment, the diagnostic and/or prognostic methods of the invention involve detection of expression of a selected set of genes in a test sample to produce a test expression pattern (TEP). The TEP is compared to a reference expression pattern (REP), which is generated by detection of expression of the selected set of genes in a reference sample (*e*.*g*., a positive or negative control sample). The selected set of genes includes at least one of the genes of the invention, which genes correspond to the polynucleotide sequences described herein. Of particular interest is a selected set of genes that includes gene differentially expressed in the disease for which the test sample is to be screened.

### IDENTIFICATION OF THERAPEUTIC TARGETS AND ANTI-CANCER THERAPEUTIC AGENTS

The present invention also encompasses methods for identification of agents having the ability to modulate activity of a differentially expressed gene product, as well as methods for identifying a differentially expressed gene product as a therapeutic target for treatment of cancer, especially breast cancer.

Identification of compounds that modulate activity of a differentially expressed gene product can be accomplished using any of a variety of drug screening techniques. Such agents are candidates for development of cancer therapies. Of particular interest are screening assays for agents that have tolerable toxicity for normal, non-cancerous human cells. The screening assays of the invention are generally based upon the ability of the agent to modulate an activity of a differentially expressed gene product and/or to inhibit or suppress phenomenon associated with cancer (*e*.*g*., cell proliferation, colony formation, cell cycle arrest, metastasis, and the like).

### Screening of candidate agents

Screening assays can be based upon any of a variety of techniques readily available and known to one of ordinary skill in the art. In general, the screening assays involve contacting a cancerous cell (preferably a cancerous cell such as a cancerous breast cell) with a candidate agent, and assessing the effect upon biological activity of a differentially expressed gene product. The effect upon a biological activity can be detected by, for example, detection of expression of a gene product of a differentially expressed gene (*e*.*g*., a decrease in mRNA or polypeptide levels, would in turn cause a decrease in biological activity of the gene product). Alternatively or in addition, the effect of the candidate agent can be assessed by examining the effect of the candidate agent in a functional assay. For example, where the differentially expressed gene product is an enzyme, then the effect upon biological activity can be assessed by detecting a level of enzymatic activity associated with the differentially expressed gene product. The functional assay will be selected according to the differentially expressed gene product. In general, where the differentially expressed gene is increased in expression in a cancerous cell, agents of interest are those that decrease activity of the differentially expressed gene product.

Assays described infra can be readily adapted in the screening assay embodiments of the invention. Exemplary assays useful in screening candidate agents include, but are not limited to, hybridization-based assays (*e*.*g*., use of nucleic acid probes or primers to assess expression levels), antibody-based assays (*e*.*g*., to assess levels of polypeptide gene products), binding assays (*e*.*g*., to detect interaction of a candidate agent with a differentially expressed polypeptide, which assays may be competitive assays where a natural or synthetic ligand for the polypeptide is available), and the like. Additional exemplary assays include, but are not necessarily limited to, cell proliferation assays, antisense knockout assays, assays to detect inhibition of cell cycle, assays of induction of cell death/apoptosis, and the like. Generally such assays are conducted *in vitro*, but many assays can be adapted for *in vivo* analyses, *e*.*g*., in an animal model of the cancer.

### Identification of therapeutic targets

In another embodiment, the invention contemplates identification of differentially expressed genes and gene products as therapeutic targets. In some respects, this is the converse of the assays described above for identification of agents having activity in modulating (*e*.*g*., decreasing or increasing) activity of a differentially expressed gene product.

In this embodiment, therapeutic targets are identified by examining the effect(s) of an agent that can be demonstrated or has been demonstrated to modulate a cancerous phenotype (*e*.*g*., inhibit or suppress or prevent development of a cancerous phenotype). Such agents are generally referred to herein as an "anti-cancer agent", which agents encompass chemotherapeutic agents. For example, the agent can be an antisense oligonucleotide that is specific for a selected gene transcript. For example, the antisense oligonucleotide may have a sequence corresponding to a sequence of a differentially expressed gene described herein, *e*.*g*., a sequence of one of SEQ ID NOS:1-499.

Assays for identification of therapeutic targets can be conducted in a variety of ways using methods that are well known to one of ordinary skill in the art. For example, a test cancerous cell that expresses or overexpresses a differentially expressed gene is contacted with an anti-cancer agent, the effect upon a cancerous phenotype and a biological activity of the candidate gene product assessed. The biological activity of the candidate gene product can be assayed be examining, for example, modulation of expression of a gene encoding the candidate gene product (*e*.*g*., as detected by, for example, an increase or decrease in transcript levels or polypeptide levels), or modulation of an enzymatic or other activity of the gene product. The cancerous phenotype can be, for example, cellular proliferation, loss of contact inhibition of growth (*e*.*g*., colony formation), tumor growth (*in vitro* or *in vivo),* and the like. Alternatively or in addition, the effect of modulation of a biological activity of the candidate target gene upon cell death/apoptosis or cell cycle regulation can be assessed.

Inhibition or suppression of a cancerous phenotype, or an increase in cell death or apoptosis as a result of modulation of biological activity of a candidate gene product indicates that the candidate gene product is a suitable target for cancer therapy. Assays described infra can be readily adapted for assays for identification of therapeutic targets. Generally such assays are conducted *in vitro*, but many assays can be adapted for *in vivo* analyses, *e*.*g*., in an appropriate, art-accepted animal model of the cancer.

### Candidate agents

The term "agent" as used herein describes any molecule, *e*.*g*. protein or pharmaceutical, with the capability of modulating a biological activity of a gene product of a differentially expressed gene. Generally a plurality of assay mixtures are run in parallel with different agent concentrations to obtain a differential response to the various concentrations. Typically, one of these concentrations serves as a negative control, *i*.*e*. at zero concentration or below the level of detection.

Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts (including extracts from human tissue to identify endogenous factors affecting differentially expressed gene products) are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, *etc*. to produce structural analogs.

Exemplary candidate agents of particular interest include, but are not limited to, antisense and RNAi polynucleotides, and antibodies, soluble receptors, and the like. Antibodies and soluble receptors are of particular interest as candidate agents where the target differentially expressed gene product is secreted or accessible at the cell-surface (*e*.*g*., receptors and other molecule stably-associated with the outer cell membrane).

For method that involve RNAi (RNA interference), a double stranded RNA (dsRNA) molecule is usually used. The dsRNA is prepared to be substantially identical to at least a segment of a subject polynucleotide (e.g. a cDNA or gene). In general, the dsRNA is selected to have at least 70%, 75%, 80%, 85% or 90% sequence identity with the subject polynucleotide over at least a segment of the candidate gene. In other instances, the sequence identity is even higher, such as 95%, 97% or 99%, and in still other instances, there is 100% sequence identity with the subject polynucleotide over at least a segment of the subject polynucleotide. The size of the segment over which there is sequence identity can vary depending upon the size of the subject polynucleotide. In general, however, there is substantial sequence identity over at least 15, 20, 25, 30, 35, 40 or 50 nucleotides. In other instances, there is substantial sequence identity over at least 100, 200, 300, 400, 500 or 1000 nucleotides; in still other instances, there is substantial sequence identity over the entire length of the subject polynucleotide, i.e., the coding and non-coding region of the candidate gene.

Because only substantial sequence similarity between the subject polynucleotide and the dsRNA is necessary, sequence variations between these two species arising from genetic mutations, evolutionary divergence and polymorphisms can be tolerated. Moreover, as described further infra, the dsRNA can include various modified or nucleotide analogs.

Usually the dsRNA consists of two separate complementary RNA strands. However, in some instances, the dsRNA may be formed by a single strand of RNA that is self-complementary, such that the strand loops back upon itself to form a hairpin loop. Regardless of form, RNA duplex formation can occur inside or outside of a cell.

The size of the dsRNA that is utilized varies according to the size of the subject polynucleotide whose expression is to be suppressed and is sufficiently long to be effective in reducing expression of the subject polynucleotide in a cell. Generally, the dsRNA is at least 10-15 nucleotides long. In certain applications, the dsRNA is less than 20, 21, 22, 23, 24 or 25 nucleotides in length. In other instances, the dsRNA is at least 50, 100, 150 or 200 nucleotides in length. The dsRNA can be longer still in certain other applications, such as at least 300, 400, 500 or 600 nucleotides. Typically, the dsRNA is not longer than 3000 nucleotides. The optimal size for any particular subject polynucleotide can be determined by one of ordinary skill in the art without undue experimentation by varying the size of the dsRNA in a systematic fashion and determining whether the size selected is effective in interfering with expression of the subject polynucleotide.

dsRNA can be prepared according to any of a number of methods that are known in the art, including in vitro and in vivo methods, as well as by synthetic chemistry approaches.

*In vitro methods*. Certain methods generally involve inserting the segment corresponding to the candidate gene that is to be transcribed between a promoter or pair of promoters that are oriented to drive transcription of the inserted segment and then utilizing an appropriate RNA polymerase to carry out transcription. One such arrangement involves positioning a DNA fragment corresponding to the candidate gene or segment thereof into a vector such that it is flanked by two opposable polymerase-specific promoters that can be same or different. Transcription from such promoters produces two complementary RNA strands that can subsequently anneal to form the desired dsRNA. Exemplary plasmids for use in such systems include the plasmid (PCR 4.0 TOPO) (available from Invitrogen). Another example is the vector pGEM-T (Promega, Madison, WI) in which the oppositely oriented promoters are T7 and SP6; the T3 promoter can also be utilized.

In a second arrangement, DNA fragments corresponding to the segment of the subject polynucleotide that is to be transcribed is inserted both in the sense and antisense orientation downstream of a single promoter. In this system, the sense and antisense fragments are cotranscribed to generate a single RNA strand that is self-complementary and thus can form dsRNA.

Various other *in vitro* methods have been described. Examples of such methods include, but are not limited to, the methods described by Sadher et al. (Biochem. Int. 14:1015, 1987); by Bhattacharyya (Nature 343:484, 1990); and by Livache, et al. (U.S. Patent No. 5,795,715), each of which is incorporated herein by reference in its entirety.

Single-stranded RNA can also be produced using a combination of enzymatic and organic synthesis or by total organic synthesis. The use of synthetic chemical methods enable one to introduce desired modified nucleotides or nucleotide analogs into the dsRNA.

*In vivo methods*. dsRNA can also be prepared *in vivo* according to a number of established methods (see, e.g., Sambrook, et al. (1989) Molecular Cloning: A Laboratory Manual, 2nd ed.; Transcription and Translation (B.D. Hames, and S.J. Higgins, Eds., 1984); DNA Cloning, volumes I and II (D.N. Glover, Ed., 1985); and Oligonucleotide Synthesis (M.J. Gait, Ed., 1984, each of which is incorporated herein by reference in its entirety).

Once the single-stranded RNA has been formed, the complementary strands are allowed to anneal to form duplex RNA. Transcripts are typically treated with DNAase and further purified according to established protocols to remove proteins. Usually such purification methods are not conducted with phenol:chloroform. The resulting purified transcripts are subsequently dissolved in RNAase free water or a buffer of suitable composition. dsRNA is generated by annealing the sense and anti-sense RNA in vitro. Generally, the strands are initially denatured to keep the strands separate and to avoid self-annealing. During the annealing process, typically certain ratios of the sense and antisense strands are combined to facilitate the annealing process. In some instances, a molar ratio of sense to antisense strands of 3:7 is used; in other instances, a ratio of 4:6 is utilized; and in still other instances, the ratio is 1:1.

The buffer composition utilized during the annealing process can in some instances affect the efficacy of the annealing process and subsequent transfection procedure. While some have indicated that the buffered solution used to carry out the annealing process should include a potassium salt such as potassium chloride (e.g. at a concentration of about 80 mM). In some embodiments, the buffer is substantially postassium free. Once single-stranded RNA has annealed to form duplex RNA, typically any single-strand overhangs are removed using an enzyme that specifically cleaves such overhangs (e.g., RNAase A or RNAase T).

Once the dsRNA has been formed, it is introduced into a reference cell, which can include an individual cell or a population of cells (e.g., a tissue, an embryo and an entire organism). The cell can be from essentially any source, including animal, plant, viral, bacterial, fungal and other sources. If a tissue, the tissue can include dividing or nondividing and differentiated or undifferentiated cells. Further, the tissue can include germ line cells and somatic cells. Examples of differentiated cells that can be utilized include, but are not limited to, neurons, glial cells, blood cells, megakaryocytes, lymphocytes, macrophages, neutrophils, eosinophils, basophils, mast cells, leukocytes, granulocytes, keratinocytes, adipocytes, osteoblasts, osteoclasts, hepatocytes, cells of the endocrine or exocrine glands, fibroblasts, myocytes, cardiomyocytes, and endothelial cells. The cell can be an individual cell of an embryo, and can be a blastocyte or an oocyte.

Certain methods are conducted using model systems for particular cellular states (e.g., a disease). For instance, certain methods provided herein are conducted with a cancer cell lines that serves as a model system for investigating genes that are correlated with various cancers.

A number of options can be utilized to deliver the dsRNA into a cell or population of cells such as in a cell culture, tissue or embryo. For instance, RNA can be directly introduced intracellularly. Various physical methods are generally utilized in such instances, such as administration by microinjection (see, e.g., Zernicka-Goetz, et al. (1997) Development 124:1133-1137; and Wianny, et al. (1998) Chromosoma 107: 430-439).

Other options for cellular delivery include permeabilizing the cell membrane and electroporation in the presence of the dsRNA, liposome-mediated transfection, or transfection using chemicals such as calcium phosphate. A number of established gene therapy techniques can also be utilized to introduce the dsRNA into a cell. By introducing a viral construct within a viral particle, for instance, one can achieve efficient introduction of an expression construct into the cell and transcription of the RNA encoded by the construct.

If the dsRNA is to be introduced into an organism or tissue, gene gun technology is an option that can be employed. This generally involves immobilizing the dsRNA on a gold particle which is subsequently fired into the desired tissue. Research has also shown that mammalian cells have transport mechanisms for taking in dsRNA (see, e.g., Asher, et al. (1969) Nature 223:715-717). Consequently, another delivery option is to administer the dsRNA extracellularly into a body cavity, interstitial space or into the blood system of the mammal for subsequent uptake by such transport processes. The blood and lymph systems and the cerebrospinal fluid are potential sites for injecting dsRNA. Oral, topical, parenteral, rectal and intraperitoneal administration are also possible modes of administration.

The composition introduced can also include various other agents in addition to the dsRNA. Examples of such agents include, but are not limited to, those that stabilize the dsRNA, enhance cellular uptake and/or increase the extent of interference. Typically, the dsRNA is introduced in a buffer that is compatible with the composition of the cell into which the RNA is introduced to prevent the cell from being shocked. The minimum size of the dsRNA that effectively achieves gene silencing can also influence the choice of delivery system and solution composition.

Sufficient dsRNA is introduced into the tissue to cause a detectable change in expression of a taget gene (assuming the candidate gene is in fact being expressed in the cell into which the dsRNA is introduced) using available detection methodologies. Thus, in some instances, sufficient dsRNA is introduced to achieve at least a 5-10% reduction in candidate gene expression as compared to a cell in which the dsRNA is not introduced. In other instances, inhibition is at least 20, 30, 40 or 50%. In still other instances, the inhibition is at least 60, 70, 80, 90 or 95%. Expression in some instances is essentially completely inhibited to undetectable levels.

The amount of dsRNA introduced depends upon various factors such as the mode of administration utilized, the size of the dsRNA, the number of cells into which dsRNA is administered, and the age and size of an animal if dsRNA is introduced into an animal. An appropriate amount can be determined by those of ordinary skill in the art by initially administering dsRNA at several different concentrations for example, for example. In certain instances when dsRNA is introduced into a cell culture, the amount of dsRNA introduced into the cells varies from about 0.5 to 3 µg per 10⁶ cells.

A number of options are available to detect interference of candidate gene expression (i.e., to detect candidate gene silencing). In general, inhibition in expression is detected by detecting a decrease in the level of the protein encoded by the candidate gene, determining the level of mRNA transcribed from the gene and/or detecting a change in phenotype associated with candidate gene expression.

### USE OF POLYPEPTIDES TO SCREEN FOR PEPTIDE ANALOGS AND ANTAGONISTS

Polypeptides encoded by differentially expressed genes identified herein can be used to screen peptide libraries to identify binding partners, such as receptors, from among the encoded polypeptides. Peptide libraries can be synthesized according to methods known in the art (*see*, *e*.*g*., USPN 5,010,175 and WO 91/17823).

Agonists or antagonists of the polypeptides of the invention can be screened using any available method known in the art, such as signal transduction, antibody binding, receptor binding, mitogenic assays, chemotaxis assays, etc. The assay conditions ideally should resemble the conditions under which the native activity is exhibited *in vivo*, that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide can require concentrations equal to or greater than the native concentration, while inhibitors capable of binding irreversibly to the polypeptide can be added in concentrations on the order of the native concentration.

Such screening and experimentation can lead to identification of a polypeptide binding partner, such as a receptor, encoded by a gene or a cDNA corresponding to a polynucleotide described herein, and at least one peptide agonist or antagonist of the binding partner. Such agonists and antagonists can be used to modulate, enhance, or inhibit receptor function in cells to which the receptor is native, or in cells that possess the receptor as a result of genetic engineering. Further, if the receptor shares biologically important characteristics with a known receptor, information about agonist/antagonist binding can facilitate development of improved agonists/antagonists of the known receptor.

### VACCINES AND USES

The differentially expressed nucleic acids and polypeptides produced by the nucleic acids of the invention can also be used to modulate primary immune response to prevent or treat cancer. Every immune response is a complex and intricately regulated sequence of events involving several cell types. It is triggered when an antigen enters the body and encounters a specialized class of cells called antigen-presenting cells (APCs). These APCs capture a minute amount of the antigen and display it in a form that can be recognized by antigen-specific helper T lymphocytes. The helper (Th) cells become activated and, in turn, promote the activation of other classes of lymphocytes, such as B cells or cytotoxic T cells. The activated lymphocytes then proliferate and carry out their specific effector functions, which in many cases successfully activate or eliminate the antigen. Thus, activating the immune response to a particular antigen associated with a cancer cell can protect the patient from developing cancer or result in lymphocytes eliminating cancer cells expressing the antigen.

Gene products, including polypeptides, mRNA (particularly mRNAs having distinct secondary and/or tertiary structures), cDNA, or complete gene, can be prepared and used in vaccines for the treatment or prevention of hyperproliferative disorders and cancers. The nucleic acids and polypeptides can be utilized to enhance the immune response, prevent tumor progression, prevent hyperproliferative cell growth, and the like. Methods for selecting nucleic acids and polypeptides that are capable of enhancing the immune response are known in the art. Preferably, the gene products for use in a vaccine are gene products which are present on the surface of a cell and are recognizable by lymphocytes and antibodies.

The gene products may be formulated with pharmaceutically acceptable carriers into pharmaceutical compositions by methods known in the art. The composition is useful as a vaccine to prevent or treat cancer. The composition may further comprise at least one co-immunostimulatory molecule, including but not limited to one or more major histocompatibility complex (MHC) molecules, such as a class I or class II molecule, preferably a class I molecule. The composition may further comprise other stimulator molecules including B7.1, B7.2, ICAM-1, ICAM-2, LFA-1, LFA-3, CD72 and the like, immunostimulatory polynucleotides (which comprise an 5'-CG-3' wherein the cytosine is unmethylated), and cytokines which include but are not limited to IL-1 through IL-15, TNF-α, IFN-γ, RANTES, G-CSF, M-CSF, IFN-α, CTAP III, ENA-78, GRO, I-309, PF-4, IP-10, LD-78, MGSA, MIP-1α, MIP-1β, or combination thereof, and the like for immunopotentiation. In one embodiment, the immunopotentiators of particular interest are those that facilitate a Th1 immune response.

The gene products may also be prepared with a carrier that will protect the gene products against rapid elimination from the body, such as a controlled release formulation, including implants and microencapsulated delivery systems. Biodegradable polymers can be used, such as ethylene vinyl acetate, polyanhydrides, polyglycolic acid, collagen, polyorthoesters, polylactic acid, and the like. Methods for preparation of such formulations are known in the art.

In the methods of preventing or treating cancer, the gene products may be administered via one of several routes including but not limited to transdermal, transmucosal, intravenous, intramuscular, subcutaneous, intradermal, intraperitoneal, intrathecal, intrapleural, intrauterine, rectal, vaginal, topical, intratumor, and the like. For transmucosal or transdermal administration, penetrants appropriate to the barrier to be permeated are used in the formulation. Such penetrants are generally known in the art, and include, for example, administration bile salts and fusidic acid derivatives. In addition, detergents may be used to facilitate permeation. Transmucosal administration may be by nasal sprays or suppositories. For oral administration, the gene products are formulated into conventional oral administration form such as capsules, tablets, elixirs and the like.

The gene product is administered to a patient in an amount effective to prevent or treat cancer. In general, it is desirable to provide the patient with a dosage of gene product of at least about 1 pg per Kg body weight, preferably at least about 1 ng per Kg body weight, more preferably at least about 1 µg or greater per Kg body weight of the recipient. A range of from about 1 ng per Kg body weight to about 100 mg per Kg body weight is preferred although a lower or higher dose may be administered. The dose is effective to prime, stimulate and/or cause the clonal expansion of antigen-specific T lymphocytes, preferably cytotoxic T lymphocytes, which in turn are capable of preventing or treating cancer in the recipient. The dose is administered at least once and may be provided as a bolus or a continuous administration. Multiple administrations of the dose over a period of several weeks to months may be preferable. Subsequent doses may be administered as indicated.

In another method of treatment, autologous cytotoxic lymphocytes or tumor infiltrating lymphocytes may be obtained from a patient with cancer. The lymphocytes are grown in culture, and antigen-specific lymphocytes are expanded by culturing in the presence of the specific gene products alone or in combination with at least one co-immunostimulatory molecule with cytokines. The antigen-specific lymphocytes are then infused back into the patient in an amount effective to reduce or eliminate the tumors in the patient. Cancer vaccines and their uses are further described in USPN 5,961,978; USPN 5,993,829; USPN 6,132,980; and WO 00/38706.

### PHARMACEUTICAL COMPOSITIONS AND USES

Pharmaceutical compositions can comprise polypeptides, receptors that specifically bind a polypeptide produced by a differentially expressed gene (*e*.*g*., antibodies, or polynucleotides (including antisense nucleotides and ribozymes) of the claimed invention in a therapeutically effective amount. The compositions can be used to treat primary tumors as well as metastases of primary tumors. In addition, the pharmaceutical compositions can be used in conjunction with conventional methods of cancer treatment, *e*.*g*., to sensitize tumors to radiation or conventional chemotherapy.

Where the pharmaceutical composition comprises a receptor (such as an antibody) that specifically binds to a gene product encoded by a differentially expressed gene, the receptor can be coupled to a drug for delivery to a treatment site or coupled to a detectable label to facilitate imaging of a site comprising breast cancer cells. Methods for coupling antibodies to drugs and detectable labels are well known in the art, as are methods for imaging using detectable labels.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature.

The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation is determined by routine experimentation and is within the judgment of the clinician. For purposes of the present invention, an effective dose will generally be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, lipid aggregates and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles.

Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins.

### DELIVERY METHODS

Once formulated, the compositions contemplated by the invention can be (1) administered directly to the subject (*e*.*g*., as polynucleotide, polypeptides, small molecule agonists or antagonists, and the like); or (2) delivered ex vivo, to cells derived from the subject (*e*.*g*., as in *ex vivo* gene therapy). Direct delivery of the compositions will generally be accomplished by parenteral injection, e.g., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the ex vivo delivery and reimplantation of transformed cells into a subject are known in the art and described in *e*.*g*., International Publication No. WO 93/14778. Examples of cells useful in ex vivo applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once differential expression of a gene corresponding to a polynucleotide described herein has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (e.g., antisense, ribozyme, etc.). In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonist for gene products that are decreased in expression in cancerous cells (*e*.*g*., to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic composition agents includes local or systemic administration, including injection, oral administration, particle gun or catheterized administration, and topical administration. In general, the therapeutic polynucleotide composition contains an expression construct comprising a promoter operably linked to a polynucleotide of at least 12, 22, 25, 30, or 35 contiguous nt of the polynucleotide disclosed herein. Various methods can be used to administer the therapeutic composition directly to a specific site in the body. For example, a small metastatic lesion is located and the therapeutic composition injected several times in several different locations within the body of the tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655; Wu et al., J. Biol. Chem. (1991) 266:338. Therapeutic compositions containing a polynucleotide are administered in a range of about 100 ng to about 200 mg of DNA for local administration in a gene therapy protocol. Concentration ranges of about 500 ng to about 50 mg, about 1 µg to about 2 mg, about 5 µg to about 500 µg, and about 20 µg to about 100 µg of DNA can also be used during a gene therapy protocol. Factors such as method of action (e.g., for enhancing or inhibiting levels of the encoded gene product) and efficacy of transformation and expression are considerations that will affect the dosage required for ultimate efficacy of the antisense subgenomic polynucleotides.

Where greater expression is desired over a larger area of tissue, larger amounts of antisense subgenomic polynucleotides or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. For polynucleotide related genes encoding polypeptides or proteins with anti-inflammatory activity, suitable use, doses, and administration are described in USPN 5,654,173.

The therapeutic polynucleotides and polypeptides of the present invention can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., WO 90/07936; WO 94/03622; WO 93/25698; WO 93/25234; USPN 5, 219,740; WO 93/11230; WO 93/10218; USPN 4,777,127; GB Patent No. 2,200,651; EP 0 345 242; and WO 91/02805), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., USPN 5,814,482; WO 95/07994; WO 96/17072; WO 95/30763; and WO 97/42338) and nucleic charge neutralization or fusion with cell membranes. Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581.

Further non-viral delivery suitable for use includes mechanical delivery systems such as the approach described in Woffendin et al., Proc. Natl. Acad. Sci. USA (1994) 91(24):11581. Moreover, the coding sequence and the product of expression of such can be delivered through deposition of photopolymerized hydrogel materials or use of ionizing radiation (see, e.g., USPN 5,206,152 and WO 92/11033). Other conventional methods for gene delivery that can be used for delivery of the coding sequence include, for example, use of hand-held gene transfer particle gun (see, e.g., USPN 5,149,655); use of ionizing radiation for activating transferred gene (see, e.g., USPN 5,206,152 and WO 92/11033).

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### EXAMPLE 1:

### SOURCE OF BIOLOGICAL MATERIALS

The cells used for detecting differential expression of breast cancer related genes were those previously described for the HMT-3522 tumor reversion model, disclosed in U.S. Patent Nos. 5,846,536 and 6,123,941, herein incorporated by reference. The model utilizes both non-tumorigenic (HMT-3522 S1) and tumorigenic (HMT-3522 T4-2) cells derived by serial passaging from a single reduction mammoplasty. In two dimensional (2D) monolayers on plastic, both S1 and T4-2 cells display similar morphology. But in three dimensional (3D) matrigel cultures, S1 form phenotypically normal mammary tissue structures while T4-2 cells fail to organize into these structures and instead disseminate into the matrix. This assay was designated as a tumor reversion model, in that the T4-2 cells can be induced to form S1-like structures in 3D by treatment with beta-1 integrin or EGFR blocking antibodies, or by treating with a chemical inhibitor of the EGFR signaling pathway (tyrophostin AG 1478). These treated T4-2 cells, called T4R cells, are non-tumorigenic.

### EXAMPLE 2:

### CELL GROWTH AND RNA ISOLATION

Growth of Cells 2D and 3D for Microarray Experiments: HMT3522 S1 and T4-2 cells were grown 2D and 3D and T4-2 cells reverted with anti-EGFR, anti-beta 1 integrin, or tyrophostin AG 1478 as previously described (Weaver et al J Cell Biol. 137:231-45, 1997; and Wang et al PNAS 95:14821-14826, 1998). Anti-EGFR (mAb 225) was purchased from Oncogene and introduced into the matrigel at the time of gelation at a concentration of 4 ug/ml purified mouse IgG1. Anti-beta 1 integrin (mAb AIIB2) was a gift from C. Damsky at the University of California at San Francisco and was also introduced into the matrigel at the time of gelation at a concentration of 100 ug/ml ascites protein (which corresponds to 4-10 ug/ml purified rat IgG1). Tyrophostin AG 1478 was purchased from Calbiochem and used at a concentration of 100 nM.

Isolation of RNA for Microarray Experiments: RNA was prepared from: S1 passage 60 2D cultures; T4-2 passage 41 2D cultures; S1 passage 59 3D cultures; and T4-2 and T4-2 revertant (with anti-EGFR, anti-beta 1 integrin, and tyrophostin) passage 35 3D cultures.

All RNA for microarray experiments was isolated using the commercially available RNeasy Mini Kit from Qiagen. Isolation of total RNA from cells grown 2D was performed as instructed in the kit handbook. Briefly, media was aspirated from the cells and kit Buffer RLT was added directly to the flask. The cell lysate was collected with a rubber cell scraper, and the lysate passed 5 times through a 20-G needle fitted to a syringe. One volume of 70% ethanol was added to the homogenized lysate and mixed well by pipetting. Up to 700 ul of sample was applied to an RNeasy mini spin column sitting in a 2-ml collection tube and centrifuged for 15 seconds at >8000 x g. 700 ul Buffer RW1 was added to the column and centrifuged for 15 seconds at >8000 x g to wash. The column was transferred to a new collection tube. 500 ul Buffer RPE was added to the column and centrifuged for 15 seconds at >8000 x g to wash. Another 500 ul Buffer RPE was added to the column for additional washing, and the column centrifuged for 2 minutes at maximum speed to dry. The column was transferred to a new collection tube and RNA eluted from the column with 30 ul RNase-free water by centrifuging for 1 minute at >8000 x g.

Isolation of total RNA from cells grown 3D was performed as described above, except cells were isolated from matrigel prior to RNA isolation. The cells were isolated as colonies from matrigel using ice-cold PBS/EDTA (0.01 M sodium phosphate pH 7.2 containing 138 mM sodium chloride and 5 mM EDTA). See Weaver et al, J Cell Biol 137:231-245, 1997; and Wang et al. PNAS 95:14821-14826, 1998.

### EXAMPLE 3:

### DETECTION AND IDENTIFICATION OF GENES EXHIBITING DIFFERENTIAL EXPRESSION

The relative expression levels of a selected sequence (which in turn is representative of a single transcript) were examined in the tumorigenic versus non-tumorigenic cell lines described above, following culturing of the cells (S1, T4-2 and T4R) in either two-dimensional (2D) monolayers or three-dimensional (3D) matrigel cultures as described above. Differential expression for a selected sequence was assessed by hybridizing mRNA from S1 and T4-2 2D cultures, and S1, T4-2 and T4R 3D cultures to microarray chips as described below, as follows: Exp1 = T4-2 2D/S1 2D; Exp2 = T4-2 3D/S1 3D; Exp3 = S1 3D/S1 2D; Exp4 = T4-2 3D/T4-2 2D; Exp5 = T4-2 3D/T4R (anti-EGFR) 3D; Exp6 = T4-2 3D/T4R (anti-betal integrin) 3D; and Exp7 = T4-2 3D/T4R (tyrophostin AG 1478) 3D.

Each array used had an identical spatial layout and control spot set. Each microarray was divided into two areas, each area having an array with, on each half, twelve groupings of 32 x 12 spots for a total of about 9,216 spots on each array. The two areas are spotted identically which provide for at least two duplicates of each clone per array. Spotting was accomplished using PCR amplified products from 0.5kb to 2.0 kb and spotted using a Molecular Dynamics Gen III spotter according to the manufacturer's recommendations. The first row of each of the 24 regions on the array had about 32 control spots, including 4 negative control spots and 8 test polynucleotides.

The test polynucleotides were spiked into each sample before the labeling reaction with a range of concentrations from 2-600 pg/slide and ratios of 1:1. For each array design, two slides were hybridized with the test samples reverse-labeled in the labeling reaction. This provided for about 4 duplicate measurements for each clone, two of one color and two of the other, for each sample.

Identification Of Differentially Expressed Genes: "Differentially expressed" in the context of the present example meant that there was a difference in expression of a particular gene between tumorigenic vs. non-tumorigenic cells, or cells grown in three-dimensional culture vs. cells grown in two-dimensional culture. To identify differentially expressed genes, total RNA was first reverse transcribed into cDNA using a primer containing a T7 RNA polymerase promoter, followed by second strand DNA synthesis. cDNA was then transcribed *in vitro* to produce antisense RNA using the T7 promoter-mediated expression (see, *e*.*g*., Luo et al. (1999) Nature Med 5:117-122), and the antisense RNA was then converted into cDNA. The second set of cDNAs were again transcribed *in vitro,* using the T7 promoter, to provide antisense RNA. Optionally, the RNA was again converted into cDNA, allowing for up to a third round of T7-mediated amplification to produce more antisense RNA. Thus the procedure provided for two or three rounds of *in vitro* transcription to produce the final RNA used for fluorescent labeling.

Fluorescent probes were generated by first adding control RNA to the antisense RNA mix, and producing fluorescently labeled cDNA from the RNA starting material. Fluorescently labeled cDNAs prepared from tumorigenic RNA sample were compared to fluorescently labeled cDNAs prepared from non-tumorigenic cell RNA sample. For example, the cDNA probes from the non-tumorigenic cells were labeled with Cy3 fluorescent dye (green) and the cDNA probes prepared from the tumorigenic cells were labeled with Cy5 fluorescent dye (red).

The differential expression assay was performed by mixing equal amounts of probes from tumorigenic cells and non-tumorigenic cells, and/or cells grown in 3D vs. those grown in 2D. The arrays were prehybridized by incubation for about 2 hrs at 60°C in 5X SSC/0.2% SDS/1 mM EDTA, and then washed three times in water and twice in isopropanol. Following prehybridization of the array, the probe mixture was then hybridized to the array under conditions of high stringency (overnight at 42°C in 50% formamide, 5X SSC, and 0.2% SDS). After hybridization, the array was washed at 55°C three times as follows: 1) first wash in 1X SSC/0.2% SDS; 2) second wash in 0.1X SSC/0.2% SDS; and 3) third wash in 0.1X SSC.

The arrays were then scanned for green and red fluorescence using a Molecular Dynamics Generation III dual color laser-scanner/detector. The images were processed using BioDiscovery Autogene software, and the data from each scan set normalized to provide for a ratio of expression relative to non-tumorigenic or tumorigenic cells grown two-dimensionally or three-dimensionally. Data from the microarray experiments was analyzed according to the algorithms described in U.S. application serial no. 60/252,358, filed November 20, 2000, by E.J. Moler, M.A. Boyle, and F.M. Randazzo, and entitled "Precision and accuracy in cDNA microarray data," which application is specifically incorporated herein by reference.

The experiment was repeated, this time labeling the two probes with the opposite color in order to perform the assay in both "color directions." Each experiment was sometimes repeated with two more slides (one in each color direction). The level fluorescence for each sequence on the array expressed as a ratio of the geometric mean of 8 replicate spots/genes from the four arrays or 4 replicate spots/gene from 2 arrays or some other permutation. The data were normalized using the spiked positive controls present in each duplicated area, and the precision of this normalization was included in the final determination of the significance of each differential. The fluorescence intensity of each spot was also compared to the negative controls in each duplicated area to determine which spots have detected significant expression levels in each sample.

A statistical analysis of the fluorescent intensities was applied to each set of duplicate spots to assess the precision and significance of each differential measurement, resulting in a p-value testing the null hypothesis that there is no differential in the expression level between the tumorigenic and non-tumorigenic cells or cells grown two-dimensionally versus three-dimensionally. During initial analysis of the microarrays, the hypothesis was accepted if p>10⁻³, and the differential ratio was set to 1.000 for those spots. All other spots have a significant difference in expression between the two samples compared. For example, if the tumorigenic sample has detectable expression and the non-tumorigenic does not, the ratio is truncated at 1000 since the value for expression in the non-tumorigenic sample would be zero, and the ratio would not be a mathematically useful value (e.g., infinity). If the non-tumorigenic sample has detectable expression and the tumorigenic does not, the ratio is truncated to 0.001, since the value for expression in the tumor sample would be zero and the ratio would not be a mathematically useful value. These latter two situations are referred to herein as "on/off." Database tables were populated using a 95% confidence level (p>0.05).

In general, a polynucleotide is said to represent a significantly differentially expressed gene between two samples when there is detectable levels of expression in at least one sample and the ratio value is greater than at least about 1.2 fold, at least about 1.5 fold, or at least about 2 fold, where the ratio value is calculated using the method described above.

A differential expression ratio of 1 indicates that the expression level of the gene in tumorigenic cells was not statistically different from expression of that gene in the specific non-tumorigenic cells compared. A differential expression ratio significantly greater than 1 in tumorigenic breast cells relative to non-tumorigenic breast cells indicates that the gene is increased in expression in tumorigenic cells relative to non-tumorigenic cells, suggesting that the gene plays a role in the development of the tumorigenic phenotype, and may be involved in promoting metastasis of the cell. Detection of gene products from such genes can provide an indicator that the cell is cancerous, and may provide a therapeutic and/or diagnostic target. Likewise, a differential expression ratio significantly less than 1 in tumorigenic breast cells relative to non-tumorigenic breast cells indicates that, for example, the gene is involved in suppression of the tumorigenic phenotype. Increasing activity of the gene product encoded by such a gene, or replacing such activity, can provide the basis for chemotherapy. Such gene can also serve as markers of cancerous cells, *e*.*g*., the absence or decreased presence of the gene product in a breast cell relative to a non-tumorigenic breast cell indicates that the cell is cancerous.

Using the above methodology, three hundred and sixty-seven (367) genes or products thereof were identified from 20,000 chip clones analyzed as being overexpressed 2-fold or more in one or more of these experiments, with a p-value of 0.001 or less. These identified genes or products thereof are listed in Table 1, according to the Spot ID of the spotted polynucleotide, the Sample ID, the corresponding GenBank Accession Number (No.), the GenBank description (if available) for the corresponding Genbank Accession Number, and the GenBank score (p-value; the probability that the association between the SEQ ID NO. and the gene or product thereof occurred by chance). The polynucleotide and polypeptide sequences, as provided by any disclosed Genbank entries are herein incorporated by reference to the corresponding Genbank accession number. The differential hybridization results from the seven differential expression microarray experiments listed above are provided in Table 2, where sequences have a measurement corresponding to its ratio of expression in the 7 experiments, e.g. spot ID 10594 is 2.2-fold overexpressed in 3D T4-2 cells as compared to 3D S1 cells. SEQ ID NOS:1-499, representing the sequences corresponding to the spot Ids listed in Tables 1 and 2 are provided in the sequence listing. Table 11 is a lookup table showing the relationship between the spot Ids (i.e. the nucleic acids spotted on the microarray) and the sequences provided in the sequence listing.

### EXAMPLE 4

### CYCLIN G ASSOCIATED KINASE (GAK)

A gene or product thereof called cyclin G associated kinase, or GAK, was identified as being overexpressed in 3D T4-2 cultures relative to both 3D S1 cultures (ratio: 7.9296) and 2D T4-2 cultures (ratio: 34.6682) (Sample ID RG:1056692:10012:C11, Spot ID 19990). GAK corresponds to Genbank Accession number XM_003450.

### EXAMPLE 5

### ANTISENSE REGULATION OF GAK EXPRESSION

Additional functional information on GAK was generated using antisense knockout technology. A number of different oligonucleotides complementary to GAK mRNA were designed (AS) with corresponding controls (RC): GGAATCACCGCTTTGCCATCTTCAA (SEQ ID NO:500; CHIR159-1AS, gak:P1868AS), AACTTCTACCGTTTCGCCACTAAGG (SEQ ID NO:501; CHIR159-1RC, gak:P1868RC); GACCGTGTACTGCGTGTCGTGCG (SEQ ID NO:502; CHIR159-7AS, gak:P0839AS) and GCGTGCTGTGCGTCATGTGCCAG (SEQ ID NO:502; CHIR159-7RC, gak:P0839RC), and tested for their ability to suppress expression of GAK in human malignant colorectal carcinoma SW620 cells, human breast cancer MDA231 cells, and human breast cancer T4-2 cells. For each transfection mixture, a carrier molecule, preferably a lipitoid or cholesteroid, was prepared to a working concentration of 0.5 mM in water, sonicated to yield a uniform solution, and filtered through a 0.45 µm PVDF membrane. The antisense or control oligonucleotide was then prepared to a working concentration of 100 µM in sterile Millipore water. The oligonucleotide was further diluted in OptiMEM™ (Gibco/BRL), in a microfuge tube, to 2 µM, or approximately 20 µg oligo/ml of OptiMEM™. In a separate microfuge tube, lipitoid or cholesteroid, typically in the amount of about 1.5-2 nmol lipitoid/µg antisense oligonucleotide, was diluted into the same volume of OptiMEM™ used to dilute the oligonucleotide. The diluted antisense oligonucleotide was immediately added to the diluted lipitoid and mixed by pipetting up and down. Oligonucleotide was added to the cells to a final concentration of 300 nM.

The level of target mRNA (GAK) in the transfected cells was quantitated in the cancer cell lines using the methods using primers CHIR159_2896 (GCCGTCTTCAGGCAACAACTCCCA; SEQ ID NO: 504; forward) and CHIR159_3089 (TGCTGGACGAGGCTGTCATCTTGC; SEQ ID NO:505; reverse). RNA was extracted as above according to manufacturer's directions.

Quantitative PCR (qPCR) was performed by first isolating the RNA from the above mentioned tissue/cells using a Qiagen RNeasy mini prep kit. A total of 0.5 micrograms of RNA was used to generate a first strand cDNA using Stratagene MuLV Reverse Transcriptase, using recommended concentrations of buffer, enzyme, and Rnasin. Concentrations and volumes of dNTP, and oligo dT, or random hexamers were lower than recommended to reduce the level of background primer dimerization in the qPCR.

The cDNA is then used for qPCR to determine the levels of expression of GAK using the GeneAmp 7000 by ABI as recommended by the manufacturer. Primers for actin were also used in order to normalized the values, and eliminate possible variations in cDNA template concentrations, pipetting error, etc.

For each 20 µl reaction, extracted RNA (generally 0.2-1 µg total) was placed into a sterile 0.5 or 1.5 ml microcentrifuge tube, and water was added to a total volume of 12.5 µl. To each tube was added 7.5 µl of a buffer/enzyme mixture, prepared by mixing (in the order listed) 2.5 µl H₂O, 2.0 µl 10X reaction buffer, 10 µl oligo dT (20 pmol), 1.0 µl dNTP mix (10 mM each), 0.5 µl RNAsin® (20u) (Ambion, Inc., Hialeah, FL), and 0.5 µl MMLV reverse transcriptase (50u) (Ambion, Inc.). The contents were mixed by pipetting up and down, and the reaction mixture was incubated at 42°C for 1 hour. The contents of each tube were centrifuged prior to amplification.

An amplification mixture was prepared by mixing in the following order: 1X PCR buffer II, 3 mM MgCl₂, 140 µM each dNTP, 0.175 pmol each oligo, 1:50,000 dil of SYBR® Green, 0.25 mg/ml BSA, 1 unit Taq polymerase, and H₂O to 20 µl. (PCR buffer II is available in 10X concentration from Perkin-Elmer, Norwalk, CT). In 1X concentration it contains 10 mM Tris pH 8.3 and 50 mM KCl. SYBR® Green (Molecular Probes, Eugene, OR) is a dye which fluoresces when bound to double stranded DNA. As double stranded PCR product is produced during amplification, the fluorescence from SYBR® Green increases. To each 20 µl aliquot of amplification mixture, 2 µl of template RT was added, and amplification was carried out according to standard protocols.

Table 3 shows that the antisense oligonucleotides described above reduced expression of GAK mRNA as compared to controls in all three cell lines. GAK mRNA reduction ranged from about 50% to about 90%, as compared to cells transfected with reverse (i.e. sense) control oligonucleotides.

**Table 3: antisense regulation of GAK mRNA**

| Oligo | Cell Line | Gene Message | Actin Message | Ratio | Percent KO |
|---|---|---|---|---|---|
| CHIR159-1AS | SW620 | 0.0923 | 0.669 | 0.138 | 90.7 |
| CHIR159-1RC | SW620 | 1.01 | 0.680 | 1.49 | |
| CHIR159-7AS | SW620 | 0.0555 | 0.678 | 0.082 | 85.4 |
| CHIR159-7RC | SW620 | 0.335 | 0.598 | 0.560 | |
| CHIR159-1AS | MDA231 | 0.358 | 0.687 | 0.521 | 59.3 |
| CHIR159-1RC | MDA231 | 1.00 | 0.784 | 1.28 | |
| CHIR159-7AS | MDA231 | 0.262 | 0.674 | 0.389 | 69.4 |
| CHIR159-7RC | MDA231 | 0.840 | 0.659 | 1.27 | |
| CHIR159-1AS | T4-2 | 0.307 | 0.707 | 0.434 | 72.9 |
| CHIR159-1RC | T4-2 | 1.23 | 0.770 | 1.60 | |
| CHIR159-7AS | T4-2 | 0.214 | 0.649 | 0.330 | 49.8 |
| CHIR159-7RC | T4-2 | 0.506 | 0.770 | 0.657 | |

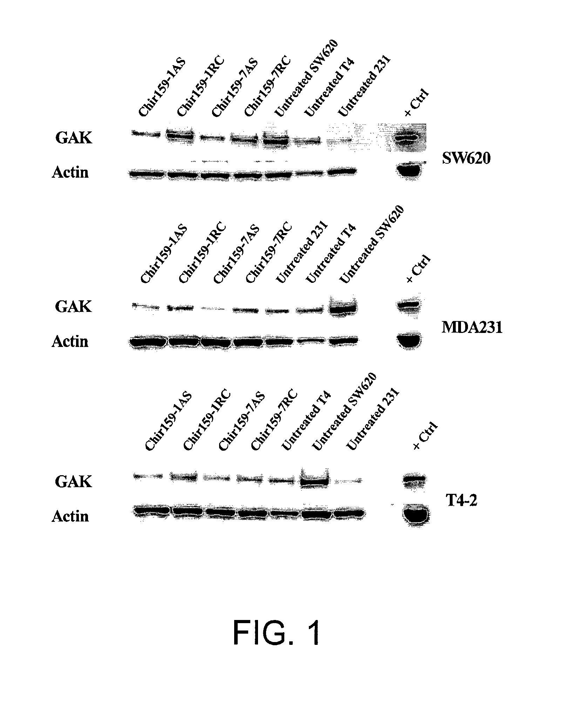

Reduction of GAK protein by antisense polynucleotides in SW620, MDA231 and T4-2 was confirmed using an antibody that specifically recognizes GAK. Figure 1 shows a western (i.e. protein) blot of protein extracts of the above cell lines decorated with anti-GAK antibodies. GAK protein expression is reduced in cell lines receiving GAK antisense oligonucleotides.

### EXAMPLE 6

### ROLE OF GAK IN ANCHORAGE INDEPENDENT CELL GROWTH

The effect of GAK gene expression upon anchorage-independent cell growth of SW620 and MBA-231 cells was measured by colony formation in soft agar. Soft agar assays were performed by first coating a non-tissue culture treated plate with PolyHEMA to prevent cells from attaching to the plate. Non-transfected cells were harvested using 0.05% trypsin and washing twice in media. The cells are counted using a hemacytometer and resuspended to 10⁴ per ml in media. 50 µl aliquots are placed in poly-HEMA coated 96-well plates and transfected. For each transfection mixture, a carrier molecule, preferably a lipitoid or cholesteroid, was prepared to a working concentration of 0.5 mM in water, sonicated to yield a uniform solution, and filtered through a 0.45 µm PVDF membrane. The antisense or control oligonucleotide was then prepared to a working concentration of 100 µM in sterile Millipore water. The oligonucleotide was further diluted in OptiMEM^{™} (Gibco/BRL), in a microfuge tube, to 2 µM, or approximately 20 µg oligo/ml of OptiMEM^{™}. In a separate microfuge tube, lipitoid or cholesteroid, typically in the amount of about 1.5-2 nmol lipitoid/µg antisense oligonucleotide, was diluted into the same volume of OptiMEM^{™} used to dilute the oligonucleotide. The diluted antisense oligonucleotide was immediately added to the diluted lipitoid and mixed by pipetting up and down. Oligonucleotide was added to the cells to a final concentration of 300 nM. Following transfection (∼30 minutes), 3% GTG agarose is added to the cells for a final concentration of 0.35% agarose. After the cell layer agar solidifies, 100 µl of media is dribbled on top of each well. Colonies form in 7 days. For a read-out of growth, 20 µl of Alamar Blue is added to each well and the plate is shaken for 15 minutes. Fluorecence readings (530nm excitation 590 nm emission) are taken after incubation for 6-24 hours.

The data presented in Table 4 shows that the application of GAK antisense oligonucleotides to SW620 and MDA 231 cells results in inhibition of colony formation and shows that GAK plays a role in production anchorage-independent cell growth. Table 4 shows the average fluorescence reading for several experiments. The standard deviation (St. Dev) of the fluorescence reading and coefficient of variation (%CV) is also shown.

**Table 4: GAK and anchorage-independent cell growth.**

| Oligo | Cell Line | Average | St. Dev | %CV |
|---|---|---|---|---|
| Blank | SW620 | 12868.17 | 208.78 | 1.78 |
| Untreated | SW620 | 31075.17 | 1944.36 | 7.66 |
| Pos Control | SW620 | 5717.17 | 1108.71 | 23.75 |
| Neg Control | SW620 | 7576.17 | 465.95 | 7.63 |
| Chir159-1AS | SW620 | 9701.5 | 2281.36 | 28.8 |
| Chir159-1RC | SW620 | 17765.5 | 1958.45 | 13.5 |
| Blank | MDA231 | 12726.83 | 232.45 | 2 |
| Untreated | MDA231 | 87272.17 | 0 | 0 |
| Pos Control | MDA231 | 10645.17 | 1591.08 | 18.31 |
| Neg Control | MDA231 | 24159.5 | 2850.58 | 14.45 |
| Chir159-1AS | MDA231 | 8613.5 | 4852.76 | 69 |
| Chir159-1RC | MDA231 | 17859.17 | 1535.55 | 10.53 |

### EXAMPLE 7

### DKFZ_{P}566I133 (DKFZ)

Several previously uncharacterized genes were identified as being induced in these experiments. One such gene was represented by two spots, Spot ID Nos 22793 and 26883 (gene assignment DKFZp566I133). This gene was expressed at a ratio of about 2.2 in two 2-dimensional (2D) T4-2 vs. 2D S1 experiments, and also at a ratio of about 2 when 3-dimensional (3D) T4-2 cells were compared to the various tumor reversion cultures. However, the ratio of expression increased to an average of 3.2 when 3-dimensional (3D) T4-2 cultures were compared to 2D S 1 cultures. In contrast, there was essentially no difference in expression levels when 3D S1 cultures were compared to 2D S1 cultures, suggesting that expression of this gene is specifically elevated in the tumorigenic cell line T4-2, and even further elevated when the tumorigenic cell line is grown in three dimensional cultures (see Table 5).

**Table 5.**

| Spot ID | 2D T4-2/ 2D S1 | 3D T42/ 3D S1 | 3D S1/ 2D S1 | 3D T4-2/ 2D T4-2 | 3D T4-2/ EGFRAb | 3D T4-2/ B1 integrin Ab | 3D T4-2/ Tyr |
|---|---|---|---|---|---|---|---|
| 22893 | 1.90387 | 2.64711 | 0.522161 | 1 | 2.17956 | 1.75287 | 2.055538 |
| 26883 | 2.43428 | 3.74613 | 0.524466 | 1 | 2.467573 | 2.029468 | 2.002817 |

These array data were confirmed by qPCR using the methods described above and the gene specific PCR primers CHIR180_1207 ACAGGGAGAAAACTGGTTGTCCTGG (SEQ ID NO:506; Forward) and CHIR180_1403 AAGGCAGAACCCATCCACTCCAA (SEQ ID NO:507; Reverse). Independent cultures were used for these experiments, and data was normalized to B-catenin. These data are shown in Table 6.

**Table 6.**

| 2D S1 | 2D TA-2 | 3D S1 | 3D TA-2 | 3D EGFR Ab | 3D B1 Integrin Ab | 3D Tyr |
|---|---|---|---|---|---|---|
| 0.165 | 0.421 | 0.14 | 0.475 | 0.231 | 0.175 | 0.174 |

DKFZ corresponds to Genbank Accession numbers NP_112200, AAH09758, and NM_030938. Orthologs of DKFZ are identified in species other than Homo sapiens include NM_138839 from *Rattus norvegicus.*

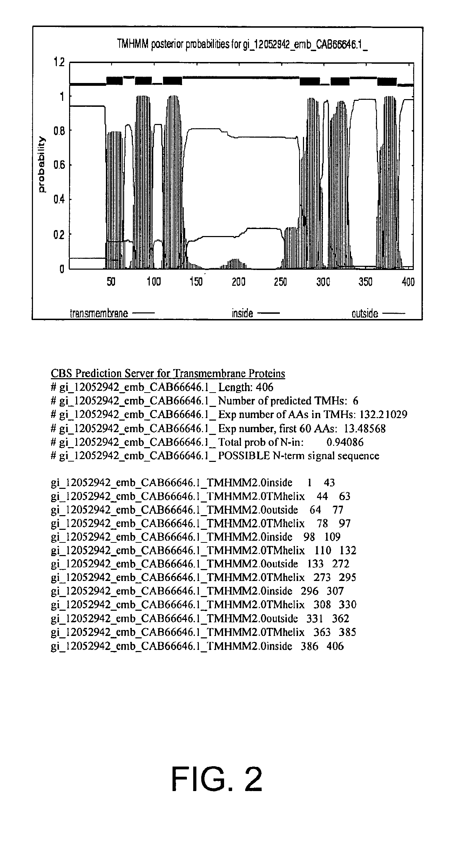

Analysis of the sequence of DKFZ using a transmembrane helix prediction algorithm (Sonhammer, et al, A hidden Markov model for predicting transmembrane helices in protein sequences, In Proc. of Sixth Int. Conf. on Intelligent Systems for Molecular Biology, p. 175-82, Ed. J. Glasgow, T. Littlejohn, F. Major, R. Lathrop, D. Sankoff, and C. Sensen, Menlo Park, CA: AAAI Press, 1998) indicates that the DKFZ protein has six transmembrane regions (Fig.2), and, as such, is likely to be a transmembrane protein.

### EXAMPLE 8

### ANTISENSE REGULATION OF DKFZ EXPRESSION

Additional functional information on DKFZ was generated using antisense knockout technology. A number of different oligonucleotides complementary to DKFZ mRNA were designed (AS) with corresponding controls (RC): GCTGCTGGATTCGTTTGGCATAACT (SEQ ID NO: 508; CHIR180-7AS, DKFZp566I1:P1301AS), TCAATACGGTTTGCTTAGGTCGTCG (SEQ ID NO:509; CHIR180-7RC, DKFZp566I1:P1301RC), TCTCCTCTGAGTTCAACCGCTGCT (SEQ ID NO:510; CHIR180-8AS, DKFZp566I1:P1320AS) and TCGTCGCCAACTTGAGTCTCCTCT (SEQ ID NO:511; CHIR180-8RC, DKFZp566I1:P1320AS), and tested for their ability to suppress expression of DKFZ in human malignant colorectal carcinoma SW620 cells, human breast cancer MDA231 cells, and human breast cancer T4-2 cells, as described above.

Table 7 shows that the antisense (AS) oligonucleotides described above reduced expression of DKFZ mRNA as compared to controls in all three cell lines. DKFZ mRNA reduction ranged from about 95% to about 99%, as compared to cells transfected with reverse (i.e. sense) control (RC) oligonucleotides.

**Table 7: antisense regulation of DKFZ mRNA**

| Oligo | Cell Line | Gene Message | Actin Message | Ratio | Percent KO |
|---|---|---|---|---|---|
| CHIR180-7AS | SW620 | 0.0157 | 0.772 | 0.020 | 99.3 |
| CHIR180-7RC | SW620 | 1.99 | 0.736 | 2.70 | |
| CHIR180-8AS | SW620 | 0.0387 | 0.681 | 0.057 | 97.9 |
| CHIR180-8RC | SW620 | 1.89 | 0.703 | 2.69 | |
| CHIR180-7AS | MDA231 | 0.0471 | 3.58 | 0.013 | 98.5 |
| CHIR180-7RC | MDA231 | 1.99 | 2.33 | 0.854 | |
| CHIR180-8AS | MDA231 | 0.00935 | 1.74 | 0.00537 | 99.5 |
| CHIR180-8RC | MDA231 | 1.14 | 1.01 | 1.13 | |
| CHIR180-7AS | T4-2 | 0.119 | 0.667 | 0.178 | 95.4 |
| CHIR180-7RC | T4-2 | 2.8 | 0.728 | 3.85 | |
| CHIR180-8AS | T4-2 | 0.0852 | 0.751 | 0.113 | 95.6 |
| CHIR180-8RC | T4-2 | 1.6 | 0.620 | 2.58 | |

### EXAMPLE 9

### EFFECT OF DKFZ EXPRESSION ON CELL PROLIFERATION

The effect of gene expression on the inhibition of cell proliferation was assessed in metastatic breast cancer cell line MDA-231 and breast cancer cell line T4-2.

Cells were plated to approximately 60-80% confluency in 96-well dishes. Antisense or reverse control oligonucleotide was diluted to 2 µM in OptiMEM^{™} and added to OptiMEM^{™} into which a delivery vehicle, preferably a lipitoid or cholesteroid, had been diluted. The oligo/delivery vehicle mixture was then further diluted into medium with serum on the cells. The final concentration of oligonucleotide for all experiments was 300 nM, and the final ratio of oligo to delivery vehicle for all experiments was 1.5 nmol lipitoid/µg oligonucleotide.

Antisense oligonucleotides were prepared. Cells were transfected for 4 hours or overnight at 37°C and the transfection mixture was replaced with fresh medium. Plates are incubated for 4 days, with a plate harvested for each day0-day4. To determine differences in cell number, a CyQuant Cell Proliferation Assay kit (Molecular Probes) was used per manufacturer's instructions. Fluorecence readings (480nm excitation 520 nm emission) are taken after incubation for 5 minutes.

The results of these assays are shown in Tables 7A and 8. The data show that DKFZ antisense polynucleotides significantly reduce cell proliferation as compared to controls, and, as such, DKFZ plays a role in production or maintenance of the cancerous phenotype in cancerous breast cells.

**Table 7A:**

| Cell proliferation | | | | | | |
|---|---|---|---|---|---|---|
| Oligo | Cell Line | Ave Day0 | Ave Day1 | Ave Day2 | Av3 Day3 | Ave Day4 |
| Untreated | MDA231 | 4233 | 4858 | 9544 | 10981 | 16776 |
| Untreated | MDA231 | 3849 | 4036 | 8686 | 9855 | 14865 |
| Pos Control | MDA231 | 3630 | 2236 | 3564 | 4536 | 7477 |
| Neg Control | MDA231 | 4913 | 5127 | 8331 | 8887 | 13620 |
| CHIR180-7AS | MDA231 | 3848 | 3476 | 6942 | 8715 | 11925 |
| CHIR180-7RC | MDA231 | 4895 | 4700 | 8484 | 10318 | 14226 |
| Untreated | T4-2 | 4062 | 3389 | 5438 | 10579 | 15617 |
| Untreated | T4-2 | 4209 | 3802 | 6346 | 11802 | 16275 |
| Pos Control | T4-2 | 3985 | 2712 | 4081 | 6404 | 9685 |
| Neg Control | T4-2 | 4051 | 3901 | 4356 | 9425 | 12964 |
| CHIR180-7AS | T4-2 | 3792 | 3201 | 3849 | 7376 | 10911 |
| CHIR180-7RC | T4-2 | 3967 | 3840 | 4321 | 8382 | 12293 |

**Table 8**

| Standard Deviations | | | | | | | P-Value of T-Test | | | |
|---|---|---|---|---|---|---|---|---|---|---|
| Oligo | Day 0 | Day 1 | Day 2 | Day 3 | Day 4 | Day0 | Day1 | Day2 | Day3 | Day4 |
| Untreated | 337 | 269 | 299 | 697 | 1333 | 0.1306 | 0.1063 | 0.1804 | 0.0926 | 0.1225 |
| Untreated | 99 | 631 | 867 | 547 | 1047 | 0.1306 | 0.1063 | 0.1804 | 0.0926 | 0.1225 |
| Pos Control | 94 | 118 | 89 | 441 | 974 | 0.0000 | 0.0001 | 0.0003 | 0.0001 | 0.0010 |
| Neg Control | 2 | 252 | 697 | 195 | 780 | 0.0000 | 0.0001 | 0.0003 | 0.0001 | 0.0010 |
| CHIR180-7 AS | 292 | 16 | 435 | 398 | 418 | 0.0072 | 0.0276 | 0.0059 | 0.0140 | 0.0028 |
| CHIR180-7 RC | 208 | 6 | 244 | 533 | 440 | 0.0072 | 0.0276 | 0.0059 | 0.0140 | 0.0028 |
| Untreated | 64 | 283 | 789 | 1593 | 1226 | 0.2550 | 0.0921 | 0.1257 | 0.2794 | 0.4352 |
| Untreated | 22 | 158 | 205 | 577 | 478 | 0.2550 | 0.0921 | 0.1257 | 0.2794 | 0.4352 |
| Pos Control | 122 | 213 | 6 | 475 | 957 | 0.4320 | 0.0065 | 0.2624 | 0.0051 | 0.0293 |
| Neg Control | 47 | 335 | 464 | 809 | 1417 | 0.4320 | 0.0065 | 0.2624 | 0.0051 | 0.0293 |
| CHIR180-7 AS | 170 | 679 | 263 | 127 | 1330 | 0.2638 | 0.0976 | 0.3516 | 0.0040 | 0.0039 |
| CHIR180-7 RC | 22 | 453 | 646 | 579 | 884 | 0.2638 | 0.0976 | 0.3516 | 0.0040 | 0.0039 |

### EXAMPLE 10

### ROLE OF DKFZ IN ANCHORAGE INDEPENDENT CELL GROWTH

The effect of DKFZ gene expression upon anchorage-independent cell growth of MDA435 and MCF7 human breast cancer cells was measured by colony formation in soft agar. Soft agar assays were conducted by the method described for GAK, above.

The data presented in Table 9 shows that the application of DKFZ antisense oligonucleotides to MDA435 and MCF7 cells results in inhibition of colony formation and shows that DKFZ plays a role in anchorage-independent cell growth of cancer cells. Table 9 shows the average fluorescence reading for several experiments. The standard deviation (St. Dev) of the fluorescence reading and coefficient of variation (%CV) and probability (P-value) is also shown.

**Table 9**

| Oligo | Cell Line | Average | St. Dev | %CV | P-Value |
|---|---|---|---|---|---|
| Untreated | MDA435 | 31190 | 5838 | 19 | 0.1342 |
| Untreated | MDA435 | 38623 | 3620 | 9 | 0.1342 |
| Pos Control | MDA435 | 4776 | 818 | 17 | 0.0156 |
| Neg Control | MDA435 | 16315 | 481 | 3 | 0.0156 |
| Chir180-7AS | MDA435 | 21161 | 3439 | 16 | 0.0274 |
| Chir180-7RC | MDA435 | 28868 | 1902 | 7 | 0.0274 |
| Untreated | MCF7 | 18954 | 1478 | 8 | 0.1476 |
| Untreated | MCF7 | 14383 | 4163 | 29 | 0.1476 |
| Pos Control | MCF7 | 1036 | 194 | 19 | 0.0036 |
| Neg Control | MCF7 | 9478 | 2382 | 25 | 0.0036 |
| Chir180-7AS | MCF7 | 4752 | 2002 | 42 | 0.0139 |
| Chir180-7RC | MCF7 | 9570 | 18 | 0 | 0.0139 |

The effect of DKFZ gene expression upon invasiveness of MDA231 human breast cancer cells was measured by a matrigel assay. A 3-dimensional reconstituted basement membrane culture of cells was generated as described previously (Peterson et al., (1992) Proc. Natl. Acad. Sci. USA 89:9064-9068) using a commercially prepared reconstituted basement membrane (Matrigel; Collaborative Research, Waltham, MA) and examined using methods well known in the art.

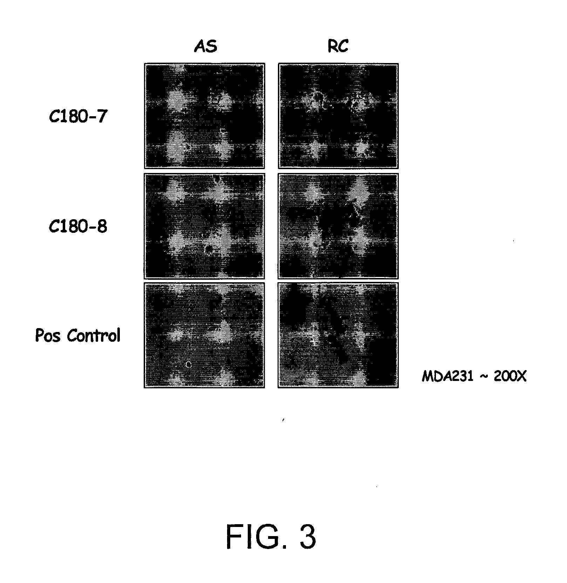

Table 10 (quantitated using Alamar Blue similar to the soft agar assay) and Figure 3 provides exemplary results of the Matrigel invasion/motility assay to test the invasiveness of MDA231 cells with reduced expression of DKFZ. In general, these data show that a reduction in the expression of DKFZ significantly decreases the invasiveness of MDA231 cells.

**Table 10:**

| Oligo | Cell Line | Average | St. Dev | %CV | P-Value |
|---|---|---|---|---|---|
| Untreated | MDA231 | 28316 | 13663 | 48 | 0.9080 |
| Untreated | MDA231 | 26840 | 15669 | 58 | 0.9080 |
| Pos Control | MDA231 | 2756 | 487 | 18 | 0.0002 |
| Neg Control | MDA231 | 14301 | 1386 | 10 | 0.0002 |
| Chir180-7AS | MDA231 | 10508 | 1963 | 19 | 0.0287 |
| Chir180-7RC | MDA231 | 14310 | 153 | 1 | 0.0287 |

### EXAMPLE 11

### EXPRESSION OF DKFZ IN CANCER TISSUES

The following peptides were used for polyclonal antibody production: peptide 809:
gvhqqyvqriek (SEQ ID NO:380), corresponding to amino acids 97-108 of the DKFZ protein and peptide 810: sgaepddeeyqef (SEQ ID NO:381), corresponding to amino acids 215-227 of the DKFZ protein.

Antibodies specific for DKFZ are used in FACS and immunolocalization analysis to show that DKFZ is associated with membrane, and up-regulated in cancer tissues of biopsies from cancer patients.

Further, antibodies specific for DKFZ are used to modulate DKFZ activity in cancerous breast, and is further used, alone or conjugated to a toxic moiety, as a treatment for breast cancer.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto

**Table 1**

| SPOTID | SAMPLE ID | GENBANK NO | GENBANK DESCRIPTION | GENBANK SCORE |
|---|---|---|---|---|
| 10594 | I:1871362:05B01:A04 | M62994 | Homo sapiens thyroid autoantigen (truncated actin binding protein) mRNA, complete cds | 8.6E-36 |
| 21851 | M00055153A:A12 | | | |
| 20990 | I:1986550:13B02:G12 | XM 005667 | Homo sapiens lipocalin 2 (oncogene 24p3) (LCN2), mRNA | 0 |
| 18641 | I:3473302:09A01:A09 | AB046098 | Macaca fascicularis brain cDNA, clone:QccE-15843 | 5.8E-57 |
| 17229 | I:1506962:09A01:G01 | AL365454 | Homo sapiens mRNA full length insert cDNA clone EUROIMAGE 926491 | 2.6E-110 |
| 25930 | 035JN020.F01 | AJ010446 | Homo sapiens mRNA for immunoglobulin kappa light chain,anti-RhD, therad 24 | 0 |
| 20701 | RG:730349:10010:G12 | U28387 | Human hexokinase II pseudogene, complete cds | 0 |
| 20346 | RG:1839794:10015:E11 | U28387 | Human hexokinase II pseudogene, complete cds | 0 |
| 21247 | M00054680C:A06 | U28387 | Human hexokinase II pseudogene, complete cds | 9.9E-80 |
| 23062 | M00056353C:E10 | XM 011013 | Homo sapiens filamin B, beta (actin-binding protein 278) (FLNB), mRNA | 0 |
| 25666 | 035Jn031.B01 | AF191633 | Homo sapiens filamin (FLNB) gene, exon 48 and complete cds | 0 |
| 19001 | I:2171401:09A02:E09 | AF123887 | Homo sapiens ER01L (ER01L) mRNA, partial cds | 3.3E-104 |
| 10897 | I:1852047:02A01:A10 | U22384 | Human lysyl oxidase gene, partial cds | 0 |
| 1960 | M00023297B:A10 | M33376 | Human pseudo-chlordecone reductase mRNA, complete cds | 0 |
| 26381 | 035JN029.H02 | AB037838 | Homo sapiens mRNA for KIAA1417 protein, partial cds | 0 |
| 26719 | 035JN030.A02 | X68277 | H.sapiens CL 100 mRNA for protein tyrosine phosphatase | 0 |
| 27152 | 037XN007.A09 | XM 048479 | Homo sapiens hypothetical protein FLJ14642 (FLJ14642), mRNA | 7.3E-58 |
| 10926 | I:2047770:08B02:G04 | AK000969 | Homo sapiens cDNA FLJ10107 fis, clone HEMBA1002583 | 3.8E-94 |
| 28980 | 035JN003.C12 | XM 027456 | Homo sapiens hypothetical gene supported by AK000584 (LOC89942), mRNA | 0 |
| 1236 | M00022024A:F02 | | | |
| 29350 | 035JN008.D06 | XM 043864 | Homo sapiens phosphoinositide-3-kinase, regulatory subunit, polypeptide 1 (p85 alpha) (PIK3R1), mRNA | 0 |
| 26242 | 035JN015.B02 | AL137717 | Homo sapiens mRNA; cDNA DKFZp434J1630 (from clone DKFZp434J1630) | 2.6E-70 |
| 4098 | MOD001439D:C09 | BC002446 | Homo sapiens, MRJ gene for a member of the DNAJ protein family, clone MGC:1152 IMAGE:3346070, mRNA, complete cds | 0 |
| 17432 | I:1965049:16B02:D07 | XM 051165 | Homo sapiens DKFZP586A0522 protein (DKFZP586A0522), mRNA | 0 |
| 1785 | SL198 | XM 051165 | Homo sapiens DKFZP586A0522 protein (DKFZP586A0522), mRNA | 0 |
| 28856 | 035JN032.E11 | X62996 | H.sapiens mitochondrial genome (consensus sequence) | 0 |
| 18791 | RG:229957:10007:D03 | D42042 | Human mRNA for KIAA0085 gene, partial cds | 0 |
| 22950 | M00056922C:C09 | | | |
| 1882 | M00022196B:D09 | Z29083 | H.sapiens 5T4 gene for 5T4 Oncofetal antigen | 0 |
| 23886 | M00055408A:F10 | | | |
| 24995 | M00055215C:E11 | XM 012880 | Homo sapiens hypothetical protein MGC1936 (MGC1936), mRNA | 0 |
| 24477 | M00055510B:F08 | AF240697 | Homo sapiens retinol dehydrogenase homolog isoform-2(RDH) mRNA, complete cds | 0 |
| 21681 | M00056771C:A12 | X02152 | Human mRNA for lactate dehydrogenase-A (LDH-A, EC 1.1.1.27) | 0 |
| 9557 | 1:1335140:05A02:C08 | X02152 | Human mRNA for lactate dehydrogenase-A (LDH-A, EC 1.1.1.27) | 0 |
| 22033 | M00056574B:A07 | | | |
| 873 | M00007979C:C05 | X00663 | Human mRNA fragment for epidermal growth factor (EGF) receptor | 0 |
| 17144 | RG:25254:10004:D07 | M97675 | Human transmembrane receptor (ror1) mRNA, complete cds | 0 |
| 26970 | 035JN015.F09 | AF097514 | Homo sapiens stearoyl-CoA desaturase (SCD) mRNA, complete cds | 0 |
| 21402 | M00054507C:D07 | | | |
| 27074 | 035Jn031.B03 | AF061741 | Homo sapiens retinal short-chain dehydrogenase/reductase retSDR1 mRNA, complete cds | 0 |
| 10963 | 1:1258790:05A02:B10 | AF072752 | Homo sapiens ten integrin EGF-like repeat domains protein precursor (ITGBL1) mRNA, complete cds | 0 |
| 29525 | 035JN026.D12 | | | |
| 25514 | 035JN011.F01 | U62961 | Human succinyl CoA:3-oxoacid CoA transferase precursor (OXCT) mRNA, complete cds | 0 |
| 26612 | 035JN016.C08 | NM 000240 | Homo sapiens monoamine oxidase A (MAOA), nuclear gene encoding mitochondrial protein, mRNA | 0 |
| 24600 | M00055490C:G11 | U57059 | Homo sapiens Apo-2 ligand mRNA, complete cds | 0 |
| 9741 | 1:3126828:12A02:G02 | U37518 | Human TNF-related apoptosis inducing ligand TRAIL mRNA. complete cds | 0 |
| 23689 | M00054752A:E11 | XM 001468 | Homo sapiens S100 calcium-binding protein A10 (annexin II ligand, calpactin 1, light polypeptide (p11)) (S100A10), mRNA | 0 |
| 22352 | M00042842B:E02 | XM 001468 | Homo sapiens S100 calcium-binding protein A10 (annexin II ligand, calpactin 1, light polypeptide (p11)) (S100A10), mRNA | 0 |
| 23806 | RG:2007319:20003:G10 | | | |
| 12285 | 1:1404669:04A01:G12 | BC002517 | Homo sapiens, Pirin, clone MGC:2083 IMAGE:3140037, mRNA, complete cds | 0 |
| 27638 | 035JN011.D10 | AK002155 | Homo sapiens cDNA FLJ11293 fis, clone PLACE1009670, highly similar to Homo sapiens genethonin 1 mRNA | 0 |
| 9663 | 1:2488567:11A02:H08 | XM 006027 | Homo sapiens brain-derived neurotrophic factor (BDNF), mRNA | 0 |
| 26850 | 035JN003.B03 | XM 031551 | Homo sapiens similar to carbohydrate (N-acetylglucosamine-6-O) sulfotransferase 2 (H. sapiens) (LOC90414), mRNA | 0 |
| 10204 | 1:1491445:02B01:F09 | AF131765 | Homo sapiens clone 24833 nonsyndromic hearing impairment protein mRNA sequence, complete cds | 0 |
| 1318 | 2192-6 | | | |
| 25922 | 035JN020.B01 | AB020673 | Homo sapiens mRNA for KIAA0866 protein, complete cds | 0 |
| 26347 | 035JN025.G02 | | | |
| 20361 | 1:395116:17A02:E05 | | | |
| 28672 | 035JN012.A05 | AF126181 | Homo sapiens breast cancer-associated gene 1 protein (BCG1) mRNA, complete cds | 0 |
| 25520 | 035JN011.A07 | D86956 | Human mRNA for KIAA0201 gene, complete cds | 0 |
| 1723 | M00005694A:A09 | BC001980 | Homo sapiens, clone IMAGE:3462291, mRNA | 0 |
| 28863 | 037XN002.A05 | | | |
| 25526 | 035JN011.D07 | AF086281 | Homo sapiens full length insert cDNA clone ZD45G11 | 0 |
| 27936 | 035JN008.A04 | X59445 | H.sapiens mRNA for colon carcinoma Manganese Superoxide Dismutase | 0 |
| 26851 | 035JN001.C03 | XM 033944 | Homo sapiens superoxide dismutase 2, mitochondrial (SOD2), mRNA | 0 |
| 25107 | M00054825A:E04 | AF075061 | Homo sapiens full length insert cDNA YP07G10 | 0 |
| 24912 | M00054505D:D06 | AF075061 | Homo sapiens full length insert cDNA YP07G10 | 0 |
| 25169 | M00055510D:D04 | M11167 | Human 28S ribosomal RNA gene | 1.2E-76 |
| 25600 | 035JN023.A01 | BC003107 | Homo sapiens, inhibitor of DNA binding 3, dominant negative helix-loop-helix protein, clone MGC:1988 IMAGE:3543936, mRNA, complete | 0 |
| 28706 | 035JN016.B05 | X55181 | Human ETS2 gene, 3'end | 0 |
| 26377 | 035JN029.F02 | Y14436 | Homo sapiens mRNA for phosphatidic acid phosphatase type 2 | 0 |
| 19460 | 1:438655:14B02:B04 | AF007133 | Homo sapiens clone 23764 mRNA sequence | 4.5E-113 |
| 25243 | RG:1667183:10014:F12 | BC000013 | Homo sapiens, insulin-like growth factor binding protein 3, clone MGC:2305 IMAGE:3506666, mRNA, complete cds | 0 |
| 20018 | 1:1213574:17B01:A11 | AB037925 | Homo sapiens MAIL mRNA, complete cds | 3.7E-106 |
| 918 | M00026895D:H03 | BC006433 | Homo sapiens, Ras-related GTP-binding protein, clone MGC:13077 IMAGE:3835186, mRNA, complete cds | 0 |
| 25027 | RG:1983823:20002:B06 | | | |
| 29089 | 035JN017.B06 | XM 037534 | Homo sapiens phosphodiesterase 7A (PDE7A), mRNA | 0 |
| 9141 | 1:1347384:02A02:C07 | U78579 | Human type I phosphatidylinositol-4-phosphate 5-kinase beta (STM7) mRNA, partial cds | 0 |
| 12005 | 1:1259230:05A01:C06 | D87075 | Human mRNA for KIAA0238 gene, partial cds | 0 |
| 12148 | 1:3360476:03B01:B12 | XM 040922 | Homo sapiens interleukin 13 receptor, alpha 2 (IL13RA2), mRNA | 0 |
| 17394 | RG:1943755:10016:A07 | AF346607 | Homo sapiens interleukin-1 receptor associated kinase 1b (IRAK) mRNA, complete cds, alternatively spliced | 0 |
| 27017 | 035JN021.F03 | XM 051742 | Homo sapiens spermine synthase (SMS), mRNA | 0 |
| 25809 | 035JN002.B07 | XM 009699 | Homo sapiens nuclear receptor interacting protein 1 (NRIP1), mRNA | 0 |
| 8719 | 1:2600080:10A01:H01 | XM 009665 | Homo sapiens Kreisler (mouse) maf-related leucine zipper homolog (KRML), mRNA | 0 |
| 21030 | RG:1714832:10015:C06 | XM 029957 | Homo sapiens Rab acceptor 1 (prenylated) (RABAC1), mRNA | 0 |
| 11436 | 1:1470085:03B01:F05 | XM 038976 | Homo sapiens N-ethylmaleimide-sensitive factor attachment protein, alpha (NAPA), mRNA | 0 |
| 10374 | 1:1513989:03B02:C03 | XM 009010 | Homo sapiens complement component 3 (C3), mRNA | 1.4E-96 |
| 19037 | 1:417827:15A01:G10 | X79538 | H.sapiens nuk_34 mRNA for translation initiation factor | 1.9E-28 |
| 398 | M00027016A:C05 | XM 031470 | Homo sapiens aldolase C, fructose-bisphosphate (ALDOC), mRNA | 4E-62 |
| 18773 | 1:1211682:14A02:C09 | XM 008477 | Homo sapiens aldolase C, fructose-bisphosphate (ALDOC), mRNA | 0 |
| 3583 | M00023407B:C10 | | | |
| 3418 | M00001470A:C03 | XM 043951 | Homo sapiens CDP-diacylglycerol--inositol 3-phosphatidyltransferase (phosphatidylinositol synthase) (CDIPT), mRNA | 0 |
| 18985 | I:1402615:09A02:E03 | AF191148 | Homo sapiens type I transmembrane protein Fn14 mRNA, complete cds | 7.9E-64 |
| 25861 | 035JN010.D01 | XM 047975 | Homo sapiens hydroxyacyl glutathione hydrolase (HAGH), mRNA | 0 |
| 3317 | M00003974D:E04 | AF136185 | Homo sapiens collagen type XVII (COL17A1) gene, 3' UTR, long form | 0 |
| 8743 | 1:1858905:04A01:D01 | U36775 | Human ribonuclease 4 gene, partial cds | 2.1E-57 |
| 26240 | 035JN015.A02 | XM 007493 | Homo sapiens ribonuclease, RNase A family, 4 (RNASE4), mRNA | 0 |
| 28562 | 037XN007.B11 | X00947 | Human alpha 1-antichymotrypsin gene fragment | 0 |
| 16877 | 1:2362945:15A01:C07 | XM 029378 | Homo sapiens checkpoint suppressor 1 (CHES1), mRNA | 1.9E-91 |
| 25955 | 035JN022.C01 | AF035620 | Homo sapiens BRCA1-associated protein 2 (BRAP2) mRNA, complete cds | 0 |
| 26308 | 035JN023.C02 | XM 041470 | Homo sapiens zinc finger protein 145 (Kruppel-like, expressed in promyelocytic leukemia) (ZNF145), mRNA | 0 |
| 4140 | 2239-4 | X03083 | Human lactate dehydrogenase-A gene exon 7 and 3' flanking region | 0 |
| 3436 | 2239-1 | X03083 | Human lactate dehydrogenase-A gene exon 7 and 3' flanking region | 0 |
| 25612 | 035JN023.G01 | M94856 | Human fatty acid binding protein homologue (PA-FABP) mRNA, complete cds | 0 |
| 12257 | 1:1448135:04A01:A06 | X15535 | H.sapiens lysosomal acid phosphatase gene (EC 3.1.3.2) Exon 11 | 0 |
| 9111 | 1:1958902:04A02:D07 | D87258 | Homo sapiens mRNA for serin protease with IGF-binding motif, complete cds | 0 |
| 17620 | 1:875567:15B01:B08 | XM 045326 | Homo sapiens MAX-interacting protein 1 (MXI1), mRNA | 0 |
| 26025 | 035JN030.F01 | XM 032511 | Homo sapiens procollagen-proline, 2-oxoglutarate 4-dioxygenase (proline 4-hydroxylase), alpha polypeptide I (P4HA1), mRNA | 0 |
| 19271 | RG:686684:10010:D04 | AF005216 | Homo sapiens receptor-associated tyrosine kinase (JAK2) mRNA, complete cds | 0 |
| 4151 | 2035-1 | D87953 | Human mRNA for RTP, complete cds | 0 |
| 26569 | 035JN010.F02 | AB004788 | Homo sapiens mRNA for BNIP3L, complete cds | 0 |
| 10344 | 1:2859338:11B02:D03 | XM 005052 | Homo sapiens angiopoietin 1 (ANGPT1), mRNA | 1.3E-97 |
| 832 | M00021649B:D05 | XM 004628 | Homo sapiens hypoxia-inducible protein 2 (HIG2), mRNA | 0 |
| 12071 | 1:1798283:06A01:D06 | S72481 | pantophysin [human, keratinocyte line HaCaT, mRNA, 2106 nt] | 0 |
| 12271 | 1:1445767:04A01:H06 | X12701 | H.sapiens mRNA for endothelial plasminogen activator inhibitor PAI | 1.8E-130 |
| 11433 | 1:1526282:03A01:E05 | XM 033627 | Homo sapiens glycoprotein (transmembrane) nmb (GPNMB), mRNA | 3.7E-117 |
| 20917 | RG:222350:10007:C12 | X00663 | Human mRNA fragment for epidermal growth factor (EGF) receptor | 1.7E-122 |
| 25810 | 035JN004.B07 | X00588 | Human mRNA for precursor of epidermal growth factor receptor | 0 |
| 12039 | 1:3506985:07A01:D06 | M24795 | Human CD36 antigen mRNA, complete cds | 0 |
| 25499 | 035JN005.G07 | XM 028224 | Homo sapiens N-acetylglucosamine-phosphate mutase (AGM1), mRNA | 0 |
| 25557 | 035JN013.D07 | BC010135 | Homo sapiens, cyclin C, clone IMAGE:4106819, mRNA | 0 |
| 9917 | I:1283532:05A01:G09 | XM 004148 | Homo sapiens 5T4 oncofetal trophoblast glycoprotein (5T4), mRNA | 2.4E-70 |
| 19505 | RG:204653:10007:A10 | XM 003789 | Homo sapiens colony stimulating factor 1 receptor, formerly McDonough feline sarcoma viral (v-fms) oncogene homolog (CSF1 R), mRNA | 0 |
| 17491 | RG:277866:10008:B07 | XM 003789 | Homo sapiens colony stimulating factor 1 receptor, formerly McDonough feline sarcoma viral (v-fms) oncogene homolog (CSF1R), mRNA | 0 |
| 10683 | I:1686726:06A01:F10 | XM 003789 | Homo sapiens colony stimulating factor 1 receptor, formerly McDonough feline sarcoma viral (v-fms) oncogene homolog (CSF1R), mRNA | 0 |
| 1936 | M00008020C:H09 | X68277 | H.sapiens CL 100 mRNA for protein tyrosine phosphatase | 0 |
| 828 | M00021638B:F03 | X68277 | H.sapiens CL 100 mRNA for protein tyrosine phosphatase | 0 |
| 9558 | I:1824443:05B02:C08 | XM 003708 | Homo sapiens gamma-aminobutyric acid (GABA) A receptor, pi (GABRP), mRNA | 0 |
| 20164 | I:1997963:14B02:B05 | XM 003631 | Homo sapiens solute carrier family 25 (mitochondrial carrier; adenine nucleotide translocator), member 4 (SLC25A4), mRNA | 0 |
| 969 | NIH50_40026 | BC008664 | Homo sapiens, clone MGC:9281 IMAGE:3871960, mRNA, complete cds | 0 |
| 9910 | I:1805840:05B01:C09 | XM 003399 | Homo sapiens mannosidase, beta A, lysosomal (MANBA), mRNA | 0 |
| 2427 | M00005767D:B03 | XM 047441 | Homo sapiens RAP1, GTP-GDP dissociation stimulator 1 (RAP1GDS1), mRNA | 0 |
| 19990 | RG:1056692:10012:C11 | XM 003450 | Homo sapiens cyclin G associated kinase (GAK), mRNA | 0 |
| 20605 | I:690313:16A01:G12 | XM 011152 | Homo sapiens insulin-like growth factor binding protein 7 (IGFBP7), mRNA | 0 |
| 10650 | I:2456393:07B01:E10 | AK001580 | Homo sapiens cDNA FLJ10718 fis, clone NT2RP3001096, weakly similar to Rattus norvegicus leprecan mRNA | 0 |
| 25963 | 035JN022.G01 | X53002 | Human mRNA for integrin beta-5 subunit | 0 |
| 25562 | 035JN015.F07 | X53002 | Human mRNA for integrin beta-5 subunit | 0 |
| 9377 | I:2782593:12A01:A02 | X60656 | H.sapiens mRNA for elongation factor 1-beta | 1.4E-46 |
| 17618 | I:707667:15B01:A08 | XM 002273 | Homo sapiens inhibitor of DNA binding 2, dominant negative helix-loop-helix protein (ID2), mRNA | 3.5E-117 |
| 12136 | I:3208994:03B01:D06 | U16267 | Human AMP deaminase isoform L, alternatively spliced (AMPD2) mRNA, exons 1A, 2 and 3, partial cds | 0 |
| 17373 | I:1538189:14A02:G07 | XM 046818 | Homo sapiens similar to receptor tyrosine kinase-like orphan receptor 1 (H. sapiens) (LOC92711), mRNA | 8.3E-123 |
| 18577 | RG:503209:10010:A09 | XM 049305 | Homo sapiens Lysosomal-associated multispanning membrane protein-5 (LAPTM5), mRNA | 0 |
| 3143 | M00001605D:C02 | BC003107 | Homo sapiens, inhibitor of DNA binding 3, dominant negative helix-loop-helix protein, clone MGC:1988 IMAGE:3543936, mRNA, complete | 1.7E-88 |
| 17737 | RG:155066:10006:E02 | AL050147 | Homo sapiens mRNA; cDNA DKFZp586E0820 (from clone DKFZp586E0820); partial cds | 0 |
| 20029 | I:1923613:17A01:G11 | AF113123 | Homo sapiens carbonyl reductase mRNA, complete cds | 0 |
| 18537 | NIH50_40304 | BC001380 | Homo sapiens, succinate dehydrogenase complex, subunit A, flavoprotein (Fp), clone MGC:1484 IMAGE:3051442, mRNA, complete cds | 0 |
| 10090 | NIH50_40304 | | | |
| 12102 | I:2832414:11B01:C06 | XM 048045 | Homo sapiens katanin p80 (WD40-containing) subunit B 1 (KATNB1), mRNA | 0 |
| 8487 | I:1375115:05A01:D01 | BC001174 | Homo sapiens, exostoses (multiple) 1, clone MGC:2129 IMAGE:3502232, mRNA, complete cds | 0 |
| 9252 | I:1673876:06B01:B02 | BC000917 | Homo sapiens, clone MGC:5184 IMAGE:3048750, mRNA, complete cds | 0 |
| 25605 | 035JN021.D01 | BC000671 | Homo sapiens, claudin 4, clone MGC:1778 IMAGE:3349211, mRNA, complete cds | 0 |
| 29652 | M00001610C:D05 | BC000588 | Homo sapiens, HIRA-interacting protein 3, clone MGC:1814 IMAGE:3345739, mRNA, complete cds | 0 |
| 10858 | I:2458933:04B01:E04 | X97544 | H.sapiens mRNA for TIM17 preprotein translocase | 8.7E-62 |
| 1261 | M00023419C:B06 | U89606 | Human pyridoxal kinase mRNA, complete cds | 0 |
| 4156 | 2243-4 | X93334 | Homo sapiens mitochondrial DNA, complete genome | 0 |
| 3452 | 2243-1 | X93334 | Homo sapiens mitochondrial DNA, complete genome | 0 |
| 2748 | 2242-6 | X93334 | Homo sapiens mitochondrial DNA, complete genome | 0 |
| 2046 | 2248-3 | X93334 | Homo sapiens mitochondrial DNA, complete genome | 0 |
| 2044 | 2242-4 | X93334 | Homo sapiens mitochondrial DNA, complete genome | 0 |
| 1342 | 2248-2 | X93334 | Homo sapiens mitochondrial DNA, complete genome | 0 |
| 1326 | 2244-3 | X93334 | Homo sapiens mitochondrial DNA, complete genome | 0 |
| 9981 | I:1720149:06A01:G09 | AF069604 | Homo sapiens myosin light chain kinase isoform 4 (MLCK) mRNA, partial cds | 0 |
| 27917 | 035JN002.H04 | XM 015978 | Homo sapiens hypothetical protein FLJ22969 (FLJ22969), mRNA | 1.8E-92 |
| 8488 | I:1808529:05B01:D01 | AJ293647 | Homo sapiens partial IL4RA gene for interleukin-4 receptor alfa chain, exon 11, ECSSQV allele | 1.1E-125 |
| 22793 | M00057283C:D06 | AF161410 | Homo sapiens HSPC292 mRNA, partial cds | 0 |
| 26883 | 035JN005.C03 | AF161410 | Homo sapiens HSPC292 mRNA, partial cds | 0 |
| 11540 | I:1909488:10B01:B11 | XM 027739 | Homo sapiens duodenal cytochrome b (FLJ23462), mRNA | 0 |
| 17707 | I:489882:14A01:F02 | X99474 | H.sapiens mRNA for chloride channel, CIC-6c | 0 |
| 20649 | NIH50_41452 | Z14136 | H.sapiens gene for spermidine/spermine N1-acetyltransferase | 0 |
| 24004 | M00056163C:H09 | AF107495 | Homo sapiens FWP001 and putative FWP002 mRNA, complete cds | 0 |
| 11836 | I:1806769:01B02:F11 | X93036 | H.sapiens mRNA for MAT8 protein | 0 |
| 24932 | M00054963C:C09 | M26152 | Homo sapiens serum amyloid A (SAA) mRNA, complete cds | 0 |
| 19143 | RG:149960:10006:D04 | AK003448 | Mus musculus 18 days embryo cDNA, RIKEN full length enriched library, clone:1110004P15, full insert sequence | 8.9E-21 |
| 26257 | 035JN013.B08 | J04056 | Human carbonyl reductase mRNA, complete cds | 0 |
| 21239 | M00054679B:B03 | J02619 | Human Z type alpha-1-antitrypsin gene, complete cds(exons 2-5) | 0 |
| 16959 | I:1426031:14B01:B07 | AY035783 | Homo sapiens laminin 5 beta 3 subunit (LAMB3) mRNA, complete cds | 3.8E-121 |
| 2568 | M00022158D:C11 | XM 036609 | Homo sapiens laminin, beta 3 (nicein (125kD), kalinin (140kD), BM600 (125kD)) (LAMB3), mRNA | 0 |
| 25936 | 035JN020.A07 | XM 036608 | Homo sapiens laminin, beta 3 (nicein (125kD), kalinin (140kD), BM600 (125kD)) (LAMB3), mRNA | 0 |
| 23041 | M00054797C:G10 | XM 046649 | Homo sapiens nuclear factor of kappa light polypeptide gene enhancer in B-cells inhibitor, alpha (NFKBIA), mRNA | 0 |
| 9206 | I:1822716:05B01:C08 | BC008059 | Homo sapiens, clone IMAGE:2967491, mRNA | 0 |
| 25105 | M00054824C:H04 | BC009110 | Homo sapiens, clone MGC: 17355 IMAGE:3453825, mRNA, complete cds | 0 |
| 24779 | M00057061D:G07 | | | |
| 22451 | M00043372B:B06 | X00947 | Human alpha 1-antichymotrypsin gene fragment | 0 |
| 22291 | M00054785D:G05 | X00947 | Human alpha 1-antichymotrypsin gene fragment | 0 |
| 21143 | M00055146A:D11 | | | |
| 24751 | M00054676B:D07 | X03083 | Human lactate dehydrogenase-A gene exon 7 and 3' flanking region | 0 |
| 24294 | M00056163D:E01 | X03083 | Human lactate dehydrogenase-A gene exon 7 and 3' flanking region | 9.4E-110 |
| 24006 | M00056163D:E01 | X03083 | Human lactate dehydrogenase-A gene exon 7 and 3' flanking region | 0 |
| 25678 | 035Jn031.H01 | AK001670 | Homo sapiens cDNA FLJ10808 fis, clone NT2RP4000879, weakly similar to UBIQUITIN ACTIVATING ENZYME E1 | 4.9E-53 |
| 22027 | M00056534C:E08 | XM 003512 | Homo sapiens amphiregulin (schwannoma-derived growth factor) (AREG), mRNA | 0 |
| 29495 | 035JN022.E12 | D83761 | Homo sapiens mRNA for mother against dpp (Mad) related protein, complete cds | 0 |
| 24577 | M00056654B:G02 | XM 038306 | Homo sapiens dual specificity phosphatase 6 (DUSP6), mRNA | 0 |
| 23527 | M00055865C:D04 | | | |
| 17090 | I:341491:13B01:A01 | BC004490 | Homo sapiens, v-fos FBJ murine osteosarcoma viral oncogene homolog, clone MGC:11074 IMAGE:3688670, mRNA, complete cds | 3.8E-98 |
| 25137 | M00057167A:C07 | | | |
| 23772 | M00056360A:E07 | BC004490 | Homo sapiens, v-fos FBJ murine osteosarcoma viral oncogene homolog, clone MGC:11074 IMAGE:3688670, mRNA, complete cds | 0 |
| 1659 | M00001350B:D10 | BC004490 | Homo sapiens, v-fos FBJ murine osteosarcoma viral oncogene homolog, clone MGC:11074 IMAGE:3688670, mRNA, complete cds | 0 |
| 8497 | I:2170638:05A01:A07 | BC006169 | Homo sapiens, Similar to SH3-domain binding protein 5 (BTK-associated), clone MGC:13234 IMAGE:4025362, mRNA, complete cds | 5.2E-125 |
| 25272 | M00054621A:D09 | AF161435 | Homo sapiens HSPC317 mRNA, partial cds | 0 |
| 21216 | M00056194B:G06 | XM 002844 | Homo sapiens procollagen-lysine, 2-oxoglutarate 5-dioxygenase (lysine hydroxylase) 2 (PLOD2), mRNA | 0 |
| 11939 | I:2938757:02A02:B05 | D43767 | Human mRNA for chemokine, complete cds | 0 |
| 9191 | I:1421929:05A01:D02 | X63629 | H.sapiens mRNA for p cadherin | 2.4E-90 |
| 3429 | 2024-3 | AF002697 | Homo sapiens E1B 19K/Bcl-2-binding protein Nip3 mRNA, nuclear gene encoding mitochondrial protein, complete cds | 0 |
| 2725 | 2024-1 | AF002697 | Homo sapiens E1B 19K/Bcl-2-binding protein Nip3 mRNA, nuclear gene encoding mitochondrial protein, complete cds | 0 |
| 19923 | I:1001356:13A01:B11 | BC006318 | Homo sapiens, erythrocyte membrane protein band 4.9 (dematin), clone MGC:12740 IMAGE:4125804, mRNA, complete cds | 1.7E-103 |
| 20457 | I:1923289:19A01:E06 | XM 035603 | Homo sapiens gap junction protein, beta 5 (connexin 31.1) (GJB5), mRNA | 0 |
| 24773 | M00057055D:B11 | | | |
| 24119 | M00042886D:H10 | BC006260 | Homo sapiens, Similar to N-myc downstream regulated, clone MGC:11293 IMAGE:3946764, mRNA, complete cds | 4.4E-114 |
| 3908 | M00027080A:E06 | M60756 | Human histone H2B.1 mRNA, 3' end | 0 |
| 8560 | I:2346704:06B01:H01 | AJ000334 | Homo sapiens mRNA for cytosolic asparaginyl-tRNA synthetase | 0 |
| 24588 | M00055411A:C10 | L19779 | Homo sapiens histone H2A.2 mRNA, complete cds | 0 |
| 4047 | M00007997C:B08 | XM 009091 | Homo sapiens glycogen synthase 1 (muscle) (GYS1), mRNA | 0 |
| 28344 | 035JN011.E11 | XM 050471 | Homo sapiens glycogen synthase 1 (muscle) (GYS1), mRNA | 0 |
| 27561 | 035JN001.F04 | XM 001472 | Homo sapiens v-jun avian sarcoma virus 17 oncogene homolog (JUN), mRNA | 0 |
| 3272 | M00022165C:E12 | NM 001024 | Homo sapiens ribosomal protein S21 (RPS21), mRNA | 0 |
| 26735 | 035JN030.A08 | XM 010408 | Homo sapiens RAB9-like protein (RAB9L), mRNA | 0 |
| 24900 | M00054500D:C08 | BC004427 | Homo sapiens, proteasome (prosome, macropain) subunit, alpha type, 7, clone MGC:3755 IMAGE:2819923, mRNA, complete cds | 0 |
| 9472 | I:2510171:04B01:H08 | X04503 | Human SLPI mRNA fragment for secretory leucocyte protease inhibitor | 0 |
| 9979 | I:1623318:06A01:F09 | L31409 | Homo sapiens creatine transporter mRNA, complete cds | 2.2E-45 |
| 21996 | M00042467B:B04 | L00160 | Human phosphoglycerate kinase (pgk) mRNA, exons 2 to last | 0 |
| 22312 | M00055035D:F05 | | | |
| 11327 | I:3139773:05A01:H11 | L00160 | Human phosphoglycerate kinase (pgk) mRNA, exons 2 to last | 2.6E-21 |
| 18240 | RG:1927470:10015:H08 | V00572 | Human mRNA encoding phosphoglycerate kinase | 0 |
| 21922 | M00054848A:D12 | AF139065 | Homo sapiens desmoplakin I mRNA, partial cds | 0 |
| 22290 | M00057002D:H01 | | | |
| 10390 | I:1405391:03B02:C09 | AF056979 | Homo sapiens clone YAN1 interferon-gamma receptor mRNA, complete cds | 0 |
| 2212 | M00008098B:F06 | U19247 | Homo sapiens interferon-gamma receptor alpha chain gene, exon 7 and complete cds | 0 |
| 20213 | RG:221172:10007:C11 | S74774 | p59fyn(T)=OKT3-induced calcium influx regulator [human, Jurkat J6 T cell line, mRNA Partial, 1605 nt] | 2.9E-103 |
| 24955 | M00055929D:D04 | | | |
| 19574 | I:635178:13B02:C10 | XM 033944 | Homo sapiens superoxide dismutase 2, mitochondrial (SOD2), mRNA | 0 |
| 19969 | RG:501476:10010:A05 | U14394 | Human tissue inhibitor of metalloproteinases-3 mRNA, complete cds | 0 |
| 8570 | I:1696224:06B01:E07 | X70684 | C.aethiops mRNA for heat shock protein 70 | 5.6E-25 |
| 18519 | I:1997703:13A01:D09 | X52947 | Human mRNA for cardiac gap junction protein | 0 |
| 9616 | I:3200341:06B02:H02 | Y00106 | Human gene for beta-adrenergic receptor (beta-2 subtype) | 0 |
| 22334 | M00055067D:H12 | | | |
| 17459 | I:2056395:13A02:B07 | M77349 | Human transforming growth factor-beta induced gene product (BIGH3) mRNA, complete cds | 2.5E-121 |
| 25193 | M00056763B:A12 | X68277 | H.sapiens CL 100 mRNA for protein tyrosine phosphatase | 0 |
| 25191 | M00056763B:A12 | X68277 | H.sapiens CL 100 mRNA for protein tyrosine phosphatase | 0 |
| 9448 | I:2455617:04B01:D02 | XM 051799 | Homo sapiens guanosine monophosphate reductase (GMPR), mRNA | 0 |
| 25224 | RG:950682:10003:D06 | BC002536 | Homo sapiens, phosphofructokinase, platelet, clone MGC:2192 IMAGE:3140233, mRNA, complete cds | 0 |
| 20218 | RG:2158297:10016:E11 | BC002536 | Homo sapiens, phosphofructokinase, platelet, clone MGC:2192 IMAGE:3140233, mRNA, complete cds | 0 |
| 3089 | NIH50_26184 | D25328 | Human mRNA for platelet-type phosphofructokinase, complete cds | 2E-108 |
| 23985 | NIH50_26184 | | | |
| 19953 | NIH50_26184 | D25328 | Human mRNA for platelet-type phosphofructokinase, complete cds | 2E-108 |
| 11506 | NIH50_26184 | | | |
| 22362 | M00056349A:F08 | M10546 | Human mitochondrial DNA, fragment M1, encoding transfer RNAs, cytochrome oxidase I, and 2 URFs | 1.2E-86 |
| 25516 | 035JN011.G01 | XM 011470 | Homo sapiens myristoylated alanine-rich protein kinase C substrate (MARCKS, 80K-L) (MACS), mRNA | 0 |
| 25757 | 037XN005.H07 | AF017116 | Homo sapiens type-2 phosphatidic acid phosphohydrolase (PAP2) mRNA, complete cds | 0 |
| 24814 | M00042773B:E09 | M17733 | Human thymosin beta-4 mRNA, complete cds | 0 |
| 21994 | M00042465B:E04 | M17733 | Human thymosin beta-4 mRNA, complete cds | 0 |
| 27117 | 037XN001.H03 | BC001631 | Homo sapiens, prothymosin beta 4, clone MGC:2219 IMAGE:3536637, mRNA, complete cds | 0 |
| 24681 | NIH50_41452 | | | |
| 22745 | M00056592A:B08 | NM 003739 | Homo sapiens aldo-keto reductase family 1, member C3 (3-alpha hydroxysteroid dehydrogenase, type II) (AKR1C3), mRNA | 0 |
| 24233 | M00055873C:B06 | | | |
| 2001 | M00001381A:F03 | XM 035387 | Homo sapiens ribosomal protein, large, P1 (RPLP1), mRNA | 0 |
| 21179 | NIH50_43550 | | | |
| 17147 | NIH50_43550 | AK026515 | Homo sapiens cDNA: FLJ22862 fis, clone KAT01966, highly similar to HSLDHAR Human mRNA for lactate dehydrogenase-A | 0 |
| 8700 | NIH50_43550 | | | |
| 21214 | M00056193B:D06 | BC006260 | Homo sapiens, Similar to N-myc downstream regulated, clone MGC:11293 IMAGE:3946764, mRNA, complete cds | 0 |
| 26422 | 037XN003.D08 | BC006260 | Homo sapiens, Similar to N-myc downstream regulated, clone MGC:11293 IMAGE:3946764, mRNA, complete cds | 0 |
| 22837 | M00055891C:B09 | | | |
| 21965 | M00057029A:G09 | | | |
| 25541 | 035JN013.D01 | AK026310 | Homo sapiens cDNA: FLJ22657 fis, clone HSI07791, highly similar to HUMCYB5 Human cytochrome b5 mRNA | 0 |
| 18302 | I:1738248:09B02:G08 | XM 016114 | Homo sapiens hypothetical protein FLJ22501 (FLJ22501), mRNA | 0 |
| 24049 | M00054706B:G04 | AF107495 | Homo sapiens FWP001 and putative FWP002 mRNA, complete cds | 0 |
| 26326 | 035JN023.D08 | AK025906 | Homo sapiens cDNA: FLJ22253 fis, clone HRC02763 | 0 |
| 2254 | M00004085C:C02 | AK025703 | Homo sapiens cDNA: FLJ22050 fis, clone HEP09454 | 0 |
| 10296 | I:2868216:07B02:D09 | AK025703 | Homo sapiens cDNA: FLJ22050 fis, clone HEP09454 | 0 |
| 20044 | I:2547084:09B01:F05 | XM 016847 | Homo sapiens hypothetical protein FLJ22002 (FLJ22002), mRNA | 0 |
| 28806 | 035JN028.D05 | AK025504 | Homo sapiens cDNA: FLJ21851 fis, clone HEP01962 | 0 |
| 17566 | I:446969:17B02:G07 | AK023217 | Homo sapiens cDNA FLJ13155 fis, clone NT2RP3003433 | 2E-115 |
| 19005 | I:2674167:09A02:G09 | AK022968 | Homo sapiens cDNA FLJ12906 fis, clone NT2RP2004373 | 0 |
| 3567 | M00023369D:C05 | | | |
| 21983 | M00057081B:H03 | | | |
| 458 | M00022134B:E08 | XM 037412 | Homo sapiens hypothetical gene supported by BC008993 (LOC91283), mRNA | 0 |
| 22331 | M00057138A:E11 | | | |
| 21411 | M00055833D:B03 | | | |
| 22972 | M00056956D:B01 | | | |
| 24533 | RG:1643392:10014:C11 | | | |
| 24853 | M00056617D:F07 | AK020869 | Mus musculus adult retina cDNA, RIKEN full-length enriched library, clone:A930017A02, full insert sequence | 6.5E-59 |
| 23753 | M00054915A:G02 | | | |
| 21502 | M00056193B:D06 | | | |
| 18180 | RG:39422:10005:B02 | | | |
| 23918 | M00056278C:E03 | | | |
| 24144 | RG:1982961:20001:H05 | | | |
| 19996 | RG:1283072:10012:F11 | BC009107 | Homo sapiens, clone MGC:17352 IMAGE:3449913, mRNA, complete cds | 0 |
| 11528 | I:1899534:10B01:D05 | | | |
| 20506 | I:1969044:18B01:E12 | AB048286 | Homo sapiens GS1999full mRNA, complete cds | 0 |
| 23833 | RG:1656861:10014:E10 | | | |
| 20042 | I:1873176:09B01:E05 | BC001909 | Homo sapiens, clone IMAGE:3537447, mRNA, partial cds | 0 |
| 24977 | M00055820D:F01 | | | |
| 11646 | I:1723142:08B02:G11 | AK014612 | Mus musculus 0 day neonate skin cDNA, RIKEN full-length enriched library, clone:4633401I05, full insert sequence | 4.6E-45 |
| 24872 | RG:773612:10011:D06 | | | |
| 10577 | I:2174196:08A01:A10 | | | |
| 21710 | RG:1091554:10003:G01 | | | |
| 18556 | RG:31082:10004:F09 | | | |
| 29433 | 035JN014.F12 | AK001805 | Homo sapiens cDNA FLJ10943 fis, clone OVARC1001360 | 0 |
| 29273 | 037XN005.F12 | | | |
| 28763 | 035JN018.G11 | AJ310543 | Homo sapiens mRNA for EGLN1 protein | 1.9E-40 |
| 27887 | RG:2364147:8119908:A10 | | | |
| 27450 | 035JN032.F09 | | | |
| 27255 | 035JN006.E09 | XM 027456 | Homo sapiens hypothetical gene supported by AK000584 (LOC89942), mRNA | 1.2E-57 |
| 27226 | 035JN004.F09 | | | |
| 26550 | 035JN008.D08 | | | |
| 26508 | 035JN004.G02 | | | |
| 26483 | RG:2377371:8119908:C08 | | | |
| 26334 | 035JN023.H08 | AF364547 | Homo sapiens methylmalonyl-CoA epimerase mRNA, complete cds; nuclear gene for mitochondrial product | 0 |
| 26027 | 035JN030.G01 | | | |
| 25977 | 035JN022.F07 | | | |
| 25965 | 035JN022.H01 | | | |
| 25844 | 035JN008.C07 | | | |
| 25834 | 035JN008.F01 | AB048289 | Bos taurus lae mRNA for lipoate-activating enzyme, complete cds | 3.1E-35 |
| 25816 | 035JN004.E07 | | | |
| 25746 | 037XN007.B07 | | | |
| 25742 | 037XN007.H01 | | | |
| 25741 | 037XN005.H01 | | | |
| 25712 | 037XN003.A07 | | | |
| 25642 | 035Jn027.F01 | | | |
| 25621 | 035JN021.D07 | AK027321 | Homo sapiens cDNA FLJ14415 fis, clone HEMBA1004889, weakly similar to Human C3f mRNA | 0 |
| 25614 | 035JN023.H01 | | | |
| 25603 | 035JN021.C01 | | | |
| 25556 | 035JN015.C07 | | | |
| 25555 | 035JN013.C07 | | | |
| 25540 | 035JN015.C01 | | | |
| 23576 | RG.1984769:20002:D10 | | | |
| 22566 | RG:1996656:20003:C03 | | | |
| 9036 | DD182 | | | |
| 4164 | M00007932B:E06 | | | |
| 4146 | 2179-5 | | | |
| 4091 | M00026845A:E01 | | | |
| 4072 | M00023398A:G12 | | | |
| 4022 | M00022127D:B06 | | | |
| 3965 | M00005406A:f04 | | | |
| 3954 | M00005400B:E1 | | | |
| 3872 | M00007974D:B04 | | | |
| 3869 | M00003868C:A03 | | | |
| 3838 | M00007052A:C09 | XM 048272 | Homo sapiens similar to Ras-related GTP-binding protein (H. sapiens) (LOC92951), mRNA | 0 |
| 3806 | 2168-2 | | | |
| 3798 | 2138-4 | | | |
| 3792 | 2171-5 | | | |
| 3788 | 2156-4 | | | |
| 3767 | M00001355D:H12 | | | |
| 3458 | M00007160D:E10 | | | |
| 3251 | M00005471A:a04 | | | |
| 3194 | DF821 | | | |
| 3102 | 2167-1 | | | |
| 3094 | 2138-3 | | | |
| 2671 | M00023431A:D02 | | | |
| 2634 | M00008025D:A04 | | | |
| 2567 | M00008061B:A12 | | | |
| 2317 | M00001502D:E09 | | | |
| 1958 | M00023296B:B09 | | | |
| 1680 | 2169-5 | | | |
| 1625 | M00001542C:G08 | | | |
| 1445 | M00023335C:C09 | | | |
| 1320 | 2207-5 | | | |
| 974 | 2161-1 | | | |
| 726 | DO15 | | | |
| 718 | ER418 | | | |
| 703 | M00004189D:A11 | | | |
| 652 | M00007070A:C08 | | | |
| 630 | 2203-2 | | | |
| 593 | M00001373A:A06 | X93036 | H.sapiens mRNA for MAT8 protein | 0 |
| 532 | M00022005A:H05 | | | |
| 272 | 2168-5 | | | |
| 256 | M00001406C:H12 | | | |
| 57 | M00023371B:H02 | | | |

**Table 2**

| SEQ ID NO. | SPOT ID | 2D T4-2/ 2D S1 | 3D T4-2/ 3D S1 | 3D S1/ 2D S1 | 3D T4-2/ 3D 2D T4-2 | 3D T4-2/ EGFR Ab | 3D T4-2/ B1 Integrin Ab | 3D T4-2/ Tyr |
|---|---|---|---|---|---|---|---|---|
| 1 | 10594 | 0.6 | 2.2 | 0.6 | 1.9 | 3.0 | 1.0 | 2.9 |
| 2 | 21851 | 1.0 | 1.0 | 1.0 | 3.5 | 1.3 | 1.0 | 1.0 |
| 3 | 20990 | 1.6 | 4.6 | 1.0 | 1.5 | 1.0 | 1.0 | 1.0 |
| 4 | 18641 | 1.0 | 0.6 | 2.6 | 1.7 | 1.0 | 1.6 | 1.0 |
| 5 | 17229 | 0.3 | 0.8 | 1.0 | 2.1 | 1.0 | 1.0 | 1.0 |
| 6 | 25930 | 1.0 | 1.0 | 1.0 | 1.0 | 1.0 | 1.0 | 1.0 |
| 7 | 20701 | 1.6 | 2.9 | 1.3 | 2.7 | 4.5 | 1.9 | 5.8 |
| 8 | 20346 | 1.7 | 2.7 | 1.4 | 2.6 | 4.3 | 2.0 | 5.2 |
| 9 | 21247 | 1.0 | 4.4 | 1.5 | 3.0 | 3.4 | 2.6 | 4.7 |
| 10 | 23062 | 0.6 | 2.5 | 0.6 | 1.8 | 3.3 | 1.4 | 2.7 |
| 11 | 25666 | 1.0 | 2.9 | 0.6 | 2.0 | 3.6 | 1.0 | 2.3 |
| 12 | 19001 | 8.5 | 14.2 | 1.0 | 1.0 | 4.8 | 1.7 | 8.0 |
| 13 | 10897 | 1.0 | 3.1 | 4.5 | 1000.0 | 13.3 | 4.6 | 18.4 |
| 14 | 1960 | 0.3 | 1.5 | 3.0 | 13.7 | 3.9 | 2.4 | 4.9 |
| 15 | 26381 | 1.0 | 1.0 | 1.0 | 0.9 | 1.0 | 1.0 | 1.0 |
| 16 | 26719 | 0.4 | 1.0 | 0.6 | 2.8 | 1.2 | 1.7 | 1.0 |
| 17 | 27152 | 4.2 | 3.0 | 2.2 | 1.5 | 1.3 | 1.0 | 1.3 |
| 18 | 10926 | 0.7 | 1.9 | 0.9 | 2.1 | 3.7 | 1.5 | 3.3 |
| 19 | 28980 | 0.6 | 1.4 | 1.0 | 2.4 | 1.0 | 1.0 | 1.0 |
| 20 | 1236 | 1.0 | 2.8 | 0.8 | 2.1 | 2.2 | 1.8 | 3.2 |
| 21 | 29350 | 0.5 | 0.6 | 1.2 | 2.1 | 1.4 | 1.0 | 10 |
| 22 | 26242 | 1.0 | 1.0 | 0.6 | 2.2 | 1.0 | 1.0 | 2.0 |
| 23 | 4098 | 1.4 | 3.9 | 0.6 | 2.1 | 2.7 | 1.3 | 3.1 |
| 24 | 17432 | 0.4 | 0.3 | 2.4 | 2.1 | 0.3 | 0.9 | 0.3 |
| 25 | 1785 | 0.5 | 0.4 | 2.4 | 2.0 | 0.3 | 1.0 | 0.3 |
| 26 | 28856 | 8.5 | 0.9 | 2.5 | 0.3 | 0.6 | 1.0 | 0.5 |
| 27 | 18791 | 1.0 | 0.2 | 0.3 | 4.1 | 1.0 | 1.0 | 1.3 |
| 28 | 22950 | 3.9 | 4.1 | 1.2 | 1.0 | 2.1 | 1.0 | 2.4 |
| 29 | 1882 | 2.4 | 4.1 | 0.9 | 1.8 | 3.2 | 1.5 | 4.7 |
| 30 | 23886 | 1.0 | 1.0 | 1.2 | 2.1 | 1.0 | 1.0 | 1.0 |
| 31 | 24995 | 2.0 | 1.6 | 2.1 | 1.0 | 1.0 | 1.0 | 1.0 |
| 32 | 24477 | 1.0 | 1.9 | 1.0 | 4.2 | 2.7 | 1.3 | 1.8 |
| 33 | 21681 | 1.7 | 7.1 | 0.6 | 2.0 | 2.8 | 1.0 | 3.6 |
| 34 | 9557 | 1.6 | 7.5 | 0.8 | 1.0 | 3.0 | 1.0 | 2.5 |
| 35 | 22033 | 2.8 | 3.7 | 1.0 | 0.9 | 2.2 | 1.0 | 2.7 |
| 36 | 873 | 1.0 | 4.0 | 1.0 | 2.7 | 1.7 | 1.0 | 1.0 |
| 37 | 17144 | 1.0 | 0.5 | 3.6 | 1.4 | 1.0 | 1.0 | 1.0 |
| 38 | 26970 | 6.0 | 15.3 | 0.2 | 0.6 | 2.9 | 1.0 | 5.4 |
| 39 | 21402 | 0.2 | 1.0 | 2.8 | 6.9 | 2.4 | 1.0 | 3.6 |
| 40 | 27074 | 1.7 | 2.5 | 2.3 | 3.2 | 1.6 | 1.0 | 2.0 |
| 41 | 10963 | 0.5 | 0.3 | 2.1 | 0.5 | 1.0 | 1.0 | 0.7 |
| 42 | 29525 | 0.6 | 1.0 | 0.7 | 2.4 | 1.7 | 1.3 | 1.0 |
| 43 | 25514 | 1000.0 | 1.0 | 1.0 | 1.0 | 0.5 | 1.0 | 1.0 |
| 44 | 26612 | 0.4 | 0.5 | 1.6 | 2.8 | 0.8 | 1.0 | 0.8 |
| 45 | 24600 | 1.6 | 2.7 | 1.0 | 2.0 | 1.0 | 1.2 | 1.4 |
| 46 | 9741 | 2.3 | 5.0 | 1.0 | 2.2 | 1.7 | 1.0 | 1.0 |
| 47 | 23689 | 1.0 | 2.6 | 0.8 | 1.8 | 2.3 | 1.0 | 2.7 |
| 48 | 22352 | 1.0 | 2.9 | 0.7 | 1.6 | 2.4 | 1.0 | 2.4 |
| 49 | 23806 | 1.0 | 0.4 | 1.3 | 2.3 | 1.0 | 1.4 | 1.4 |
| 50 | 12285 | 1.0 | 1.0 | 1.0 | 1.0 | 0.8 | 1.0 | 0.5 |
| 51 | 27638 | 0.6 | 1.0 | 0.8 | 2.2 | 2.1 | 1.0 | 1.0 |
| 52 | 9663 | 1.0 | 1.0 | 1.0 | 1000.0 | 1.0 | 1.0 | 1.0 |
| 53 | 26850 | 1.0 | 0.2 | 9.1 | 2.1 | 1.3 | 1.6 | 2.2 |
| 54 | 10204 | 2.9 | 2.3 | 0.8 | 0.6 | 3.1 | 1.4 | 2.4 |
| 55 | 1318 | 2.0 | 0.9 | 2.3 | 0.5 | 0.6 | 1.1 | 0.7 |
| 56 | 25922 | 1.0 | 0.8 | 1.0 | 1.0 | 1.0 | 1.0 | 1.0 |
| 57 | 26347 | 1.0 | 1.0 | 1.0 | 1.0 | 1.0 | 1.0 | 1.0 |
| 58 | 20361 | 1.0 | 1.0 | 1.0 | 2.0 | 1.0 | 1.0 | 1.0 |
| 59 | 28672 | 0.6 | 2.1 | 0.6 | 2.1 | 1.4 | 1.0 | 1.7 |
| 60 | 25520 | 0.5 | 0.3 | 2.3 | 1.3 | 1.0 | 0.7 | 0.5 |
| 61 | 1723 | 1.0 | 0.5 | 5.1 | 3.5 | 1.0 | 3.1 | 1.0 |
| 62 | 28863 | 0.8 | 1.3 | 1.0 | 2.3 | 1.7 | 1.7 | 1.7 |
| 63 | 25526 | 5.9 | 1.7 | 1.0 | 0.6 | 0.6 | 0.7 | 0.4 |
| 64 | 27936 | 1.0 | 1.0 | 3.2 | 3.1 | 1.9 | 3.1 | 1.5 |
| 65 | 26851 | 1.0 | 0.7 | 3.2 | 2.7 | 1.6 | 2.4 | 1.3 |
| 66 | 25107 | 1.0 | 5.8 | 1.0 | 2.6 | 2.6 | 1.6 | 2.6 |
| 67 | 24912 | 1.0 | 2.9 | 1.0 | 2.4 | 1.6 | 1.3 | 1.8 |
| 68 | 25169 | 1.0 | 0.7 | 2.5 | 1.5 | 1.0 | 1.0 | 1.0 |
| 69 | 25600 | 1.6 | 1.4 | 2.9 | 2.1 | 0.7 | 0.9 | 0.5 |
| 70 | 28706 | 0.2 | 0.5 | 0.6 | 2.1 | 1.3 | 1.2 | 1.0 |
| 71 | 26377 | 0.6 | 0.3 | 2.2 | 1.0 | 1.2 | 1.3 | 1.0 |
| 72 | 19460 | 2.4 | 1.5 | 2.5 | 1.3 | 1.0 | 1.0 | 0.8 |
| 73 | 25243 | 1.0 | 0.7 | 2.2 | 1.0 | 1.0 | 1.0 | 1.0 |
| 74 | 20018 | 1.0 | 1.0 | 1.0 | 2.6 | 1.0 | 1.0 | 1.0 |
| 75 | 918 | 1.0 | 1.7 | 1.3 | 2.1 | 2.0 | 1.6 | 2.4 |
| 76 | 25027 | 1.0 | 1.0 | 1.0 | 1.0 | 1.0 | 1.0 | 1.0 |
| 77 | 29089 | 0.6 | 0.5 | 0.8 | 2.1 | 1.0 | 1.0 | 1.0 |
| 78 | 9141 | 1.0 | 1.0 | 1.0 | 1.0 | 1.0 | 1.0 | 1.0 |
| 79 | 12005 | 1.0 | 1.0 | 2.2 | 1.0 | 1.0 | 1.0 | 1.0 |
| 80 | 12148 | 1.0 | 1.0 | 1.0 | 1.0 | 1.0 | 1.0 | 1.0 |
| 81 | 17394 | 0.4 | 0.6 | 2.1 | 2.0 | 1.0 | 1.0 | 1.0 |
| 82 | 27017 | 2.8 | 3.3 | 0.8 | 1.0 | 2.4 | 1.8 | 2.8 |
| 83 | 25809 | 1.0 | 1.0 | 1000.0 | 1.0 | 1.0 | 1.0 | 1.0 |
| 84 | 8719 | 0.1 | 1.0 | 2.3 | 2.1 | 0.4 | 0.5 | 0.3 |
| 85 | 21030 | 0.4 | 1.0 | 1.3 | 2.1 | 1.4 | 1.6 | 1.4 |
| 86 | 11436 | 0.7 | 0.4 | 2.0 | 1.0 | 0.6 | 0.8 | 0.6 |
| 87 | 10374 | 1.5 | 1.5 | 3.5 | 2.7 | 0.4 | 1.0 | 0.3 |
| 88 | 19037 | 3.0 | 3.3 | 0.9 | 1.5 | 2.7 | 1.4 | 3.7 |
| 89 | 398 | 1.6 | 6.9 | 1.1 | 3.3 | 2.4 | 1.0 | 4.5 |
| 90 | 18773 | 1.9 | 5.1 | 1.0 | 3.9 | 3.8 | 2.0 | 6.1 |
| 91 | 3583 | 0.5 | 0.7 | 1.0 | 2.0 | 2.5 | 1.0 | 1.5 |
| 92 | 3418 | 1.8 | 3.2 | 1.2 | 2.4 | 1.6 | 1.0 | 1.2 |
| 93 | .18985 | 9.2 | 3.1 | 1.0 | 0.6 | 2.3 | 1.1 | 2.5 |
| 94 | 25861 | 3.4 | 1.5 | 2.0 | 0.8 | 0.8 | 0.9 | 0.6 |
| 95 | 3317 | 0.9 | 2.3 | 1.0 | 3.4 | 1.9 | 1.0 | 1.0 |
| 96 | 8743 | 0.2 | 0.7 | 1.0 | 4.3 | 1.8 | 1.0 | 1.7 |
| 97 | 26240 | 0.2 | 1.0 | 1.0 | 5.3 | 1.9 | 1.9 | 1.1 |
| 98 | 28562 | 0.3 | 0.2 | 2.0 | 1.0 | 0.5 | 0.5 | 0.6 |
| 99 | 16877 | 1.0 | 2.6 | 1.1 | 2.6 | 1.7 | 1.5 | 1.3 |
| 100 | 25955 | 1.0 | 1.0 | 1.0 | 1.0 | 1.0 | 1.0 | 1.0 |
| 101 | 26308 | 0.2 | 0.4 | 1.0 | 2.2 | 0.7 | 0.8 | 0.6 |
| 102 | 4140 | 1.9 | 6.7 | 0.7 | 2.1 | 3.0 | 1.0 | 3.5 |
| 103 | 3436 | 1.8 | 6.3 | 0.6 | 2.2 | 3.1 | 1.3 | 3.3 |
| 104 | 25612 | 1.0 | 12.5 | 1.0 | 1.0 | 2.1 | 1.0 | 2.9 |
| 105 | 12257 | 1.0 | 1.0 | 2.0 | 1.0 | 0.8 | 0.9 | 0.8 |
| 106 | 9111 | 0.5 | 0.5 | 2.2 | 1.3 | 1.5 | 1.0 | 0.7 |
| 107 | 17620 | 0.3 | 0.8 | 1.0 | 3.2 | 2.7 | 2.1 | 1.0 |
| 108 | 26025 | 1.0 | 2.9 | 1.1 | 2.2 | 2.3 | 1.0 | 2.6 |
| 109 | 19271 | 0.5 | 1.3 | 0.7 | 2.2 | 1.6 | 1.2 | 1.5 |
| 110 | 4151 | 0.4 | 4.2 | 1.2 | 11.1 | 4.2 | 1.0 | 2.9 |
| 111 | 26569 | 0.7 | 2.2 | 0.8 | 2.9 | 2.3 | 1.7 | 2.6 |
| 112 | 10344 | 1.0 | 1.0 | 1.0 | 1.0 | 1.0 | 1.0 | 1.0 |
| 113 | 832 | 1.0 | 3.3 | 1.0 | 2.4 | 3.7 | 2.2 | 4.0 |
| 114 | 12071 | 1.8 | 1.5 | 2.2 | 1.0 | 1.3 | 0.8 | 1.4 |
| 115 | 12271 | 0.6 | 4.9 | 1.9 | 14.9 | 20.8 | 4.0 | 24.1 |
| 116 | 11433 | 0.5 | 0.4 | 5.7 | 3.0 | 1.7 | 1.8 | 1.0 |
| 117 | 20917 | 1.0 | 2.8 | 0.9 | 2.6 | 1.7 | 1.4 | 1.7 |
| 118 | 25810 | 1.1 | 3.8 | 1.0 | 2.9 | 1.5 | 1.3 | 1.5 |
| 119 | 12039 | 1.0 | 1.0 | 3.6 | 1.0 | 1.0 | 1.0 | 1.0 |
| 120 | 25499 | 1.0 | 1.0 | 1.0 | 1.0 | 1.0 | 1.0 | 1.0 |
| 121 | 25557 | 1.0 | 1.8 | 1.0 | 0.8 | 1.0 | 1.0 | 1.0 |
| 122 | 9917 | 2.5 | 2.7 | 0.7 | 1.6 | 3.8 | 1.2 | 3.6 |
| 123 | 19505 | 0.4 | 1.7 | 0.7 | 3.8 | 1.7 | 1.6 | 1.4 |
| 124 | 17491 | 0.6 | 1.7 | 0.7 | 2.5 | 1.6 | 1.3 | 1.4 |
| 125 | 10683 | 0.4 | 1.9 | 0.6 | 3.6 | 1.7 | 1.4 | 1.1 |
| 126 | 1936 | 0.2 | 0.6 | 0.6 | 3.1 | 1.0 | 1.8 | 1.0 |
| 127 | 828 | 0.1 | 1.0 | 0.5 | 3.0 | 1.0 | 1.7 | 1.2 |
| 128 | 9558 | 1.0 | 1.0 | 1.0 | 1.0 | 1.0 | 1.0 | 1.0 |
| 129 | 20164 | 2.0 | 1.1 | 2.5 | 1.7 | 1.0 | 1.0 | 0.8 |
| 130 | 969 | 1.0 | 1.0 | 2.7 | 1.0 | 1.0 | 1.5 | 0.7 |
| 131 | 9910 | 0.4 | 1.0 | 0.8 | 3.2 | 1.9 | 1.3 | 1.4 |
| 132 | 2427 | 1.3 | 0.7 | 3.0 | 2.8 | 0.8 | 1.9 | 1.0 |
| 133 | 19990 | 1.0 | 7.9 | 2.8 | 34.7 | 1.0 | 1.0 | 1.0 |
| 134 | 20605 | 3.0 | 1.2 | 2.1 | 1.0 | 1.3 | 1.2 | 0.8 |
| 135 | 10650 | 0.5 | 1.7 | 0.5 | 2.9 | 2.8 | 0.6 | 3.4 |
| 136 | 25963 | 2.6 | 3.5 | 0.7 | 1.0 | 3.3 | 1.0 | 2.3 |
| 137 | 25562 | 3.2 | 5.9 | 0.7 | 1.0 | 4.2 | 1.0 | 4.8 |
| 138 | 9377 | 0.6 | 1.0 | 1.0 | 2.1 | 1.9 | 2.0 | 1.6 |
| 139 | 17618 | 1.0 | 0.7 | 2.3 | 3.2 | 0.8 | 0.7 | 0.8 |
| 140 | 12136 | 1.0 | 1.0 | 3.8 | 1.0 | 1.0 | 1.0 | 1.0 |
| 141 | 17373 | 1.0 | 0.4 | 6.1 | 2.4 | 1.0 | 1.0 | 1.0 |
| 142 | 18577 | 1.0 | 0.3 | 0.3 | 4.6 | 1.0 | 1.0 | 1.0 |
| 143 | 3143 | 1.7 | 1.3 | 2.6 | 2.3 | 0.7 | 1.0 | 0.5 |
| 144 | 17737 | 6.1 | 0.7 | 3.4 | 0.3 | 0.5 | 1.3 | 0.4 |
| 145 | 20029 | 1.0 | 0.6 | 2.3 | 1.0 | 1.0 | 1.0 | 0.5 |
| 146 | 18537 | 1.0 | 1.3 | 2.1 | 2.6 | 1.3 | 1.0 | 1.2 |
| 147 | 10090 | 1.0 | 1.7 | 2.1 | 2.8 | 1.5 | 1.0 | 1.2 |
| 148 | 12102 | 1.0 | 1.0 | 3.9 | 1.0 | 1.0 | 1.0 | 1.0 |
| 149 | 8487 | 4.7 | 2.4 | 1.0 | 1.0 | 2.3 | 1.1 | 2.2 |
| 150 | 9252 | 1.3 | 3.8 | 0.3 | 1.0 | 2.1 | 1.6 | 2.5 |
| 151 | 25605 | 1.0 | 1.0 | 1.0 | 1.0 | 0.5 | 0.5 | 1.0 |
| 152 | 29652 | 1.0 | 2.9 | 1.5 | 2.9 | 2.0 | 1.5 | 2.1 |
| 153 | 10858 | 1.0 | 0.8 | 2.0 | 1.0 | 1.0 | 1.0 | 0.7 |
| 154 | 1261 | 0.2 | 0.6 | 1.0 | 2.9 | 0.8 | 0.8 | 0.9 |
| 155 | 4156 | 12.4 | 0.8 | 3.1 | 0.2 | 0.6 | 1.0 | 0.3 |
| 156 | 3452 | 10.6 | 0.8 | 2.8 | 0.3 | 0.6 | 1.0 | 0.4 |
| 157 | 2748 | 10.8 | 0.8 | 3.1 | 0.2 | 0.5 | 1.0 | 0.4 |
| 158 | 2046 | 9.2 | 1.0 | 2.4 | 0.3 | 0.5 | 1.2 | 0.4 |
| 159 | 2044 | 11.7 | 0.8 | 2.8 | 0.2 | 0.6 | 1.4 | 0.4 |
| 160 | 1342 | 10.5 | 0.9 | 2.8 | 0.2 | 0.5 | 1.2 | 0.4 |
| 161 | 1326 | 12.2 | 1.0 | 2.7 | 0.2 | 0.5 | 1.0 | 0.4 |
| 162 | 9981 | 0.2 | 1.5 | 0.3 | 2.5 | 1.2 | 1.6 | 0.5 |
| 163 | 27917 | 1.9 | 2.5 | 0.5 | 1.0 | 2.1 | 1.4 | 2.3 |
| 164 | 8488 | 4.3 | 2.4 | 1.0 | 0.5 | 2.9 | 0.9 | 3.6 |
| 165 | 22793 | 1.9 | 2.6 | 0.5 | 1.0 | 2.2 | 1.8 | 2.1 |
| 166 | 26883 | 2.4 | 3.7 | 0.5 | 1.0 | 2.5 | 2.0 | 2.0 |
| 167 | 11540 | 0.7 | 1.0 | 1.3 | 2.8 | 0.8 | 1.0 | 0.5 |
| 168 | 17707 | 1.0 | 0.6 | 2.6 | 1.0 | 1.0 | 1.0 | 1.0 |
| 169 | 20649 | 2.3 | 2.6 | 0.5 | 0.4 | 3.0 | 1.0 | 3.1 |
| 170 | 24004 | 1.0 | 2.5 | 1.8 | 3.6 | 2.3 | 1.0 | 2.8 |
| 171 | 11836 | 1.2 | 5.0 | 0.9 | 3.7 | 1.3 | 1.0 | 0.8 |
| 172 | 24932 | 1.8 | 0.8 | 6.5 | 2.1 | 0.8 | 1.0 | 0.5 |
| 173 | 19143 | 0.6 | 1.6 | 0.7 | 2.0 | 1.7 | 1.2 | 1.4 |
| 174 | 26257 | 1.9 | 1.3 | 2.2 | 1.7 | 0.7 | 1.0 | 0.6 |
| 175 | 21239 | 9.4 | 9.2 | 0.5 | 0.4 | 2.4 | 1.0 | 2.7 |
| 176 | 16959 | 0.6 | 2.1 | 0.8 | 2.1 | 3.0 | 1.4 | 2.5 |
| 177 | 2568 | 0.7 | 1.9 | 0.7 | 2.2 | 3.0 | 1.3 | 2.4 |
| 178 | 25936 | 1.0 | 2.4 | 0.7 | 2.0 | 3.1 | 1.5 | 2.4 |
| 179 | 23041 | 0.7 | 1.0 | 2.1 | 2.6 | 1.0 | 1.4 | 1.0 |
| 180 | 9206 | 5.7 | 1.8 | 4.6 | 1.0 | 1.0 | 0.7 | 0.9 |
| 181 | 25105 | 1.6 | 1.3 | 2.1 | 1.0 | 1.0 | 0.7 | 0.8 |
| 182 | 24779 | 1.0 | 1.0 | 1.0 | 2.9 | 2.3 | 1.4 | 1.2 |
| 183 | 22451 | 1.0 | 0.2 | 2.1 | 1.0 | 1.4 | 0.6 | 1.0 |
| 184 | 22291 | 0.2 | 0.2 | 2.1 | 1.0 | 0.6 | 0.6 | 0.5 |
| 185 | 21143 | 1.0 | 7.2 | 0.7 | 2.0 | 2.6 | 1.1 | 2.4 |
| 186 | 24751 | 1.7 | 5.0 | 0.7 | 2.1 | 2.4 | 1.3 | 4.0 |
| 187 | 24294 | 1.7 | 3.9 | 0.8 | 2.4 | 2.6 | 1.1 | 3.9 |
| 188 | 24006 | 1.7 | 6.3 | 0.8 | 2.5 | 2.4 | 1.0 | 4.0 |
| 189 | 25678 | 1.0 | 1.0 | 1.0 | 1.0 | 1.0 | 1.0 | 1.0 |
| 190 | 22027 | 8.7 | 7.0 | 0.4 | 0.2 | 5.1 | 2.0 | 5.2 |
| 191 | 29495 | 1.0 | 1.0 | 1.0 | 1.0 | 1.0 | 1.0 | 1.0 |
| 192 | 24577 | 6.8 | 3.2 | 0.8 | 0.4 | 3.8 | 1.3 | 2.1 |
| 193 | 23527 | 0.3 | 2.1 | 1.6 | 6.4 | 2.7 | 2.1 | 3.4 |
| 194 | 17090 | 1.0 | 4.9 | 0.7 | 2.3 | 3.1 | 2.3 | 3.6 |
| 195 | 25137 | 1.0 | 1.0 | 0.4 | 3.8 | 1.0 | 2.5 | 4.1 |
| 196 | 23772 | 0.6 | 6.8 | 0.5 | 3.7 | 12.6 | 3.6 | 9.2 |
| 197 | 1659 | 1.0 | 7.5 | 0.3 | 3.2 | 17.8 | 4.1 | 20.3 |
| 198 | 8497 | 1.3 | 0.4 | 2.2 | 0.5 | 1.0 | 1.0 | 1.0 |
| 199 | 25272 | 8.0 | 6.0 | 1.0 | 0.6 | 2.2 | 1.0 | 2.9 |
| 200 | 21216 | 1.0 | 1.0 | 0.6 | 2.0 | 2.5 | 2.0 | 2.2 |
| 201 | 11939 | 1.0 | 1.0 | 1.0 | 1.0 | 1.0 | 1.0 | 1.0 |
| 202 | 9191 | 1.8 | 2.2 | 1.3 | 1.1 | 2.2 | 1.0 | 2.0 |
| 203 | 3429 | 0.7 | 3.4 | 0.8 | 3.5 | 3.0 | 1.5 | 3.7 |
| 204 | 2725 | 0.8 | 3.4 | 1.0 | 3.4 | 2.6 | 1.6 | 4.1 |
| 205 | 19923 | 1.0 | 1.1 | 2.9 | 1.0 | 1.7 | 1.4 | 1.2 |
| 206 | 20457 | 1.0 | 2.0 | 1.0 | 2.3 | 2.9 | 1.0 | 2.3 |
| 207 | 24773 | 0.2 | 1.0 | 0.8 | 2.0 | 1.6 | 1.0 | 1.0 |
| 208 | 24119 | 0.2 | 4.6 | 1.1 | 15.9 | 2.7 | 1.0 | 3.4 |
| 209 | 3908 | 0.3 | 0.5 | 1.1 | 2.3 | 1.7 | 1.0 | 1.0 |
| 210 | 8560 | 1.9 | 0.7 | 2.2 | 0.5 | 1.0 | 1.0 | 0.7 |
| 211 | 24588 | 0.3 | 0.5 | 1.0 | 2.0 | 1.0 | 1.0 | 1.4 |
| 212 | 4047 | 0.5 | 1.2 | 1.0 | 2.1 | 1.9 | 1.0 | 1.8 |
| 213 | 28344 | 0.8 | 1.0 | 1.0 | 2.0 | 1.7 | 1.5 | 2.7 |
| 214 | 27561 | 1.0 | 1.0 | 1.0 | 2.4 | 1.2 | 1.2 | 1.3 |
| 215 | 3272 | 0.6 | 0.8 | 1.0 | 2.1 | 1.3 | 1.6 | 1.0 |
| 216 | 26735 | 1.0 | 1.0 | 1.0 | 1.0 | 1.0 | 1.0 | 1.0 |
| 217 | 24900 | 0.3 | 0.8 | 2.9 | 5.7 | 2.2 | 1.1 | 3.0 |
| 218 | 9472 | 2.2 | 5.0 | 1.0 | 2.0 | 1.5 | 0.8 | 1.7 |
| 219 | 9979 | 1.3 | 3.3 | 1.5 | 3.9 | 3.4 | 1.4 | 2.5 |
| 220 | 21996 | 1.0 | 4.7 | 1.0 | 3.4 | 2.5 | 1.0 | 2.4 |
| 221 | 22312 | 1.2 | 4.4 | 1.2 | 3.3 | 2.2 | 1.1 | 2.2 |
| 222 | 11327 | 1.4 | 6.2 | 1.4 | 2.7 | 2.7 | 1.0 | 2.2 |
| 223 | 18240 | 2.0 | 4.5 | 1.0 | 2.2 | 2.1 | 1.0 | 2.7 |
| 224 | 21922 | 0.7 | 1.4 | 0.8 | 2.1 | 1.8 | 1.0 | 1.3 |
| 225 | 22290 | 0.7 | 1.6 | 0.9 | 2.1 | 1.5 | 1.2 | 1.3 |
| 226 | 10390 | 1.3 | 1.0 | 2.6 | 1.6 | 0.8 | 1.0 | 0.6 |
| 227 | 2212 | 1.9 | 1.0 | 2.8 | 1.0 | 0.6 | 1.6 | 0.8 |
| 228 | 20213 | 0.4 | 1.0 | 1.0 | 1.0 | 1.0 | 1.0 | 1.0 |
| 229 | 24955 | 0.9 | 2.9 | 1.0 | 3.3 | 0.8 | 0.8 | 1.0 |
| 230 | 19574 | 1.0 | 0.6 | 3.7 | 3.1 | 1.5 | 2.6 | 1.4 |
| 231 | 19969 | 1.0 | 1.0 | 1.0 | 3.1 | 1.0 | 1.0 | 1.0 |
| 232 | 8570 | 0.4 | 1.2 | 1.0 | 2.6 | 1.2 | 0.8 | 0.6 |
| 233 | 18519 | 3.5 | 2.9 | 2.6 | 1.8 | 1.8 | 1.0 | 2.0 |
| 234 | 9616 | 0.6 | 2.0 | 1.0 | 2.3 | 1.2 | 1.2 | 1.0 |
| 235 | 22334 | 0.2 | 0.7 | 2.9 | 8.5 | 1.7 | 1.1 | 3.4 |
| 236 | 17459 | 0.1 | 0.7 | 2.7 | 18.8 | 4.0 | 1.3 | 4.6 |
| 237 | 25193 | 1.0 | 0.8 | 1.0 | 2.3 | 1.0 | 1.3 | 1.0 |
| 238 | 25191 | 0.2 | 0.8 | 0.7 | 2.5 | 1.3 | 1.5 | 1.2 |
| 239 | 9448 | 0.6 | 1.0 | 1.0 | 2.3 | 0.8 | 0.8 | 0.5 |
| 240 | 25224 | 5.6 | 14.4 | 1.0 | 2.3 | 6.0 | 1.5 | 9.6 |
| 241 | 20218 | 6.1 | 12.3 | 0.7 | 1.7 | 5.6 | 1.6 | 9.0 |
| 242 | 3089 | 7.0 | 15.7 | 0.7 | 2.3 | 7.3 | 1.8 | 8.0 |
| 243 | 23985 | 5.8 | 17.2 | 0.9 | 2.1 | 6.8 | 1.8 | 8.1 |
| 244 | 19953 | 6.2 | 13.5 | 0.8 | 1.8 | 6.4 | 1.7 | 10.4 |
| 245 | 11506 | 4.1 | 13.3 | 1.0 | 1.4 | 4.4 | 1.6 | 7.2 |
| 246 | 22362 | 1.0 | 0.7 | 4.1 | 2.1 | 1.2 | 1.8 | 1.0 |
| 247 | 25516 | 0.7 | 10.1 | 0.4 | 4.0 | 14.7 | 4.7 | 8.1 |
| 248 | 25757 | 0.6 | 0.4 | 2.4 | 1.0 | 1.0 | 1.3 | 0.9 |
| 249 | 24814 | 0.5 | 2.8 | 0.3 | 1.0 | 3.5 | 1.4 | 4.4 |
| 250 | 21994 | 0.5 | 3.2 | 0.3 | 1.0 | 3.6 | 1.0 | 4.3 |
| 251 | 27117 | 1.0 | 2.8 | 0.3 | 1.0 | 3.9 | 1.0 | 4.9 |
| 252 | 24681 | 1.8 | 2.6 | 0.6 | 0.5 | 3.2 | 1.5 | 3.0 |
| 253 | 22745 | 0.3 | 2.4 | 1.4 | 8.1 | 2.8 | 2.3 | 3.5 |
| 254 | 24233 | 1.9 | 3.9 | 1.3 | 2.3 | 1.3 | 0.8 | 2.2 |
| 255 | 2001 | 1.0 | 1.0 | 1.5 | 2.1 | 1.0 | 1.0 | 1.0 |
| 256 | 21179 | 2.0 | 7.9 | 0.7 | 1.9 | 2.1 | 1.0 | 4.3 |
| 257 | 17147 | 1.3 | 4.3 | 0.7 | 1.7 | 2.4 | 1.2 | 3.9 |
| 258 | 8700 | 1.5 | 7.3 | 0.7 | 1.6 | 3.1 | 1.0 | 2.7 |
| 259 | 21214 | 0.3 | 5.4 | 1.2 | 15.5 | 3.1 | 1.0 | 3.6 |
| 260 | 26422 | 0.4 | 3.7 | 1.0 | 12.7 | 3.9 | 1.0 | 3.3 |
| 261 | 22837 | 0.7 | 1.0 | 2.1 | 2.4 | 1.2 | 1.5 | 0.9 |
| 262 | 21965 | 1.0 | 1.0 | 1.0 | 2.2 | 2.4 | 1.0 | 1.0 |
| 263 | 25541 | 4.5 | 2.7 | 2.7 | 0.8 | 1.0 | 1.3 | 0.8 |
| 264 | 18302 | 1.1 | 0.9 | 2.1 | 1.0 | 1.0 | 1.0 | 1.0 |
| 265 | 24049 | 1.0 | 2.6 | 1.5 | 2.5 | 2.3 | 1.4 | 2.4 |
| 266 | 26326 | 9.2 | 1.5 | 3.2 | 0.7 | 0.7 | 0.9 | 1.0 |
| 267 | 2254 | 1.6 | 3.3 | 1.0 | 2.8 | 2.0 | 1.1 | 3.1 |
| 268 | 10296 | 0.9 | 1.7 | 2.9 | 5.0 | 2.1 | 1.0 | 1.3 |
| 269 | 20044 | 1.0 | 0.8 | 2.0 | 1.0 | 1.0 | 1.0 | 1.0 |
| 270 | 28806 | 2.8 | 1.1 | 2.1 | 1.0 | 0.9 | 1.2 | 0.8 |
| 271 | 17566 | 7.5 | 4.2 | 0.7 | 0.5 | 2.5 | 1.0 | 2.5 |
| 272 | 19005 | 1.0 | 0.8 | 1.0 | 2.1 | 1.0 | 1.0 | 1.0 |
| 273 | 3567 | 1.0 | 1.0 | 1.0 | 1.0 | 1.0 | 1.0 | 1.0 |
| 274 | 21983 | 0.1 | 1.0 | 3.1 | 25.6 | 3.4 | 1.0 | 4.7 |
| 275 | 458 | 1.0 | 2.1 | 0.6 | 1.0 | 1.6 | 2.1 | 2.3 |
| 276 | 22331 | 0.6 | 2.1 | 0.4 | 1.0 | 2.2 | 1.0 | 2.8 |
| 277 | 21411 | 0.7 | 1.5 | 1.0 | 2.5 | 1.0 | 1.0 | 1.0 |
| 278 | 22972 | 1.0 | 2.2 | 0.5 | 1.0 | 2.2 | 1.4 | 2.4 |
| 279 | 24533 | 1.0 | 2.5 | 1.0 | 2.0 | 2.0 | 2.7 | 3.2 |
| 280 | 24853 | 1.0 | 2.6 | 2.1 | 2.1 | 2.4 | 1.3 | 2.1 |
| 281 | 23753 | 0.7 | 1.5 | 1.3 | 2.1 | 2.0 | 1.7 | 2.3 |
| 282 | 21502 | 0.3 | 4.8 | 1.0 | 10.8 | 2.6 | 1.0 | 2.9 |
| 283 | 18180 | 0.3 | 0.8 | 0.8 | 2.4 | 0.9 | 1.4 | 0.7 |
| 284 | 23918 | 0.7 | 2.3 | 0.4 | 1.0 | 2.4 | 1.2 | 3.5 |
| 285 | 24144 | 1.0 | 1.0 | 1.0 | 1.0 | 1.0 | 1.6 | 1.0 |
| 286 | 19996 | 1.5 | 2.5 | 0.7 | 1.2 | 2.1 | 0.9 | 2.5 |
| 287 | 11528 | 1.0 | 1.0 | 1.0 | 2.1 | 1.0 | 1.0 | 1.0 |
| 288 | 20506 | 2.2 | 0.9 | 3.2 | 0.8 | 1.3 | 1.6 | 1.0 |
| 289 | 23833 | 1.0 | 0.5 | 2.1 | 1.0 | 1.0 | 1.0 | 0.7 |
| 290 | 20042 | 3.8 | 1.6 | 2.3 | 0.8 | 1.0 | 1.0 | 1.0 |
| 291 | 24977 | 1.0 | 1.0 | 2.1 | 1.0 | 2.3 | 1.4 | 1.4 |
| 292 | 11646 | 1.0 | 1.0 | 0.8 | 1000.0 | 1.0 | 1.0 | 1.7 |
| 293 | 24872 | 1.0 | 1.4 | 0.8 | 2.5 | 1.4 | 1.2 | 1.3 |
| 294 | 10577 | 1.0 | 1.0 | 1.0 | 1.0 | 1.0 | 1.0 | 1.0 |
| 295 | 21710 | 1.0 | 0.2 | 2.2 | 0.7 | 1.6 | 1.0 | 1.2 |
| 296 | 18556 | 0.0 | 1.0 | 1.0 | 1.0 | 1.0 | 1.0 | 1.0 |
| 297 | 29433 | 1.0 | 0.5 | 1.0 | 2.1 | 1.0 | 1.0 | 1.0 |
| 298 | 29273 | 1.0 | 2.2 | 1.0 | 2.2 | 1.0 | 1.3 | 1.0 |
| 299 | 28763 | 1.6 | 2.7 | 1.0 | 2.2 | 1.8 | 1.3 | 2.5 |
| 300 | 27887 | 0.1 | 0.2 | 1.1 | 2.7 | 0.8 | 1.0 | 0.6 |
| 301 | 27450 | 2.6 | 11.3 | 0.2 | 1.0 | 4.4 | 3.3 | 7.3 |
| 302 | 27255 | 0.6 | 1.6 | 0.8 | 2.3 | 1.7 | 1.4 | 1.5 |
| 303 | 27226 | 1.0 | 1.3 | 1.0 | 2.6 | 1.8 | 1.0 | 1.0 |
| 304 | 26550 | 4.2 | 17.9 | 0.2 | 1.0 | 6.9 | 2.9 | 9.2 |
| 305 | 26508 | 1.0 | 1.4 | 1.0 | 1.0 | 1.0 | 1.0 | 1.0 |
| 306 | 26483 | 1.2 | 2.2 | 0.6 | 1.0 | 2.1 | 1.4 | 2.7 |
| 307 | 26334 | 1.0 | 0.5 | 3.0 | 1.0 | 0.6 | 0.8 | 0.5 |
| 308 | 26027 | 1.0 | 1.0 | 1.0 | 1.5 | 1.0 | 1.0 | 1.0 |
| 309 | 25977 | 1.0 | 1.0 | 1.0 | 1.0 | 1.0 | 1.0 | 1.0 |
| 310 | 25965 | 1.0 | 1.0 | 1.0 | 1.0 | 1.0 | 1.0 | 1.0 |
| 311 | 25844 | 1.0 | 1.0 | 1.0 | 1.0 | 1.0 | 1.0 | 1.0 |
| 312 | 25834 | 1000.0 | 1.0 | 1.0 | 1.0 | 0.4 | 1.0 | 1.0 |
| 313 | 25816 | 1.0 | 1.0 | 1.0 | 1.0 | 1.0 | 1.0 | 1.0 |
| 314 | 25746 | 1.0 | 1.0 | 1.0 | 1.0 | 1.0 | 1.0 | 1.0 |
| 315 | 25742 | 1.0 | 1.0 | 1.0 | 1.0 | 0.5 | 1.0 | 1.0 |
| 316 | 25741 | 1.0 | 1.0 | 1.0 | 1.0 | 1.0 | 1.0 | 1.0 |
| 317 | 25712 | 1.0 | 1.0 | 1.0 | 1.0 | 1.0 | 1.0 | 1.0 |
| 318 | 25642 | 1.0 | 1.0 | 1.0 | 1.0 | 1.0 | 1.0 | 1.0 |
| 319 | 25621 | 0.6 | 0.8 | 2.1 | 2.0 | 1.3 | 1.2 | 1.0 |
| 320 | 25614 | 1.0 | 1.0 | 1.0 | 1.0 | 1.0 | 1.0 | 1.0 |
| 321 | 25603 | 1.0 | 1.0 | 1.0 | 1.0 | 1.0 | 1.0 | 1.0 |
| 322 | 25556 | 1.8 | 0.7 | 1.0 | 0.8 | 1.0 | 1.0 | 1.0 |
| 323 | 25555 | 1.0 | 2.9 | 1.0 | 1.0 | 1.5 | 1.3 | 1.0 |
| 324 | 25540 | 1.0 | 1.0 | 1.0 | 1.2 | 1.0 | 1.0 | 1.0 |
| 325 | 23576 | 0.0 | 1.0 | 1.0 | 1.0 | 1.0 | 1.5 | 1.4 |
| 326 | 22566 | 1.0 | 1.0 | 1.0 | 1.0 | 1.0 | 1.3 | 1.0 |
| 327 | 9036 | 1.9 | 3.8 | 0.6 | 1.8 | 2.6 | 1.3 | 3.1 |
| 328 | 4164 | 1.0 | 1.0 | 2.2 | 1.0 | 1.5 | 1.0 | 0.9 |
| 329 | 4146 | 0.8 | 3.7 | 0.9 | 4.4 | 3.3 | 1.0 | 4.3 |
| 330 | 4091 | 1.0 | 1.0 | 1.0 | 2.5 | 1.7 | 1.0 | 0.9 |
| 331 | 4072 | 1.0 | 1.0 | 2.1 | 1.0 | 5.9 | 2.0 | 5.1 |
| 332 | 4022 | 3.5 | 4.5 | 0.8 | 1.0 | 3.0 | 1.0 | 3.2 |
| 333 | 3965 | 1.9 | 5.6 | 0.4 | 1.0 | 5.5 | 2.3 | 4.1 |
| 334 | 3954 | 1.0 | 2.7 | 1.3 | 3.6 | 2.8 | 1.9 | 2.6 |
| 335 | 3872 | 1.0 | 3.2 | 1.3 | 2.8 | 4.0 | 1.8 | 3.9 |
| 336 | 3869 | 1.0 | 1.0 | 5.8 | 3.8 | 1.0 | 0.7 | 0.6 |
| 337 | 3838 | 1.0 | 1.6 | 1.2 | 2.0 | 2.6 | 1.7 | 1.9 |
| 338 | 3806 | 0.6 | 2.6 | 0.9 | 3.7 | 3.0 | 1.0 | 3.4 |
| 339 | 3798 | 10.2 | 0.9 | 2.9 | 0.3 | 0.7 | 1.0 | 0.4 |
| 340 | 3792 | 1.0 | 1.0 | 1.0 | 2.7 | 2.9 | 1.0 | 2.5 |
| 341 | 3788 | 1.7 | 5.4 | 1.2 | 3.4 | 2.5 | 1.3 | 2.7 |
| 342 | 3767 | 1.1 | 2.2 | 0.7 | 1.7 | 2.5 | 1.0 | 2.5 |
| 343 | 3458 | 1.2 | 3.3 | 0.7 | 2.0 | 2.6 | 1.0 | 2.2 |
| 344 | 3251 | 0.4 | 0.5 | 1.4 | 2.7 | 1.4 | 1.0 | 1.0 |
| 345 | 3194 | 1.0 | 2.3 | 1.3 | 3.1 | 2.2 | 1.3 | 3.2 |
| 346 | 3102 | 0.5 | 3.2 | 1.0 | 4.8 | 2.9 | 1.0 | 2.3 |
| 347 | 3094 | 11.5 | 0.8 | 2.7 | 0.3 | 0.6 | 1.0 | 0.4 |
| 348 | 2671 | 0.8 | 1.6 | 1.0 | 2.2 | 2.8 | 1.9 | 1.0 |
| 349 | 2634 | 0.9 | 2.8 | 0.4 | 1.0 | 3.8 | 1.7 | 4.0 |
| 350 | 2567 | 4.6 | 3.3 | 0.8 | 0.6 | 2.6 | 1.0 | 3.3 |
| 351 | 2317 | 1.0 | 1.0 | 2.4 | 1.0 | 1.0 | 1.0 | 1.1 |
| 352 | 1958 | 0.3 | 0.6 | 1.0 | 2.6 | 0.9 | 0.8 | 0.9 |
| 353 | 1680 | 0.3 | 4.7 | 1.0 | 17.7 | 2.7 | 1.0 | 4.5 |
| 354 | 1625 | 2.2 | 7.8 | 0.5 | 1.8 | 3.1 | 1.7 | 3.4 |
| 355 | 1445 | 0.2 | 0.6 | 1.0 | 2.7 | 0.8 | 0.9 | 0.9 |
| 356 | 1320 | 4.9 | 1.0 | 2.4 | 0.4 | 0.6 | 1.2 | 0.5 |
| 357 | 974 | 0.6 | 3.1 | 1.1 | 3.2 | 2.4 | 1.4 | 3.7 |
| 358 | 726 | 1.0 | 1.0 | 1.0 | 1.0 | 1.0 | 1.0 | 1.0 |
| 359 | 718 | 0.4 | 2.6 | 0.5 | 2.7 | 1.6 | 1.0 | 1.0 |
| 360 | 703 | 1.0 | 4.1 | 1.0 | 2.4 | 1.6 | 1.0 | 1.7 |
| 361 | 652 | 2.8 | 4.4 | 1.6 | 2.3 | 1.0 | 1.6 | 1.0 |
| 362 | 630 | 6.9 | 1.0 | 2.2 | 0.3 | 0.6 | 1.0 | 0.5 |
| 363 | 593 | 1.0 | 4.3 | 1.0 | 2.3 | 1.0 | 1.0 | 1.0 |
| 364 | 532 | 1.3 | 4.7 | 1.0 | 2.4 | 2.6 | 2.2 | 4.0 |
| 365 | 272 | 0.7 | 2.7 | 0.9 | 3.1 | 2.4 | 1.3 | 4.3 |
| 366 | 256 | 0.6 | 3.2 | 0.5 | 1.9 | 2.8 | 1.0 | 3.4 |
| 367 | 57 | 0.5 | 1.4 | 1.0 | 2.3 | 0.9 | 1.0 | 0.7 |

**Table 11**

| **SEQ ID NO** | **SPOT ID** |
|---|---|
| 1 | 10594 |
| 2 | 21851 |
| 3 | 20990 |
| 4 | 18641 |
| 5 | 19037 |
| 6 | 398 |
| 7 | 18773 |
| 8 | 3583 |
| 9 | 3418 |
| 10 | 145306 |
| 11 | 3418 |
| 12 | 3418 |
| 13 | 18985 |
| 14 | 17229 |
| 15 | 25930 |
| 16 | 25930 |
| 17 | 20701 |
| 18 | 20346 |
| 19 | 20346 |
| 20 | 21247 |
| 21 | 21247 |
| 22 | 23062 |
| 23 | 25666 |
| 24 | 25666 |
| 25 | 19001 |
| 26 | 10897 |
| 27 | 10897 |
| 28 | 10897 |
| 29 | 1960 |
| 30 | 146262 |
| 31 | 26381 |
| 32 | 26381 |
| 33 | 26719 |
| 34 | 26719 |
| 35 | 27152 |
| 36 | 10926 |
| 37 | 28980 |
| 38 | 1236 |
| 39 | 29350 |
| 40 | 29350 |
| 41 | 26242 |
| 42 | 4098 |
| 43 | 145253 |
| 44 | 4098 |
| 45 | 17432 |
| 46 | 17432 |
| 47 | 1785 |
| 48 | 1785 |
| 49 | 1785 |
| 50 | 28856 |
| 51 | 28856 |
| 52 | 18791 |
| 53 | 18791 |
| 54 | 22950 |
| 55 | 22950 |
| 56 | 1882 |
| 57 | 23886 |
| 58 | 24995 |
| 59 | 24995 |
| 60 | 24477 |
| 61 | 21681 |
| 62 | 21681 |
| 63 | 9557 |
| 64 | 9557 |
| 65 | 22033 |
| 66 | 873 |
| 67 | 17144 |
| 68 | 26970 |
| 69 | 26970 |
| 70 | 21402 |
| 71 | 27074 |
| 72 | 27074 |
| 73 | 10963 |
| 74 | 10963 |
| 75 | 29525 |
| 76 | 29525 |
| 77 | 25514 |
| 78 | 25514 |
| 79 | 26612 |
| 80 | 26612 |
| 81 | 24600 |
| 82 | 9741 |
| 83 | 9741 |
| 84 | 9741 |
| 85 | 23689 |
| 86 | 23689 |
| 87 | 22352 |
| 88 | 23806 |
| 89 | 12285 |
| 90 | 27638 |
| 91 | 27638 |
| 92 | 9663 |
| 93 | 9663 |
| 94 | 26850 |
| 95 | 10204 |
| 96 | 10204 |
| 97 | 10204 |
| 98 | 25922 |
| 99 | 25922 |
| 100 | 26347 |
| 101 | 26347 |
| 102 | 20361 |
| 103 | 20361 |
| 104 | 28672 |
| 105 | 28672 |
| 106 | 25520 |
| 107 | 25520 |
| 108 | 1723 |
| 109 | 1723 |
| 110 | 28863 |
| 111 | 25526 |
| 112 | 25526 |
| 113 | 27936 |
| 114 | 27936 |
| 115 | 26851 |
| 116 | 25107 |
| 117 | 25107 |
| 118 | 25107 |
| 119 | 24912 |
| 120 | 24912 |
| 121 | 25169 |
| 122 | 25600 |
| 123 | 25600 |
| 124 | 28706 |
| 125 | 28706 |
| 126 | 26377 |
| 127 | 26377 |
| 128 | 19460 |
| 129 | 25243 |
| 130 | 20018 |
| 131 | 20018 |
| 132 | 918 |
| 133 | 25027 |
| 134 | 29089 |
| 135 | 29089 |
| 136 | 9141 |
| 137 | 9141 |
| 138 | 9141 |
| 139 | 12005 |
| 140 | 12148 |
| 141 | 12148 |
| 142 | 17394 |
| 143 | 27017 |
| 144 | 27017 |
| 145 | 25809 |
| 146 | 8719 |
| 147 | 8719 |
| 148 | 21030 |
| 149 | 21030 |
| 150 | 11436 |
| 151 | 11436 |
| 152 | 10374 |
| 153 | 10374 |
| 154 | 25861 |
| 155 | 25861 |
| 156 | 3317 |
| 157 | 3317 |
| 158 | 8743 |
| 159 | 26240 |
| 160 | 26240 |
| 161 | 28562 |
| 162 | 16877 |
| 163 | 25955 |
| 164 | 26308 |
| 165 | 26308 |
| 166 | 4140 |
| 167 | 3436 |
| 168 | 25612 |
| 169 | 25612 |
| 170 | 12257 |
| 171 | 12257 |
| 172 | 9111 |
| 173 | 9111 |
| 174 | 17620 |
| 175 | 26025 |
| 176 | 26025 |
| 177 | 19271 |
| 178 | 4151 |
| 179 | 4151 |
| 180 | 26569 |
| 181 | 26569 |
| 182 | 10344 |
| 183 | 10344 |
| 184 | 10344 |
| 185 | 832 |
| 186 | 832 |
| 187 | 12071 |
| 188 | 12071 |
| 189 | 12271 |
| 190 | 11433 |
| 191 | 20917 |
| 192 | 25810 |
| 193 | 12039 |
| 194 | 12039 |
| 195 | 25499 |
| 196 | 25499 |
| 197 | 25557 |
| 198 | 25557 |
| 199 | 9917 |
| 200 | 19505 |
| 201 | 17491 |
| 202 | 10683 |
| 203 | 10683 |
| 204 | 1936 |
| 205 | 828 |
| 206 | 9558 |
| 207 | 9558 |
| 208 | 20164 |
| 209 | 969 |
| 210 | 969 |
| 211 | 9910 |
| 212 | 2427 |
| 213 | 19990 |
| 214 | 20605 |
| 215 | 20605 |
| 216 | 10650 |
| 217 | 10650 |
| 218 | 25963 |
| 219 | 25963 |
| 220 | 25562 |
| 221 | 25562 |
| 222 | 3429 |
| 223 | 2725 |
| 224 | 19923 |
| 225 | 20457 |
| 226 | 20457 |
| 227 | 24773 |
| 228 | 24119 |
| 229 | 3908 |
| 230 | 3908 |
| 231 | 8560 |
| 232 | 8560 |
| 233 | 9377 |
| 234 | 9377 |
| 235 | 17618 |
| 236 | 12136 |
| 237 | 17373 |
| 238 | 18577 |
| 239 | 18577 |
| 240 | 3143 |
| 241 | 17737 |
| 242 | 17737 |
| 243 | 20029 |
| 244 | 20029 |
| 245 | 18537 |
| 246 | 18537 |
| 247 | 12102 |
| 248 | 12102 |
| 249 | 8487 |
| 250 | 9252 |
| 251 | 9252 |
| 252 | 25605 |
| 253 | 25605 |
| 254 | 29652 |
| 255 | 10858 |
| 256 | 1261 |
| 257 | 4156 |
| 258 | 4156 |
| 259 | 3452 |
| 260 | 3452 |
| 261 | 2748 |
| 262 | 2046 |
| 263 | 2046 |
| 264 | 2044 |
| 265 | 2044 |
| 266 | 1342 |
| 267 | 1342 |
| 268 | 1326 |
| 269 | 1326 |
| 270 | 9981 |
| 271 | 9981 |
| 272 | 27917 |
| 273 | 8488 |
| 274 | 22793 |
| 275 | 22793 |
| 276 | 26883 |
| 277 | 26883 |
| 278 | 11540 |
| 279 | 17707 |
| 280 | 20649 |
| 281 | 20649 |
| 282 | 24004 |
| 283 | 24004 |
| 284 | 11836 |
| 285 | 11836 |
| 286 | 11836 |
| 287 | 24932 |
| 288 | 19143 |
| 289 | 19143 |
| 290 | 26257 |
| 291 | 26257 |
| 292 | 21239 |
| 293 | 21239 |
| 294 | 16959 |
| 295 | 2568 |
| 296 | 25936 |
| 297 | 25936 |
| 298 | 23041 |
| 299 | 9206 |
| 300 | 25105 |
| 301 | 25105 |
| 302 | 24779 |
| 303 | 22451 |
| 304 | 22451 |
| 305 | 22291 |
| 306 | 22291 |
| 307 | 21143 |
| 308 | 24751 |
| 309 | 24751 |
| 310 | 24294 |
| 311 | 24294 |
| 312 | 24006 |
| 313 | 24006 |
| 314 | 25678 |
| 315 | 25678 |
| 316 | 22027 |
| 317 | 29495 |
| 318 | 29495 |
| 319 | 24577 |
| 320 | 24577 |
| 321 | 24577 |
| 322 | 23527 |
| 323 | 17090 |
| 324 | 25137 |
| 325 | 23772 |
| 326 | 1659 |
| 327 | 8497 |
| 328 | 25272 |
| 329 | 21216 |
| 330 | 21216 |
| 331 | 21216 |
| 332 | 11939 |
| 333 | 11939 |
| 334 | 11939 |
| 335 | 9191 |
| 336 | 3429 |
| 337 | 24588 |
| 338 | 4047 |
| 339 | 28344 |
| 340 | 28344 |
| 341 | 27561 |
| 342 | 3272 |
| 343 | 26735 |
| 344 | 26735 |
| 345 | 24900 |
| 346 | 24900 |
| 347 | 9472 |
| 348 | 9472 |
| 349 | 9979 |
| 350 | 21996 |
| 351 | 22312 |
| 352 | 11327 |
| 353 | 18240 |
| 354 | 18240 |
| 355 | 21922 |
| 356 | 21922 |
| 357 | 22290 |
| 358 | 10390 |
| 359 | 10390 |
| 360 | 2212 |
| 361 | 20213 |
| 362 | 20213 |
| 363 | 24955 |
| 364 | 19574 |
| 365 | 19969 |
| 366 | 8570 |
| 367 | 18519 |
| 368 | 9616 |
| 369 | 9616 |
| 370 | 17459 |
| 371 | 17459 |
| 372 | 25193 |
| 373 | 25193 |
| 374 | 25193 |
| 375 | 25191 |
| 376 | 22566 |
| 377 | 4164 |
| 378 | 4146 |
| 379 | 4072 |
| 380 | 4022 |
| 381 | 3954 |
| 382 | 3838 |
| 383 | 3806 |
| 384 | 3798 |
| 385 | 3792 |
| 386 | 3788 |
| 387 | 3458 |
| 388 | 3194 |
| 389 | 3102 |
| 390 | 25191 |
| 391 | 25191 |
| 392 | 9448 |
| 393 | 9448 |
| 394 | 25224 |
| 395 | 20218 |
| 396 | 3089 |
| 397 | 3089 |
| 398 | 19953 |
| 399 | 19953 |
| 400 | 22362 |
| 401 | 25516 |
| 402 | 25516 |
| 403 | 25757 |
| 404 | 24814 |
| 405 | 21994 |
| 406 | 27117 |
| 407 | 22745 |
| 408 | 24233 |
| 409 | 2001 |
| 410 | 2001 |
| 411 | 2001 |
| 412 | 17147 |
| 413 | 21214 |
| 414 | 21214 |
| 415 | 21214 |
| 416 | 26422 |
| 417 | 21965 |
| 418 | 25541 |
| 419 | 25541 |
| 420 | 18302 |
| 421 | 18302 |
| 422 | 24049 |
| 423 | 24049 |
| 424 | 26326 |
| 425 | 26326 |
| 426 | 2254 |
| 427 | 162502 |
| 428 | 10296 |
| 429 | 20044 |
| 430 | 28806 |
| 431 | 17566 |
| 432 | 17566 |
| 433 | 19005 |
| 434 | 3567 |
| 435 | 159223 |
| 436 | 3567 |
| 437 | 3567 |
| 438 | 458 |
| 439 | 21411 |
| 440 | 22972 |
| 441 | 24853 |
| 442 | 21502 |
| 443 | 18180 |
| 444 | 23918 |
| 445 | 24144 |
| 446 | 19996 |
| 447 | 11528 |
| 448 | 20506 |
| 449 | 20506 |
| 450 | 23833 |
| 451 | 20042 |
| 452 | 20042 |
| 453 | 11646 |
| 454 | 10577 |
| 455 | 10577 |
| 456 | 18556 |
| 457 | 29433 |
| 458 | 28763 |
| 459 | 27450 |
| 460 | 27450 |
| 461 | 27255 |
| 462 | 26550 |
| 463 | 26550 |
| 464 | 26508 |
| 465 | 26334 |
| 466 | 26334 |
| 467 | 26027 |
| 468 | 26027 |
| 469 | 25977 |
| 470 | 25977 |
| 471 | 25965 |
| 472 | 25965 |
| 473 | 25844 |
| 474 | 25844 |
| 475 | 25834 |
| 476 | 25816 |
| 477 | 25746 |
| 478 | 25712 |
| 479 | 25621 |
| 480 | 25621 |
| 481 | 25614 |
| 482 | 25614 |
| 483 | 25603 |
| 484 | 25603 |
| 485 | 25556 |
| 486 | 25556 |
| 487 | 25555 |
| 488 | 25555 |
| 489 | 3094 |
| 490 | 2567 |
| 491 | 1958 |
| 492 | 1680 |
| 493 | 1445 |
| 494 | 1320 |
| 495 | 974 |
| 496 | 652 |
| 497 | 630 |
| 498 | 593 |
| 499 | 256 |

The following represent further embodiments of the present invention:
1. A method for inhibiting a cancerous phenotype of a cell, said method comprising:
   contacting a cancerous mammalian cell with an agent for inhibition of DKFZP566I133 activity.
2. The method of embodiment 1, wherein said test cell is a breast cell.
3. The method of embodiments 1-2, wherein said cancerous phenotype is aberrant cellular proliferation relative to a normal cell.
4. The method of embodiments 1-3, wherein said cancerous phenotype is loss of contact inhibition of cell growth.
5. The method of embodiments 1-4, wherein said agent is selected from the group consisting of a small molecule, an antibody, an antisense polynucleotide, and an RNAi molecule.
6. The method of embodiments 1-6, wherein said inhibition is associated with a reduction in a level of DKFZP566I133 protein.
7. The method of embodiments 1-7, wherein said inhibition is associated with a reduction in a level of DKFZP566I133 RNA.
8. The method of embodiments 1-8, wherein said inhibition is associated with a reduction in a level of activity of DKFZP566I133 protein.
9. A method for detecting a cancerous cell, said method comprising:
   detecting a level of DKFZP566I133 or fragment thereof in a test sample obtained from a cell of a subject,
   comparing the level of DKFZP566I133 to a control level of DKFZP566I133,
   wherein the presence of a cancerous cell is indicated by detection of said level and comparison to a control level of DKFZP566I133.
10. The method of embodiment 9, wherein said cancerous cell is a cancerous breast cell.
11. The method of embodiments 9-10, wherein said gene product is nucleic acid.
12. The method of embodiments 9-11, wherein said gene product is a polypeptide.
13. The method of embodiments 9-12, wherein said detecting step uses a polymerase chain reaction.
14. The method of embodiments 9-13, wherein said detecting step uses hybridization.
15. The method of embodiments 9-14, wherein said sample is a sample of breast tissue.
16. The method of embodiments 9-15, wherein said level of said product is indicative of the cancerous state of the cell of the test sample.
17. A method of treating a subject with cancer, said method comprising: administering to a subject a pharmaceutically effective amount of an agent, wherein said agent modulates the activity of DKFZP566I133.
18. The method of embodiment 17, wherein said cancer is breast cancer.
19. The method of embodiments 17-18, wherein said agent is selected from the group consisting of a small molecule, an antibody, an antisense polynucleotide, and an RNAi molecule.
20. A method for assessing the tumor burden of a subject, said method comprising:
   detecting a level of DKFZP566I133 in a test sample from a subject, wherein the level of DKFZP566I133 in the test sample is indicative of the tumor burden in the subject.
21. A method for identifying an agent that modulates a biological activity of a gene product differentially expressed in a cancerous cell as compared to a normal cell, said method comprising:
   contacting a candidate agent with a DKFZP566I133; and
   detecting modulation of a biological activity of DKFZP566I133 relative to a level of biological activity of DKFZP566I133 in the absence of the candidate agent.
22. The method of embodiment 21, wherein said cancerous cell and said normal cell are breast cells.
23. The method of embodiments 21-22, wherein said detecting is by assessing expression of said gene product.
24. The method of embodiment 23, wherein expression is assessed by detecting a polynucleotide gene product.
25. The method of embodiments 23-24, wherein expression is assessed by detecting a polypeptide gene product.
26. The method of embodiments 21-25, wherein said candidate agent is selected from the group consisting of a small molecule, an antibody, an antisense polynucleotide, and an RNAi molecule.
27. The method of embodiments 21-26, wherein said biological activity is modulation of a cancerous phenotype.
28. The method of embodiment 27, wherein said cancerous phenotype is abnormal cellular proliferation.
29. The method of embodiment 27-28, wherein said cancerous phenotype is loss of contact inhibition.
30. An isolated polynucleotide comprising at least 15 contiguous nucleotides of a sequence selected from the group consisting of SEQ ID NO: 1-499.

## Claims

1. A method for inhibiting a cancerous phenotype of a cell, said method comprising: contacting a cancerous mammalian cell with an agent for inhibition of DKFZp566I133 activity.

2. The method of claim 1, wherein said test cell is a breast cell.

3. The method of claims 1-2, wherein said agent is selected from the group consisting of a small molecule, an antibody, an antisense polynucleotide, and an RNAi molecule.

4. The method of claims 1-3, wherein said inhibition is associated with a reduction in a level of DKFZp566I133 protein.

5. The method of claims 1-4, wherein said inhibition is associated with a reduction in a level of DKFZp566I133 RNA.

6. The method of claims 1-5, wherein said inhibition is associated with a reduction in a level of activity of DKFZp566I133 protein.

7. A method for detecting a cancerous cell, said method comprising:
detecting a level of DKFZp566I133 or fragment thereof in a test sample obtained from a
cell of a subject, comparing the level of DKFZp566I133 to a control level of DKFZp566I133, wherein the presence of a cancerous cell is indicated by detection of said level and comparison to a control level of DKFZp566I133.

8. The method of claim 7, wherein said cancerous cell is a cancerous breast cell.

9. A method of treating a subject with cancer, said method comprising: administering to a subject a pharmaceutically effective amount of an agent, wherein said agent modulates the activity of DKFZp566I133.

10. The method of claim 9, wherein said cancer is breast cancer.

11. A method for assessing the tumor burden of a subject, said method comprising:
detecting a level of DKFZp566I133 in a test sample from a subject, wherein the level of DKFZp566I133
in the test sample is indicative of the tumor burden in the subject

12. A method for assessing the tumor burden of a subject, said method comprising: detecting a level of DKFZp566I133 in a test sample from a subject, wherein the level of DKFZp566I133 in the test sample is indicative of the tumor burden in the subject.

13. A method for assessing the tumor burden of a subject, said method comprising:
detecting a level of DKFZp566I133 in a test sample from a subject, wherein the level of DKFZp566I133 in the test sample is indicative of the tumor burden in the subject.

14. A method for identifying an agent that modulates a biological activity of a gene product differentially expressed in a cancerous cell as compared to a normal cell, said method comprising:
contacting a candidate agent with a DKFZp566I133; and detecting modulation of a biological activity of DKFZp566I133 relative to a level of biological activity of DKFZP566I133 in the absence of the candidate agent.

15. An isolated polynucleotide comprising at least 15 contiguous nucleotides of a sequence selected from the group consisting of SEQ ID NO: 1-499.


SEQUENCE LISTING

<110> Hansen, Rhonda

<120> GENE PRODUCTS DIFFERENTIALLY EXPRESSED
  IN CANCEROUS BREAST CELLS AND THEIR METHODS OF USE

<130> 2300-17767WO

<150> 60/345,637
<151> 2002-01-08

<160> 516

<170> FastSEQ for Windows Version 4.0

<210> 1
<211> 114
<212> DNA
<213> Homo sapiens

<220>
<221> misc_feature
<222> 70
<223> n = A,T,C or G

<400> 1
catcctcgga cgccagcaag gtgacctcta agggggcagg gctctcaaag gcctttgtgg 60
gccagaaggn aaggttcctt cctggtggac tgcagcaaag ctggctccaa catg 114

<210> 2
<211> 430
<212> DNA
<213> Homo sapiens

<400> 2
gggactcgcc acctcctctt gcacccctgc caggcccagc agccaccaca gcgcctgctt 60
cctcggccct gaaatcatgc ccctaggtct cctgtggctg ggcctagccc tgttgggggc 120
tctgcatgcc caggcccagg actccacctc agacctgatc ccagccccac ctctgaacaa 180
ggtccctctg cagcagaact tgcaggacaa ccaattccag gggaagtggt atgtggtacg 240
cctggcaggg aatgcaattc tcagagaaga caaagacccg caaaagatgt atgccaccat 300
ctatgagctg aaagaagaca agagctacaa tgtcacctcc gtcctgttta ggaaaaagaa 360
gtgtgactac tggatcacga cttttgttcc aggttgccag cccggcgagt tcacgctggg 420
caacattaag 430

<210> 3
<211> 527
<212> DNA
<213> Homo sapiens

<400> 3
ctgctaatac agccctggct gtggaatcct tcaccgtctc agctggtatc agccccagcc 60
tgccttgtgc catatctcag cttggatctc tgctagagtc cccccaacca tatatcatag 120
agttgaatca caatgagacc gttggctttg aatttgagtc gttggttccc atggtgagat 180
gcttgttaag actttatact tgggtcaatc tctcacttta ttttgtagaa ccatttgaaa 240
tcctaggatg tgcttgttct ggaaggatga catgggccca gactgaacaa gtcagcttga 300
tgatcttaaa tgatggaagt ataggacgtt gcttatttta aaacaaggga aggacacaaa 360
atggaatgac tgcttagtcc tttctcagat actcttaaaa caatttttta ttgttaaatt 420
tgtggtaata catggtcaca accgtggatc aaacaaggtc agtctaaagt ggcaggtcct 480
aggtgtgacc tgataccacc accctttgtg gcagcaccgg gctggac 527

<210> 4
<211> 262
<212> DNA
<213> Homo sapiens

<220>
<221> misc_feature
<222> 186, 188
<223> n = A,T,C or G

<400> 4
ccggcctcgt ggaccagcct gggctctcgc tggaggaagt ggcttgcaag gaggcttggg 60
aggagtgtgg ctaccacttg gccccctctg atctgcgccg ggtcgccaca tactggtctg 120
gagtgggact gactggctcc agacagacca tgttctacac agaggtgaca gatgcccagc 180
gtacgntncc aggtgggggc ctggtggagg agggtgagct cattgaggtg gtgcacctgc 240
ccctggaagg cgcccaggcc tt 262

<210> 5
<211> 201
<212> DNA
<213> Homo sapiens

<400> 5
gccactgaaa atccttgtta aaaaccagat cacaaatctg gggctcttgg tcccattgga 60
gaaggaagga agagcctcaa aataagtgtg cacccatgca catattcagg aacagcttgt 120
ttagtcttta cactttgcct gaaagttgct tctcctcgtc cctttgtgtg cctgggtggc 180
ctcggccctg tgcgttggca a 201

<210> 6
<211> 621
<212> DNA
<213> Homo sapiens

<400> 6
tgagggtccc cgctcagctc ctggggctcc tgctactctg gctccgaggt gccagatgtg 60
acatccagat gacccagtct ccatcctccc tgtctgcatc tgttggagac agagtcacca 120
tcgcttgccg ggcaagtcag agcattggca tctatttaaa ttggtatcaa caaaaaccag 180
ggaaagcccc taaactcctg atctatgatt catccagatt gcaaagtggg gtcccatcaa 240
ggttcagtgg cagtggaggt gggacacact tcactctcac catcagcagt ctgcaacctg 300
aagatttagc aacttactac tgtcaacaag ggtacagtac acctggcacc ttcggccaag 360
ggacacgact ggaaattaaa cgaactgtgg ctgcaccatc tgtcttcatc ttcccgccat 420
ctgatgagca gttgaaatct ggaactgcct ctgttgtgtg cctgctgaat aacttctatc 480
ccagagaggc caaagtacag tggaaggtgg ataacgccct ccaatcgggt aactcccagg 540
aggggtgtca cagagcagga cagcaaggac agcacctaca gcctcagcag caccctgacg 600
ctgagcaaag cagactacga g 621

<210> 7
<211> 548
<212> DNA
<213> Homo sapiens

<400> 7
gacagcatgg acatgagggt ccccgctcag ctcctggggc tcctgctact ctggctccga 60
ggtgccagat gtgacatcca gatgacccag tctccatcct ccctgtctgc atctgttgga 120
gacagagtca ccatcgcttg ccgggcaagt cagagcattg gcatctattt aaattggtat 180
caacaaaaac cagggaaagc ccctaaactc ctgatctatg attcatccag attgcaaagt 240
ggggtcccat caaggttcag tggcagtgga ggtgggacac acttcactct caccatcagc 300
agtctgcaac ctgaagattt agcaacttac tactgtcaac aagggtacag tacacctggc 360
accttcggcc aagggacacg actggaaatt aaacgaactg tggctgcacc atctgtcttc 420
atcttcccgc catctgatga gcagttgaaa tctggaactg cctctgttgt gtgcctgctg 480
aataacttct atcccagaga ggccaaagta cagtggaagg tggataacgc cctccaatcg 540
ggtaactc 548

<210> 8
<211> 430
<212> DNA
<213> Homo sapiens

<400> 8
tatacacaac atttatttca aactattggg agggatgaga gtggcttaaa aacttccatc 60
cctacttttc aagagtgcag ttgattctga atctgaaagc ccgcctctgt cctaaaatac 120
aaacaagcac agacattaaa cctggatact atatgataaa gagggatgta actattgaat 180
tggatacaag gatcagaatg gaaagaaact cacgatgaaa ttgaacctgg tttttgtata 240
tttatcaaac ttgtgctgag aatagtgtct gattatacga cttttaagca aagttgggtg 300
taattaggtg aaaacagccc aggtcctccc gggagcacag aggggctagg ggctggtcct 360
tctcgtttgc tctagtcttg ctttgctgtc tggtgtagct cctctgctgc tcccatctgc 420
actaattgac 430

<210> 9
<211> 493
<212> DNA
<213> Homo sapiens

<400> 9
ctcactattt ggaatttggc cctcgaggcc aagaattcgg cacgaggcgg cacgaggtgt 60
aactattgaa ttggatacaa ggatcagaat ggaaagaaac tcacgatgaa attgaacctg 120
gtttttgtat atttatcaaa cttgtgctga gaatagtgtc tgattatacg acttttaagc 180
aaagttgggt gtaattaggt gaaaacagcc caggtcctcc cgggagcaca gaggggctag 240
gggctggtcc ttctcgtttg ctctagtctt gctttgctgt ctggtgtagc tcctctgctg 300
ctcccatctg cactaattga cccaaaacgt gggtatttcc tgctacacaa aagccaaaag 360
gtttcatgta gattttagtt cactaaaggg tgcccacaaa atagagatta attttaactt 420
aaattttaag cttgaagatt aggtactatc tgtgaagtta cacttttttt ttttttttaa 480
aaggaaaaaa tgt 493

<210> 10
<211> 472
<212> DNA
<213> Homo sapiens

<400> 10
cggcacgagg tgtaactatt gaattggata caaggatcag aatggaaaga aactcacgat 60
gaaattgaac ctggtttttg tatatttatc aaacttgtgc tgagaatagt gtctgattat 120
acgactttta agcaaagttg ggtgtaatta ggtgaaaaca gcccaggtcc tcccgggagc 180
acagaggggc taggggctgg tccttctcgt ttgctctagt cttgctttgc tgtctggtgt 240
agctcctctg ctgctcccat ctgcactaat tgacccaaaa cgtgggtatt tcctgctaca 300
caaaagccaa aaggtttcat gtagatttta gttcactaaa gggtgcccac aaaatagaga 360
ttaattttaa cttaaatttt aagcttgaag attaggtact atctgtgaag ttacactttt 420
ttattttttt ttaaaggtag agatgtgtgt gtgtgtaggt attaaagatg tg 472

<210> 11
<211> 271
<212> DNA
<213> Homo sapiens

<400> 11
gtttttcttt tttttataca caacatttat ttcaaactat tgggagggat gagagtggct 60
taaaaacttc catccctact tttcaagagt gcagttgatt ctggggggga aagcccgcct 120
ctgtcctaaa atacaaacaa gcacagacat taaacctgga tactatatga taaagaggga 180
tgtaactatt gaattggata caaggatcag aatggaaaga aactcacgat gaaattgaac 240
ctggtttttg tatatttatc aaacttgtgc t 271

<210> 12
<211> 343
<212> DNA

<213> Homo sapiens

<400> 12
gtttttcttt tttttataca caacatttat ttcaaactat tgggagggat gagagtggct 60
taaaaacttc catccctact tttcacgagt gcagctgatt ctgaatctga aagcccgcct 120
ctgtcctaaa atacaaacac gcacagacat tagacctgga tactatatga tacagaggga 180
tgtaactatt gaattggata cacggatcac aatggaaaga aactcacgat gaaattgaac 240
ctggcttttg tatatttatc aaacttgtgc tgagaatagc gcctgattat acgactttta 300
agcaaagctg ggtgtaatta ggtgaaaaca gcccacgtcc tcc 343

<210> 13
<211> 345
<212> DNA
<213> Homo sapiens

<400> 13
agtggcgagc aggttcccac ttgccaaaga tcccttttaa ccaacactag cccttgtttt 60
taacacacgc tccagccctt catcagcctg ggcagtctta ccaaaatgtt taaagtgatc 120
tcagaggggc ccatggatta acgccctcat cccaaggtcc gtcccatgac ataacactcc 180
acacccgccc cagccaactt catgggtcac tttttctgga aaataatgat ctgtacagac 240
aggacagaat gaaactcctg cgggtctttg gcctgaaagt tgggaatggt tgggggagag 300
aagggcagca gcttattggt ggtcttttca ccattggcag aaacg 345

<210> 14
<211> 401
<212> DNA
<213> Homo sapiens

<400> 14
ttttccaagt ccgtttcagt cccttccttg gtctgaagaa attctgcagt ggcgagcagt 60
ttcccacttg ccaaagatcc cttttaacca acactagccc ttgtttttaa cacacgctcc 120
agcccttcat cagcctgggc agtcttacca aaatgtttaa agtgatctca gaggggccca 180
tggattaacg ccctcatccc aaggtccgtc ccatgacata acactccaca cccgccccag 240
ccaacttcat gggtcacttt ttctggaaaa taatgatctg tacagacagg acagaatgaa 300
actcctgcgg ctctttggcc tgaaagttgg gaatggttgg gggagagaag ggcagcagct 360
tattggtggt cttttcacca ttggcagaaa cagtgagagc t 401

<210> 15
<211> 442
<212> DNA
<213> Homo sapiens

<400> 15
ggcagccggc ccacatgtct ctcaagtacc tgtcccctcg ctctggtgat tatttcttgc 60
agaatcacca cacgagacca tcccggcagt catggttttg ctttagtttt ccaagtccgt 120
ttcagtccct tccttggtct gaagaaattc tgcagtggcg agcagtttcc cacttgccaa 180
agatcccttt taaccaacac tagcccttgt ttttaacaca cgctccagcc cttcatcagc 240
ctgggcagtc ttaccaaaat gtttaaagtg atctcagagg ggcccatgga ttaacgccct 300
catcccaagg tccgtcccat gacataacac tccacacccg ccccagccaa cttcatgggt 360
cactttttct ggaaaataat gatctgtaca gacaggacag aatgaaactc ctgcggctct 420
ttggcctgaa agtgggaatg gt 442

<210> 16
<211> 256
<212> DNA
<213> Homo sapiens

<220>
<221> misc_feature
<222> 96
<223> n = A,T,C or G

<400> 16
gaatatgtag atttgcttct taatcctgag cgctacactg gttacaaggg accagatgct 60
tggaaaatat ggaatgtcat ctacgaagaa aactgnttta agccacagac cattaaaaga 120
ccttaaatcc tttggcttct ggtcaaggga caagtgaaga gaacactttt tacagttggc 180
tagaaggtct ctgtgtagaa aaaagagctt ctacagactt atatctggcc tacatgcaag 240
ccattaatgt gcattt 256

<210> 17
<211> 405
<212> DNA
<213> Homo sapiens

<400> 17
attctgtgat ttatttgaaa ctgtgaaacc atgtgccata atagaatttt gagaattttg 60
cttttaccta aattcaagaa aatgaaatta cacttttaag ttagtggtgc ttaagcataa 120
tttttcctat attaaccagt attaaaatct caagtaagat tttccagtgc cagaacatgt 180
taggtggaat tttaaaagtg cctcggcatc ctgtattaca tgtcatagaa ttgtaaagtc 240
aacatcaatt actagtaatc attctgcact cactgggtgc atagcatggt tagaggggct 300
agagatggac agtcatcaac tggcggatat agcggtacat atgatcctta gccaccaggg 360
cacaagctta ccagtagaca atacagacag agcttttgtt gagct 405

<210> 18
<211> 447
<212> DNA
<213> Homo sapiens

<400> 18
tgtgatttca tttgaaactg tgaaaccatg tgccataata gaattttgag aattttgctt 60
ttacctaaat tcaagaaaat gaaattacac ttttaagtta gtggtgctta agcataattt 120
ttcctatatt aaccagtatt aaaatctcaa gtaagatttt ccagtgccag aacatgttag 180
gtggaatttt aaaagtgcct cggcatcctg tattacatgt catagaattg taaagtcaac 240
atcaattact agtaatcatt ctgcactcac tgggtgcata gcatggttag aggggctaga 300
gatggacagt catcaactgg cggatatagc ggtacatatg atccttagcc accagggcac 360
aagcttacca gtagacaata cagacagagc ttttgttgag ctgtaactga gctatggaat 420
agcttctttg atgtacctct ttgcctt 447

<210> 19
<211> 294
<212> DNA
<213> Homo sapiens

<400> 19
tgtgatttca tttgaaactg tgaaaccatg tgccataata gaattttgag aattttgctt 60
ttacctaaat tcaagaaaat gaaattacac ttttaagtta gtggtgctta agcataattt 120
ttcctatatt aaccagtatt aaaatctcaa gtaagatttt ccagtgccag aacatgttag 180
gtggaatttt aaaagtgcct cggcatcctg tattacatgt catagaattg taaagtcaac 240
atcaattact agtaatcatt ctgcactcac tgggtgcata gcatggttag aggg 294

<210> 20
<211> 562
<212> DNA
<213> Homo sapiens

<400> 20
aggagcaggt tggactggcc atccgaagca agattgcaga tggcagtgtg aagagagaag 60
acatattcta cacttcaaag ctttggagca attcccatcg accagagttg gtccgaccag 120
ccttggaaag gtcactgaaa aatcttcaat tggactatgt tgacctctat cttattcatt 180
ttccagtgtc tgtaaagcca ggtgaggaag tgatcccaaa agatgaaaat ggaaaaatac 240
tatttgacac agtggatctc tgtgccacat gggaggccat ggagaagtgt aaagatgcag 300
gattggccaa gtccatcggg gtgtccaact tcaaccacag gctgctggag atgatcctca 360
acaagccagg gctcaagtac aagcctgtct gcaaccaggt ggaatgtcat ccttacttca 420
accagagaaa actgctggat ttctgcaagt caaaagacat tgttctggtt gcctatagtg 480

ctctgggatc ccatcgagaa gaaccatggg tggacccgaa ctccccggtg ctcttggagg 540
acccagtcct ttgtgccttg gc 562

<210> 21
<211> 721
<212> DNA
<213> Homo sapiens

<220>
<221> misc_feature
<222> 626, 685, 696
<223> n = A,T,C or G

<400> 21
ggcacgagat gaggagcagg ttggactggc catccgaagc aagattgcag atggcagtgt 60
gaagagagaa gacatattct acacttcaaa gctttggagc aattcccatc gaccagagtt 120
ggtcccgacc agccttggaa aggtcactga aaaatcttca attggactat gttgacctct 180
atcttattca ttttccagtg tctgtaaagc caggtgagga agtgatccca aaagatgaaa 240
atggaaaaat actatttgac acagtggatc tctgtgccac atgggaggcc atggagaagt 300
gtaaagatgc aggattggcc aagtccatcg gggtgtccaa cttcaaccac aggctgctgg 360
agatgatcct caacaagcca gggctcaagt acaagcctgt ctgcaaccag gtggaatgtc 420
atccttactt caaccagaga aaactgctgg atttctgcaa gtcaaaagac attgttctgg 480
ttgcctatag tgctctggga tcccatcgag aagaaccatg ggtggacccg aactccccgg 540
tgctcttgga ggacccagtc ctttgtgcct tggcaaaaaa gcacaagcga accccaccct 600
gattgccctg cgctaccagc ttgcancgtg gggttgtggt cctggccaag agcttcaatg 660
agcacgcatc agacagaacg tgcangtgtt tgaatncagt tgacttcaga aggagatgaa 720
a 721

<210> 22
<211> 496
<212> DNA
<213> Homo sapiens

<400> 22
agatgataac cagaagtctg catttgaagt tcacaaaagt aatcaagctc aaacagttag 60
tgagaggcag aagaacagac ctaaatcttg taaaaaagga aaaaatatta gggaagatga 120
tcctgtaaga atgttgcaaa ctgttgcaaa gaaattcgac ttcagtaatt tgagtagtag 180
gttagatgga gtcagatttg aaaatgaaaa aaattaatgt tattgccaag aacactggta 240
ataaactgaa gctaagtcag aaaaaatggt tgtttgctag atcccaatgg agaaaagtgt 300
gtaactgctc ctcgtcaggt ctctgctctt caccataaag acattgctct gtctttggtt 360
gctgcaagtg atggagctac agtctgtgtt accacaaggg gagatattta cttacttgca 420
gactatcagt gcaagaagat ggcttctaaa cagttgaact tgaaaaaagt tcttgtgtct 480
gggggtcata tggaat 496

<210> 23
<211> 549
<212> DNA
<213> Homo sapiens

<400> 23
ctgcatttga agttcacaaa agtaatcaag ctcaaacagt tagtgagagg cagaagagca 60
gacctaaatc ttgtaaaaaa ggaaaaaata ttagggaaga tgatcctgta agaatgttgc 120
aaactgttgc aaagaaattc gacttcagta atttgagtag taggttagat ggagtcagat 180
ttgaaaatga aaaaaattaa tgttattgcc aagaacactg gtaataaact gaagctaagt 240
cagaaaaaat ggttgtttgc tagatcccaa tggagaaaag tgtgtaactg ctcctcgtca 300
ggtctctgct cttcaccata aagacattgc tctgtctttg gttgctgcaa gtgatggagc 360
tacagtctgt gttaccacaa ggggagatat ttacttactt gcagactatc agtgcaagaa 420
gatggcttct aaacagttga acttgaaaaa agttcttgtg tctgggggtc atatggaata 480
caaggttgat cctgaacatt tgaaagaaaa tgggggtcaa aaaatttgca ttcttgcaat 540
ggatggagc 549

<210> 24

<211> 55
<212> DNA
<213> Homo sapiens

<400> 24
gtgtctgcct tcacaaatgt cattgtctac tcctagaaga accaaatacc tcaat 55

<210> 25
<211> 498
<212> DNA
<213> Homo sapiens

<400> 25
tccttattta tttaacttca cccgagttcc tctgggtttc taagcagtta tggtgatgac 60
ttagcgtcaa gacatttgct gaactcagca cattcgggac caatatatag tgggtacatc 120
aagttcatct gacaaaatgg ggcagaagag aaaggactca gtgtgtgatc cggtttcttt 180
ttgctcgccc ctgttttttg tagaatctct tcatgcttga catacctacc agtattattc 240
ccgacgacac atatacatat gagaatatac cttatttatt tttgtgtagg tgtctgcctt 300
cacaaatgtc attgtctact cctagaagaa ccaaatacct caatttttgt ttttgagtac 360
tgtactatcc tgtaaatata tcttaagcag gtttgttttc agcactgatg gaaaatacca 420
gtgttgggtt tttttttagt tgccacagtt gtatgtttgc tgattattta tgacccgaaa 480
aatatatttc ttctccta 498

<210> 26
<211> 325
<212> DNA
<213> Homo sapiens

<400> 26
gtcgctgcct ctgggggcgc tgtacaccgc ggccgtcgcg gctttagtgc tgtacaagtg 60
tgtggggggg ggagatgaaa ctgcggttct ccaccaggag gcaagcaagc agcagccact 120
gcagtcagag caacagctgg cccagttgac acaacagctg gcccagacag agcagcacct 180
gaacaacctg atggcccagc tggaccccct ttttgagccg tgtgactact ctggctggag 240
cccagcagga gcttctgaac atgaagctat ggaccatcca cgagctgctg caagatagca 300
agccggacaa ggatatggag gcttc 325

<210> 27
<211> 166
<212> DNA
<213> Homo sapiens

<400> 27
gaatccagca tcttaaagtt gcatatgtgt agcactaatg tttcttttta aatagttggg 60
ggaaaatgac ctagaaaacc aaattgcagt ttggtagcca aaattaactc ttggtttatt 120
tgtcctttgt gtgtgaaaag tcctactatt ccgtgcgtca gacttc 166

<210> 28
<211> 501
<212> DNA
<213> Homo sapiens

<400> 28
tttttttttt tttttttttt tttttcgcag ctgaattaca tttactgtac aaagaacggt 60
tcggagagaa ccaggaatgg cggagtgtct aacagcagcg cgggtagtgt tgatgccgtg 120
aatgcaggac catccaggtc ctcaaagtct gcgaggtttg ttcataatcc caaacaaggg 180
ccctgctggc agcaacagga caggtggggc caggacaggg aagctggagc aggaggccag 240
tgtctttggg ggctgtggca gggccgcctg cctggggttc ccttactcat ctggtagttc 300
atgcaggcca cggccctcat ctcccaggaa cgggccatgg ggcgagtcca ctggtgccca 360
gtaacaccct ccgtgggacc accttgggaa gcatgtgccg cggagtccac cacggggggt 420
cctgggtccc gggagggctc cttctgcgtg ctggccatgt cgtgccgcac ggcctgagga 480
caggaggtag aggtgagcac c 501

<210> 29
<211> 149
<212> DNA
<213> Homo sapiens

<400> 29
cgtcccggag gtgcggtgtg gggcaccggg cggggccgcg ggaaccggcg ccccacggag 60
ctgctgctgt cagaccaacc ccgggccccc atcatcactg cgccgcgctt tcaggcgccg 120
agaactaccg ttcccggcat gccatgaaa 149

<210> 30
<211> 475
<212> DNA
<213> Homo sapiens

<400> 30
agcagtaaac agggctgcta tgcctgctct gtagtggtgg acggcgaagt aaagcattgt 60
gtcataaaca aaacagcaac tggctatggc tttgccgagc cctataactt gtacagctct 120
ctgaaagaac tggtgctaca ttaccaacac acctcccttg tgcagcacaa cgactccctc 180
aatgtcacac tagcctaccc agtatatgca cagcagaggc gatgaagcgc ttactctttg 240
atccttctcc tgaagttcag ccaccctgag gcctctggaa agcaaagggc tcctctccag 300
tctgatctgt gaattgagct gcagaaacga agccatcttt ctttggatgg gactagagct 360
ttctttcaca aaaaagaagt aggggaagac atgcagccta aggctgtatg atgaccacac 420
gttcctaagc tggagtgctt atcccttctt tttctttttt tctttggttt aattt 475

<210> 31
<211> 570
<212> DNA
<213> Homo sapiens

<400> 31
cttttttttt tttttttttt tactggcatc ctgtacattt acttttaaaa aaggataaca 60
aaaatgaata ttaacaaaaa tccgggacaa caatattttc aagcaacaaa aactggggtg 120
gggaagctta ttctgaaggt acatttaaaa ctgaaataac aacttaatga aaattaagaa 180
ttgcatagcg ctgtgaattt agccttcagc aaaacaaaac agaagctatt tggtattgat 240
acaaatccat ctatttgata gttagtcatc caatattatg tacatatttt atatactgaa 300
tgtcatttta agtcctgttt tccaaactcc atttttctgt tgctgggttt ttgttttttg 360
acaagttaaa cactttctgg cactttctat gacagaattt cttctgaaca tacatgaact 420
gacattctcc caaagcgtcc cttgtgagtg gacgcgcctt tctgctacat atcgttcatt 480
tgttacaaaa tgaaataatc cacagtgcga tgtgtctggg tccaccgtgc acagcaacat 540
ccaggctaaa ccaggctgga ccaaaccttc 570

<210> 32
<211> 645
<212> DNA
<213> Homo sapiens

<400> 32
tccgagcgtc gggagcctgt ggaagagaag agcgcgcggg cgacagttaa acaggcccga 60
ggcagagaaa ccgccctagc agctctcgcg cgcccggtgc aggcggcggt tgctgcggag 120
gtccgtgcac agactgcttt gcctgttgtt gctcttcgga ggcggcgatc cccgaaggcg 180
agctgaaata cggctgcagg ctacaatttg cagccgacga ttaaggaaga cgacgagcgg 240
gagaggtggc ccaccctcat ggagcgcttg tgctcggatg gcttcgcatt tccccattac 300
tacattaaac cgtatcatct gaagaggatc cacagagctg tcttacgtgg taatctggag 360
aaactgaagt accttctgct cacgtattat gacgccaata agagagacag gaaggaaagg 420
actgccctac atttggcctg tgccactggc caaccggaaa tggtacatct cctggtgtcc 480
agaagatgtg agcttaacct ctgcgaccgt gaagacagga cacctctgat caaggctgta 540
caactgaggc aggaggcttg tgcaactctt ctgctgcaaa tggcgccgat ccaaatatta 600
cggatgtctt tggaaggact gctctgcact acgctgtgta taatg 645

<210> 33
<211> 572

<212> DNA
<213> Homo sapiens

<400> 33
ctaactgagt aacattcatg aaatgaggct ttctgtggcg gcgtagtgtt tggaattaga 60
aggtaattca gtagagtgta acttagagaa tattgcaagt gacacattga atcctgcccg 120
tcagggcacc ttttcctcag agcaatccgg ccacacgaat agaaggctgt cgtgaatcac 180
atcagatgta aaatcattcc ttctgtttac tcttttaatt ttcatccttt gcaggtagtg 240
caaattcaac ttcaaatatg gtgtaggttt tgctagattc catatttttt tcttggattt 300
ttgctaatta tttttagcaa aaaatttttg ctcagtggca ccctccctag tgtccatggg 360
ttagggccat gctggggaaa acgggccggt atttacacac gcgcaaaaca cccagagacg 420
gcacaaggag gttgaactca tgtttcagtt cgcgaacatt gactccttac gaaagtcact 480
tcattctaac tagatgcgcc cacttctggt cattatttcg tttgcatgat gtattgcttc 540
ttcacgtttt gtttttattg agcacggagt ag 572

<210> 34
<211> 701
<212> DNA
<213> Homo sapiens

<220>
<221> misc_feature
<222> 34, 41, 43, 52, 58, 72, 180, 204, 205, 211, 214, 228, 243,
253, 269, 271, 295, 315, 343, 429, 439, 457, 483, 517, 529,
546, 554, 555, 557, 560, 561, 565, 627, 632, 637, 644, 655,
659, 662, 672, 680, 689, 690, 698
<223> n = A,T,C or G

<400> 34
ggcacgaggc taactgtgta acatttatga aatntgctct ntntggcggc gnaggggncg 60
gaatgagaag gnaattcagt agagtgtaac ttagagaata ttgcaaggga cacattgaat 120
cctgcccgtc agggcacctt ttcctcagag caatccggcc acacgaatag aaggctgcgn 180
gaatcacatc agatgtaaaa tcannccttc nggngactct tttaattntc atcctttgca 240
ggnagggcaa atncaacttc aaatatggng naggttttgc tagattccat atttntttct 300
tggatttttg ctaantattt ttagcaaaaa atttttgctc agnggcaccc tccctagtgt 360
ccatgggtta gggccatgct ggggaaaacg ggccggtatt tacacacgcg caaaacaccc 420
agagacggna caaggaggnt gaactcatgt ttcagtncgc gaacattgac tccttacgaa 480
agncacttca ttctaactag atgcgcccac ttctggncat tattacgant gcatgaagga 540
ttgctncttc acgnntnggn nttantgagc acgggagtag aaattccagg gctggcttga 600
catcttccct gcatgctccc tcccagngga cngtccntcc cttncacatg agganctgnc 660
gnccatggtg gntttctccn ttgggcctnn tgggactngg a 701

<210> 35
<211> 300
<212> DNA
<213> Homo sapiens

<400> 35
gctaactgag taacattcat gaaatgaggc tttctgtggc ggcgtagtgt ttggaattag 60
aaggtaattc agtagagtgt aacttagaga atattgcaag tgacacattg aatcctgccc 120
gtcagggcac cttttcctca gagcaatccg gccacacgaa tagaaggctg tcgtgaatca 180
catcagatgt aaaatcattc cttctgttta ctcttttaat tttcatcctt tgcaggtagt 240
gcaaattcaa cttcaaatat ggtgtaggtt ttgctagatt ccatattttt ttcttggatt 300

<210> 36
<211> 374
<212> DNA
<213> Homo sapiens

<400> 36
tggtacgcct gcaggtaccg gtccggaatt cccgggtcga cccacgcgtc cggaggggtc 60

ctggagaatg ggttacccca gttgtcttat ttaaatggtt acccatcaga ttttaatttt 120
atcttctctt tgagagcttg gtaataagaa gcacttaaat cactccaaag aagactttaa 180
aaagggagca gtgaaaaggt cttaataatt tattgattga attaagaaat actagctaat 240
taagaatctg agtctaaaca gcacagattt tttctttctg cttttaaatt gtgttttaaa 300
aaaagagaca gggggctggg cgtggtggct cacgcctgta atcctagcac tttgggaggc 360
cgaggcgggt ggat 374

<210> 37
<211> 290
<212> DNA
<213> Homo sapiens

<400> 37
gaggggtcct ggagaaatgg gttaccccag ttgtcttatt taaatggtta cccatcagat 60
tttaatttta tcttctcttt gagagcttgg taataagaag cacttaaatc actccaaaga 120
agactttaaa aagggagcag tgaaaaggtc ttaataattt attgattgaa ttaagaaata 180
ctagctaatt aagaatctga gtctaaacag cacagatttt ttctttctgc ttttaaattg 240
tgttttaaaa aaagagacag ggggctgggc gtggtggctc acgcctgtaa 290

<210> 38
<211> 405
<212> DNA
<213> Homo sapiens

<400> 38
gccctttcga gcggccgccc gggcaggtac ctgggattac aggcacccac caccacgcct 60
ggctaatttt tttttgtatc tttagtaggg ttttgccatg ttggccaggc tggtctttaa 120
ctcctacctc gtgatccacc cgcctcggcc ccccaaagtg ctaggaccac aggcgtgagc 180
caccacgccc agccccctgt ctcttttttt aaaacacaat ttaaaagcag aaagaaaaaa 240
tctgtgctgt ttagactcag attcttaatt agctagtatt tcttaattca atcaataaat 300
tattaagacc ttttcactgc tcccttttta aagtcttctt tggagtgatt taagtgcttc 360
ttattaccaa gctctcaaag agaagataaa attaaaatct gatgg 405

<210> 39
<211> 736
<212> DNA
<213> Homo sapiens

<220>
<221> misc_feature
<222> 2, 3, 4, 5, 6, 7, 8, 9, 14, 15, 16, 17, 18, 19, 20, 21, 22,
23, 24, 25, 26, 27, 28, 29, 30, 31, 32, 33, 34, 35, 36,
37, 38, 39, 40, 41, 42, 43, 44, 45, 46, 47, 48, 49, 50,
51, 52, 53, 54, 55, 56, 57, 58, 59, 60, 61, 62, 63, 64,
65
<223> n = A,T,C or G

<221> misc_feature
<222> 66, 67, 68, 69, 70, 71, 72, 73, 74, 75, 76, 77, 78, 79, 80,
81, 82, 83, 84, 85, 86, 87, 88, 89, 90, 91, 92, 93, 94,
95, 96, 97, 98, 99, 100, 101, 102, 103, 104, 105, 106, 107,
108, 109, 110, 111, 112, 113, 114, 115, 116, 117, 118
<223> n = A,T,C or G

<221> misc_feature
<222> 119, 626, 627, 628, 629, 630, 631, 632, 633, 634, 635, 636,
637, 638, 639, 640, 641, 642, 643, 644, 645, 646, 647, 648,
649, 650, 651, 652, 653, 654, 655, 656, 657, 658, 659, 660,
661, 662, 663, 664, 665, 666, 667, 668, 669, 670, 671
<223> n = A,T,C or G

<221> misc_feature

<222> 672, 673, 674, 675, 676, 677, 678, 679, 680, 681, 682, 683,
684, 685, 686, 687, 688, 689, 690, 691, 692, 693, 694, 695,
696, 697, 698, 699, 700, 701, 702, 703, 704, 705, 706, 707,
708, 709, 710, 711, 712, 713, 714, 729, 736
<223> n = A,T,C or G

<400> 39
gnnnnnnnna gacnnnnnnn nnnnnnnnnn nnnnnnnnnn nnnnnnnnnn nnnnnnnnnn 60
nnnnnnnnnn nnnnnnnnnn nnnnnnnnnn nnnnnnnnnn nnnnnnnnnn nnnnnnnnna 120
cctgggatta caggcaccca ccaccacgcc tggctaattt ttttttgtat ctttagtagg 180
gttttgccat gttggccagg ctggtcttta actcctacct cgtgatccac ccgcctcggc 240
cccccaaagt gctaggacca caggcgtgag ccaccacgcc cagccccctg tctctttttt 300
taaaacacaa tttaaaagca gaaagaaaaa atctgtgctg tttagactca gattcttaat 360
tagctagtat ttcttaattc aatcaataaa ttattaagac cttttcactg ctcccttttt 420
aaagtcttct ttggagtgat ttaagtgctt cttattacca agctctcaaa gagaagataa 480
aattaaaatc tgatgggtaa ccatttaaat aagacaactg gggtaaccca tttctccagg 540
acccctctct gcaacagaga gctattctct ttctttggcc tagtaaacct ctgctcttaa 600
cctttaaaaa aaaaaaaaaa gtaccnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnncatagt 720
ggttcctgng tgaaan 736

<210> 40
<211> 725
<212> DNA
<213> Homo sapiens

<220>
<221> misc_feature
<222> 2, 3, 4, 5, 6, 7, 8, 9, 10, 12, 13, 15, 16, 17, 18, 19, 20,
21, 22, 23, 24, 25, 26, 27, 28, 29, 30, 31, 32, 33, 34,
35, 36, 37, 38, 39, 40, 41, 42, 43, 44, 45, 46, 47, 48,
49, 50, 51, 52, 53, 54, 55, 56, 57, 58, 59, 60, 61, 62,
63
<223> n = A,T,C or G

<221> misc_feature
<222> 64, 65, 66, 67, 68, 69, 70, 71, 72, 73, 74, 75, 76, 77, 78,
79, 80, 81, 82, 83, 84, 85, 86, 87, 88, 89, 90, 91, 92,
93, 94, 95, 96, 97, 98, 605, 606, 607, 608, 609, 610, 611,
612, 613, 614, 615, 616, 617, 618, 619, 620, 621, 622, 623
<223> n = A,T,C or G

<221> misc_feature
<222> 624, 625, 626, 627, 628, 629, 630, 631, 632, 633, 634, 635,
636, 637, 638, 639, 640, 641, 642, 643, 644, 645, 646, 647,
648, 649, 650, 651, 652, 653, 654, 655, 656, 657, 658, 659,
660, 661, 662, 663, 664, 665, 666, 667, 668, 669, 670
<223> n = A,T,C or G

<221> misc_feature
<222> 671, 672, 673, 674, 675, 676, 677, 678, 679, 680, 681, 682,
683, 684, 685, 686, 687, 688, 689, 690, 691, 692, 693, 694,
695, 696, 697, 698, 699, 700, 701, 702, 703, 704, 705, 706,
707, 708, 709, 710, 711, 712, 713, 714, 715, 716, 717
<223> n = A,T,C or G

<400> 40
gnnnnnnnnn anngnnnnnn nnnnnnnnnn nnnnnnnnnn nnnnnnnnnn nnnnnnnnnn 60
nnnnnnnnnn nnnnnnnnnn nnnnnnnnnn nnnnnnnnac ctgggattac aggcacccac 120
caccacgcct ggctaatttt tttttgtatc tttagtaggg ttttgccatg ttggccaggc 180
tggtctttaa ctcctacctc gtgatccacc cgcctcggcc ccccaaagtg ctaggaccac 240
aggcgtgagc caccacgccc agccccctgt ctcttttttt aaaacacaat ttaaaagcag 300

aaagaaaaaa tctgtgctgt ttagactcag attcttaatt agctagtatt tcttaattca 360
atcaataaat tattaagacc ttttcactgc tcccttttta aagtcttctt tggagtgatt 420
taagtgcttc ttattaccaa gctctcaaag agaagataaa attaaaatct gatgggtaac 480
catttaaata agacaactgg ggtaacccat ttctccagga cccctctctg caacagagag 540
ctattctctt tctttggcct agtaaacctc tgctcttaac ctttaaaaaa aaaaaaaaag 600
taccnnnnnn nnnnnnnnnn nnnnnnnnnn nnnnnnnnnn nnnnnnnnnn nnnnnnnnnn 660
nnnnnnnnnn nnnnnnnnnn nnnnnnnnnn nnnnnnnnnn nnnnnnnnnn nnnnnnnggt 720
atccg 725

<210> 41
<211> 474
<212> DNA
<213> Homo sapiens

<220>
<221> misc_feature
<222> 243, 267
<223> n = A,T,C or G

<400> 41
ccggaaaaaa agaaccattt ggatacatag gtatggtctg agctatgata tcaattggct 60
tcctagggtt tatcgtgtga gcacaccata tatttacagt aggaatagac gtagacacac 120
gagcatattt cacctccgct accataatca tcgctatccc caccggcgtc aaagtattta 180
gctgactcgc cacactccac ggaagcaata tgaaatgatc tgctgcagtg ctctgagccc 240
tangattcat ctttcttttc accgtangtg gcctgactgg cattgtatta gcaaactcat 300
cactagacat cgtactacac gacacgtact acgttgtagc ccacttccac tatgtcctat 360
caataggagc tgtatttgcc atcataggag gcttcattca ctgatttccc ctattctcag 420
gctacaccct agaccaaacc tacgccaaaa tccatttcac tatcatattc atcg 474

<210> 42
<211> 540
<212> DNA
<213> Homo sapiens

<400> 42
cataggtatg gtctgagcta tgatatcaat tggcttccta gggtttatcg tgtgagcaca 60
ccatatattt acagtaggaa tagacgtaga cacacgagca tatttcacct ccgctaccat 120
aatcatcgct atccccaccg gcgtcaaagt atttagctga ctcgccacac tccacggaag 180
caatatgaaa tgatctgctg cagtgctctg agccctagga ttcatctttc ttttcaccgt 240
aggtggcctg actggcattg tattagcaaa ctcatcacta gacatcgtac tacacgacac 300
gtactacgtt gtagcccact tccactatgt cctatcaata ggagctgtat ttgccatcat 360
aggaggcttc attcactgat ttcccctatt ctcaggctac accctagacc aaacctacgc 420
caaaatccat ttcactatca tattcatcgg cgtaaatcta actttcttcc cacaacactt 480
tctcggccta tccggaatgc cccgacgtta ctcggactac cccgatgcat acaccacatg 540

<210> 43
<211> 587
<212> DNA
<213> Homo sapiens

<400> 43
gaccatgagt catttagaat agtgataaat agaatacaca gaatagtgat gaaattcaat 60
ttaaaaaatc acgttagcct ccaaaccatt taattcaaat gaacccatca actggatgcc 120
aactctggcg aatgtaggac ctctgagtgg ctgtataatt gttaattcaa atgaaattca 180
tttaaacagt tgacaaactg tcattcaaca attagctcca gtaaataaca gttatttcat 240
cataaaacag tcccttcaaa cacacaattg ttctgctgaa gagttgtcat caacaatcca 300
atgctcacct attcagttgc tctgtggtca gtgtggctgc atagcagtgg attccatgaa 360
aggagtcatt ttagtgatga gctgccagtc cattcccagg ccaggctgtc gctggccatc 420
cattcagtcg attcagtcat aggcgaatct gttctgcccg aggcttgtgg tcaagcaaaa 480
attcagccct gaaatcaggc acatctgttc gttggactaa acccacaggt tagttcagtc 540
aaagcaggca acccccttgt gggcactgac cctgccactg gggtcat . 587

<210> 44
<211> 622
<212> DNA
<213> Homo sapiens

<220>
<221> misc_feature
<222> 491, 541, 556, 561, 568, 578, 585
<223> n = A,T,C or G

<400> 44
accatgagtc atttagaata gtgataaata gaatacacag aatagtgatg aaattcaatt 60
taaaaaatca cgttagcctc caaaccattt aattcaaatg aacccatcaa ctggatgcca 120
actctggcga atgtaggacc tctgagtggc tgtataattg ttaattcaaa tgaaattcat 180
ttaaacagtt gacaaactgt cattcaacaa ttagctccag taaataacag ttatttcatc 240
ataaaacagt cccttcaaac acacaattgt tctgctgaag agttgtcatc aacaatccaa 300
tgctcaccta ttcagttgct ctgtggtcag tgtggctgca tagcgtggga ttccatgaaa 360
ggagtcattt tagtggtgga gctgccagtc cttcccgggc cgggtgtcgc tgggccatcc 420
ttcagtcgtt tcgtcatagg cgatctgttc tgcccgaggg ttgtggtcag gcaaaattca 480
gccctgaatt ngggcactct gttcgttggg ctaaaccccc ggttagttca gtcaaggcgg 540
naaccccctt gtgggnactg ncctggcntt ggggtctngg cggtntggcc gttggggagg 600
tttggcccca cggcctctgt gg 622

<210> 45
<211> 340
<212> DNA
<213> Homo sapiens

<400> 45
aaggcaggaa tgtcaggcct ctgagcccaa gccaagccat cgcatcccct gtgacttgca 60
cgtatacacc cagatggcct gaagtaactg aagaatcaca aaagaagtga aaaggccctg 120
ccccgcctca actgatgaca ttccaccatg gtgatttgtt cctgccccac cttaactgag 180
tgattaaccc tgtgaatttc cttctcctgg ctcagaagct cccccactga gcaccttgtg 240
acccccgccc ctgcccacca gagaacaacc ccctttgact gtaatttccc atcaccttcc 300
caaatcctat aaaacggccc cacccctatc tccctttgct 340

<210> 46
<211> 394
<212> DNA
<213> Homo sapiens

<400> 46
aaggcaggaa tgtcaggcct ctgagcccaa gccaagccat cgcatcccct gtgacttgca 60
cgtatacacc cagatggcct gaagtaactg aagaatcaca aaagaagtga aaaggccctg 120
ccccgcctca actgatgaca ttccaccatg gtgatttgtt cctgccccac cttaactgag 180
tgattaaccc tgtgaatttc cttctcctgg ctcagaagct cccccactga gcaccttgtg 240
acccccgccc ctgcccacca gagaacaacc ccctttgact gtaatttccc atcaccttcc 300
caaatcctat aaaacggccc cacccctatc tccctttgct gactctcttt ttggactcag 360
cccgcctgca cccaggtgaa ataaacagcc atgt 394

<210> 47
<211> 246
<212> DNA
<213> Homo sapiens

<400> 47
tagccctgat aggcgctatt ttcctcctgg ttttgtattt gaaccgcaag gggataaaaa 60
agtggatgca taacatcaga gatgcctgca gggatcacat ggaagggtat cattacagat 120
atgaaatcaa tgcggacccg gggattaaca aacctcagtt ctaactcgga tgtctgagaa 180
atattagagg acagaccaag gacaactctg catgagatgt agacttaagc tttatcccta 240
ctaggc 246

<210> 48
<211> 336
<212> DNA
<213> Homo sapiens

<400> 48
acatatttcc ttttcctcca ttggccacaa tgggctccaa acaaccacat gcagatttta 60
caaaaagaaa gttccaaaac tgctcaatca aaagaaagat tcaactctgt gagatgaata 120
cacacatcac aacgaagttt ctcagaatgc ttctgtgttg tttttatgtg aagatatttc 180
cttttccatc ataggcctct aagtgcatca actatccact tgcagattct acaaaaagag 240
tgtttcaaaa ctgctcaatc aaaagaaagt atcaactctg tgaggaaatg cacacatcac 300
aaagaagttt ctcagaatga ttctgtgtag ttttta 336

<210> 49
<211> 518
<212> DNA
<213> Homo sapiens

<400> 49
cagaagggtc tgcaagatgc tgttcttggc cactttcttt cccacctggg aaggcggcat 60
ctatgacttc attggggagt tcatgaaggc cagcgtggat gtgccagacc tgataggtct 120
aaaccttgtc atgtcccgga atgccggcaa gggagagtac aagatcatgg ttgctgccct 180
gggctgggcc actgctgagc ttattatgtc ccgctgcatt cccctatggg tcggagcccg 240
gggcattgag tttgactgga agtacatcca gatgagcata gactccaaca tcagtctggt 300
ccattacatc gtcgcgtctg ctcaggtctg gatgataaca cgctatgatc tgtaccacac 360
cttccggcca gctgtcctcc tgctgatgtt cctcagtgtc tacaaggcct ttgttatgga 420
gaccttcgtc cacctctgct cgctgggcag ttgggcagct ctactggccc gagcagtggt 480
aacggggctg ctggccctca acactttggc cctgtatg 518

<210> 50
<211> 326
<212> DNA
<213> Homo sapiens

<400> 50
tctgcaagat gctgttcttg gccactttct ttcccacctg ggaaggcggc atctatgact 60
tcattgggga gttcatgaag gccagcgtgg atgtgccaga cctgataggt ctaaaccttg 120
tcatgtcccg gaatgccggc aagggagagt acaagatcat ggttgctgcc ctgggctggg 180
ccactgctga gcttattatg tcccgctgca ttcccctatg ggtcggagcc cggggcattg 240
agtttgactg gaagtacatc cagatgagca tagactccaa catcagtctg gtccattaca 300
tcgtcgcgtc tgctcaggtc tggatg 326

<210> 51
<211> 331
<212> DNA
<213> Homo sapiens

<400> 51
acattgaaaa aagtctagac aaactgaaag gcaataaatc ctatgtgaac atggacctct 60
ctccggtggt agagtgcatg gaccacgctc taacaagtct cttccctaag actcattatg 120
ccgctggaaa agatgccaaa attttctgga tacctctgtc tcacatgcca gcagctttgc 180
aagacttttt attgttgaaa cagaaagcag agctggctaa tcccaaggca gtgtgactca 240
gctaaccaca aatgtctcct ccaggctatg aaattggccg atttcaagaa cacatctcct 300
tttcaacccc attccttatc tgctccaacc g 331

<210> 52
<211> 253
<212> DNA
<213> Homo sapiens

<400> 52

acagaaggga tcgaagacaa attgaaggga gagatgatcg atctccaaca tggcagcctt 60
ttccttagaa caccaaagat tgtctctggc aaagactcta atgtaactgc aaactccaag 120
ctggtcatta tcacggctgg ggcacgtcag caagagggag aaagccgtct taatttggtc 180
cagcgtaacg tgaacatatt taaattcatc attcctaatg ttgtaaaata cagcccgaac 240
tgcaagttgc tta 253

<210> 53
<211> 356
<212> DNA
<213> Homo sapiens

<400> 53
atcgaagaca aattgaaggg agagatgatg gatctccaac atggcagcct tttccttaca 60
acaccaaaga ttgtctctgg caaagactat aatgtaactg caaactccaa gctggtcatt 120
atcacggctg gggcacgtca gcaagaggga gaaagccgtc ttaatttggt ccagcgtaac 180
gtgaacatat ttaaattcat cattcctaaa gttgtaaaat acagcccgaa ctgcaagttg 240
cttattgttt caaatccagt ggatatcttg acctacgtgg cttggaagat aagtggtttt 300
cccaaaaacc gtgttattgg aagaggttgc aatctggatt caacccgatt ccgcta 356

<210> 54
<211> 570
<212> DNA
<213> Homo sapiens

<400> 54
ccgctgccgc cgattccgga tctcattgcc acgcgccccc gacgaccgcc cgacgtgcat 60
tcccgattcc ttttggttcc aagtccaata tggcaactct aaaggatcag ctgatttata 120
atcttctaaa ggaagaacag accccccaga ataagattac agttgttggg gttggtgctg 180
ttggcatggc ctgtgccatc agtatcttaa tgaaggactt ggcagatgaa cttgctcttg 240
ttgatgtcat cgaagacaaa ttgaagggag agatgatgga tctccaacat ggcagccttt 300
tccttagaac accaaagatt gtctctggca aagactataa tgtaactgca aactccaagc 360
tggtcattat cacggctggg gcacgtcagc aagagggaga aagccgtctt aatttggtcc 420
agcgtaacgt gaacatattt aaattcatca ttcctaatgt tgtaaaatac agcccgaact 480
gcaagttgct tattgtttca aatccagtgg atatcttgac ctacgtggct tggaagataa 540
gtggttttcc caaaaaccgt gttattggaa 570

<210> 55
<211> 223
<212> DNA
<213> Homo sapiens

<400> 55
gccgctgccg ccgattccgg atctcattgc cacgcgcccc cgacgaccgc ccgacgtgca 60
ttcccgattc cttttggttc caagtccaat atggcaactc taaaggatca gctgatttat 120
aatcttctaa aggaagaaca gaccccccag aataagatta cagttgttgg ggttggtgct 180
gttggcatgg cctgtgccat cagtatctta atgaaggact tgg 223

<210> 56
<211> 337
<212> DNA
<213> Homo sapiens

<400> 56
gatgcccata agatatggga agctatgtta tcaagccata ttagatatca agcattaata 60
tggaaataaa ccagcctgtt tggtgggctc ttcacatgga cgcgcatgaa atttggtgcc 120
gtgactagga tcgggggacc tcccttggga gatcaatccc ctgtcctcct gctctttgct 180
ccgtgagaaa catgcaccta tggcctcatg ttctcaaacc gaccaaacca agaaacatct 240
caccaatttt aaatccgcct ggcttgtgag gccttttgac cccaattcaa gtcttttgat 300
accctgtgaa ttgcacccat actgcccaga tggctag 337

<210> 57
<211> 473

<212> DNA
<213> Homo sapiens

<400> 57
aaagatcaaa gtgctgggct ccggtgcgtt cggcacggtg tataagggac tctggatccc 60
agaaggtgag aaagttaaaa ttcccgtcgc tatcaaggaa ttaagagaag caacatctcc 120
gaaagccaac aaggaaatcc tcgatgaagc ctacgtgatg gccagcgtgg acaaccccca 180
cgtgtgccgc ctgctgggca tctgcctcac ctccaccgtg caactcatca cgcagctcat 240
gcccttcggc tgcctcctgg actatgtccg ggaacacaaa gacaatattg gctcccagta 300
cctgctcaac tggtgtgtgc agatcgcaaa gggcatgaac tacttggagg accgtcgctt 360
ggtgcaccgc gacctggcag ccaggaacgt actggtgaaa acaccgcagc atgtcaagat 420
cacagatttt gggctggcca aactgctggg tgcggaagag aaagaatacc atg 473

<210> 58
<211> 487
<212> DNA
<213> Homo sapiens

<220>
<221> misc_feature
<222> 7
<223> n = A,T,C or G

<400> 58
actatcnccc aggacatggg accatgctca gctggttgct atcaagacct tgaaagacta 60
taacaacccc cagcaatgga tggaatttca acaagaagcc tccctaatgg cagaactgca 120
ccaccccaat attgtctgcc ttctaggtgc cgtcactcag gaacaacctg tgtgcatgct 180
ttttgagtat attaatcagg gggatctcca tgagttcctc atcatgagat ccccacactc 240
tgatgttggc tgcagcagtg atgaagatgg gactgtgaaa tccagcctgg accacggaga 300
ttttctgcac attgcaattc agattgcagc tggcatggaa tacctgtcta gtcacttctt 360
tgtccacaag gaccttggca gctcgcaata ttttaatcgg agaggcaact ttcatgttaa 420
aggttttcag gacttggggg ctttccagag gaaattttac tccgctgatt tactacaggg 480
tacccaa 487

<210> 59
<211> 532
<212> DNA
<213> Homo sapiens

<400> 59
atagaagtct gggaaaaaaa taaaaacaga atttgagaac cttggaccac tcctgtccct 60
gtagctcagt catcaaagca gaagtctggc tttgctctat taagattgga aatgtacact 120
accaaacact cagtccactg ttgagcccca gtgctggaag ggaggaaggc ctttcttctg 180
tgttaattgc gtaaaggcta caggggttag cctggactaa aggcatcctt gtcttttgag 240
ctattcacct cagtagaaaa ggatctaagg gaagatcact gtagtttagt tctgttgacc 300
tgtgcaccta ccccttggaa atgtctgctg gtatttctaa ttccacaggt catcagatgc 360
ctgcttgata atatataaac aataaaaaca accttcactt cttcctattg taatcgtgtg 420
ccatggatct gatctgtacc atgaccctac ataaggctgg atggcacccc aggctgaggg 480
ccccaatgta tgtgtggctg tgggtgtggg tgggagtgtg tctgctgagt aa 532

<210> 60
<211> 608
<212> DNA
<213> Homo sapiens

<400> 60
tacggccggg atagagtctg gaaaaaataa aaacagaatt tgagaacctt ggaccactcc 60
tgtccctgta gctcagtcat caaagcagaa gtctggcttt gctctattaa gattggaaat 120
gtacactacc aaacactcag tccactgttg agccccagtg ctggaaggga ggaaggcctt 180
tcttctgtgt taattgcgta gaggctacag gggttagcct ggactaaagg catccttgtc 240
ttttgagcta ttcacctcag tagaaaagga tctaagggaa gatcactgta gtttagttct 300
gttgacctgt gcacctaccc cttggaaatg tctgctggta tttctaattc cacaggtcat 360

cagatgcctg cttgataata tataaacaat aaaaacaacc ttcacttctt cctattgtaa 420
tcgtgtgcca tggatctgat ctgtaccatg accctacata aggctggatg gcaccccagg 480
ctgagggccc caatgtatgt gtggctgtgg gtgtgggtgg gagtgtgtct gctgagtaag 540
gaacacgatt ttcaagattc taaagctcaa ttcaagtgac acattaatga taaactcaga 600
tctgatca 608

<210> 61
<211> 480
<212> DNA
<213> Homo sapiens

<400> 61
tagatgacac tgatgattct caccagtctt atgagtctca ccattctgat gaatctgatg 60
aactggtcac tgattttccc acggacctgc cagcaaccga agttttcact ccagttgtcc 120
ccacagtaga cacatatgat ggccgaggtg atagtgtggt ttatggactg aggtcaaaat 180
ctaagaagtt tcgcagacct gacatccagt accctgatgc tacagacgag gacatcacct 240
cacacatgga aagcgaggag ttgaatggtg catacaaggc catccccgtt gcccaggacc 300
tgaacgcgcc ttctgattgg gacagccgtg ggaaggacag ttatgaaacg agtcagctgg 360
atgaccagag tgctgaaacc cacagccaca agcagtccag attatataag cggaaagcca 420
atgatgagag caatgagcat tccgatgtga ttgatagtca ggaactttcc aaagtcagcc 480

<210> 62
<211> 440
<212> DNA
<213> Homo sapiens

<400> 62
aggagatccg gcagatgggc actgagtgcc attacttcat ctgtgatgtg ggcaaccggg 60
aggaggtgta ccagacggcc aaggccgtcc gggagaaggt gggtgacatc accatcctgg 120
tgaacaatgc cgccgtggtc catgggaagg gcctaatgga cagtgatgat gatgccctcc 180
tcaagtccca acacatcaac accctgggcc agttctggac caccaaggcc ttcctgccgc 240
gtatgctgga gctgcagaat ggccacatcg tgtgcctcaa ctccgtgctg gcactgtctg 300
ccatccccgg tgccatcgac taccgcacat ccaaagcgtc agccttcgcc ttcatggaga 360
gcctgaccct ggggctgctg gactgtccgg gagtcagcgc caccacagtg ctgcccttcc 420
acaccagcac cgagatgttc 440

<210> 63
<211> 589
<212> DNA
<213> Homo sapiens

<400> 63
ggcactgagt gccattactt catctgtgat gtgggcaacc gggaggaggt gtaccagacg 60
gccaaggccg tccgggagaa ggtgggtgac atcaccatcc tggtgaacaa tgccgccgtg 120
gtccatggga agggcctaat ggacagtgat gatgatgccc tcctcaagtc ccaacacatc 180
aacaccctgg gccagttctg gaccaccaag gccttcctgc cgcgtatgct ggagctgcag 240
aatggccaca tcgtgtgcct caactccgtg ctggcactgt ctgccatccc cggtgccatc 300
gactaccgca catccaaagc gtcagccttc gccttcatgg agagcctgac cctggggctg 360
ctggactgtc cgggagtcag cgccaccaca gtgctgccct tccacaccag caccgagatg 420
ttccagggca tgagagtcag gtttcccaac ctctttcccc cactgaagcc ggagacggtg 480
gcccggagga cagtggaagc tgtgcagctc aaccaggccc tcctcctcct cccatggaca 540
atgcatgccc tcgttatctt gaaaagcata cttccacagg ctgcactcg 589

<210> 64
<211> 313
<212> DNA
<213> Homo sapiens

<400> 64
gcatattgtg ctcggggaag ggttcttgtc attgtgggaa gtgcatttgt tctgctgaag 60
agtggtatat ttctggggag ttctgtgact gtgatgacag agactgcgac aaacatgatg 120

gtctcatttg tacagggaat ggaatatgta gctgtggaaa ctgtgaatgc tgggatggat 180
ggaatggaaa tgcatgtgaa atctggcttg gctcagaata tccttaacaa ttacatgaga 240
gaggtctgga ttcttatttt ttctgggcca ttagaacata taaatgcgaa ggaaaccatg 300
tatattcacc act 313

<210> 65
<211> 223
<212> DNA
<213> Homo sapiens

<400> 65
tgtgaatcag cagatggcat attgtgctcg gggaagggtt cttgtcattg tgggaagtgc 60
atttgttctg ctgaagagtg gtatatttct ggggagttct gtgactgtga tgacagagac 120
tgcgacaaac atgatggtct catttgtaca gggaatggaa tatgtagctg tggaaactgt 180
gaatgctggg atggatggaa tggaaatgca tgtgaaatct ggc 223

<210> 66
<211> 424
<212> DNA
<213> Homo sapiens

<400> 66
ggtacagatt tagagcctgt aatcccagct acttgggagt ctaaggcaag agaatccctt 60
gaacctggga ggtggagatt gcagtgagct gagatcacac cattgcccta cagcctgggt 120
gacagtgaga ctgccccaag aaaaaacaaa agagacagcc ctagtgatct tgtaagttgc 180
ctttggtggg tcagtctttc cttttcttaa agaatagtac acattgacag ccaggtagct 240
ctatgatcct gttctataga attcaaaaag tcgacaacct tcctttgttc ctttctgttt 300
tctctgccta cgttagttta aattggcagt gtctctgctg gaataatccc atctctcttc 360
ctggcttctg ctgagatggc tgattaaatc cttgggtcac acccattatc tctttatcaa 420
atgg 424

<210> 67
<211> 487
<212> DNA
<213> Homo sapiens

<400> 67
ctgtaatccc agctacttgg gagtctaagg caagagaatc ccttgaacct gggaggtgga 60
gattgcagtg agctgagatc acaccattgc cctacagcct gggtgacagt gagactgccc 120
caagaaaaaa caaaagagac agccctagtg atcttgtaag ttgcctttgg tgggtcagtc 180
tttccttttc ttaaagaata gtacacattg acagccaggt agctctatga tcctgttcta 240
tagaattcaa aaagtcgaca accttccttt gttcctttct gttttctctg cctacgttag 300
tttaaattgg cagtgtctct gctggaataa tcccatctct cttcctggct tctgctgaga 360
tggctgatta aatccttggg tcacacccat tatctcttta tcaaatggtt gttcaggcta 420
ggctcagtgt ttcacgcctg taatcccaac actttgggag actgaggagg gcagatcact 480
tgagctc 487

<210> 68
<211> 492
<212> DNA
<213> Homo sapiens

<400> 68
agtcgcgcac cgacgctcaa acgcgcgctc caacccgcag cctcctcctg cctcaccgcc 60
cgaagatggc ggctctcaaa ctcctctcct ccgggcttcg gctctgcgcc tctgcccgcg 120
gatctggggc aacctggtac aagggatgtg tttgttcctt ttccaccagt gctcatcgcc 180
ataccaagtt ttatacagat ccagtagaag ctgtaaaaga catccctgat ggtgccacgg 240
ttttggttgg tggttttggg ctatgtggaa ttccagagaa tcttatagat gctttactga 300
aaactggagt aaaaggacta actgcagtca gcaacaatgc aggggttgac aattttggtt 360
tggggctttt gcttcggtcc aagcagataa aacgcatggt ctcttcatat gtgggagaaa 420
atgcagaatt tgaacgacag tacttatctg gtgaattaga agtggagctg acaccacagg 480
gcacacttgc tg 492

<210> 69
<211> 494
<212> DNA
<213> Homo sapiens

<400> 69
tttttttttt tttttttttc tccctttata aggcgatgta cataaatctg aggaatatgg 60
atgtcttctg gagcaaatgc tccaatatcc acaatttctt caacctctac cactgtggtt 120
tctgcagctt tgcacattgg caagttgaaa ttccttgcac ttttcctgaa aatcacgttt 180
cctgctcggt ccgccttcca ggctttcacc aaagcaaaat cccctgtaat tgcttcctcc 240
aaaataaagt gctgaccatt gaactccctc acctctcttg gcttattggc aatggcaaca 300
ctgccatctt tgttgtattt gatgggcgat cctccttctt gtaccagggt cccataccct 360
gttggggtgt aaaatgcagg aactccagcc ccgcctgcac ggatcctctc agcaagtgtg 420
ccctgtggtg tcagctccac ttctaattca ccagataagt actgtcgttc aaattctgca 480
ttttctccca cata 494

<210> 70
<211> 462
<212> DNA
<213> Homo sapiens

<400> 70
catgatgtat tacaaggagg ccttctggaa gaagaaggat tactgtggct gcatgatcat 60
tgaagatgaa gatgctccaa tttcaataac cttggatgac accaagccag atgggtcact 120
gcctgccatc atgggcttta ttcttgcccg gaaagctggt cgacttgcta agctacataa 180
ggaaataagg aagaagaaaa tctgtgagct ctatgccaaa gtgctgggat cccaagaagc 240
tttacatcca gtgcattatg aagagaagaa ctggtgtgag gagcagtact ctgggggctg 300
ctacacggcc tacttccctc ctgggatcat gactcaatat ggaagggtga ttcgtcaacc 360
cgtgggcagg attttctttg cgggcacaga gactgccaca aagtggagcg gctacatgga 420
aggggcagtt gaggctggag aacgagcagc tagggaggtc tt 462

<210> 71
<211> 626
<212> DNA
<213> Homo sapiens

<400> 71
catgatgtat tacaaggagg ccttctggaa gaagaaggat tactgtggct gctgatcatt 60
gaaaatgaag atgctcaatt tcaataacct tggatgacac caagccagat gggtcactgc 120
ctgccatcat gggcttcatt cttgcccgga aagctggtcg acttgctaag ctacataagg 180
aaataaggaa gaagaaaatc tgtgagctct atgccaaagt gctgggatcc caagaagctt 240
tacatccagt gcattatgaa gagaagaact ggtgtgagga gcagtactct gggggctgct 300
acacggccta cttccctcct gggatcatga ctcaatatgg aagggtgatt cgtcaacccg 360
tgggcaggat tttctttgcg ggcacagaga ctgccacaaa gtggagcggc tacatggaag 420
gggcagttga ggctggagaa cgagcagcta gggaggtctt aaatggtctc gggaaggtga 480
ccgagaaaga catctgggta caagaacctg aatcaaagga cgttccagcg gtagaaatca 540
cccacacctt ctgggaaagg aacctgccct ctgtttctgg cctgctgaag atcattggat 600
ttccacatca gtaactgccc tggggc 626

<210> 72
<211> 348
<212> DNA
<213> Homo sapiens

<400> 72
tggtgaactg gtcatccatg aaaaagggtt ttactacatc tattcccaaa catactttcg 60
atttcaggag gaaataaaag aaaacacaaa gaacgacaaa caaatggtcc aatatattta 120
caaatacaca agttatcctg accctatatt gttgatgaaa agtgctagaa atagttgttg 180
gtctaaagat gcagaatatg gactctattc catctatcaa gggggaatat ttgagcttaa 240
ggaaaatgac agaatttttg tttctgtaac aaatgagcac ttgatagaca tggaccatga 300
agccagtttt ttcggggcct ttttagttgg ctaactgacc tggaaaga 348

<210> 73
<211> 207
<212> DNA
<213> Homo sapiens

<220>
<221> misc_feature
<222> 122, 123
<223> n = A,T,C or G

<400> 73
tcaactcagt ggaacacggt tctcccaaac agattttgta attccgaaaa ccacgcatgc 60
gcaaacatac gcatacactc ccatgttcct ggacagttta tagctaccat aacctggcat 120
tnnccaaaac ataccatggt agactcttgg atacacaagg taattttaga gccacattag 180
gatgaacctt ctgaaaaagt tatgcat 207

<210> 74
<211> 497
<212> DNA
<213> Homo sapiens

<400> 74
gagcttaagg aaaatgacag aatttttgtt tctgtaacaa atgagcactt gatagacatg 60
gaccatgaag ccagtttttt cggggccttt ttagttggct aactgacctg gaaagaaaaa 120
gcaataacct caaagtgact attcagtttt caggatgata cactatgaag atgtttcaaa 180
aaatctgacc aaaacaaaca aacagaaaac agaaaacaaa aaaacctcta tgcaatctga 240
gtagagcagc cacaaccaaa aaattctaca acacacactg ttctgaaagt gactcactta 300
tcccaagaaa atgaaattgc tgaaagatct ttcaggactc tacctcatat cagtttgcta 360
gcagaaatct agaagactgt cagcttccaa acattaatgc aatggttaac atcttctgtc 420
tttataatct actccttgta aagactgtag aagaaagcgc aacaatccat ctctcaagta 480
gtgtatcaca gtagtag 497

<210> 75
<211> 275
<212> DNA
<213> Homo sapiens

<220>
<221> misc_feature
<222> 96
<223> n = A,T,C or G

<400> 75
tgagcttaag gaaaatgaca gaatttttgt ttctgtaaca aatgagcact tgatagacat 60
ggaccatgaa gccagttttt tcggggcctt tttagntggc taactgaccc tggaaagaaa 120
aagcaataac ctcaaagtga ctattcagtt ttcaggatga tacactatga agatgtttca 180
aaaaatctga ccaaaacaaa caaacagaaa acagaaaaca aaaaaacctc tatgcaatct 240
gagtagagca gccacaacca aaaaattcta caaca 275

<210> 76
<211> 530
<212> DNA
<213> Homo sapiens

<400> 76
gacagaaggg gcctctccgc cccgcgtcca gctcgcccag ctcgcccagc gtccgccgcg 60
cctcggccaa ggcttcaacg gaccacacca aaatgccatc tcaaatggaa cacgccatgg 120
aaaccatgat gtttacattt cacaaattcg ctggggataa aggctactta acaaaggagg 180
acctgagagt actcatggaa aaggagttcc ctggattttt ggaaaatcaa aaagaccctc 240
tggctgtgga caaaataatg aaggacctgg accagtgtag agatggcaaa gtgggcttcc 300
agagcttctt ttccctaatt gcgggcctca ccattgcatg taatgactat tttgtagtac 360

acatgaagca gaagggaaag aagtaggcag aaatgagcag ttcgctcctc cttgataaga 420
gttgtcccaa agggtcgctt aaggaatctg ccccacagct tcccccatag aaggatttca 480
tgagcagatc aggacactta gcaaatgtaa aaataaaatc taactctcat 530

<210> 77
<211> 341
<212> DNA
<213> Homo sapiens

<400> 77
gcctctccgc cccgcgtcca gctcgcccag ctcgcccagc gtccgccgcg cctcggccaa 60
ggcttcaacg gaccacacca aaatgccatc tcaaatggaa cacgccatgg aaaccatgat 120
gtttacattt cacaaattcg ctggggataa aggctactta acaaaggagg acctgagagt 180
actcatggaa aaggagttcc ctggattttt ggaaaatcaa aaagaccctc tggctgtgga 240
caaaataatg aaggacctgg accagtgtag agatggcaaa gtgggcttcc agagcttctt 300
ttccctaatt gcgggcctca ccattgcatg taatgactat t 341

<210> 78
<211> 350
<212> DNA
<213> Homo sapiens

<400> 78
ggcctctccg ccccgcgtgc agctcgccca gctcgcccag cgtccgccgc gcctcggcca 60
aggcttcaac ggaccacacc aaaatgccat ctcaaatgga acacgccatg gaaaccatga 120
tgtttacatt tcacaaattc gctggggata aaggctactt aacaaaggag gacctgagag 180
tactcatgga aaaggagttc cctggatttt tggaaaatca aaaagaccct ctggctgtgg 240
acaaaataat gaaggacctg gaccagtgta gagatggcaa agtgggcttc cagagcttct 300
tttccctaat tgcgggcctc accattgcat gcaatgacta ttttgtagta 350

<210> 79
<211> 171
<212> DNA
<213> Homo sapiens

<400> 79
acagaaggga caaagagatc tggacagaat cgccggacag gtggcagctg ccaacaagaa 60
gcattagaac aaaccatgct gggttaataa attgcctcat tcgtaaacaa aaaaaaaaaa 120
aaaaaaaaaa agtttttttt ttttcccccc attttttatt ttttttcccc c 171

<210> 80
<211> 389
<212> DNA
<213> Homo sapiens

<400> 80
tggcgctgtg ttctatggag gaaaacaaag caggagaggg gagagtgact gctgggtaag 60
gtcttcctcc acctcctttg catctttgct cacatgccag cttctcctgg gcttcacaga 120
ccaccaattt ataatttcca tttaaaactt ccattttatt tttttaattt ttatttattt 180
atttatttat tacgagatgg ggtttcgctc ttgttgccca agattgcacc actgcactgc 240
agcctgggtg acagagcgag actttgtcaa aaagaaagaa agaaagaagg aaaggaagga 300
aggaaggaag gaaggaagga aaagaaaaga aagggaaaga aaaaagaaaa agaaagaaag 360
aaagaaaaaa aaaaaaaagg ggggccccc 389

<210> 81
<211> 430
<212> DNA
<213> Homo sapiens

<400> 81
tgcagataca gtggtggagt ggaagtttgc gttggtagag aatgggggag ttacccgctg 60
ggaagaatgc agcaatagat tcctagaaac tggccatgag gataaagtgg ttcacgcatg 120

gtgggggatt cactgattca gtttgcatag taatggagaa gctgtagaac aatgtggaag 180
aagctgaggt tgtggaacac actgaataaa ataaaggcag tgtgactcca aattcagcca 240
tctgaattgt ttaaatttgc tagtggattt tgtctactgt gcagaaatat atatgtctaa 300
tgtgcagaaa tatatatgtg tgtatgtgtg tatatatatg cacacacaca cagataatgc 360
ttccagtgaa tgtgaacttc ttttccctgt ggcactgatt gacagacttg tgctgatcca 420
ttattacttt 430

<210> 82
<211> 556
<212> DNA
<213> Homo sapiens

<400> 82
tttttttttt tttttttttt ttttttttaa gatattaaaa ttcaggtttt attatttgtt 60
cagttataat aatttaagtt aatatttgct gtattctcag agcaaagatg tatttctgta 120
ccactgtcct gtataaattt gttacccaag atagtgactg gtatgaaagg agagggaaga 180
gggtgacaga tggaaacgat tgctgtagga cagtccatct ggccagatgc ggtgggggag 240
gggagaagaa gtgggagaga gatggtccta cagatgctcc catgggtaaa tgatgggtgc 300
atccctccct gcagtcgggc tgtgcctgaa cttcacagtc ctctaagagg tgtcattcag 360
gccacctcac tcagcctatg cccaacccca ctcactttcc ctttccttat gggctgcccc 420
cgcaactgac ttccatggtg attggttctc attaggccct ttgtttctac accagcctta 480
gatcattaag acaaagacgt acttgctacc ctcatagcac ataacaacgc ctggcagatg 540
aaaatcaaac aaaaag 556

<210> 83
<211> 543
<212> DNA
<213> Homo sapiens

<400> 83
tgcagtggac atgtcgggcg ggacggtcac agtccttgaa aagtccctgt atcaaaaggc 60
caactgaagc aatacttcta cgagaccaag tgcaatccca tgggttacac aaaagaaggc 120
tgcaggggca tagacaaaag gcattggaac tcccagtgcc gaactaccca gtcgtacgtg 180
cgggccctta ccatggatag caaaaagaga attggctggc gattcataag gatagacact 240
tcttgtgtat gtacattgac cattaaaagg ggaagatagt ggatttatgt tgtatagatt 300
agattatatt gagacaaaaa ttatctattt gtatatatac ataacagggt aaattattca 360
gttaagaaaa aaataatttt atgaactgca tgtataaatg aagtttatac agtacagtgg 420
ttctacaatc tatttattgg acatgtccat gaccagaagg gaaacagtca tttgcgcaca 480
acttaaaaag tctgcattac attccttgat aatgttgtgg tttgttgccg ttgccaagaa 540
ctg 543

<210> 84
<211> 242
<212> DNA
<213> Homo sapiens

<400> 84
cggcggcaga caaaaagact gcagtggaca tgtcgggcgg gacggtcaca gtccttgaaa 60
aggtccctgt atcaaaaggc caactgaagc aatacttcta cgagaccaag tgcaatccca 120
tgggttacac aaaagaaggc tgcaggggca tagacaaaag gcattggaac tcccagtgcc 180
gaactaccca gtcgtacgtg cgggccctta ccatggatag caaaaagaga attggctggc 240
ga 242

<210> 85
<211> 350
<212> DNA
<213> Homo sapiens

<400> 85
tttttttttt tttttttttt tctttttttt tttttttttt tttattatta attatcttct 60
ttattaatac tcacatgtaa cctttgcttt ttacacaaaa gtctgcttta gaagaatgcc 120
tcctcggctt atcatgccca atggggcttt ttgtttctgg accacttccc ctttctccac 180

ccccaccccc acatccaaat tactcttaac atgttcacag ataccacgaa tattttgtaa 240
acaagatttg ggttactgga acttgatttc attaacatcc cacttcaaaa tggaaggcag 300
gtggaggaca gggtaagaaa taggagaaag aggacaagag aaggcaaaga 350

<210> 86
<211> 448
<212> DNA
<213> Homo sapiens

<400> 86
acagtttaag aagtggtgac attttgcatg atgaatgacc tgacttttag ccaccaggta 60
ctctttaaac agttttcctt atcagaggcc ctcctgtgct ggtgacccag catctgagtt 120
aggttccagc atgtaaagag ctgggagggc ggagaattct tagcatacat tcagacgttt 180
tttctgcaca ataataagtc catctgtcac ttgcattcca ctttttgtta catagaaaga 240
gtctgaccct ttaatccaaa aggtcttttt acattgtgaa tgctgtggga aggcaatttc 300
tctgcacaca agaggctacg ttttggaagt gatgtatgtt atttgatgac tgaaaatgaa 360
ctgtaaatgc tcctagagta tattcctctg ctgaacaaaa ttaaacttca aaaaaatcta 420
acagtaacac acccctgctt gggaccct 448

<210> 87
<211> 586
<212> DNA
<213> Homo sapiens

<400> 87
aatttacaga acagtttaag aagtggtgac attttgcatg atgaatgacc tgacttttag 60
ccaccaggta ctctttaaac agttttcctt atcagaggcc ctcctgtgct ggtgacccag 120
catctgagtt aggttccagc atgtaaagag ctgggagggc ggagaattct tagcatacat 180
tcagacgttt tttctgcaca ataataagtc catctgtcac ttgcattcca ctttttgtta 240
catagaaaga gtctgaccct ttaatccaaa aggtcttttt acattgtgaa tgctgtggga 300
aggcaatttc tctgcacaca agaggctacg ttttggaagt gatgtatgtt atttgatgac 360
tgaaaatgaa ctgtaaatgc tcctagagta tattcctctg ctgaacaaaa ttaaacttca 420
aaaaaatcta acagtaacac acccctgctt gggaccctag ctatatgcat tttatgtgac 480
cttgccatgc ttcagtgaac atactaattc tatgtctagc acatgttgat ttcctatgta 540
ttctgggtat tctattaaag gaaactttga actatgtcaa aaaaaa 586

<210> 88
<211> 203
<212> DNA
<213> Homo sapiens

<400> 88
   aatgaattta cagaacagtt taagaagtgg tgacattttg catgatgaat gacctgactt 60
   ttagccacca ggtactcttt aaacagtttt ccttatcaga ggccctcctg tgctggtgac 120
   ccagcatctg agttaggttc cagcatgtaa agagctggga gggcggagaa ttcttagcat 180
   acattcagac gttttttctg cac 203

<210> 89
<211> 548
<212> DNA
<213> Homo sapiens

<400> 89
tgctggaagg cattcgcatc tgccggcgag ggcttccgca accggatcgt cttccaggag 60
ttccgccaac gctacgagat cctggcggcg aatgccatcc ccaaaggctt catggacggg 120
aagcaggcct gcattctcat gatcaaagcc ctggaacttg accccaactt atacaggata 180
gggcagagca aaatcttctt ccgaactggc gtcctggccc acctagagga ggagcgagat 240
ttgaagatca ccgatgtcat catggccttc caggcgatgt gtcgtggcta cttggccaga 300
aaggcttttg ccaagaggca gcagcagctg accgccatga aggtgattca gaggaactgc 360
gctgcctacc tcaagctgcg gaactggcag tggtggaggc ttttcaccaa agtgaagcca 420
ctgctgcagg tgacacggca ggaggaggag atgcaagcca aggaggatga actgcagaag 480
accaaggagc ggcagcagaa ggcagagaat gagcttaagg agctggaaca gaagcactcg 540

cagctgac 548

<210> 90
<211> 595
<212> DNA
<213> Homo sapiens

<400> 90
tgcaatgggg tgctggaagg cattcgcatc tgccggcagg gcttccccaa ccggatcgtc 60
ttccaggagt tccgccaacg ctacgagatc ctggcggcga atgccatccc caaaggcttc 120
atggacggga agcaggcctg cattctcatg atcaaagccc tggaacttga ccccaactta 180
tacaggatag ggcagagcaa aatcttcttc cgaactggcg tcctggccca cctagaggag 240
gagcgagatt tgaagatcac cgatgtcatc atggccttcc aggcgatgtg tcgtggctac 300
ttggccagaa aggcttttgc caagaggcag cagcagctga ccgccatgaa ggtgattcag 360
aggaactgcg ctgcctacct caagctgcgg aactggcagt ggtggaggct tttcaccaaa 420
gtgaagccac tgctgcaggt gacacggcag gaggaggaga tgcaggccaa ggaggatgaa 480
ctgcagaaga ccaaggagcg gcagcagaag gcagagaatg agcttaagga gctggaacag 540
aagcactcgc agctgaccga ggagaagaac ctgctacagg aacagctgca ggcag 595

<210> 91
<211> 498
<212> DNA
<213> Homo sapiens

<400> 91
tgacagagca agacttggtt tcaaaaaaga gaaacacagt tggccctcca tatctgagtt 60
tcacagacga aaaatattca gaagaaaaaa aaatcaatgg ctgtatttgt actaaacatg 120
cccaggcttt ttttcttatt gttatcccct aaacaataca acaactattt ttatagcatt 180
tacattgtat tagatgttat aactactcta aagaggattt aaagtatatg gaatgatgtg 240
cataggttat atgcaaatac tatactattt atatcaggga cttgagcatc cttggatttt 300
ggtatgtgtg ggaggtcctg aaaccaatgt cctgtggata ctgaaggata actgtactaa 360
tttggagatt tctctctact atgatcaaga ttttcaaaca ttacattgct gattacatta 420
catcgttaca ttgtgattct ttccaagact tgagataaag tttgggaaga agtaccactt 480
gtttcagttt atgaaata 498

<210> 92
<211> 510
<212> DNA
<213> Homo sapiens

<400> 92
aaacacagtt ggccctccat atctgagttt cacagacgaa aaatattcag aagaaaaaaa 60
aatcaatggc tgtatttgta ctaaacatgc ccaggctttt tttcttattg ttatccccta 120
aacaatacaa caactatttt tatagcattt acattgtatt agatgttata actactctaa 180
agaggattta aagtatatgg aatgatgtgc ataggttata tgcaaatact atactattta 240
tatcagggac ttgagcatcc ttggattttg gtatgtgtgg gaggtcctga aaccaatgtc 300
ctgtggatac tgaaggataa ctgtactaat ttggagattt ctctctacta tgatcaagat 360
tttcaaacat tacattgctg attacattac atcgttacat tgtgattctt tccaagactt 420
gagataaagt ttgggaagaa gttaccactt gtttcagttt atgaaataga aaaaaaaaaa 480
aggggtaaag catgaaataa aaacctaaac 510

<210> 93
<211> 299
<212> DNA
<213> Homo sapiens

<400> 93
tggatccccc gggctgcagg aattcggcac gagcagaagt gcctgagacg cggagacatg 60
gctggtgtta aatggagcta ttcaatagca gtgacgcgct ctcctcagcc accaaatgtc 120
cctgacaccc tccccagccc ccacagataa catcagctga ggtttttttc agtatgaacc 180
tgtcctaaat caattcctca aagtgtgcac aaaactaaag aatataaata aacaaaagaa 240
aggtgaaaaa aaaaaaaaaa aaaaaaactc gggggggggc ccgggcccca attcccccct 299

<210> 94
<211> 234
<212> DNA
<213> Homo sapiens

<220>
<221> misc_feature
<222> 163, 189, 219, 222, 225, 226, 228, 233
<223> n = A,T,C or G

<400> 94
cagaagtgcc tgagacgcgg agacatggct ggtgttaaat ggagctattc aatagcagtg 60
acgcgctctc ctcagccacc aaatgtccct gacaccctcc ccagccccca cagataacat 120
cagctgaggt ttttttcagt atgaacctgt cctaaatcaa ttnctcaaag tgtgcacaaa 180
actaaagant ataaataaac aaaagaaagg tgaaaaaana anaannanaa aana 234

<210> 95
<211> 534
<212> DNA
<213> Homo sapiens

<400> 95
tgaagcagaa gtacctggac tatgccagag tccccaatag caatccccct gaatatgagt 60
tcttctgggg cctgcgctct tactatgaga ccagcaagat gaaagtcctc aagtttgcct 120
gcaaggtaca aaagaaggat cccaaggaat gggcagctca gtaccgagag gcgatggaag 180
cagatttgag ggctgcagct gaggctgcag ctgaagccaa ggctagggcc gagattagag 240
ctcgaatggg cattgggctc ggctcggaga atgctgccgg gccctgcaac tgggacgaag 300
ctgatatcgg accctgggcc aaagcccgga tccaggcggg agcagaagct aaagccaaag 360
cccaagagag tggcagtgcc agcactgctg ccagtaccag taccaataac agtgccagtg 420
ccagtgccag caccagtggt ggcttcagtg ctggtgccag cctgaccgcc actctcacat 480
ttgggctctt cgctggcctt ggtggagctg gtgccagcac cagtggcagc tctg 534

<210> 96
<211> 351
<212> DNA
<213> Homo sapiens

<400> 96
tttttttttt tttttttttt tttctgaaat ggcaaataga tttaatgcag agtgtcaact 60
tcaattgatt gatagtggct gcctagagtg ctgtgttgag taggtttctg aggatgcacc 120
ctggcttgaa gagaaagact ggcaggatta acaatatcta aaatctcact tgtaggagaa 180
accacaggca ccagagctgc cactggtgct ggcaccagct ccaccaaggc cagcgaagag 240
cccaaatgtg agagtggcgg tcaggctggc accagcactg aagccaccac tggtgctggc 300
actggcactg gcactgttat tggtactggt actggcacca gtgctggcac t 351

<210> 97
<211> 610
<212> DNA
<213> Homo sapiens

<400> 97
tttatgaatg ataaagatgt ttccggaaag atgaacaggt cacaatttga agaactctgt 60
gctgaacttc tgcaaaagat agaagtaccc ctttattcac tgttggaaca aactcatctc 120
aaagtagaag atgtgagtgc agttgagatt gttggaggca ctacacgaat tccagctgtg 180
aaggaaagaa ttgccaaatt ctttggaaaa gatattagca caacactcaa tgcagatgaa 240
gcagtagcca gaggatgtgc attacagtgt gcaatacttt ccccggcatt taaagttaga 300
gaattttccg tcacagatgc agttcctttt ccaatatctc tgatctggaa ccatgattca 360
gaagatactg aaggtgttca tgaagtcttt agtcgaaacc atgctgctcc tttctccaaa 420
gttctcacct ttctgagaag gggccctttt gagctagaag ctttctattc tgatccccaa 480
ggagttccat atccagaagc aaaaataggc cgctttgtag ttcagaatgt ttctgcacag 540
aaagatggag aaaaatctag agtaaaagtc aaagtgcgag tcaacaccca tggcattttc 600

accatctcta                                                          610

<210> 98
<211> 551
<212> DNA
<213> Homo sapiens

<400> 98
tttttttttt tttttttttt tagcattatc atcttaccct ctgtctcaat atacatgtta 60
agaaggtctt tccctaactg ccagaccaag ttggcttcaa taggcagctc aacattcacc 120
acctttattt tgggcttttt agcttctgga ggctggtcaa cttttttttc atttgctttg 180
tcagcatctg ggattttgtt ttcttctgag gtaagttcag gtgaaggggg agactgtgag 240
gtttgttgag catcagtttg tacctggggc tgtgttccag cttcactgtt gtcttgctgg 300
acatttttat cagtgtctgg gttttctggt ggtctctgat tcagacactc catgtcagct 360
tcagaagaca tttcattctc ctcagttggg actttctcca ccatagatgc cgtagagatg 420
gtgaaaatgc catgggtgtt gactcgcact ttgactttta ctctagattt ttctccatct 480
ttctgtgcag aaacattctg aactacaaag cggcctattt ttgcttctgg atatggaact 540
ccttggggat c                                                        551

<210> 99
<211> 550
<212> DNA
<213> Homo sapiens

<400> 99
tgtggggctc tatttttgct ttggctttct ggtgagagag tgaggaagca ttctttcctt 60
cactaagttt gtctttcttg tcttctggat agattgattt taagagacta agggaattta 120
caaactaaag attttagtca tctggtggaa aaggagactt taagattgtt tagggctggg 180
cggggtgact cacatctgta atcccagcac tttgggaggc caaggcaggc agaacacttg 240
aaggagttcg agaccagcgt ggccaacgtg gtgaaaccct gtctctacta aaaatacaaa 300
aattgtttag ctctgttttt cataatagaa atagaaaagg taaaattgct tttcttctga 360
aaagaacaag tattgttcat ccaagaaggg tttttgtgac tgaatcagca gtgcctgccc 420
tagtcatagc tgtgcttcaa aaacctcagc atgattagtg ttggagcaaa acaaggaagc 480
aaagcaaata ctgtttttga aattctatct gttgcttgaa ctattttgta ataattaaac 540
tttgatgttg                                                          550

<210> 100
<211> 300
<212> DNA
<213> Homo sapiens

<400> 100
ctaagcttta agatttaaaa aatgttcaat gttgaaattt ctgtggggct ctatttttgc 60
tttggctttc tggtgagaga gtgaggaagc attctttcct tcactaagtt tgtctttctt 120
gtcttctgga tagattgatt ttaagagact aagggaattt acaaactaaa gattttagtc 180
atctggtgga aaaggagact ttaagattgt ttagggctgg gcggggtgac tcacatctgt 240
aatcccagca ctttgggagg ccaaggcagg cagaacactt gaaggagttc gagaccagcg 300

<210> 101
<211> 583
<212> DNA
<213> Homo sapiens

<400> 101
gtttgagtca tgaccatgct gttgtctaga gtgggcgggg atgacgtggt tggagtgggt 60
gcgctgctct gtacttgatt tttttgagtc tgaaattagc tttccaggct ggggcaggga 120
ggggagcaca ggtggatcag tactgccccc aagcggtgga gctttggtgg tggatcaaat 180
actgctgccg cctgtctgca caaacatatt tctctcttcc agcccttcag aagtgtattg 240
gaatatgtcg ataacaataa tgatggtggt gaagatgatg atgatgtggg taattctggc 300
taccttattg ggtccaagct ccccacaatt cgttgcacaa agcactctac atacattctc 360
tttagtcctg atcaaaccac ctttcagagt aggatttagt gtcctatttt aaagatgaag 420

gagctcgggc tcagagagag atcgtttaga cacacacaca actttggaat gaaacattta 480
cagccgggcg cggtggcgcg tgcctgtagt cccagctact tgggaggctg aggctggagg 540
atcgcttgag tccaggagtt ctgggctgta gtgcgctatg ccg 583

<210> 102
<211> 517
<212> DNA
<213> Homo sapiens

<400> 102
cccggaaggc gacgggaagg agccgagctt gggtcatggc ggcgccgggc gcgctgctgg 60
tgatgggcgt gagcggctcg gggaaatcca ccgtgggcgc cctgctggca tctgagctgg 120
gatggaaatt ctatgatgct gatgattatc acccggagga aaatcgaagg aagatgggaa 180
aaggcatacc gctcaatgac caggaccgga ttccatggct ctgtaacttg catgacattt 240
tactaagaga tgtagcctcg ggacagcgtg tggttctagc ctgttcagcc ctgaagaaaa 300
cgtacagaga catattaaca caaggaaaag atggtgtagc tctgaagtgt gaggagtcgg 360
gaaaggaagc aaagcaggct gagatgcagc tcctggtggt ccatctgagc gggtcgtttg 420
aggtcatctc tggacgctta ctcaaaagag agggacattt tatgccccct gaattattgc 480
agtcccagtt tgagactctg gagcccccag cagctcc 517

<210> 103
<211> 590
<212> DNA
<213> Homo sapiens

<400> 103
tttttttttt tttttttttt ttttttacta gcgaagtttc atttatttgt gcaaatacag 60
gcatgagcaa gaatgttcta aacaatgtaa cgatttccag cattgattac agaatttcct 120
ctgatcattt gatttggtta tagatgaatt taaacttcaa tttaagcttg acttttaaaa 180
ctccccctct gcttcctgat gaaccagcat aattcctaaa attacaccta aacaagtctg 240
tcttgacaca ttggggtttg cctttagaaa catttagaat ctattatggg caaggcggct 300
ggaacgaggt ttgggatggc acaatgattt atgcttagtt ctgtttggac cactgataca 360
aaatcattgt catttcattt ttagggtttc cataattgta gcaattatct ctgaaacatt 420
tttgtccaca cttatttgga taaagttttc tggagctgct gggggctcca gagtctcaaa 480
ctgggactgc aataattcag ggggcataaa atgtccctct cttttgagta agcgtccaga 540
gatgacctca aacgacccgc tcagatggac caccaggagc tgcatctcag 590

<210> 104
<211> 116
<212> DNA
<213> Homo sapiens

<400> 104
gacacttaca aattgctgct tgtccaaatc aggatccact gcaaggaaca acaggcctta 60
ttccactgct ggggattggt gtgtgggagc acgcttacta ccttcagtat aaaaat 116

<210> 105
<211> 574
<212> DNA
<213> Homo sapiens

<400> 105
ttcttttttt tttttttttt tttgcacaaa gcatttacta ttttcaatca cttgcccaat 60
aacaaaatgt ttagtaagaa attattcaga acattaagtt gtttatgaaa taagtgacta 120
agcaacatca agaaatgcta caatagagca gcttactgta ttctgcagta ctctatacca 180
ctacaaaaac agtcataaag agcttaacat actcagcata acgatcgtgg tctacttttt 240
gcaagccatg tatctttcag ttacattctc ccagttgatt acattccaaa tagcttttag 300
ataatcaggc ctgacatttt tatactgaag gtagtaagcg tgctcccaca caccaatccc 360
cagcagtgga ataaggcctg ttgttccttg cagtggatcc tgatttggac aagcagcaat 420
ttgtaagtgt ccccgttcct tattgaaacc aagccaaccc caacctgagc cttggacacc 480
aacagatgca gccgtcagct tctccttaaa cttgtcaaag gaaccaaagt cacgtttgat 540
ggcttccagc aactcccctt tgggttctcc acca 574

<210> 106
<211> 474
<212> DNA
<213> Homo sapiens

<400> 106
tttttttttt tttttttttt ttgggggggt gacagattct tttattaaca gtcaaaaact 60
tcacacaatt ggaaaataaa tgtttcttca atgaataatc aaacaaaaat tatccaggac 120
cttatagggt tttcagtatg taccaggctt gatgcacatc ttagaagaca ggacattatc 180
ttgctgggat cattagggta tgatcagcat aacgatcgtg gtttactttt tgcaagccat 240
gtatctttca gttacattct cccagttgat tacattccaa atagctttta gataatcagg 300
cctgacattt ttatactgaa ggtagtaagc gtgctcccac acatcaatcc ccagcagtgg 360
aataaggcct gttgttcctt gcagtggatc ctgatttgga caagcagcaa tttgtaagtg 420
tccccgttcc ttattgaaac caagccaacc ccaacctgag ccttggacac caac 474

<210> 107
<211> 526
<212> DNA
<213> Homo sapiens

<400> 107
gggaacccgg ggcgcggcgc actgcgcagg cggccggact ccgctcagtt tccggtgcgg 60
cgaacaccaa agtccgggaa cttaagcatt ttcggtttct agggttgtta cgaagctgca 120
ggagcgagat ggaggtggac gcaccgggtg ttgatggtcg agatggtctc cgggagcggc 180
gaggctttag cgagggaggg aggcagaact tcgatgtgag gcctcagtct ggggcaaatg 240
ggcttcccaa acactcctac tggttggacc tctggctttt catccttttc gatgtggtgg 300
tgtttctctt tgtgtatttt ttgccatgac ttgttcgctg atatctaaat taagaagttg 360
gttcttgagt gaattctgaa aatggctaca aacttcttga ataaagaaga caggactctc 420
aatagaagaa tttcacatct ccaagggacc cttcctttca ttttacactt tgttactaat 480
ttgcagaact ctattaattg ggtaggattt cacccattcc tagcta 526

<210> 108
<211> 344
<212> DNA
<213> Homo sapiens

<400> 108
gaacccgggg cgcggcgcac tgcgcatgcg gccggactcc gctcagtttc cggtgcggcg 60
aacaccaaag tccgggaact taagcatttt cggtttctag ggttgttacg aagctgcagg 120
agcgagatgg aggtggacgc accgggtgtt gatggtcgag atggtctccg ggagcggcga 180
ggctttagcg agggagggag gcagaacttc gatgtgaggc ctcagtctgg ggcaaatggg 240
cttcccaaac actcctactg gttggacctc tggcttttca tccttttcga tgggggggag 300
cttctctctg tgtatttctc gccatgacct gttcagtgac accc 344

<210> 109
<211> 332
<212> DNA
<213> Homo sapiens

<400> 109
gaacccgggg cgcggcgcac tgcgcaggcg gccggactcc gctcagtttc cggtgcggcg 60
aacaccaaag tccgggaact taagcatttt cggtttctag ggttgttacg aagctgcagg 120
agcgagatgg aggtggacgc accgggtgtt gatggtcgag atggtctccg ggagcggcga 180
ggctttagcg agggagggag gcagaacttc gatgtgaggc ctcagtctgg ggcaaatggg 240
cttcccaaac actcctactg gttggacctc tggcttttca tccttttcga tgtggaggag 300
attctctttg tgtatttttt gccatgactt gt 332

<210> 110
<211> 545
<212> DNA
<213> Homo sapiens

<400> 110
cggctgcgag aagacgacag aaggggagtt tccggtgcgg cgaacaccaa agtccgggaa 60
cttaagcatt ttcggtttct agggttgtta cgaagctgca ggagcgagat ggaggtggac 120
gcaccgggtg ttgatggtcg agatggtctc cgggagcggc gaggctttag cgagggaggg 180
aggcagaact tcgatgtgag gcctcagtct ggggcaaatg ggcttcccaa acactcctac 240
tggttggacc tctggctttt catccttttc gatgtggtgg tgtttctctt tgtgtatttt 300
ttgccatgac ttgttcgctg atatctaaat taagaagttg gttcttgagt gaattctgaa 360
aatggctaca aacttcttga ataaagaaga caggactctc aatagaagaa tttcacatct 420
ccaagggacc cttcctttca ttttacactt tgttactaat ttgcagaact ctattaattg 480
ggtaggattt cacccattcc tagctaagtt cttaaaatta aaccctttgg ttcgtgttta 540
aaaac 545

<210> 111
<211> 329
<212> DNA
<213> Homo sapiens

<400> 111
gagtttccgg tgcggcgaac accaaagtcc gggaacttaa gcattttcgg tttctagggt 60
tgttacgaag ctgcaggagc gagatggagg tggacgcacc gggtgttgat ggtcgagatg 120
gtctccggga gcggcgaggc tttagcgagg gagggaggca gaacttcgat gtgaggcctc 180
agtctggggc aaatgggctt cccaaacact cctactggtt ggacctctgg cttttcatcc 240
ttttcgatga ggaggtgttt ctctttgtgt attttttgcc atgacttgtt cgctgatatc 300
taaatttaca agttggatct tgagtgaaa 329

<210> 112
<211> 284
<212> DNA
<213> Homo sapiens

<400> 112
gcgcggcgcc tcgcctcggc cggcgcctat cagccgactt agaactggtg cggaccaggg 60
gaatccgact gtttaattaa aacaaagcat cgcgaaggcc cgcggcgggt gttgacgcga 120
tgtgatttct gcccagtgct ctgaatgcca tattaaaaat aaactttaaa atttaaaagg 180
gggccgtttt tctctgattc ccaccccgtt aaaaaccctt ttgggggggg ggcccccccc 240
ccctcatggg gcggggaaaa aaggcctttt ttgggaaatt tggg 284

<210> 113
<211> 522
<212> DNA
<213> Homo sapiens

<400> 113
gttgcaggtc actgtagcgg gacttctttt ggttttcttt ctctttgggg cacctctgga 60
ctcactcccc agcatgaagg cgctgagccc ggtgcgcggc tgctacgagg cggtgtgctg 120
cctgtcggaa cgcagtctgg ccatcgcccg gggccgaggg aagggcccgg cagctgagga 180
gccgctgagc ttgctggacg acatgaacca ctgctactcc cgcctgcggg aactggtacc 240
cggagtcccg agaggcactc agcttagcca ggtggaaatc ctacagcgcg tcatcgacta 300
cattctcgac ctgcaggtag tcctggccga gccagcccct ggaccccctg atggccccca 360
ccttcccatc cagacagccg agcccgctcc ggaacttgtc atctccaacg acaaaaggag 420
cttttgccac tgactccggc cgtgtcctga cacctccaga acgcaggtgc tggcgcccgt 480
tctgcctggg accccgggaa cctctcctgc cggaagccgg ac 522

<210> 114
<211> 510
<212> DNA
<213> Homo sapiens

<400> 114
   gttgcaggtc actgtagcgg gacttctttt ggttttcttt ctctttgggg cacctctgga 60
   ctcactcccc agcatgaagg cgctgagccc ggtgcgcggc tgctacgagg cggtgtgctg 120
   cctgtcggaa cgcagtctgg ccatcgcccg gggccgaggg aagggcccgg cagctgagga 180
   gccgctgagc ttgctggacg acatgaacca ctgctactcc cgcctgcggg aactggtacc 240
   cggagtcccg agaggcactc agcttagcca ggtggaaatc ctacagcgcg tcatcgacta 300
   cattctcgac ctgcaggtag tcctggccga gccagcccct ggaccccctg atggccccca 360
   ccttcccatc cagacagccg agcccgctcc ggaacttgtc atctccaacg acaaaaggag 420
   cttttgccac tgactcggcc gtgtcctgac acctccagaa cgcaggtgct ggcgcccgtt 480
   ctgcctggga ccccgggaac ctctcctgcc 510

<210> 115
<211> 385
<212> DNA
<213> Homo sapiens

<400> 115
aatagtctgt gtccaagaaa ataagaatca cgtcatctag ctgtggacac tgagcaaaaa 60
ggagcagcat gctattaaga tggttgagac acacgagtga acaaagatgg gacaaactgt 120
gcttcgttca agaagtttca tcaagacccc taccgccccc cgtccttcag ctctgtacag 180
taactttagc tttacataga gctgagataa aaataaagct ttcttacaaa ttacattttt 240
ttccagtgaa ttacttttgc agtaaaaata gctgctacat aaatccctcc tgatctctga 300
aaaggagttg catatttcca aaaataatat tcttatttta atcacacaga agaacgtgga 360
gcacaggaag gaaatggctg gctgg 385

<210> 116
<211> 645
<212> DNA
<213> Homo sapiens

<400> 116
   tacggccggg tcttttaaag aggccgggaa tacacatgac tcaggtgctc ttttgaaacg 60
   actacaaaag tctccatttt gatcaaaacg ttttctccga atgaatggct ccgatgcttt 120
   ctctttccca tcttaagtcc ccgctctgtg cctcagaata gtctgtgtcc aagaaaataa 180
   gaatcacgtc atctagctgt ggacactgag caaaaaggag cagcatgcta ttaagatggt 240
   tgagacacac gagtgaacaa agatgggaca aactgtgctt cgttcaagag gtttcatcaa 300
   gacccctacc gccccccgtc cttcagctct gtacagtaac tttagcttta catagagctg 360
   agataaaaat aaagctttct tacaaattac atttttttcc agtgaattac ttttgcagta 420
   aaaatagctg ctacataaat ccctcctgat ctctgaaaag gagttgcata tttccaaaaa 480
   taatattctt attttaatca cacagaagaa cgtggagcac aggaaggaaa tggctggctg 540
   gtcagggaga ggtgagctgt cggagaaaca cagtaaaact aaaaaataaa atccattttg 600
   tgtataaact gacttaaacg catgcaaaga agtggaaaac atatg 645

<210> 117
<211> 500
<212> DNA
<213> Homo sapiens

<400> 117
atgtcgaggg aatgcagaaa gagttaagga aggcaggttg tccttctatt caggccactc 60
ttcgttttcc atgtactgca tgctgtttgt ggcactttat cttcaagcca ggatgaaggg 120
agactgggca agactcttac gccccacact gcaatttggt cttgttgccg tatccattta 180
tgtgggcctt tctcgagttt ctgattataa acaccactgg agcgatgtgt tgactggact 240
cattcaggga gctctggttg caatattagt tgctgtatat gtatcggatt tcttcaaaga 300
aagaacttct tttaaagaaa gaaaagagga ggactctcat acaactctgc atgaaacacc 360
aacaactggg aatcactatc cgagcaatca ccagccttga aaggcagcag ggtgcccagg 420
tgaggctggc ctgttttcta aaggaagatg attgccacaa ggcaagaaga tgcatctttc 480
ttcctggtgt acaagccttt 500

<210> 118
<211> 592
<212> DNA
<213> Homo sapiens

<400> 118

taaggaaggc aggttgtcct tctattcagg ccactcttcg ttttccatgt actgcatgct 60
gtttgtggca ctttatcttc aagccaggat gaagggagac tgggcaagac tcttacgccc 120
cacactgcaa tttggtcttg ttgccgtatc catttatgtg ggcctttctc gagtttctga 180
ttataaacac cactggagcg atgtgttgac tggactcatt cagggagctc tggttgcaat 240
attagttgct gtatatgtat cggatttctt caaagaaaga acttctttta aagaaagaaa 300
agaggaggac tctcatacaa ctctgcatga aacaccaaca actgggaatc actatccgag 360
caatcaccag ccttgaaagg cagcagggtg cccaggtgag gctggcctgt tttctaaagg 420
aagatgattg ccacaaggca agaggatgca tctttcttcc tggtgtacaa gcctttaaag 480
acttctgctg ctgctatgcc tcttggatgc acactttgtg tgtacatagt tacctttaac 540
tcagtggtta tctaatagct ctaaactcat taaaaaaact ccaagccttc ca 592

<210> 119
<211> 197
<212> DNA
<213> Homo sapiens

<400> 119
ggccgccctt tttttttttt tttttttttt ttttttttgg ggaaaagggg gtcttttttg 60
ggtccccccc ccccttttaa aaaacccccc taaaaaatgc ccccaaaaaa aaaaattttt 120
tttttttgggg gggggggaaaa aaaggggggaa aaaacccccc cccccccgggg ggggaaaaaa 180
acccccccaa aacccccc 197

<210> 120
<211> 493
<212> DNA
<213> Homo sapiens

<400> 120
tttttttttt ttaatggtaa aaactttatt tactatttat aaatacattg caagacaaac 60
ttctcaaaaa tacttttccc cccaaaaagt taaaaaaata aagaaaagct aataggtagg 120
cagaatgtct tgagacccct ctgttttcaa ggagagctct atgcagcgtg tgtccacacc 180
gaggtctgca gcagggcaga gtctccctga gcctgacttt gccagacctt cttgggtttg 240
gcctccggga gagcagccca gtctctgggt cgacgtcctt tcctcagtca tggccacagt 300
tgtatcatat agcatctcta acatttcatc taggattatc tagtatagat cttactatat 360
ttggggctat gttgtataca atgttaacaa gaacatatct tctctgcata tatgtgtgaa 420
ttataaagaa aagcatgaga atgactctaa gttcaacaaa catgggtgaa tctctatgtg 480
ctcccagtgt cct 493

<210> 121
<211> 265
<212> DNA
<213> Homo sapiens

<400> 121
tggtacgcct gcagtaccgg tccggaattc ccgggtcgac ccacgcgtcc gcttcctgtt 60
ttctgttgtc aaatgatgat aatgtgccat gatgttttat atatatcatt cagaaaaagt 120
tttatttttt aataacattc tattaacatt attttgcttg ccgctggcat gcctgaggaa 180
tgtatttggc tttgattaca cactaagttt ttgtaataaa tttgactcat taaaaacctt 240
tttttttaaa aaaaaaaaaa aaaaa 265

<210> 122
<211> 186
<212> DNA
<213> Homo sapiens

<400> 122
tttctgtttt ctgttgtcaa atgatgataa tgtgccatga tgttttatat atatcattca 60
gaaaaagttt tattttttaa taacattcta ttaacattat tttgcttgcc gctggcatgc 120
ctgaggaatg tatttggctt tgattacaca ctaagttttt gtaataaatt tgactcatta 180
aaaacc 186

<210> 123

<211> 475
<212> DNA
<213> Homo sapiens

<400> 123
cagcccgtcc gcggcctctc cagccccggg ttcgcgctct cgactccccc gacccagtcc 60
gcggtgcccc ggcgggtgat gccaaataca gccatgaaga aaaaggtgct gctgatgggg 120
aagagcgggt cggggaagac cagcatgagg tcgataatct tcgccaatta cattgctcgc 180
gacacccggc gcctgggggc caccattgac gtggaacact cccacgtccg attcctaggg 240
aacctggtgc tgaacctgtg ggactgtggc ggtcaggaca ccttcatgga aaattacttc 300
accagccagc gagacaatat cttccgtaac gtggaagttt tgatttacgt gtttgacgtg 360
gagagccgcg aactggaaaa ggacatgcat tattaccagt cgtgtctgga ggccatcctc 420
cagaactctc ctgacgccaa aatcttctgc ctggtgcaca aaatggatct ggttc 475

<210> 124
<211> 122
<212> DNA
<213> Homo sapiens

<400> 124
agaagggttg ctggagccta ggacgtcgag gctgcagtga gatatgatca caccactgca 60
ctccagcatg actgagtgag accctgtctc aaaaaaaaaa aaaaaaaagt tttttttttt 120
tc 122

<210> 125
<211> 147
<212> DNA
<213> Homo sapiens

<400> 125
ggaggggaag gttggtaggt aagctgtaac agattgctcc agttgcctta aactacgcac 60
atagctaagt gaccaaactt cttgttttga tttgaaaaag tgcattgttt tcttgtccct 120
ccctttgatg aaacgttacc ctttgac 147

<210> 126
<211> 607
<212> DNA
<213> Homo sapiens

<400> 126
cagtgaagac ttgcatgttg ttttcactac tgtacacttg acctgcacat gcgagaaaaa 60
ggtggaatgt ttaaaacacc ataatcagct cagggtattt gccaatctga aataaaagtg 120
ggatgggaga gtgtgtcctt cagatcaagg gtactaaagt ccctttcgct gcagtgagtg 180
agaggtatgt tgtgtgtgaa tgtacggatg tgtgtttgcg tgcatgtttg tgcatgtgtg 240
actgtgcatg ttatgtttct ccatgtgggc aaagatttga aatgtaagct tttatttatt 300
attttagaat gtgacataat gagcagccac actcggggga ggggaaggtt ggtaggtaag 360
ctgtaacaga ttgctccagt tgccttaaac tacgcacata gctaagtgac caaacttctt 420
gttttgattt gaaaaaagtg cattgttttc ttgtccctcc ctttgatgaa acgttaccct 480
ttgacgggcc ttttgatgtg aacagatgtt ttctaggaca aactataagg actaatttta 540
aacttcaaac attccacttt tgtaatttgt tttaaattgt tttatgtata gtaagcacaa 600
ctgtaat 607

<210> 127
<211> 463
<212> DNA
<213> Homo sapiens

<400> 127
attccaatta gccaggaatg gaaggatgag aagcgggatt tgctgactga aggacaaagt 60
tttagcagcc ttgatgaaga agccctggga tcccgacaca ggccagacct ggtccctagc 120
actccatcac tgtttgaagc tgcttccttg gcaaccacaa tttcatcttc ttccttatac 180
gtcaatgagc actatccaca cgacaggcct acactctatt caaacagcaa agggttacct 240

tccagttcaa catttacctt ggaagagggg accatctact tgaccgctga gcccaacact 300
ctggaagtgc aggatgacaa tgcttctgtg cttgacgtct atttataagt gaaaatggtg 360
atcacctaag cacatggatg agacgtgagc acagttatgg cagagaagtt tctccgcacc 420
agaattatcc acagcaactt ggctgagccc cactacacac aga 463

<210> 128
<211> 592
<212> DNA
<213> Homo sapiens

<400> 128
ccaattagcc aggaatggaa ggatgagaag cgggatttgc tgactgaagg acaaagtttt 60
agcagccttg atgaagaagc cctgggatcc cgacacaggc cagacctggt ccctagcact 120
ccatcactgt ttgaagctgc ttccttggca accacaattt catcttcttc cttatacgtc 180
aatgagcact atccacacga caggcctaca ctctattcaa acagcaaagg gttaccttcc 240
agttcaacat ttaccttgga agaggggacc atctacttga ccgctgagcc caacactctg 300
gaagtgcagg atgacaatgc ttctgtgctt gacgtctatt tataagtgaa aatggtgatc 360
acctaagcac atggatgaga cgtgagcaca gttatggcag agaagtttct ccgcaccaga 420
attatccaca gcaacttggc tgagccccac tacacacaga gaaatcatca acctgactta 480
agagttttca agatgtcaac ttcaggctga tcagcagatg ggatgtgaaa aatactaccc 540
tattctatca tttgctgttg cttgctgaac tgtgaagaac tgcatgaact at 592

<210> 129
<211> 251
<212> DNA
<213> Homo sapiens

<400> 129
caattagcca ggaatggaag gatgagaagc gggatttgct gactgaagga caaagtttta 60
gcagccttga tgaagaagcc ctgggatccc gacacaggcc agacctggtc cctagcactc 120
catcactgtt tgaagctgct tccttggcaa ccacaatttc atcttcttcc ttatacgtca 180
atgagcacta tccacacgac aggcctacac tctattcaaa cagcaaaggg ttaccttcca 240
gttcaacatt t 251

<210> 130
<211> 229
<212> DNA
<213> Homo sapiens

<400> 130
gtagcagaag cctcattcca gaacccatct ggccagagaa gcagcagcat cctgggggat 60
ggccgtgcat ggggtgtaca ctcgctatag gcataggccc ggcatggctg tcgctggacg 120
ccagctgtgc acacccagcc acacctgctg cacgccgcgt tagtgtgcgg ctccgggcct 180
gagcattcgc aaagctcgct tctccaggga gcctcctctt ggctttgga 229

<210> 131
<211> 316
<212> DNA
<213> Homo sapiens

<400> 131
cgccataacc tggtcagaag tgtgcctgtc ggcggggaga gaggcaatat caaggtttta 60
aatctcggag aaatggcttt cgtttgcttg gctatcggat gcttatatac ctttctgata 120
agcacaacat ttggctgtac ttcatcttca gacaccgaga taaaagttaa ccctcctcag 180
gattttgaga tagtggatcc cggatactta ggttatctct atttgcaatg gcaaccccca 240
ctgtctctgg atcattttaa ggaatgcaca gtggaatatg aactaaaata ccgaaacatt 300
ggtagtgaaa catgga 316

<210> 132
<211> 270
<212> DNA
<213> Homo sapiens

<220>
<221> misc_feature
<222> 37
<223> n = A,T,C or G

<400> 132
agtcgccata acctggtcag aagtgtgcct gtcggcnggg agagaggcaa tatcaaggtt 60
ttaaatctcg gagaaatggc tttcgtttgc ttggctatcg gatgcttata tacctttctg 120
ataagcacaa catttggctg tacttcatct tcagacaccg agataaaagt taaccctcct 180
caggattttg agatagtgga tcccggatac ttaggttatc tctatttgca atggcaaccc 240
ccactgtctc tggatcattt taaggaatgc 270

<210> 133
<211> 341
<212> DNA
<213> Homo sapiens

<400> 133
ttacatacgt ttttattact cgggggggac ctgtacgtca ccaatgccca gcttcacggg 60
ggcatgtagt gtgactcacg gctgaacaca aaatcactgt gaagcctgtg ctacagaagg 120
atgtccagtc gctgaggcca ggagagaggt gggcaggcct gggtctggca gtggagacgg 180
tcctccaggg agccgttggg caggaagccg tacaccaggc agtagaagcc gttctgagca 240
cagtagccag caaagtccac aatgtttggg tgacgaaacc tggacagctg ctccacctcg 300
gtcaggaagc tctgcttcac tgcagtccac tccaggtcag c 341

<210> 134
<211> 466
<212> DNA
<213> Homo sapiens

<400> 134
attatgtgat taatgatttg acagccgttc caatctccac gtctccagaa gagattccac 60
atgggagttt ctcagactga ttcttgacct ctcaatgaaa gtgttgaaac aggatgggaa 120
atattttaca caggggaact gtgtcaatct gacagaagca ctgtcgctct atgaagaaca 180
gctggggcgc ctgtattgtc ctgtggaatt ttcaaaggag atcgtctgtg tcccttcata 240
cttggaattg tgggtatttt acactgtttg gaagaaagct aaaccctgaa gatcagtagc 300
ccctaatcac atgtgctgca aatagccttc ctgacctcca tatgctgtac atgacatcaa 360
aatgagtcag gcaattgatt gtgaattcct taaagttttc ctttttttaa taattatttt 420
taatttaaaa aagcaaatgg aaaatgtata ttttgatgag cttagg 466

<210> 135
<211> 70
<212> DNA
<213> Homo sapiens

<400> 135
agttttcctt tttttaataa ttatttttaa tttaaaaaag caaatggaaa atgtatattt 60
tgatgagctt 70

<210> 136
<211> 442
<212> DNA
<213> Homo sapiens

<400> 136
tttttttttt tttttttcgg ctcagtataa agcttccttt tcttagggac catgcaaaga 60
ttctttgatt ctagaagtgc catttcatta tttctgtgac tcctgtctga atcatctgcc 120
aggtaactat cttgattttg tcttagcaat cgacttagca gaccattctt ggagaaagaa 180
aaatcctgag gtgaaacagg ctccgattta aagtcttcgg acactggtaa ggcaggtgcg 240
cttctctgca cagcaggagc catacccaag aatggggcac tcttagcatc atggctcaag 300
tgcacatttg tgttaggaat ttgtaagtca tcacaaggct cagattttat tttcaccatc 360

agtatttgtt cacttaaagc tctctctgag tgttcctgag tactttcatc tcttaaggga 420
gttttctctt ttttttcact ct 442

<210> 137
<211> 275
<212> DNA
<213> Homo sapiens

<220>
<221> misc_feature
<222> 244
<223> n = A,T,C or G

<400> 137
agaaaaatac aaaaaatctg cattaaaaat attaatcctg catgctggac atgtatggta 60
ataatttcta ttttgtacca ttttctgttt aactttagca tgttgttgat catggatcat 120
actcccctgt ttcttgggtg agaagggatc gccagtttgg aaactccggc ggctgcgtgc 180
ggggtttcag tcccactgta ggcttgtaaa taccgccccg ccaaaccgca tagagacgtg 240
gcancactga gggctttgtt gggttatata cgtat 275

<210> 138
<211> 353
<212> DNA
<213> Homo sapiens

<400> 138
taagctcgga attcgggtcg aggaaaaata caaaaaatct gcattaaaaa tattaatcct 60
gcatgctgga catgtatggt aataatttct attttgtacc attttcttgt ttaactttag 120
catgttgttg atcatggatc atactcccct tgtttctttg ggtgagaagg gatcgcagtt 180
tggaaactcc ggcggctgcg tgcggggttt cagtcccagc tgtaggcttg taaatacccg 240
ccccgccaaa ccgcatagag aacgtggcag caagctgagg gtctttgttt gggtttatta 300
ttacggtatt tttgtttgta agttaaaaaa aaaaaaaaaa gggggggccc cca 353

<210> 139
<211> 559
<212> DNA
<213> Homo sapiens

<400> 139
gaatttggcc ctcgaggcca agaattcggc actagggcgc agaaggacca gcagaaagat 60
gccgaggcgg aagggctgag cggcacgacc ctgctgccga agctgattcc ctccggtgca 120
ggccgggagt ggctggagcg gcgccgcgcg accatccggc cctggagcac cttcgtggac 180
cagcagcgct tctcacggcc ccgcaacctg ggagagctgt gccagcgcct cgtacgcaac 240
gtggagtact accagagcaa ctatgtgttc gtgttcctgg gcctcatcct gtactgtgtg 300
gtgacgtccc ctatgttgct ggtggctctg gctgtctttt tcggcgcctg ttacattctc 360
tatctgcgca ccttggagtc caagcttgtg ctctttggcc gagaggtgag cccagcgcat 420
cagtatgctc tggctggagg catctccttc cccttcttct ggctggctgg tgcgggctcg 480
gccgtcttct gggtgctggg agccaccctg gtggtcatcg gctcccacgc tgccttccac 540
cagattgagg ctgtggacg 559

<210> 140
<211> 711
<212> DNA
<213> Homo sapiens

<220>
<221> misc_feature
<222> 444
<223> n = A,T,C or G

<400> 140
tttttttttt tttttttttg acacccataa cagctttatt ttcaaaggcg ggatccctcc 60

ccgggcttgt gatgggacgg cgctgtgggc ccgagcagca aagccgtgca ggacaggcat 120
gggcaggggt ggggcagctg gcccgggagg ccggcaggtc ccaaaagaca cctcacacgg 180
gttccatctg cagctcctcc ccgtccacag cctcaatctg gtggaaggca gcgtgggagc 240
cgatgaccac cagggtggct cccagcaccc agaagacggc cgagcccgca ccagccagcc 300
agaagaaggg gaaggagatg cctccagcca gagcatactg atgcgctggg ctcacctctc 360
ggccaaagag cacaagcttg gactccaagg tgcgcagata gagaatgtaa caggcgccga 420
aaaagaccag ccagagccac cagncacata ggggacgtca ccacacagta caggatgagg 480
cccaggaaca cgaacacata gttgctctgg tagtactcca cgttgcgtac gaggcgctgg 540
cacagctctc ccaggttgcg gggccgtgag aagcgctgct ggtccacgaa ggtgctccag 600
gggccggatg gtcgcgcggc gccgctccag ccactcccgg cctgcacccg gaggaatcag 660
cttcggcagc aaggtcgtgc cggtcagccc ttccgcctcg gcattctttc t 711

<210> 141
<211> 468
<212> DNA
<213> Homo sapiens

<400> 141
actcgcagtc cttcttctct ggcctctttg gaggctcatc caaaatagag gaagcatgcg 60
aaatctacgc cagagcagca aacatgttca aaatggccaa aaactggagt gctgctggaa 120
acgcgttctg ccaggctgca cagctgcacc tgcagctcca gagcaagcac gacgcagcca 180
cctgctttgt ggacgctggc aacgcattca agaaagccga cccccaagag gccattaact 240
gtttgatgcg agcaatcgag atctacacag acatggccgg attcacgatt gcggccaagc 300
accacatctc cattgctgag atctatgaga cagagttggt ggacatcgag aaggccattg 360
cccactacga gcagtctgca gactactaca aaggcgagga gtccaacagc tcagccaaca 420
agtgtctgct gaaggtggct ggttacgctg cgctgctgga gcagtatc 468

<210> 142
<211> 203
<212> DNA
<213> Homo sapiens

<400> 142
cgcaaagtga agaactcgca gtccttcttc tctggcctct ttggaggctc atccaaaata 60
gaggaagcat gcgaaatcta cgccagagca gcaaacatgt tcaaaatggc caaaaactgg 120
agtgctgctg gaaacgcgtt ctgccaggct gcacagctgc acctgcagct ccagagcaag 180
cacgacgcag ccacctgctt tgt 203

<210> 143
<211> 212
<212> DNA
<213> Homo sapiens

<400> 143
tctgcgggga acagaacatg atcggcatga cgcccacggt catcgctgag cattacctgg 60
ctgaaacgga gcagcgggag aagttcgggc taaagaagcg ggagggggcc tgggagctca 120
tgaagaaggg gtacacccag caactggcct tcatacaacc cagctctgcc tttgcggcct 180
tcgtgaaacg ggcacccagc acctggctga cc 212

<210> 144
<211> 226
<212> DNA
<213> Homo sapiens

<220>
<221> misc_feature
<222> 109, 128, 153, 161, 167, 174, 175, 178, 196, 202, 206, 211,
213
<223> n = A,T,C or G

<400> 144
gaagcacctc attgtgaccc cctcgggctg cggggaacag aacatgatcg gcatgacgcc 60

cacggtcatc gctgtgcatt acctggatga aacggagcag tgggagaant tcggcctaga 120
gaagcggnag ggggccttgg agctcatcaa ganggggtac ncccagnagc tggnnttnag 180
acaacccagc tctgcntttg cnggcnttcg nanaaagggc ccccac 226

<210> 145
<211> 97
<212> DNA
<213> Homo sapiens

<400> 145
ctgggctgcg gctgatgcgc atccgttttc ctgccctggg catgtgtctc tgaaaccgta 60
tggcgggcgg tgggcaacgg gcactgctaa gggaggc 97

<210> 146
<211> 120
<212> DNA
<213> Homo sapiens

<400> 146
ggcacgagct catctgtttg cggatcagaa cccgagctgt gcttgtggct gcggctgcta 60
actggctgcg cacagggagc tgtcaccatg cctcactcgt acccagccct ttctgctgag 120

<210> 147
<211> 273
<212> DNA
<213> Homo sapiens

<400> 147
ggccgccctt tttttttttt ttttttttcc cccctttttt ttggtggggg ggtttttcca 60
aggggttgaa tgggggtttt ttttcccccc ttttacccca gaaaaagggg gaggaaaaaa 120
ggaacccccg gggaaaattt tccttttttt ggaaaatttg ggggacccga aaaagggggg 180
gggaaccccc cccctttttt ttttctttta aaaaattttt ttgcccccaa aaaaaggggg 240
gccccctttc ccccccttct tgggccccgg ggg 273

<210> 148
<211> 90
<212> DNA
<213> Homo sapiens

<400> 148
cacttcatgc aaggcacatg tgctgtcctg caggtctgca gggaaccgac ccagagagcc 60
cagcggcagg ccctggaaca cccgcctctg 90

<210> 149
<211> 463
<212> DNA
<213> Homo sapiens

<400> 149
gacttgtccg ggaatccggt gcttcggatc tactacacct cgaggcctgc tctgttcacc 60
ttgtgtgctg ggaatgagct cttctactgc ctcctctacc tgttccattt ctctgaggga 120
cctttagttg gctctgtggg actgttccgg atgggcctct gggtcactgc ccccatcgcc 180
ttgctgaagt cgctcatcag cgtcatccac ctgatcacgg ccgcccgcaa catggctgcc 240
ctggacgcag cagaccgcgc caagaagaag tgacgctgga gccccgggtc ctggctgccc 300
acctgccctg ggagtcttgc tgtgccacac agctccccac cccctgctag gaggtcccag 360
tctcacgcct tcctcatgtg ttgttctacc tgctgggatg ggggtcagcc tctctttggt 420
gacgtcacgt tctctgggat cctgaggacc cgggcctcaa atc 463

<210> 150
<211> 693
<212> DNA

<213> Homo sapiens

<220>
<221> misc_feature
<222> 285, 455, 597, 606, 636, 667, 686
<223> n = A,T,C or G

<400> 150
ggcacgagga gagagagagt cacaagatga tcgacttgtt cgggaatccg gtgcttcgga 60
tctactacac ctcgaggcct gctctgttca ccttgtgtgc tgggaatgag ctcttctact 120
gcctcctcta cctgttccat ttctctgagg gacctttagt tggctctgtg ggactgttcc 180
ggatgggcct ctgggtcact gcccccatcg ccttgctgaa gtcgctcatc agcgtcatcc 240
acctgatcac ggccgcccgc aacatggctg ccctggacgc agcanaccgc gccaagaaga 300
agtgacgctg gagccccggg tcctggctgc cacctgccct gggagtcttg ctgtgccaca 360
cagctcccca ccccctgcta ggaggtccca gtctcacgcc ttcctcatgt gttgttctac 420
ctgctgggat gggggtcagc ctctctttgg tgacntcacg ttcttctggg atcctgagga 480
ccgggcctca aatcagggag gatacccggg agggcccctt catccaagcg gtgcttctgg 540
ggtgccggga ccgggcagtg tcacaccctg cctgctagtc ctggggtcca gatctangga 600
ccttantgaa ggagtggtgt gaggcagttc tgaagnggat aactcgccca caacaagttg 660
ggacatncag aggaaactca actctnacgt ctt 693

<210> 151
<211> 300
<212> DNA
<213> Homo sapiens

<400> 151
gagagagaga gtcacaagat gatcgacttg tccgggaatc cggtgcttcg gatctactac 60
acctcgaggc ctgctctgtt caccttgtgt gctgggaatg agctcttcta ctgcctcctc 120
tacctgttcc atttctctga gggaccttta gttggctctg tgggactgtt ccggatgggc 180
ctctgggtca ctgcccccat cgccttgctg aagtcgctca tcagcgtcat ccacctgatc 240
acggccgccc gcaacatggc tgccctggac gcagcagacc gcgccaagaa gaagtgacgc 300

<210> 152
<211> 300
<212> DNA
<213> Homo sapiens

<220>
<221> misc_feature
<222> 37, 41
<223> n = A,T,C or G

<400> 152
gacttgtccg ggaatccggt gcttcggatc tactacncct ngaggcctgc tctgttcacc 60
ttgtgtgctg ggaatgagct cttctactgc ctcctctacc tgttccattt ctctgaggga 120
cctttagttg gctctgtggg actgttccgg atgggcctct gggtcactgc ccccatcgcc 180
ttgctgaagt cgctcatcag cgtcatccac ctgatcacgg ccgcccgcaa catggctgcc 240
ctggacgcag cagaccgcgc caagaagaag tgacgctgga gccccgggtc ctggctgccc 300

<210> 153
<211> 239
<212> DNA
<213> Homo sapiens

<220>
<221> misc_feature
<222> 168, 190, 203, 229
<223> n = A,T,C or G

<400> 153
gttgccctgc ctctggctcc agaacagaaa gggagcctca cgctggctca cacaaaacag 60
ctgacactga ctaaggaact gcagcatttg cacaggggag gggggtgcct ccttcctaga 120
ggccctgggg gccaggctga ttggggggca gattgacata ggccccantc atcagatgtc 180
tgaaattcan cacgggggta acntgggggg ttagggacta tttttaaant aggggtggc 239

<210> 154
<211> 113
<212> DNA
<213> Homo sapiens

<400> 154
gacacatttg ttacttcgtg agcaagcccg gaggctcgga gccccctgcc gtgttcacag 60
gtgacacctt gtttgtggct ggctgcggga agttctatga agggactgcg gat 113

<210> 155
<211> 294
<212> DNA
<213> Homo sapiens

<400> 155
tttttttttt tttttttttt ttttggcggg aataaatact tgttaaactt ctcttataaa 60
tatgcattaa aacgtccgat aacacaagcc aagggctgta aaattaaggt taaatcaaga 120
ctgaatttcc cgcacggacc agcaggaaag ccagttacct aaaagagcct aatccccaaa 180
tccgctgaag gtgcagggcg gcctcagtcc cggggcatct tgaactggtc cttctccctg 240
cgcacggccc gcatggtggt caccgggtcc gtctcacctg cgtgctgctg cacc 294

<210> 156
<211> 419
<212> DNA
<213> Homo sapiens

<400> 156
tagccatggc aggacagctc ctggaccagg tctcataatg catgtggcac ttaggtccaa 60
gctctccaga gggtgaaagc tggagtctgt caatgtccta ctgagacagc acagccaacc 120
tagctagcaa catttgtttt agtctgaaca atatatactt atagaattca gtcaaagata 180
cacaatctga aacagcttca tggggtggac tctaacagta gttgcaatgt tttagaatga 240
gacttacttc tctgctatct agatctgaac tccttggctt ctttacttag ttcaagcccc 300
agcctaggaa agccagttac ataaaagttg gctcaggagt cttagagctt tacctaaata 360
gagcccagaa aacggaggat gggggtgggg cgccttcctg gaggtgacac ttgatgggg 419

<210> 157
<211> 357
<212> DNA
<213> Homo sapiens

<400> 157
cgtattgctg tcaagccgtg agctagccat ggcaggacag ctcctggacc aggtctcata 60
atgcatgtgg cacttaggtc caagctctcc agagggtgaa agctggagtc tgtcaatgtc 120
ctactgagac agcacagcca acctagctag caacatttgt tttagtctga acaatatata 180
cttatagaat tcagtcaaag atacacaatc tgaaacagct tcatggggtg gactctaaca 240
gtagttgcaa tgttttagaa tgagacttac ttctctgcta tctagatctg aactccttgg 300
cttctttact tagttcaagc cccagcctag gaaagccagt tacataaaag ttggctc 357

<210> 158
<211> 408
<212> DNA
<213> Homo sapiens

<400> 158
actttgtatc actgcagcgc ttcacacctt catcctgaag atatctggaa cattcgtagt 60
atctgcagca ccaccaatat ccaatgcaag aacggcaaga tgaactgcca tgagggtgta 120

gtgaaggtca cagattgcag ggacacagga agttccaggg cacccaactg cagatatcgg 180
gccatagcga gcactagacg tgttgtcatt gcctgtgagg gtaacccaca ggtgcctgtg 240
cactttgacg gttagatgcc accatgtagg gattatcgcg agtggttgac cttacactta 300
ctccttaaat agcagtgagt aatgcatttg agctgcccca ggctctgtct cctcagctca 360
tttcttactc tttttctcta tataactcat tctattaaat acattgca 408

<210> 159
<211> 550
<212> DNA
<213> Homo sapiens

<400> 159
acaaggacgc caaccccacc tagatgcaaa gcaggattca aaagaacatc tttgcgtttt 60
ctaccggctc cccatcatcg tactagggag gaagaagcgg gtgagaaaca aaacttcttt 120
ccattgtcct gcccttttct gcggacttgt tctgaggccg aggcacctct aagatactga 180
tggctctgca gaggacccat tcattgcttc tgcttttgct gctgaccctg ctggggctgg 240
ggctggtcca gccctcctat ggccaggatg gcatgtacca gcgattcctg cggcaacacg 300
tgcaccctga ggagacaggt ggcagtgatc gctactgcaa cttgatgatg caaagacgga 360
agatgacttt gtatcactgc aagcgcttca acaccttcat ccatgaagat atctggaaca 420
ttcgtagtat ctgcagcacc accaatatcc aatgcaagaa cggcaagatg aactgccatg 480
agggtgtagt gaaggtcaca gattgcaggg acacaggaag ttccagggca cccaactgca 540
gatatcgggc 550

<210> 160
<211> 554
<212> DNA
<213> Homo sapiens

<400> 160
ccaaccccac ctagatgcaa agcaggattc aaaagaacat ctttgcgttt tctaccggct 60
ccccatcatc gtactaggga ggaagaagcg ggtgagaaac aaaacttctt tccattgtcc 120
tgcccgtttc tgcggacttg ttctgaggcc gaggcacctc taagatactg atggctctgc 180
agaggaccca ttcattgctt ctgcttttgc tgctgaccct gctggggctg gggctggtcc 240
agccctccta tggccaggat ggcatgtacc agcgattcct gcggcaacac gtgcaccctg 300
aggagacagg tggcagtgat cgctactgca acttgatgat gcaaagacgg aagatgactt 360
tgtatcactg caagcgcttc aacaccttca tccatgaaga tatctggaac attcgtagta 420
tctgcagcac caccaatatc caatgcaaga acggcaagat gaactgccat gagggtgtag 480
tgaaggtcac agattgcagg gacacaggaa gttccagggc acccaactgc agatatcggg 540
ccatagcgag cact 554

<210> 161
<211> 313
<212> DNA
<213> Homo sapiens

<400> 161
aattacatct tctttaaagc caaatgggag atgccctttg acccccaaga tactcatcag 60
tcaaggggcg tacttgagca ggaaaaagtg ggtaatggtg cccatgatga gtttgcatca 120
cctgactata ccttacttcc gggacgagga gctgtcctgc accgtggtgg agctgaagta 180
cacaggcaat gccagcgcac tcttcatcct ccctgatcaa gacaagatgg aggaagtgga 240
agccatgctg ctcccagaga ccctgaagcg gtggagagac tctctggagt tcagagagat 300
aggtgagctc tac 313

<210> 162
<211> 519
<212> DNA
<213> Homo sapiens

<400> 162
cggccgccct tttttttttt tttggccccc cggggccccc ttattttaaa aacccccccc 60
ccccctgggg ggggggcccc gaccttttaa gttttttttt tttcccccgg gggaaaaaaa 120
ggggggaaaa aaaaaaaaaa ttcccccccc tttttccccc ccccaaaaaa gggggggacc 180

ccccgggggg ggggggtttt ccccgggggg gaaaaaaaaa acccccgggg gccccccccc 240
aattttttcc ccccccccct tggggggggg gggggggggg gggggggggg gggggccccc 300
cccccccccc ccccccccat tttggggggt tgggttgggg gaaatttttt tttaaaaaaa 360
aaaaaaaaaa atttgggggt cccccccccc ctttttttcc cccccctttt ttccaaaagg 420
ggaccccccc cccccccccc caaaaaaacc cccccccccc ccccaaaaaa aacccccccc 480
cgggggggga aaaaaaaggg gggggggggg ggccccccc 519

<210> 163
<211> 422
<212> DNA
<213> Homo sapiens

<400> 163
aactaaaaac tacagtggaa gaaaggaagt cttcagaagc ctcccccact gcgcaaagaa 60
gtaaagatca cagtaaggaa tgcataaacg ctgccccaga ttctccgtcc aaacagcttc 120
cagaccagat ttcattcttc agtggaaatc catcagttga aatagttcat ggtattatgc 180
acctatataa gacaaataag atgacctcct taaaagaaga tgtgcggcgc agtgccatgc 240
tgtgtattct cacagtccct gctgcaatga ccagtcatga ccttatgaag tttgttgccc 300
catttaacga agtaattgaa caaatgaaaa ttatcagaga ctctactccc aaccaatata 360
tggtgctgat aaagtttcgt gcacaggctg atgcggatag tttttatatg acatgcaatg 420
gc 422

<210> 164
<211> 626
<212> DNA
<213> Homo sapiens

<400> 164
tacggccggg tgcgagctct gcgggaagcg gttcctggat agtttgcggc tgagaatgca 60
cttactggct cattcagcgg gtgccaaagc ctttgtctgt gatcagtgcg gtgcacagtt 120
ttcgaaggag gatgccctgg agacacacag gcagacccat actggcactg acatggccgt 180
cttctgtctg ctgtgtggga agcgcatcca ggcgcagagc gcactgcagc agcacatgga 240
ggtccacgcg ggcgtgcgca gctacatctg cagtgagtgc aaccgcacct tccccagcca 300
cacggctctc aaacgccacc tgcgctcaca tacaggcgac cacccctacg agtgtgagtt 360
ctgtggcagc tgcttccggg atgagagcac actcaagagc cacaaacgca tccacacggg 420
tgagaaaccc tacgagtgca atggctgtgg caagaagttc agcctcaagc atcagctgga 480
gacgcactat agggtgcaca caggtgagaa gccctttgag tgtaggctct gccaccagcg 540
ctcccgggac tactcggcca tgatcaagca cctgagaacg cacaacggcg cctcgcccta 600
ccagtgcacc atctgcacag agtact 626

<210> 165
<211> 515
<212> DNA
<213> Homo sapiens

<400> 165
gatagtttgc ggctgagaat gcacttactg gctcattcag cgggtgccaa agcctttgtc 60
tgtgatcagt gcggtgcaca gttttcgaag gaggatgccc tggagacaca caggcagacc 120
catactggca ctgacatggc cgtcttctgt ctgctgtgtg ggaagcgcat ccaggcgcag 180
agcgcactgc agcagcacat ggaggtccac gcgggcgtgc gcagctacat ctgcagtgag 240
tgcaaccgca ccttccccag ccacacggct ctcaaacgcc acctgcgctc acatacaggc 300
gaccacccct acgagtgtga gttctgtggc agctgcttcc gggatgagag cacactcaag 360
agccacaaac gcatccacac gggtgagaaa ccctacgagt gcaatggctg tggcaagaag 420
ttcagcctca agcatcagct ggagacgcac tatagggtgc acacaggtga gaagcccttt 480
gagtgtaggc tctgccacca gcgctcccgg gacta 515

<210> 166
<211> 615
<212> DNA
<213> Homo sapiens

<400> 166

actgttcaag gtttattggg ggttttagtt ggtataacac ttggatagtt ggttgcattg 60
tttgtatgta gatcttttta cattatatgg taatgtacac tactgatata gttcacaaaa 120
taagatcctt tggaagaatt atgcacaaga catgatattg gatttataca ctggatccca 180
ggatgtgact cactgggaaa aaatgttgga ctaggcatgt tcagtgaagg agccaggaag 240
ttatataaca cacggtaaac atccacctgg ctcaaggggc aaatgcagta cgtacagcat 300
tggcagtggt gcgtcagagg tggcagaact atttcacact aaccagttga agactacaca 360
agattaatac catccagcat caggatatag ctgtggattt tacaaaccat tcttatttct 420
aacttcagga gttgatgttt ttcccagtcc atcttaaaat attactgctt taatcacaga 480
tcagataaaa aggacaacat gcacaacctc cacctagaat cctgttgtag cctagacagt 540
gaaatgatat gacatcagaa gactttaaaa ttgcagctcc ttttggatcc cccaaagtgt 600
atctgcactc ttctt 615

<210> 167
<211> 99
<212> DNA
<213> Homo sapiens

<400> 167
tttttttttt ccactgttca aggtttattg gggggtttag ttggtataac acttggatag 60
tgggttgcat tgtttgtatg taaatctttt tacattata 99

<210> 168
<211> 612
<212> DNA
<213> Homo sapiens

<400> 168
tacggccggg acatgaagga gctaggagtg ggaatagctt tgcgaaaaat gggcgcaatg 60
gccaagccag attgtatcat cacttgtgat ggtaaaaacc tcaccataaa aactgagagc 120
actttgaaaa caacacagtt ttcttgtacc ctgggagaga agtttgaaga aaccacagct 180
gatggcagaa aaactcagac tgtctgcaac tttacagatg gtgcattggt tcagcatcag 240
gagtgggatg ggaaggaaag cacaataaca agaaaattga aagatgggaa attagtggtg 300
gagtgtgtca tgaacaatgt cacctgtact cggatctatg aaaaagtaga ataaaaattc 360
catcatcact ttggacagga gttaattaag agaatgtcca agctcagttc aatgagcaaa 420
tctccatact gtttctttct ttttttttca ttactgtgtt caattatctt tatcataaac 480
attttacatg cagctatttc aaagtgtgct ggattaatta ggatcatccc tttggttaat 540
aaataaatgg gtttgtgcta atatatcttg tatgcattct ttaaacctta caggaaatta 600
gtgatgagtt tt 612

<210> 169
<211> 410
<212> DNA
<213> Homo sapiens

<400> 169
gaaaacaaca cagttttctt gtaccctggg agagaagttt gaagaaacca cagctgatgg 60
cagaaaaact cagactgtct gcaactttac agatggtgca ttggttcagc atcaggagtg 120
ggatgggaag gaaagcacaa taacaagaaa attgaaagat gggaaattag tggtggagtg 180
tgtcatgaac aatgtcacct gtactcggat ctatgaaaaa gtagaataaa aattccatca 240
tcactttgga caggagttaa ttaagagaat gtccaagctc agttcaatga gcaaatctcc 300
atactgtttc tttctttttt tttcattact gtgttcaatt atctttatca taaacatttt 360
acatgcagct atttcaaagt gtgctggatt aattaggatc atccctttgg 410

<210> 170
<211> 310
<212> DNA
<213> Homo sapiens

<400> 170
gctcgggaat tcgctcgagt gctgctcccc acccatggac aggagatcct gggttgggcc 60
tccctctgat gaccccagcc agatgagcga gtggggctca gcgtggccca tggtgcctgt 120
cactcagcat tcccatgcct gatgtttacc aagtgctgtg ttggacactg gctttctcca 180

aacaggattt gcctcctcca cgctccctac acacctgaga tgtaaactgg cagtcagtgt 240
tcactcagga cctaggatta gaaaatggca gagttggtgc tggatccacc ttgcacttct 300
atcaagccct 310

<210> 171
<211> 257
<212> DNA
<213> Homo sapiens

<400> 171
tgctgctccc cagcccatgg acaggagatc ctgggttggg cctccctctg atgaccccag 60
ccagatgagc gagtggggct cagcgtggcc catggtgcct gtcactcagc attcccatgc 120
ctgatgttta ccaagtgctg tgttggacac tgactttctc caaacaggat ttgcctcctc 180
cacgctccct acacacctga gatgtaaact ggcagtcagt gttcactcag gacctaggat 240
tagaaaatgg cagagtt 257

<210> 172
<211> 593
<212> DNA
<213> Homo sapiens

<400> 172
tgaagaacgg tgccacttac gaagccaaaa tcaaggatgt ggatgagaaa gcagacatcg 60
cactcatcaa aattgaccac cagggcaagc tgcctgtcct gctgcttggc cgctcctcag 120
agctgcggcc gggagagttc gtggtcgcca tcggaagccc gttttccctt caaaacacag 180
tcaccaccgg gatcgtgagc accacccagc gaggcggcaa agagctgggg ctccgcaact 240
cagacatgga ctacatccag accgacgcca tcatcaacta tggaaactcg ggaggcccgt 300
tagtaaacct ggacggtgaa gtgattggaa ttaacacttt gaaagtgaca gctggaatct 360
cctttgcaat cccatctgat aagattaaaa agttcctcac ggagtcccat gaccgacagg 420
ccaaaggaaa agccatcacc aagaagaagt atattggtat ccgaatgatg tcactcacgt 480
ccagcaaagc caaagagctg aaggaccggc accgggactt cccagacgtg atctcaggag 540
cgtatataat tgaagtaatt cctgataccc cagcagaagc tggtggtctc aag 593

<210> 173
<211> 304
<212> DNA
<213> Homo sapiens

<220>
<221> misc_feature
<222> 106, 113, 125, 137
<223> n = A,T,C or G

<400> 173
gggtcaaagt tgagctgaag aacggtgcca cttacgaagc caaaatcaag gatgtggatg 60
agaaagcaga catcgcactc atcaaaattg accaccaggg caagcngcct gtnctgctgc 120
ttggncgctc ctcaganctg cggccgggag agttcgtggt cgccatcgga agcccgtttt 180
cccttcaaaa cacagtcacc accgggatcg tgagcaccac ccagcgaggc ggcaaagagc 240
tggggctccg caactcagac atggactaca tccagaccga cgccatcatc aactatggaa 300
actc 304

<210> 174
<211> 258
<212> DNA
<213> Homo sapiens

<400> 174
ggtcagaaga gttgtgcacg cagattagca ggccaaggtc tgagccacag cagcattttt 60
atttcagatt ttgataactg tttatatgtg ttgaaaacca aaatgacatc tttttaaagc 120
ttatccataa aaaaaaatag atgtctttta tagtggaaaa acacatgggg aaaaaaatca 180
tctattttga tgcagcattt gataatgata aaacacctca cacctcactc tttatagtgc 240
acaaaatgaa tgaggtct 258

<210> 175
<211> 442
<212> DNA
<213> Homo sapiens

<400> 175
aagtagccgc tccgagtgga ggcgactggg ggctgaagag cgcgccgccc tctcgtccca 60
ctttccaggt gtgtgatcct gtaaaattaa atcttccaag atgatctggt atatattaat 120
tataggaatt ctgcttcccc agtctttggc tcatccaggc ttttttactt caattggtca 180
gatgactgat ttgatccata ctgagaaaga tctggtgact tctctgaaag attatattaa 240
ggcagaagag gacaagttag aacaaataaa aaaatgggca gagaagttag atcggctaac 300
tagtacagcg acaaaagatc cagaaggatt tgttgggcat ccagtaaatg cattcaaatt 360
aatgaaacgt ctgaatactg agtggagtga gttggagaat ctggtcctta agggtatgtc 420
agatggcttt atctctaacc ta 442

<210> 176
<211> 611
<212> DNA
<213> Homo sapiens

<400> 176
gggctgaggt aggaagtagc cgctccgagt ggaggcgact gggggctgaa gagcgcgccg 60
ccctctcgtc ccactttcca ggtgtgtgat cctgtaaaat taaatcttcc aagatgatct 120
ggtatatatt aattatagga attctgcttc cccagtcttt ggctcatcca ggctttttta 180
cttcaattgg tcagatgact gatttgatcc atactgagaa agatctggtg acttctctga 240
aagattatat taaggcagaa gaggacaagt tagaacaaat aaaaaaatgg gcagagaagt 300
tagatcggct aactagtaca gcgacaaaag atccagaagg atttgttggg catccagtaa 360
atgcattcaa attaatgaaa cgtctgaata ctgagtggag tgagttggag aatctggtcc 420
ttaagggtat gtcagatggc tttatctcta acctaaccat tcagagacag tactttccta 480
atgatgaaga tcaggttggg gcagccaaag ctctgttacg tctccaggat acctacaatt 540
tggatacaga taccatctca aagggtaatc ttccaggagt gaaacacaaa tcttttctac 600
ggctgaggac t 611

<210> 177
<211> 416
<212> DNA
<213> Homo sapiens

<400> 177
ttacaaactc ctgaaccata atattctcgt ctccacagac acatactcca taatttaaaa 60
ccaaatgctt gtgagaaagc ttgctcatca tacttgctgc ttcaaagaaa gactctgaat 120
agtttctgtg tgctttatcc agaactttta aaagaacttc tgtttcatgc agttgaccgt 180
agtctcctac ttctcttcgt acgcctttaa aaatctttgt aaaagtgcct tggccaaggc 240
tttcattaaa tatcaaatct tcatttctga ttttgtgaaa caccatttgg ttcatatgag 300
taggcctctg taatgttggt gaggttggta catcagaaac accattcgtt ctgaagacta 360
gaaggtttga tttatctttt cggctttggg ggacagcatt tagtacacgg gaaaat 416

<210> 178
<211> 163
<212> DNA
<213> Homo sapiens

<400> 178
gggctttttt tttttgcaaa gttccaaatt tatgggtcgg gaaataaatc caaatttctc 60
attaaaaaac tcctttggaa aaacttgggc ccaaaagttt cccatccgaa ctcagccttt 120
tttgccccga tccccgactt ttttactcaa ggcccgggaa ggc 163

<210> 179
<211> 285
<212> DNA
<213> Homo sapiens

<400> 179
aaagttacaa atttattggt ctggaaataa atacaaatat ctcattaaga aactcctctg 60
gaaagacttg tgcacaatag tttcccatcc gtactcagcc tctcttgccc cgatccccga 120
cttttctact caaggccagg gaaaggcctc caaggtgatg ggcggcaggt aacgagtcat 180
tgcctctcac gccacctgga aggctggact acttcctcct cccaactgcg gggtcccaga 240
aatcctcggg tcccagtggc tgacttacaa tattcaattc actct 285

<210> 180
<211> 458
<212> DNA
<213> Homo sapiens

<400> 180
tcgagccgcc gccgcccctg tacaacaaca acaacaactg cgaggaaaat gagcagtctc 60
tgcccccgcc ggccggcctc aacagttcct gggtggagct acccatgaac agcagcaatg 120
gcaatgataa tggcaatggg aaaaatgggg ggctggaaca cgtaccatcc tcatcctcca 180
tccacaatgg agacatggag aagattcctt tggatgcaca acatgaatca ggacagagta 240
gttccagagg cagttctcac tgtgacagcc cttcgccaca agaagatggg cagatcatgt 300
ttgatgtgga aatgcacacc agcagggacc atagctctca gtcagaagaa gaagttgtag 360
aaggagagaa ggaagtcgag gctttgaaga aaagtgcgga ctgggtatca gactggtcca 420
gtagacccga aaacattcca cccaaggagt tccacttc 458

<210> 181
<211> 329
<212> DNA
<213> Homo sapiens

<400> 181
tttttttttt tttttttttt tttcttttta ataactatca actcaaactt agggaaactt 60
gcctttgtct tgggggaaaa aaacaactag acaataaagc ttcttttaca tcatttgcta 120
acctgatctc gttttaagag agagatggta gttatgttgc aagagtaaaa tttataccat 180
gaatgataca ggtctagtct ggtggcacta attagagata atagcattgc tgacaaaatt 240
ataatctgct ggtggcattt gcggaaaaga ggcccttgca aatttctaaa caacagtaaa 300
ctctgttagg aaattctaaa atgtcttca 329

<210> 182
<211> 527
<212> DNA
<213> Homo sapiens

<400> 182
atacatgtaa cttcattatt ttaaaaatat ttttagaact ccaatactca ccctgttatg 60
tcttgctagt ttaaattttg ctaattaact gaaacatgct taccagattc acactgttcc 120
agtgtctata aaagaaacac tttgaagtct ataaaaaata aaataattat aaatgtcatt 180
gtacatagca tgtttatatc tgcaaaaaac ctaatagcta attaatctgg aatatgcaac 240
attgtcctta attgatgcaa ataacacaaa tgctgcaaag aaatctacta tatcccttaa 300
tgaaatacat cattcttcat atatttctcc ttcagtccat tcccttaggc aatttttaat 360
ttttaaaaat tattatcagg ggagaaaaat tggcaacgct attatatgta agggaaatat 420
atacaaaaag aaaattaatc atagtcacct gactaagaaa ttctgactgc tagttgccat 480
aaataactca atggaaatat tcctatggga taatgtattt taagtga 527

<210> 183
<211> 530
<212> DNA
<213> Homo sapiens

<400> 183
atacatacat gtaacttcat tattttaaaa atatttttag aactccaata ctcaccctgt 60
tatgtcttgc taatttaaat tttgctaatt aactgaaaca tgcttaccag attcacactg 120
ttccagtgtc tataaaagaa acactttgaa gtctataaaa aataaaataa ttataaatat 180
cattgtacat agcatgttta tatctgcaaa aaacctaata gctaattaat ctggaatatg 240

caacattgtc cttaattgat gcaaataaca caaatgctca aagaaatcta ctatatccct 300
taatgaaata catcattctt catatatttc tccttcagtc cattccctta ggcaattttt 360
aatttttaaa aattattatc aggggagaaa aattggcaaa actattatat gtaagggaaa 420
tatatacaaa aagaaaatta atcatagtca cctgactaag aaattctgac tgctagttgc 480
cataaataac tcaatggaaa tattcctatg ggataatgta ttttaagtga 530

<210> 184
<211> 253
<212> DNA
<213> Homo sapiens

<220>
<221> misc_feature
<222> 98, 141, 162, 213
<223> n = A,T,C or G

<400> 184
tatacataca tgtaacttca ttattttaaa aatattttta gaactccaat actcaccctg 60
ttatgtcttg ctaatttaaa ttttgctaat taactganac atgcttacca gattcacact 120
gttccagtgt ctataaaaga nacactttga agtctataaa anataaaata attataaata 180
tcattgtaca tagcatgttt atatctgcaa aanacctaat agctaattaa tctggaatat 240
gcaacattgt cct 253

<210> 185
<211> 421
<212> DNA
<213> Homo sapiens

<400> 185
ccgttgctgt cgatcccagc tccttgggag gctgaggcgg gagaattgcg ggaaggcggg 60
gacggaggtt gcagtgagcc gagatcgcac tgctgtaccc agcctgggcc acagtgcaag 120
actccatctc aaaaaaaaaa gaaaagaaaa agcctgttta atgcacaggt gtgagtggat 180
tgcttatggc tatgagatag gttgatctcg cccttacccc ggggtctggt gtatgctgtg 240
ctttcctcag cagtatggct ctgacatctc ttaaatgtcc caacttcagc tgttgggaga 300
tggtgatatt ttcaacccta cttcctaaac atctgtctgg ggttccttta gtcttgaatg 360
tcttatgctc aattatttgg tgttgagcct ctcttccaca agagctcctc catgtttgga 420
t 421

<210> 186
<211> 377
<212> DNA
<213> Homo sapiens

<400> 186
cagctccttg ggaggctgag gcgggagaat tgcttgaacc cggggacgga ggttgcagtg 60
agccgagatc gcactgctgt acccagcctg ggccacagtg caagactcca tctcaaaaaa 120
aaaagaaaag aaaaagcctg tttaatgcac aggtgtgagt ggattgctta tggctatgag 180
ataggttgat ctcgccctta ccccggggtc tggtgtatgc tgtgctttcc tcagcagtat 240
ggctctgaca tctcttagat gtcccaactt cagctgttgg gagatggtga tattttcaac 300
cctacttcct aaacatctgt ctggggttcc tttagtcttg aatgtcttat gctcaattat 360
ttggtgttga gcctctc 377

<210> 187
<211> 243
<212> DNA
<213> Homo sapiens

<220>
<221> misc_feature
<222> 228
<223> n = A,T,C or G

<400> 187
gaggtattcc acctcctacc ggaatataat taaagggaga aatacactgt atgaagtata 60
tgttgatact atgacatgtt gccaacacct tgagaagcat tatttgtttc taataaaagt 120
aatggctttg tcaatatatt ggtgggttta aaactttgct gcttttttac ataaagcctg 180
tgcctttcct agaaagttaa gatgtaaatg tattctcaca tgtaaatntg aaagttcagg 240
ggt 243

<210> 188
<211> 544
<212> DNA
<213> Homo sapiens

<400> 188
tattccacct cctaccggaa tataattaaa gggagaaata cactgtatga agtatatgtt 60
gatactatga catgttgcca acaccttgag aagcattatt tgtttctaat aaaagtaatg 120
gctttgtcaa tatattggtg ggtttaaaac tttgctgctt ttttacataa agcctgtgcc 180
tttcctagaa agttaagatg taaatgtatt ctcacatgta aatttgaaag ttcaggggtc 240
tattatgaaa tgatacacat ttttaaatga accataattt ttttcactaa gctgtttgcc 300
ttccaaagtg tttacacctt aagccttaac atgtatcttc attcagaaaa cagttatatt 360
gtcataccat agtaggaaga aaaaccttta tttggaatat acactactgt aagtttgtac 420
agatcatata cctaccacct gtctttgctt aaagagcctt gattacataa atatgtagga 480
aaaaacatat tgagttcaaa atttatatct aacattgttt atgttatgat ttttttttaa 540
ttgc 544

<210> 189
<211> 244
<212> DNA
<213> Homo sapiens

<220>
<221> misc_feature
<222> 210
<223> n = A,T,C or G

<400> 189
cacaaaaggt atgatcagca acttgcttgg gaaaggagcc gtggaccagc tgacacggct 60
ggtgctggtg aatgccctct acttcaacgg ccagtggaag actcccttcc ccgactccag 120
cacccaccgc cgcctcttcc acaaatcaga cggcagcact gtctctgtgc ccatgatggc 180
tcagaccaac aagttcaact atactgagtn caccacgccc gatggccatt atacgacatc 240
ctgg 244

<210> 190
<211> 209
<212> DNA
<213> Homo sapiens

<220>
<221> misc_feature
<222> 140
<223> n = A,T,C or G

<400> 190
gaacactgtt gctcttggtg gacgggccca gaggaattca gagttaaacc ttgagtgcct 60
gcgtccgtga gaattcagca tggaatgtct ctactatttc ctgggatttc tgctcctggc 120
tgcaagattg ccacttgatn ccgccaaacg atttcatgat gtgctgggca atgaaagacc 180
ttctgcttac atgagggagc acaatcaat 209

<210> 191
<211> 254
<212> DNA
<213> Homo sapiens

<220>
<221> misc_feature
<222> 85, 100, 143, 155, 182, 203, 229, 245, 254
<223> n = A,T,C or G

<400> 191
ctcccaacca agctctcttg aggatcttga aggaaactga attcaaaaag atcaaagtcc 60
tgggctccgg tgcgttcggc acggngtata agggactctn gatcccagaa ggtgagaaag 120
ttaaaattcc cgtcgctatc aangaattaa gagangcaac atctccgaaa gccaacaagg 180
anatcctcga tgaagcctac gtnatggcca gcgtggacaa cccccacgng tgccgcctgc 240
tgggnatctg tctn 254

<210> 192
<211> 484
<212> DNA
<213> Homo sapiens

<400> 192
tttttttttt tttttttttc aaatatacct ctttgaaaga taaatttctg ctcaaaggga 60
caatattctt gctggatgcg ttcctgtaaa tgcttcacag tttgaagaca aaggaatgca 120
acttcccaaa atgtgcccga ggtggaagta cttcctggct agtcggtgta aacgttgcaa 180
aaccagtctg tgggtctaag agctaatgcg ggcatggctg ttgggatgga ggacctgctg 240
tggcttggtc ctgggtatcg aaagagtctg gatttttagg gctcatacta tcctccgtgg 300
tcatactcca ataaattcac tgctttgtgg cgcgaccctt aggtattctg cattttcagc 360
tgtggagccc ttaaagatgc catttggctt ggcttccttg ggaaagaagt cctgctggta 420
gtcagggttg tccaggctaa tttggtggct gcctttctgg gcccagtggg cagggctgtc 480
gaat 484

<210> 193
<211> 660
<212> DNA
<213> Homo sapiens

<400> 193
tttaatcata tccaggagtt tgcaagaaac aggtgcttaa cactaattca cctcctgaac 60
aagaaaaatg ggctgtgacc ggaactgtgg gctcatcgct ggggctgtca ttggtgctgt 120
cctggctgtg tttggaggta ttctaatgcc agttggagac ctgcttatcc agaagacaat 180
taaaaagcaa gttgtcctcg aagaaggtac aattgctttt aaaaattggg ttaaaacagg 240
cacagaagtt tacagacagt tttggatctt tgatgtgcaa aatccacagg aagtgatgat 300
gaacagcagc aacattcaag ttaagcaaag aggtccttat acgtacagag ttcgttttct 360
agccaaggaa aatgtaaccc aggacgctga ggacaacaca gtctctttcc tgcagcccaa 420
tggtgccatc ttcgaacctt cactatcagt tggaacagag gctgacaact tcacagttct 480
caatctggct gtggcagctg catcccatat ctatcaaaat caatttgttc aaatgatcct 540
caattcactt attaacaagt caaaatcttc tatgttccaa gtcagaactt tgagagaact 600
gttatggggc tatagggatc catttttgag tttggttccg taccctgtta ctaccacagt 660

<210> 194
<211> 277
<212> DNA
<213> Homo sapiens

<400> 194
ctttaatcat atccaggagt ttgcaagaaa caggtgctta acactaattc acctcctgaa 60
caagaaaaat gggctgtgac cggaactgtg ggctcatcgc tggggctgtc attggtgctg 120
tcctggctgt gtttggaggt attctaatgc cagttggaga cctgcttatc cagaagacaa 180
ttaaaaagca agttgtcctc gaagaaggta caattgcttt taaaaattgg gttaaaacag 240
gcacagaagt ttacagacag ttttggatct ttgatgt 277

<210> 195
<211> 457
<212> DNA

<213> Homo sapiens

<400> 195
gactgggttt gggtgcagac gttgttgctt gggcgcttct ccgctgcgtg taggtgaagg 60
gggcttcctg accgagacat ggatttaggt gctattacaa aatactcagc attacacgcc 120
aagcccaatg gactgatcct tcaatacggg actgctggat ttcgaacgaa ggcagaacat 180
cttgatcatg tcatgtttcg catgggatta ttagctgtcc tgaggtcaaa acagacaaaa 240
tccactatag gagtcatggt aacagcgtcc cacaatcctg aggaagacaa tggtgtaaaa 300
ttggttgatc ctttgggtga aatgttggca ccatcctggg aggaacatgc cacctgttta 360
gcaaatgctg aggaacaaga tatgcagaga gtgcttattg acatcagcga gaaagaagct 420
gtgaatctgc aacaagatgc ctttgtagtt attggta 457

<210> 196
<211> 361
<212> DNA
<213> Homo sapiens

<400> 196
tttttttttt tttttttttt tttgggcagg agaccatgtt actttattca tttgtttaac 60
tttaaccatg ttcaataaac ttttcacctg tttggtgagt tccacaaaag ccttagagag 120
tttctggtag taaccttcta tagttgcctt tccatatcgg ccacccgtgt ttcgacaata 180
caccatgtag tgcagctggg gtgttgttaa caagccataa tcatggaatt gacctcctag 240
aacagtcaca ccatctatta cagattgtga aagtttctca ctgctgggcc tggtatctct 300
accaataact acaaaggcat cttgttgcag attcacagct tctttctcgc tgatgtcaat 360
a 361

<210> 197
<211> 551
<212> DNA
<213> Homo sapiens

<400> 197
gagccgagct gatttgatcg aggagcgcgg ttaccggacg ggctgggtct atggtcgctc 60
cgcgggccgc tccgccggct ggtgcttttt tatcagggca agctgtgttc catggcaggg 120
aacttttggc agatctccca ctatttgcaa tggattttgg ataaacaaga tctgttgaag 180
gagcgccaaa aggatttaaa gtttctctca gaggaagaat attggaagtt acaaatattt 240
tttacaaatg ttatccaagc attaggtgaa catcttaaat taagacaaca agttattgcc 300
actgctacgg tatatttcaa gagattctat gccaggtatt ctctgaaaag tatagatcct 360
gtattaatgg ctcctacatg tgtgtttttg gcatccaaag tagaggaatt tggagtagtt 420
tcaaatacaa gattgattgc tgctgctact tctgtattaa aaactagatt ttcatatgcc 480
tttccaaagg aatttcctta taggatgaat catatattag aatgtgaatt ctatctgtta 540
gaactaatgg a 551

<210> 198
<211> 637
<212> DNA
<213> Homo sapiens

<400> 198
tacggccggg agtcgagccg agctgatttg atcgaggagc gcggttaccg gacgggctgg 60
gtctatggtc gctccgcggg ccgctccgcc ggctggtgct tttttatcag ggcaagctgt 120
gttccatggc agggaacttt tggcagagct cccactattt gcaatggatt ttggataaac 180
aagatctgtt gaaggagcgc caaaaggatt taaagtttct ctcagaggaa gaatattgga 240
agttacaaat attttttaca aatgttatcc aagcattagg tgaacatctt aaattaagac 300
aacaagttat tgccactgct acggtatatt tcaagagatt ctatgccagg tattctctga 360
aaagtataga tcctgtatta atggctccta catgtgtgtt tttggcatcc aaagtagagg 420
aatttggagt agtttcaaat acaagattga ttgctgctgc tacttctgta ttaaaaacta 480
gattttcata tgcctttcca aaggaatttc cttataggat gaatcatata ttagaatgtg 540
aattctatct gttagaacta atggattgtt gcttgatagt gtatcatcct tatagacctt 600
tgctccagta tgtgcaggac atgggccaag aagacat 637

<210> 199

<211> 130
<212> DNA
<213> Homo sapiens

<400> 199
tagaaagcct ccacctggag tacaatgccc tcaaggtcct tcacaatggc accctggctg 60
agttgcaagg tctaccccac attagggttt tcctggacaa caatccctgg gtctgcgact 120
gccacatggc 130

<210> 200
<211> 372
<212> DNA
<213> Homo sapiens

<220>
<221> misc_feature
<222> 29, 100, 297, 298, 353, 357
<223> n = A,T,C or G

<400> 200
gtgctgtttg accaatggtc atgtggccna gattggggac ttcgggctgg ctagggacat 60
catgaatgac tccaactaca ttgtcaaggg caatgccgcn ctgcctgtga agtggatggc 120
cccagagagc atctttgact gtgtctacac ggttcagagc gacgtctggt cctatggcat 180
cctcctctgg gagatcttct cacttgggct gaatccctac cctggcatcc tggtgaacag 240
caagttctat aaactggtga aggatggata ccaaatggcc cagcctgcat ttgcccnnaa 300
gaatatatac agcatcatgc aggcctgctg ggcttgggag cccacccaca ganccanctt 360
ccagcagatc tg 372

<210> 201
<211> 478
<212> DNA
<213> Homo sapiens

<220>
<221> misc_feature
<222> 3, 10, 11, 78, 112, 130, 150, 231, 457
<223> n = A,T,C or G

<400> 201
gancacctgn nacaaggagg atggacggcc cctggagctc cgggacctgc ttcacttctc 60
cagccaagta gcccaggnat ggccttcctc gcttccaaga attgcatcca cngggacgtg 120
gcagcgcgtn acgtgctgtt gaccaatggn catgtggcca agattgggga cttcgggctg 180
gctagggaca tcatgaatga ctccaactac attgtcaagg gcaatgccgc nctgcctgtg 240
aagtggatgg ccccagagag catctttgac tgtgtctaca cggttcagag cgacgtctgg 300
tcctatggca tcctcctctg ggagatcttc tcacttgggc tgaatcccta ccctggcatc 360
ctggtgaaca gcaagttcta taaactgggt gaaaggatgg ataccaaatg gcccagcctg 420
cattttgccc ccaaagaata tatacaagca tccatgnagg cccttctggg ccttggag 478

<210> 202
<211> 218
<212> DNA
<213> Homo sapiens

<400> 202
gcgagcaagg ggatatcgcc cagcccttgc tgcagcccaa caactatcag ttctgctgag 60
gagttgacga cagggagtac cactctcccc tcccacaaac ttcaactcct ccatggatgg 120
ggcgacacgg ggagaacata caaactctgc cttcggtcat ttcactcaac agctcggccc 180
agctctgaaa cttgggaagg tgagggattc aggggagg 218

<210> 203
<211> 556
<212> DNA

<213> Homo sapiens

<400> 203
taagctcgga attcggctcg aggcgagcaa ggggatatcg cccagccctt gctgcagccc 60
aacaactatc agttctgctg aggagttgac gacagggagt accactctcc cctcccacaa 120
acttcaactc ctccatggat ggggcgacac ggggagaaca tacaaactct gccttcggtc 180
atttcactca acagctcggc ccagctctga aacttgggaa ggtgagggat tcaggggagg 240
tcagaggatc ccacttcctg agcatgggcc atcactgcca gtcaggggct gggggctgag 300
ccctcacccc cccctcccct actgttctca tggtgttggc ctcgtgtttg ctatgccaac 360
tagtagaacc ttctttccta atccccttat cttcatggaa atggactgac tttatgccta 420
tgaagtcccc aggagctaca ctgatactga gaaaaccagg ctctttgggg ctagacagac 480
tggcagagag tgagatctcc ctctctgaga ggagcagcag atgctcacag accacactca 540
gctcaggccc cttgga 556

<210> 204
<211> 319
<212> DNA
<213> Homo sapiens

<400> 204
tccttattta tttaacttca cccgagttcc tctgggtttc taagcagtta tggtgatgac 60
ttagcgtcaa gacatttgct gaactcagca cattcgggac caatatatag tgggtacatc 120
aagtccatct gacaaaatgg ggcagaagag aaaggactca gtgtgtgatc cggtttcttt 180
ttgctcgccc ctgttttttg tagaatctct tcatgcttga catacctacc agtattattc 240
ccgacgacac atatacatat gagaatatac cttatttatt tttgtgtagg tgtctgcctt 300
cacaaatgtc atgtctact 319

<210> 205
<211> 456
<212> DNA
<213> Homo sapiens

<400> 205
attccgttgc tgtcgagggt cactaccagt acaagagcat ccctgtggag gacaaccaca 60
aggcagacat cagctcctgg ttcaacgagg ccattgactt catagactcc atcaagaatg 120
ctggaggaag ggtgtttgtc cactgccagg caggcatttc ccggtcagcc accatctgcc 180
tggcttacct tatgaggact aatcgagtca agctggacga ggcctttgag tttgtgaagc 240
agaggcgaag catcatctct cccaacttca gcttcatggg ccagctgctg cagtttgagt 300
cccaggtgct ggctccgcac tgttcggcag aggctgggag ccccgccatg gctgtgctcg 360
accgaggcac ctccaccacc accgtgttca acttccccgt ctccatccct gtccactcca 420
cgaacagtgc gctgagctac cttcagagcc ccatta 456

<210> 206
<211> 533
<212> DNA
<213> Homo sapiens

<400> 206
agtttttaaa taatgaatat tatttaatac cacaacagaa ttatccccaa tttccaataa 60
gtcctatcat tgaaaattca aatataagtg aagaaaaaat tagtagatca acaatctaaa 120
caaatccctc ggttctaaga tacaatggat tccccatact ggaaggactc tgaggcttta 180
ttcccccact atgcatatct tatcatttta ttattataca cacatccatc ctaaactata 240
ctaaagccct tttcccatgc atggatggaa atggaagatt tttttttaac ttgttctaaa 300
agtcttaata tgggctgttg ccatgaaggc ttgcagaatt gagtccattt tctagctgcc 360
tttattcaca tagtggacgg ggtacctaaa agtactgggg ttgactcaga gagtcgctgt 420
cattctgtca ttgctgctac tctaacactg agcaacactc tcccagtggc agatcccctg 480
tatcattcca agaggagcat tcatcccttt gctctaatga tcaggaatga tgc 533

<210> 207
<211> 246
<212> DNA
<213> Homo sapiens

<400> 207
aatgcactaa ctcaatacca agatgagttt ttaaataatg aatattattt aataccacaa 60
cagaattatc cccaatttcc aataagtcct atcattgaaa attcaaatat aagtgaagaa 120
aaaattagta gatcaacaat ctaaacaaat ccctcggttc taagatacaa tggattcccc 180
atactggaag gactctgagg ctttattccc ccactatgca tatcttatca ttttattatt 240
atacac 246

<210> 208
<211> 407
<212> DNA
<213> Homo sapiens

<400> 208
   ggccgccctt tttttttttt tttttttttt ttttttttgg gcaaaaaggg gctttttttt 60
   ttttcccccc cccttttttt aacccttccc ctaatatttc ccccaaaaaa aaaaattttt 120
   tttttttggg ggggggaaaa aaaagggaaa aaaaaccccc ccccccgggg ggggaaaaaa 180
   accccccaaa aacccccctt ttgggggggt ccccccccat gggggttccc cccccaattt 240
   ttttcccccc ccccaaaaaa tttttaaccc ccccccaagg gggtggaaaa ccttaaaaaa 300
   aaccccccgg aaaaaccaaa accccttttt taaaaaaaaa aaaaaaattt ttggggggca 360
   aaaccccccc cccccaaaaa accccccccc ccccccttaa aaaaaaa 407

<210> 209
<211> 359
<212> DNA
<213> Homo sapiens

<220>
<221> misc_feature
<222> 1, 53, 121, 123, 128, 133, 142, 150, 174, 179, 183, 186,
196, 200, 201, 204, 207, 212, 215, 218, 224, 229, 230, 231,
243, 244, 249, 260, 261, 267, 268, 270, 273, 279, 289, 291,
295, 301, 303, 305, 312, 315, 337, 345, 357
<223> n = A,T,C or G

<400> 209
ncggggactg cgcggcggtg cagagccggg cgtgggcgag aacgaacggg ctncctgcgg 60
ctgagagcgt cgagtgtcac catgggtatc acgcttggag cttcctaaag gacttcctgg 120
ncngggcntc gcnctgcccg tntccaagan cccggtcggc cccaatcgag aggncaaanc 180
tgntgntgaa ggtgcnagan nccnagnaac angtnaantc ttangaagnn ntacaaaggg 240
gtnnattant tttttggtan nattccnnan gancaaggnt ttcctttcnt nttgnagggt 300
nancntggca angtnattcc ttaatttccc aaccaangtt ttaantttgg ctttaangg 359

<210> 210
<211> 394
<212> DNA
<213> Homo sapiens

<400> 210
tttttttttt gcattaagtg gtctttattg atgtttcaca ttcagttatt atcaattctt 60
cagttaattg tacaagtatg ataaattatt ttctatttgc tgtgggaatt taaatgtaaa 120
ataaatacaa aatacatgtg tggtttaatg aacactcaat gaagcatctc ttctgaggta 180
ttcctttcag tctggtttta tcccaggatc tttttacttc ccctaggaat agtctattaa 240
accacacaat ggatctgtga acttgtagat caagttcact gtaaatctgt gaacttgtgt 300
tttaattaca ttagacatat tttttgatct catcatacaa caccaataca aaaggcaccg 360
cccatgcctc tcaggcacat tgggaccggg cacc 394

<210> 211
<211> 292
<212> DNA
<213> Homo sapiens

<400> 211
gggagcccac cagcaagaat gagttggagc aatcttttca tgtgacctcc ttaacagata 60
tttactgaag gaatctaggt tgtattttca gtggacaatg ggaataaagc atttctaaag 120
caccgactgg agaggaaggc aacagagaca aggagagaag ccgagagaca tgtctgcgtg 180
ctgccacgca tttgagcgat tgctctgtga agagttgtac actgaacact ttcaggggag 240
gctgtttacc caggcaatgt cctcaaacaa gcctgtgccg gggtgtcctg ga 292

<210> 212
<211> 495
<212> DNA
<213> Homo sapiens

<400> 212
aattccgttg ctgtcgctgc gcccaggtaa tttgagcaaa ggccacagtg aactccggcg 60
tggctgagga aggaggaggc acccacaggc tgctgggagg agagcataag gctcaaaatg 120
gaaaatcata aatccaataa taaggaaaac ataacaattg ttgatatatc cagaaaaatt 180
aaccagcttc cagaagcaga aaggaatcta cttgaaaatg gatcggttta tgttggatta 240
aatgctgctc tttgtggcct catagcaaac agtctttttc gacgcatctt gaatgtgaca 300
aaggctcgca tagctgctgg cttaccaatg gcagggatac cttttcttac aacagactta 360
acttacagat gttttgtaag ttttcctttg aatacaggtg atttggattg tgaaacctgt 420
accataacac ggagtggact gactggtctt gttattggtg gtctataccc tgttttcttg 480
gctatacctg taaat 495

<210> 213
<211> 358
<212> DNA
<213> Homo sapiens

<400> 213
tgcgaccgcg atctcctgca gctggtgcac cacctcggcg atggacagcc gctcctccgg 60
gttcacctgc agcatggcga ggatgaggct gtggaagacc gtgtactgcg tgtcgtgcgg 120
ggggatcgag tacttcccat tgactattcg aagtttcgct ccatcctcaa aagggtgctg 180
ccggaagcac agcaggtaca agatgcagcc cagggcccag atatcctgct tctcgccgat 240
cgggaagttg gaatacaagt ctatgatttc tggtgttcta tacattggtg ttgtattcct 300
cgtgatctga aaaaatacaa acatttcaaa ggaaaagttg catcccacaa acagtatt 358

<210> 214
<211> 406
<212> DNA
<213> Homo sapiens

<400> 214
tggtacgcct gcaggtaccg gtccggaatt cccgggtcga cccacgcgtc cgaggacatc 60
tggaatgtca ctggtgccca ggtgtacttg agctgtgagg tcatcggaat cccgacacct 120
gtcctcatct ggaacaaggt aaaaaggggt cactatggag ttcaaaggac agaactcctg 180
cctggtgacc gggacaacct ggccattcag acccggggtg gcccagaaaa gcatgaagta 240
actggctggg tgctggtatc tcctctaagt aaggaagatg ctggagaata tgagtgccat 300
gcatccaatt cccaaggaca ggcttcagca tcagcaaaaa ttacagtggt tgatgcctta 360
catgaaatac cagtgaaaaa aggtgaaggt gccgagctat aaacct 406

<210> 215
<211> 300
<212> DNA
<213> Homo sapiens

<220>
<221> misc_feature
<222> 66, 71, 259
<223> n = A,T,C or G

<400> 215
aggacatctg gaatgtcact ggtgcccagg tgtacttgag ctgtgaggtc atcggaatcc 60

cgacanctgt nctcatctgg aacaaggtaa aaaggggtca ctatggagtt caaaggacag 120
aacttctgcc tggtgaccgg gacaacctgg ccattcagac ccggggtggc ccagaaaagc 180
atgaagtaac tggctgggtg ctggtatctc ctctaagtaa ggaagatgct ggagaatatg 240
agtgccatgc atccaattnc caaggacagg cttcagcatc agcaaaaatt acagtggttg 300

<210> 216
<211> 232
<212> DNA
<213> Homo sapiens

<400> 216
ttcaaaagct tagagagaat aagcttcttg gtggtgaaat acaactctca cgtgtgctcc 60
agttctaaaa ttaacctgtg cctggtctct gaagcccttt cttgctctgt gcctttcagc 120
cacatcctta ggtgctaacg gccatgagct ccgactctcc aaagtgagct ccactttggg 180
tctgaggagc ccctggcaga gtccacgctg cctcaggtat catgggcgta at 232

<210> 217
<211> 453
<212> DNA
<213> Homo sapiens

<400> 217
ataagcttct tggtggtgaa actacaactc tcacgtgtgc tccagttcta aaattaacct 60
gtgcctggtc tctgaagccc tttcttgctc tgtgcctttc agccacatcc ttaggtgcta 120
acggccatga gctccgactc tccaaagtga gctccacttt gggtctgagg agcccctggc 180
agagtccacg ctgcctcagg tatcatgggc gtaatgatca cccaggctcc gggagatctc 240
atggatgatt actgtatgag acagagggga cttcagtctt tccagggcct tggtggaatt 300
tttggctctg gtgttttcgc cagacaataa acttacactg gaagctttga ttcaccctcc 360
acagtactcc agaaaggact gtcctataag ttgtacactt taaaaggtca tgtagaggtt 420
gtagtagaat ggcttttcac cctggtgact ttg 453

<210> 218
<211> 520
<212> DNA
<213> Homo sapiens

<400> 218
agatgtgtga gaagtgcccc acctgcccgg atgcatgcag caccaagaga gattgcgtcg 60
agtgcctgct gctccactct gggaaacctg acaaccagac ctgccacagc ctatgcaggg 120
atgaggtgat cacatgggtg gacaccatcg tgaaagatga ccaggaggct gtgctatgtt 180
tctacaaaac cgccaaggac tgcgtcatga tgttcaccta tgtggagctc cccagtggga 240
agtccaacct gaccgtcctc agggagccag agtgtggaaa cacccccaac gccatgacca 300
tcctcctggc tgtggtcggt agcctcctcc ttgttgggct tgcactcctg gctatctgga 360
agctgcttgt caccatccac gaccggaggg agtttgcaaa gtttcagagc gagcgatcca 420
gggcccgcta tgaaatggct tcaaatctat tatacagaaa gcctatctcc acgcacactg 480
tggacttcac cttcaacaag ttcaacaaat cctacaatgg 520

<210> 219
<211> 404
<212> DNA
<213> Homo sapiens

<400> 219
agatgtgtga gaagtgcccc acctgcccgg atgcatgcag caccaagaga gattgcgtcg 60
agtgcctgct gctccactct gggaaacctg acaaccagac ctgccacagc ctatgcaggg 120
atgaggtgat cacatgggtg gacaccatcg tgaaagatga ccaggaggct gtgctatgtt 180
tctacaaaac cgccaaggac tgcgtcatga tgttcaccta tgtggagctc cccagtggga 240
agtccaacct gaccgtcctc agggagccag agtgtggaaa cacccccaac gccatgacca 300
tcctcctggc tgtggtcggt agcctcctcc ttgttgggct tgcactcctg gctatctgga 360
agctgcttgt caccatccac gaccggaggg agtttgcaaa gttt 404

<210> 220
<211> 80
<212> DNA
<213> Homo sapiens

<400> 220
atggcttcaa atccattata cagaaagcct atctccacgc acactgtgga cttcaccttc 60
aacaagttca acaaatccta 80

<210> 221
<211> 607
<212> DNA
<213> Homo sapiens

<400> 221
tgccccacct gcccggatgc atgcagcacc aagagagatt gcgtcgagtg cctgctgctc 60
cactctggga aacctgacaa ccagacctgc cacagcctat gcagggatga ggtgatcaca 120
tgggtggaca ccatcgtgaa agatgaccag gaggctgtgc tatgtttcta caaaaccgcc 180
aaggactgcg tcatgatgtt cacctatgtg gagctcccca gtgggaagtc caacctgacc 240
gtcctcaggg agccagagtg tggaaacacc cccaacgcca tgaccatcct cctggctgtg 300
gtcggtagca tcctccttgt tgggcttgca ctcctggcta tctggaagct gcttgtcacc 360
atccacgacc ggagggagtt tgcaaagttt cagagcgagc gatccagggc ccgctatgaa 420
atggcttcaa atccattata cagaaagcct atctccacgc acactgtgga cttcaccttc 480
aacaagttca acaaatccta caatggcact gtggactgat gtttccttct ccgaggggct 540
ggagcgggga tctgatgaaa aggtcagact gaaacgcctt gcacggctgc tcggcttgat 600
cacaact 607

<210> 222
<211> 583
<212> DNA
<213> Homo sapiens

<400> 222
ggtatgtgcc atcacaagca gatgtggcag tatttgaagc cgtgtccagc ccaccgcctg 60
ccgacttgtg tcatgcccta cgttggtata atcacatcaa gtcttacgaa aaggaaaagg 120
ccagcctgcc aggagtgaag aaagctttgg gcaaatatgg tcctgccgat gtggaagaca 180
ctacaggaag tggagctaca gatagtaaag atgatgatga cattgacctc tttggatctg 240
atgatgagga ggaaagtgaa gaagcaaaga ggctaaggga agaacgtctt gcacaatatg 300
aatcaaagaa agccaaaaaa cctgcacttg ttgccaagtc ttccatctta ctagatgtga 360
aaccttggga tgatgagaca gatatggcga aattagagga gtgcgtcaga agcattcaag 420
cagacggctt agtctggggc tcatctaaac tagttccagt gggatacgga attaagaaac 480
ttcaaataca gtgtgtagtt gaagatgata aagttggaac agatatgctg gaggagcaga 540
tcactgcttt tgaggactat gtgcagtcca tggatgtggc tgc 583

<210> 223
<211> 296
<212> DNA
<213> Homo sapiens

<400> 223
tacatcgagg ggtatgtgcc atcacaagca gatgtggcag tatttgaagc cgtgtccagc 60
ccaccgcctg ccgacttgtg tcatgcccta cgttggtata atcacatcaa gtcttacgaa 120
aaggaaaagg ccagcctgcc aggagtgaag aaagctttgg gcaaatatgg tcctgccgat 180
gtggaagaca ctacaggaag tggagctaca gatagtaaag atgatgatga cattgacctc 240
tttggatctg atgatgagga ggaaagtgaa aaagcaaaga ggctaaggga agaacg 296

<210> 224
<211> 208
<212> DNA
<213> Homo sapiens

<220>

<221> misc_feature
<222> 97
<223> n = A,T,C or G

<400> 224
gactacatct tggacctgca gatcgccctg gactcgcatc ccactattgt cagcctgcat 60
caccagagac ccgggcagaa ccaggcgtcc aggacgncgc tgaccaccct caacacggat 120
atcagcatcc tgtccttgca ggcttctgaa ttcccttctg agttaatgtc aaatgacagc 180
aaagcactgt gtggctgaat aagcggtg 208

<210> 225
<211> 274
<212> DNA
<213> Homo sapiens

<220>
<221> misc_feature
<222> 133
<223> n = A,T,C or G

<400> 225
   gcagcggctg gagcggcaga tcagccagga tgtcaagctg gagccagaca tcctgcttcg 60
   ggccaagcaa gatttcctga agacggacag tgactcggac ctacagctct acaaggaaca 120
   gggtgagggg canggtgacc ggagcctgcg ggagcgtgat gtgctggaac gggagtttca 180
   gcgggtcacc atctctgggg aggagaagtg tggggtgccg ttcacagacc tgctggatgc 240
   agccaagatg tggtgcgggc gtcttcatcc ggga 274

<210> 226
<211> 330
<212> DNA
<213> Homo sapiens

<400> 226
ggccgccctt tttttttttt tttttttttg ggcccagggg gggccccctt gggaaaaaca 60
cccgggaaac ttcccaaagg ggccttgggg gaattttttt taaaaaaaaa ccttttttta 120
aaaaaaactt tgggatttaa attttttttc cggccccttt tttgggccgg gtaccccaat 180
ttaaaaaagg ggggcttttt aaaggttggg aaaaaaaaaa aattgggggg gcccaaaaaa 240
ttggggggcc cccaaaaaaa aagcggggtt tggaaaaatt ttgggggggt ttggaaattt 300
gggccccaaa acgggggacc cctttccccc 330

<210> 227
<211> 525
<212> DNA
<213> Homo sapiens

<400> 227
gaatttggcc ctcgaggcca agaattcggc acgagggttc acatagcaat ttaatcaagt 60
aatggttaat tagttacccc ctatatataa atatatgtaa tcaatttctt caaatagctt 120
gcttacatga taatcaatta gccaaccatg agtcatttag aatagtgata aatagaatac 180
acagaatagt gatgaaattc aatttaaaaa atcacgttag cctccaaacc atttaattca 240
aatgaaccca tcaactggat gccaactctg gcgaatgtag gacctctgag tggctgtata 300
attgttaatt caaatgaaat tcatttaaac agttgacaaa ctgtcattca acaattagct 360
ccaggaaata acagttattt catcataaaa cagtcccttc aaacacacaa ttgttctgct 420
gaagagttgt catcaacaat ccaatgctca cctattcagt tgctctgtgg tcagtgtggc 480
tgcataacag tggattccat gaaaggagtc attttagtga tgagc 525

<210> 228
<211> 788
<212> DNA
<213> Homo sapiens

<220>

<221> misc_feature
<222> 42, 44, 48, 49, 51, 52, 53, 54, 55, 57, 59, 61, 62, 63, 64,
68, 69, 70, 71, 73, 74, 75, 76, 77, 79, 80, 83, 87, 89,
92, 93, 94, 95, 97, 98, 107, 112, 113, 117, 122, 125, 127,
130, 131, 133, 671, 677, 685, 706, 713, 718, 725, 757, 771
<223> n = A,T,C or G

<221> misc_feature
<222> 783
<223> n = A,T,C or G

<400> 228
gttcacatag caatttaatc aagtaatcat taattagggg gngngggnng nnnnngngnt 60
nnnngtgnnn ngnnnnngnn ggngtgngng tnnnngnngg gaggtgngga anngttnttt 120
tntgngngan nantagaata cacagaatag tgatgaaatt caatttaaaa aatcacgtta 180
gcctccaaac catttaattc aaatgaaccc atcaactgga tgccaactct ggcgaatgta 240
ggacctctga gtggctgtat aattgttaat tcaaatgaaa ttcatttaaa cagttgacaa 300
actgtcattc aacaattagc tccaggaaat aacagttatt tcatcataaa acagtccctt 360
caaacacaca attgttctgc tgaagagttg tcatcaacaa tccaatgctc acctattcag 420
ttgctctgtg gtcagtgtgg ctgcataaca gtggattcca tgaaaggagt cattttagtg 480
atgagctgcc agtccattcc caggccaggc tgtcgctggc catccattca gtcgattcag 540
tcataggcga atctgttctg cccgaagctt gtggtcaagc aaaaattcag ccctgaaaat 600
cagcacatct gttcggtgga ctaaaccaca gttagttcgt caagcagcaa cccctgtggc 660
atgaccgcca ntgggtncat gcgtntgcac tgggagttgg ccaaanctcc ggnggtcncg 720
gggtnttttt tgtgggtttt ttttttttag tcttgtnttt gggtaagtgg nttttttttt 780
tcnttggg 788

<210> 229
<211> 156
<212> DNA
<213> Homo sapiens

<400> 229
gccgagggaa gggcccggca gctgaggagc cgctgagctt gctggacgac atgaaccact 60
gctactcccg cctgcgggaa ctggtacccg gagtcccgag aggcactcag cttagccagg 120
tggaaatcct acagcgcgtc atcgactaca ttctcg 156

<210> 230
<211> 538
<212> DNA
<213> Homo sapiens

<400> 230
tacgactcct atagggaatt tggccctcga ggccaagaat tcggcacgag ggtgactttg 60
gctttgctcg catcatcggc gagaagtcgt tccgccgctc agtggtgggc acgccggcct 120
acctggcacc cgaggtgctg ctcaaccagg gctacaaccg ctcgctggac atgtggtcag 180
tgggcgtgat catgtacgtc agcctcagcg gcaccttccc tttcaacgag gatgaggaca 240
tcaatgacca gatccagaac gccgccttca tgtacccggc cagcccctgg agccacatct 300
cagctggagc cattgacctc atcaacaacc tgctgcaggt gaagatgcgc aaacgctaca 360
gcgtggacaa atctctcagc cacccctggt tacaggagta ccagacgtgg ctggacctcc 420
gagagctgga ggggaagatg ggagagcgat acatcacgca tgagagtgac gacgcgcgct 480
gggagcagtt tgcagcagag catccgctgc ctgggtctgg gctgcccacg gacaggga 538

<210> 231
<211> 232
<212> DNA
<213> Homo sapiens

<220>
<221> misc_feature
<222> 18, 56, 94, 103, 117, 128, 145, 184, 204, 219
<223> n = A,T,C or G

<400> 231
tggctttgct cgcatcancg gcgagaagtc gtcccgccgc tcagtggtgg gcacgncggc 60
ctacctggca cccgaggtct tgctcaacca gggntacaac cgntcgctcg acatgtngtc 120
agtgggcntg atcatgtacg tcagnctcag cggcaccttc cctttcaacg aggatgagga 180
catnaatgac cagatccaga acgncgactt catgtaccng gccagaccct gg 232

<210> 232
<211> 420
<212> DNA
<213> Homo sapiens

<400> 232
taccggtccg gaattcccgg gtcgacccac gcgtccggcg tctctgctcc accaaggtgc 60
cctggacatg ctgaccaagg tgatggccct agagctcggg ccccacaaga tccgagtgaa 120
tgcagtaaac cccacagtgg tgatgacgtc catgggccag gccacctgga gtgaccccca 180
caaggccaag actatgctga accgaatccc acttggcaag tttgctgagg tagagcacgt 240
ggtgaacgcc atcctctttc tgctgagtga ccgaagtggc atgaccacgg gttccacttt 300
gccggtggaa gggggcttct gggcctgctg agctccctcc acacacctca agccccatgc 360
cgtgctcatc ctacccccaa tccctccaat aaacctgatt ctgctgccca aaaaaaaaaa 420

<210> 233
<211> 294
<212> DNA
<213> Homo sapiens

<220>
<221> misc_feature
<222> 2, 170
<223> n = A,T,C or G

<400> 233
gngtctactg ctccaccaag ggtgccctgg acatgctgac caaggtgatg gccctagagc 60
tcgggcccca caagatccga gtgaatgcag taaaccccac agtggtgatg acgtccatgg 120
gccaggccac ctggagtgac ccccacaagg ccaagactat gctgaaccgn atcccacttg 180
gcaagtttgc tgaggtagag cacgtggtga acgccatcct ctttctgctg agtgaccgaa 240
gtggcatgac cacgggttcc actttgccgg tggaaggggg ttctggggct gctg 294

<210> 234
<211> 55
<212> DNA
<213> Homo sapiens

<220>
<221> misc_feature
<222> 42
<223> n = A,T,C or G

<400> 234
gtctcggtcc atgactctgg agatccgaga aggaagaggc tntggcctga gaaag 55

<210> 235
<211> 394
<212> DNA
<213> Homo sapiens

<220>
<221> misc_feature
<222> 22, 335, 365, 377, 383, 391
<223> n = A,T,C or G

<400> 235
ttttttgttc atttatattt tntttaagag ctgtgcccag ttttatcatc tcacaagaat 60
gaagcaaggg acaaaggtaa gtgccacgct ccctggccac tgggttcctg gcaagctccc 120
agccactagg tgccaatctc ccttcaatgt actccttctt ccccagagtg cagaagcgta 180
tgaagacagt tatgacatgg acacatgcat gagctattat acataattac aaaagctgat 240
tctgtcatca ccacatcttg tctcatcagt aggagcgaat ggctggcggg acggtggcac 300
agtcagcctt gttcaaagtt ttgtcgatca cgggncctat attccagagt gacctttccc 360
agtgnccaac gttccanata ggncagggtc ntgc 394

<210> 236
<211> 468
<212> DNA
<213> Homo sapiens

<400> 236
agctcgggat tcggctcgag gacctggaaa ttccaggtgg tgagctgcat cgaaggggag 60
cctgggcccg tcaggagcgt cctcttcaac ccagacggct gctgcctgta cagcggctgc 120
caggactcac tgcgtgtcta cggctgggaa cctgagcggt gctttgatgt ggtcctcgtc 180
aactggggca aggtggccga cctggccatc tgcaatgacc agttgatagg tgtggccttc 240
tcccagagca acgtctcctc ctacgtggtg gatctgacgc gtgtcaccag gactggcacg 300
gtggcccggg accctgtgca ggaccaccgg cccctggcac agccactgcc caaccccagc 360
gcccccctcc ggcgcatcta tgagcggccc agcacaacct gcagcaagcc tcagagggtg 420
aagcagaact cagagagcga gcgccgcacc cccagcagcg aggatgac 468

<210> 237
<211> 254
<212> DNA
<213> Homo sapiens

<220>
<221> misc_feature
<222> 48, 85, 97
<223> n = A,T,C or G

<400> 237
gacctggaga agttccaggt ggtgagctgc atcgaagggg agcctggncc cgtcaggagc 60
gtcctcttca acccagacgg ctgcngcctg tacagcngct gccaggactc actgcgtgtc 120
tacggctggg aacctgagcg gtgctttgat gtggtcctcg tcaactgggg caaggtggcc 180
gacctggcca tctgcaatga ccagttgata ggtgtggcct tctcccagag caacgtctcc 240
tcctacgtgg tgga 254

<210> 238
<211> 419
<212> DNA
<213> Homo sapiens

<400> 238
gacccacgcg tccgtcttca acttctttag tcctcctgag attcctatga ttgggaagct 60
ggaaccacga gaagatgcta tcctggatga ggactttgaa attgggcaga ttttacatga 120
taatgtcatc ctgaaatcaa tctattacta tactggagaa gtcaatggta cctactatca 180
atttggcaaa cattatggaa acaagaaata cagaaaataa gtcaatctga aagatttttc 240
aagaatctta aaatctcaag aagtgaagca gattcataca gccttgaaaa aagtaaaacc 300
ctgacctgta acctgaacac tattattcct tatagtcaag tttttgtggt ttcttggtag 360
tctatatttt aaaaatagtc ctaaaaagtg tctaagtgcc agtttattct atctaggct 419

<210> 239
<211> 228
<212> DNA
<213> Homo sapiens

<220>
<221> misc_feature

<222> 190
<223> n = A,T,C or G

<400> 239
gaaccccgcc cgcggccaca gcgtctgctc cacctccagc ttgtacctgc aggatctgag 60
cgccgccgcc tcagagtgca tcgacccctc ggtggtcttc ccctaccctc tcaacgacag 120
cagctcgccc aagtcctgcg cctcgcaaga ctccagcgcc ttctctccgt cctcggattc 180
tctgcactcn tcgacggagt cctccccgca gggcagcccc gagcccct 228

<210> 240
<211> 525
<212> DNA
<213> Homo sapiens

<400> 240
aaccccgccc gcggccacag cgtctgctcc acctccagct tgtacctgca ggatctgagc 60
gccgccgcct cagagtgcat cgacccctcg gtggtcttcc cctaccctct caacgacagc 120
agctcgccca agtcctgcgc ctcgcaagac tccagcgcct tctctccgtc ctcggattct 180
ctgctctcct cgacggagtc ctccccgcag ggcagccccg agcccctggt gctccatgag 240
gagacaccgc ccaccaccag cagcgactct gaggaggaac aagaagatga ggaagaaatc 300
gatgttgttt ctgtggaaaa gaggcaggct cctggcaaaa ggtcagagtc tggatcacct 360
tctgctggag gccacagcaa acctcctcac agcccactgg tcctcaagag gtgccacgtc 420
tccacacatc agcacaacta cgcagcgcct ccctccactc ggaaggacta tcctgctgcc 480
aagagggtca agttggacag tgtcagagtc ctgagacaga tcagc 525

<210> 241
<211> 552
<212> DNA
<213> Homo sapiens

<400> 241
tggaaggaac tggtctgctc acacttgctg gcttgcgcat caggactggc tttatctcct 60
gactcacggt gcaaaggtgc actctgcgaa cgttaagtcc gtcccagcgc ttggaatcct 120
acggccccca cagccggatc ccctcagcct tccaggtcct caactcccgc ggacgctgaa 180
caatggcctc catggggcta caggtaatgg gcatcgcgct ggccgtcctg ggctggctgg 240
ccgtcatgct gtgctgcgcg ctgcccatgt ggcgcgtgac ggccttcatc ggcagcaaca 300
ttgtcacctc gcagaccatc tgggagggcc tatggatgaa ctgcgtggtg cagagcaccg 360
gccagatgca gtgcaaggtg tacgacttgc tgctggcact gccgcaggac ctgcaggcgg 420
cccgcgccct cgtcatcatc agcatcatcg tggctgctct gggcgtgctg ctgtccgtgg 480
tggggggcaa gtgtaccaac tgcctggagg atgaaagcgc caaggccaag accatgatcg 540
tggcgggcgt gg 552

<210> 242
<211> 519
<212> DNA
<213> Homo sapiens

<400> 242
tggaaggaac tggtctgctc acacttgctg gcttgcgcat caggactggc tttatctcct 60
gactcacggt gcaaaggtgc actctgcgaa cgttaagtcc gtcccagcgc ttggaatcct 120
acggccccca cagccggatc ccctcagcct tccaggtcct caactcccgc ggacgctgaa 180
caatggcctc catggggcta caggtaatgg gcatcgcgct ggccgtcctg ggctggctgg 240
ccgtcatgct gtgctgcgcg ctgcccatgt ggcgcgtgac ggccttcatc ggcagcaaca 300
ttgtcacctc gcagaccatc tgggagggcc tatggatgaa ctgcgtggtg cagagcaccg 360
gccagatgca gtgcaaggtg tacgacttgc tgctggcact gccgcaggac ctgcaggcgg 420
cccgcgccct cgtcatcatc agcatcatcg tggctgctct gggcgtgctg ctgtccgtgg 480
tggggggcaa gtgtaccaac tgcctggagg atgaaagcg 519

<210> 243
<211> 296
<212> DNA
<213> Homo sapiens

<220>
<221> misc_feature
<222> 64, 187, 195
<223> n = A,T,C or G

<400> 243
aggttcctca tctgctcgcg aggatgcctt ttctcttctg ccttgcgaaa taacagcagc 60
ctanctgttg cccgtgacca gtgagaaagg cagcgtcacg ggctgattag gtttcaccca 120
aagggtgccg gcgccgaatt ggtttctaac gagaactttt aaaatgatcc gttccaaaaa 180
agggtangag ccgcnagacc ctccaactgc ccagagaaaa caagtctcgt ctggcaaaat 240
tctcggccca cgcggtccgc ggccaagggg caaaggtcct cgccccacgt tgccga 296

<210> 244
<211> 273
<212> DNA
<213> Homo sapiens

<400> 244
cttgcccatg gcgaattgtg gatgactgtg gtggggcctt tacgatgggt accattggtg 60
gtggtatctt tcaagcaatc aaaggttttc gcaattctcc agtgggagta aaccacagac 120
tacgagggag tttgacagct attaaaacca gggctccaca gttaggaggt agctttgcag 180
tttggggagg gctgttttcc atgattgact gtagtatggt tcaagtcaga ggaaaggaag 240
atccctggaa ctccatcaca agtggtgcct taa 273

<210> 245
<211> 386
<212> DNA
<213> Homo sapiens

<400> 245
ttcgaattcg gcacgaggct cgatgtacgt cccggaggac ctccttcccg tctacaaaga 60
aaaagtggtg ccgcttgcag acattatcac gcccaaccag tttgaggccg agttactgag 120
tggccggaag atccacagcc aggaggaagc cttgcgggtg atggacatgc tgcactctat 180
gggccccgac accgtggtca tcaccagctc cgacctgccc tccccgcagg gcagcaacta 240
cctgattgtg ctggggagtc agaggaggag gaatcccgct ggctccgtgg tgatggaacg 300
catccggatg gacattcgca aagtggacgc cgtctttgtg ggcactgggg acctgtttgc 360
tggcatgctc ctggcgtgga cacaca 386

<210> 246
<211> 239
<212> DNA
<213> Homo sapiens

<400> 246
tttttttttt caaaaaagtc atggaggcca tggggttggc ttgaaaccag ctttgggggg 60
ttcgattcct tccttttttg cctaaatttt atgtatacgg gttcttcaaa tgtgtggtag 120
ggtggggggc atccatatag tcactccagg tttatggagg gttcttctac tattaggact 180
tttcgcttca aagcgaaggc ttctcaaatc atgaaaatta ttaatattac tgctgttaa 239

<210> 247
<211> 623
<212> DNA
<213> Homo sapiens

<400> 247
aaaaagtcat ggaggccatg gggttggctt gaaaccagct ttggggggtt cgattccttc 60
cttttttgtc tagattttat gtatacgggt tcttcgaatg tgtggtaggg tggggggcat 120
ccatatagtc actccaggtt tatggagggt tcttctacta ttaggacttt tcgcttcgaa 180
gcgaaggctt ctcaaatcat gaaaattatt aatattactg ctgttagaga aatgaatgag 240
cctacagatg ataggatgtt tcatgtggtg tatgcatcgg ggtagtccga gtaacgtcgg 300
ggcattccgg ataggccgag aaagtgttgt gggaagaaag ttagatttac gccgatgaat 360

atgatagtga aatggatttt ggcgtaggtt tggtctaggg tgtagcctga gaatagggga 420
aatcagtgaa tgaagcctcc tatgatggca aatacagctc ctattgatag gacatagtgg 480
aagtgagcta caacgtagta cgtgtcgtgt agtacgatgt ctagtgatga gtttgctaat 540
acaatgccag tcaggccacc tacggtgaaa agaaagatga atcctagggc tcagagcact 600
gcagcagatc atttcatatt gct 623

<210> 248
<211> 265
<212> DNA
<213> Homo sapiens

<400> 248
ggcttagcgg ataacaattt cacacaggag ttgcaccata atcatcgcta tccccaccgg 60
cgtcaaagta tttagctgac tcgccacact ccacggaagc aatatgaaat gatctgctgc 120
agtgctctga gccctaggat tcatctttct tttcaccgta ggtggcctga ctggcattgt 180
attagcaaac tcatcactag acatcgtact acacgacacg tactacgttg tagctcactt 240
ccactatgtc ctatcaatag gagct 265

<210> 249
<211> 625
<212> DNA
<213> Homo sapiens

<400> 249
aatcatcgct atccccaccg gcgtcaaagt atttagctga ctcgccacac tccacggaag 60
caatatgaaa tgatctgctg cagtgctctg agccctagga ttcatctttc ttttcaccgt 120
aggtggcctg actggcattg tattagcaaa ctcatcacta gacatcgtac tacacgacac 180
gtactacgtt gtagctcact tccactatgt cctatcaata ggagctgtat ttgccatcat 240
aggaggcttc attcactgat ttcccctatt ctcaggctac accctagacc aaacctacgc 300
caaaatccat ttcactatca tattcatcgg cgtaaatcta actttcttcc cacaacactt 360
tctcggccta tccggaatgc cccgacgtta ctcggactac cccgatgcat acaccacatg 420
aaacatccta tcatctgtag gctcattcat ttctctaaca gcagtaatat taataatttt 480
catgatttga gaagccttcg cttcgaagcg aaaagtccta atagtagaag aaccctccat 540
aaacctggag tgactatatg gatgcccccc accctaccac acattcgaag aacccgtata 600
cataaaatct agacaaaaaa ggaag 625

<210> 250
<211> 253
<212> DNA
<213> Homo sapiens

<400> 250
ggcttgtaat acgactcact atagggcttt ttttttttca aaaaagtcat ggaggccatg 60
gggttggctt gaaaccagct ttggggggtt cgattccttc cttttttgtc taaattttat 120
gtatacgggt tcttcaaatg tgtggtaggg tggggggcat ccatatagtc actccaggtt 180
tatggagggt tcttctacta ttaggacttt tcgcttcaaa gcgaaggctt ctcaaatcat 240
gaaaattatt aat 253

<210> 251
<211> 290
<212> DNA
<213> Homo sapiens

<400> 251
   caaactcatc actagacatc gtactacacg acacgtacta cgttgtagct cacttccact 60
   atgtcctatc aataggagct gtatttgcca tcataggagg cgtcattcac tgatttcccc 120
   tattctcagg ctacacccta gaccaaacct acgccaaaat ccatttcact atcatattca 180
   tcggcgtaaa tctaactttc ttcccacaac actttctcgg cctatccgga atgccccgac 240
   gttattcgga ctaccccgat gcatacacca catgaaacat cctatcatct 290

<210> 252
<211> 638

<212> DNA
<213> Homo sapiens

<220>
<221> misc_feature
<222> 522, 634, 636
<223> n = A,T,C or G

<400> 252
atatttacag taggaataga cgtagacaca cgagcatatt tcacctccgc taccataatc 60
atcgctatcc ccaccggcgt caaagtattt agctgactcg ccacactcca cggaagcaat 120
atgaaatgat ctgctgcagt gctctgagcc ctaggattca tctttctttt caccgtaggt 180
ggcctgactg gcattgtatt agcaaactca tcactagaca tcgtactaca cgacacgtac 240
tacgttgtag ctcacttcca ctatgtccta tcaataggag ctgtatttgc catcatagga 300
ggcttcattc actgatttcc cctattctca ggctacaccc tagaccaaac ctacgccaaa 360
atccatttca ctatcatatt catcggcgta aatctaactt tcttcccaca acactttctc 420
ggcctatccg gaatgccccg acgttattcg gactaccccg atgcatacac cacatgaaac 480
atcctatcat ctgtaggctc attcatttct ctaacagcag tnatattaat aattttcatg 540
atttgagaag ccttcgcttc gaagcgaaaa gtcctaatag tagaagaacc cttcataaac 600
ctggagtgac tatatggatg ccccccaccc tacnanca 638

<210> 253
<211> 531
<212> DNA
<213> Homo sapiens

<400> 253
ggcttagcgg ataacaattt cacacaggag ttgcaccata tatttacagt aggaatagac 60
gtagacacac gagcatattt cacctccgct accataatca tcgctatccc caccggcgtc 120
aaagtattta gctgactcgc cacactccac ggaagcaata tgaaatgatc tgctgcagtg 180
ctctgagccc taggattcat ctttcttttc accgtaggtg gcctgactgg cattgtatta 240
gcaaactcat cactagacat cgtactacac gacacgtact acgttgtagc tcacttccac 300
tatgtcctat caataggagc tgtatttgcc atcataggag gcttcattca ctgatttccc 360
ctattctcag gctacaccct agaccaaacc tacgccaaaa tccatttcac tatcatattc 420
atcggcgtaa atctaacttt cttcccacaa cactttctcg gcctatccgg aatgccccga 480
cgttactcgg actaccccga tgcatacacc acatgaaaca tcctatcatc t 531

<210> 254
<211> 625
<212> DNA
<213> Homo sapiens

<400> 254
atatttacag taggaataga cgtagacaca cgagcatatt tcacctccgc taccataatc 60
atcgctatcc ccaccggcgt caaagtattt agctgactcg ccacactcca cggaagcaat 120
atgaaatgat ctgctgcagt gctctgagcc ctaggattca tctttctttt caccgtaggt 180
ggcctgactg gcattgtatt agcaaactca tcactagaca tcgtactaca cgacacgtac 240
tacgttgtag ctcacttcca ctatgtccta tcaataggag ctgtatttgc catcatagga 300
ggcttcattc actgatttcc cctattctca ggctacaccc tagaccaaac ctacgccaaa 360
atccatttca ctatcatatt catcggcgta aatctaactt tcttcccaca acactttctc 420
ggcctatccg gaatgccccg acgttactcg gactaccccg atgcatacac cacatgaaac 480
atcctatcat ctgtaggctc attcatttct ctaacagcag taatattaat aattttcatg 540
atttgagaag tcttcgcttc gaagcgaaaa gtcctaatag tagaagaacc cttcataaac 600
ctggagtgac tatatggatg ccccc 625

<210> 255
<211> 217
<212> DNA
<213> Homo sapiens

<400> 255
   tttttttttt taaaaagtca tggaggccat ggggttggct tgaaaccacc tttggggggt 60
   tcaatccctt ccttctttgt ctaaatttta tgtatacggg ttcttcaaat gtgtggtagg 120
   ggggggggca tccatatagc ccctccaggt ttatggaggg ttcttctact attagaactt 180
   ttcccttcaa agcaaaggct tctcaaatca tgaaaat 217

<210> 256
<211> 636
<212> DNA
<213> Homo sapiens

<220>
<221> misc_feature
<222> 496, 562, 564, 605, 635
<223> n = A,T,C or G

<400> 256
aaagtcatgg aggccatggg gttggcttga aaccagcttt ggggggttcg attccttcct 60
tctttgtcta gattttatgt atacgggttc ttcgaatgtg tggtagggtg gggggcatcc 120
atatagtcac tccaggttta tggagggttc ttctactatt aggacttttc gcttcgaagc 180
gaaggcttct caaatcatga aaattattaa tattactgct gttagagaaa tgaatgagcc 240
tacagatgat aggatgtttc atgtggtgta tgcatcgggg tagtccgagt aacgtcgggg 300
cattccggat aggccgagaa agtgttgtgg gaagaaagtt agatttacgc cgatgaatat 360
gatagtgaaa tggattttgg cgtaggtttg gtctagggtg tagcctgaga ataggggaaa 420
tcagtgaatg aagcctccta tgatggcaaa tacagctcct attgatagga catagtggaa 480
gtgagctaca acgtantacg tgtcgtgtag tacgatgtct agtgatgagt ttgctaatac 540
aatgccagtc aggccaccta cngngaaaaa gaaagatgaa tcctagggct caaaacacct 600
gcacnagatc atttcatatt ggcttccgtg gagtnc 636

<210> 257
<211> 279
<212> DNA
<213> Homo sapiens

<400> 257
ggcttagcgg ataacaattt cacacaggag ttgcaccata atcatcgcta tccccaccgg 60
cgtcaaagta tttagctgac tcgccacact ccacggaagc aatatgaaat gatctgctgc 120
agtgctctga gccctaggat tcatctttct tttcaccgta ggtggcctga ctggcattgt 180
attagcaaac tcatcactag acatcgtact acacgacacg tactacgttg tagctcactt 240
ccactatgtc ctatcaatag gagctgtatt tgccatcat 279

<210> 258
<211> 623
<212> DNA
<213> Homo sapiens

<220>
<221> misc_feature
<222> 537
<223> n = A,T,C or G

<400> 258
aatcatcgct atccccaccg gcgtcaaagt atttagctga ctcgccacac tccacggaag 60
caatatgaaa tgatctgctg cagtgctctg agccctagga ttcatctttc ttttcaccgt 120
aggtggcctg actggcattg tattagcaaa ctcatcacta gacatcgtac tacacgacac 180
gtactacgtt gtagctcact tccactatgt cctatcaata ggagctgtat ttgccatcat 240
aggaggcttc attcactgat ttcccctatt ctcaggctac accctagacc aaacctacgc 300
caaaatccat ttcactatca tattcatcgg cgtaaatcta actttcttcc cacaacactt 360
tctcggccta tccggaatgc cccgacgtta ctcggactac cccgatgcat acaccacatg 420
aaacatccta tcatctgtag gctcattcat ttctctaaca gcagtaatat taataatttt 480
catgatttga gaagccttcg cttcgaagcg aaaagtccta atagtagaag aaccctncat 540
aaacctggag tgactatatg gatgcccccc accctaccac acattcgaag aacccgtata 600
cataaaatct agacaaaaaa gga 623

<210> 259
<211> 189
<212> DNA
<213> Homo sapiens

<220>
<221> misc_feature
<222> 170, 173
<223> n = A,T,C or G

<400> 259
tggcctttcc cccttcatgg gagacaacga taacgaaacc ttggccaacg ttacctcagc 60
cacctgggac ttcgacgacg aggcattcga tgagatctcc gacgatgcca aggatttcat 120
cagcaatctg ctgaagaaag atatgaaaaa ccgcctggac tgcacgcagn ctntcagcat 180
ccatggcta 189

<210> 260
<211> 507
<212> DNA
<213> Homo sapiens

<400> 260
cctttccccc ttcatgggag acaacgataa cgaaaccttg gccaacgtta cctcagccac 60
ctgggacttc gacgacgagg cattcgatga gatctccgac gatgccaagg atttcatcag 120
caatctgctg aagaaagata tgaaaaaccg cctggactgc acgcagtgcc ttcagcatcc 180
atggctaatg aaagatacca agaacatgga ggccaagaaa ctctccaagg accggatgaa 240
gaagtacatg gcaagaagga aatggcagaa aacgggcaat gctgtgagag ccattggaag 300
actgtcctct atggcaatga tctcagggct cagtggcagg aaatcctcaa cagggtcacc 360
aaccagcccg ctcaatgcag aaaaactaga atctgaagaa gatgtgtccc aagctttcct 420
tgaggctgtt gctgaggaaa agcctcatgt aaaaccctat ttctctaaga ccattcgcga 480
tttagaagtt gtggagggaa gtgctgc 507

<210> 261
<211> 193
<212> DNA
<213> Homo sapiens

<400> 261
tttttttttt tttttttttt ttttttggcc gagactccaa gactattatt tttatttccg 60
gacaaaaaca tctgcttcac acagtgcacg gcatcaaatg aagaggaaag aacttgtatc 120
ccaaagcctg gctttctgta tcatccacaa attaagacag catctgctga gcccatgctg 180
agcctgtcac agt 193

<210> 262
<211> 235
<212> DNA
<213> Homo sapiens

<220>
<221> misc_feature
<222> 183, 184, 185, 193
<223> n = A,T,C or G

<400> 262
cccacttccc caggagcagg ccacagaccc ccttgtggac agcctgggca gtggcattgt 60
ctactcagcc cttacctgcc acctgtgcgg ccacctgaaa cagtgtcatg gccaggagga 120
tggtggccag acccctgtca tggccagtcc ttgctgtggc tgctgctgtg gagacaggtc 180
ctnnncccct acnacccccc tgagggcccc agacccctct ccaggtgggg ttcca 235

<210> 263
<211> 493
<212> DNA

<213> Homo sapiens

<400> 263
agaatttcag cagttctctg atttttatat tttattcctc ttcctatcca atccctgcct 60
tttgagtcca ggtggtaagt acattttctt taacgttttt cctgcttttc ttcccaaatg 120
tgtctttttc tttgggctac tgtaccctgc ttccagtgct gtccccggca taggtccatc 180
tctgcagaag ccatttcagg agtacctgga ggctcaacgg cagaagcttc accacaaaag 240
cgaaatgggc acaccacagg gagaaaactg cttgtcctgg atgtttgaaa agtcggtcga 300
tgtcatggtg tgttacttca tcctatctat cattaactcc atggcacaaa gttatgccaa 360
acgaatccag cagcggttga actcagagga gaaaactaaa taagtagaga aagttttaaa 420
ctgcagaaat tggagtggat gggttctgcc ttatattggg aggactccaa gccgggaagg 480
aaaattccct ttt 493

<210> 264
<211> 345
<212> DNA
<213> Homo sapiens

<400> 264
agaatttcag cagttctctg atttttatat tttattcctc ttcctatcca atccctgcct 60
tttgagtcca ggtggtaagt acattttctt taacgttttt cctgcttttc ttcccaaatg 120
tgtctttttc tttgggctac tgtaccctgc ttccagtgct gtccccggca taggtccatc 180
tctgcagaag ccatttcagg agtacctgga ggctcaacgg cagaagcttc accacaaaag 240
cgaaatgggc acaccacagg gagaaaactg gttgtcctgg atgtttgaaa agtcggtcgt 300
tgtcatggtg tgttacttca tcctatctat cattaactcc atggt 345

<210> 265
<211> 374
<212> DNA
<213> Homo sapiens

<400> 265
tagaagagct aacctcacac tcatcccact ctaaactatg tgattcaaca ctgattttac 60
atccaacaaa gtgaaatctt gatagttggg tgtaaaaagg agagtaatgg agatttcaga 120
gtagttgggg ttgcttactt ttcattttta attctttagg ttttgtaagt tacacacttc 180
aagcattata gatgatcctc tttttactac tgaactaatg aagccttttt cattgcattg 240
ttctgcattt atttctacag ggagaaaact ggttgtcctg gatgtttgaa aagttggtcg 300
ttgtcatggt gtgttacttc atcctatcta tcattaactc catggcacaa agttatgcca 360
aacgaatcca gcag 374

<210> 266
<211> 360
<212> DNA
<213> Homo sapiens

<400> 266
   tttttttttt tttttttttg tgcggtggga attctctaat tgtatcatgt gggccttttg 60
   aaagtaacaa acagaaggcc agtctgctgc aagtttgctg ctgaacatca cattccaccc 120
   taagaaaaca caaggtggat tgcatcgagg gtggatacct taccttagca cggaaggaaa 180
   aagtatgtca gtgcaaagta tggactaaac tgctttcagg aaaaaagttg taaaaattga 240
   tacaggttgg aaaagggaat tttccttccc ggcttggagt cctcccaatt taaggcagaa 300
   cccatccact ccaatttctg cagtttaaaa ctttctctac ttatttagtt ttctcctctg 360

<210> 267
<211> 247
<212> DNA
<213> Homo sapiens

<400> 267
ctggaattgt catctttgga acagtgattg caacagcact tatgggattg acagagaaac 60
tgat[?]ttttc cctgagagat cctgcataca gtacattccc gccagaaggt gttttcgtaa 120

atacgcttgg ccttctgatc ctggtgttcg gggccctcat tttttggata gtcaccagac 180
cgcaatggaa acgtcctaag gagccaaatt ctaccattct tcatccaaat ggaggcatga 240
acaggga 247

<210> 268
<211> 350
<212> DNA
<213> Homo sapiens

<400> 268
   taatggattt gtttggagat ggcatgttgg tagacgactg aatatggaaa ggatatcaag 60
   ttatctattt tgttaatttt atttttgttt tttatcatct agatttttat catggattag 120
   tctgaaattt aaagttctgg ccagtcggtt ttctttcatc ttgtagtttt tacagtattt 180
   ccactgtgca tatgcaaaat gggtattaca taactgtatc atatttggta ttgataattt 240
   tttttttttt ttggaaacgg gtttttgttt tggcccagcc caaaaacatc ccttggttac 300
   cccttccggg gaaaaaaaac caaacccctt tttcggggaa aaaaaaaggg 350

<210> 269
<211> 455
<212> DNA
<213> Homo sapiens

<220>
<221> misc_feature
<222> 81, 195, 231, 247, 298, 307, 317, 395, 427, 446, 451
<223> n = A,T,C or G

<400> 269
ttttttttaa atcaaagagt agtttattaa aaaaggaatc aaacaggaaa ctctaagtac 60
cagtgtgtac attgtacaat nttaaatgac tcacgagaat gaagtttttt tcaaatatat 120
taagatcaca ccaccttgtt gtttatcgaa agatattcaa ggagaaagat ctgactctcc 180
aaactgcatc tgagnattgc cactttaaac aggacctcat ttcaaacatg ncaacaacgc 240
cactggntaa taaaggcttt gggaatgggg tgctcattct attatttcac tacaaacngc 300
atagganagg caggagnagt tggggaattt attctaaaat aggaatggga gggttgtcca 360
tctacagcag gcactccttc acttcctctg tttgnccttt ttaggcagta ctccttggtc 420
ggtcttngaa cggttttcca accctnttca ntggg 455

<210> 270
<211> 444
<212> DNA
<213> Homo sapiens

<220>
<221> misc_feature
<222> 17, 20, 391, 430
<223> n = A,T,C or G

<400> 270
ttttctgacg tctgttnctn aggctggaag aaatgagcag aaaacaaggg atgagtactt 60
tttagagtat gtgcatgtta cgtaatacct gtttctgggc aatgctgctt cttctgactc 120
aacaaatggg gagagcaaat tgaaaatgcg taaattggaa ggcaagttct gaaattaaac 180
gttgtacttt ggcctgatgt tctgaccttt aaggaagcaa gagtttgtaa acttccaaat 240
atttactatt ctgaactgcc gtgtaaacct gacgtattcc caagtcaaca taccagtata 300
ccaataggat gtgaataatg tttgtgttga gtttaaaacc atagcagttt tgctctggca 360
agtaatggaa agcgttctcg cttcctgagt ntgagctcca gcagactgca gagtggccag 420
tgccacagtn gtagcctgac tttc 444

<210> 271
<211> 502
<212> DNA
<213> Homo sapiens

<400> 271
ggttctgcgc tggtcggcgg agtagcaagt ggccatgggg agcctcagcg gtctgcgcct 60
ggcagcagga agctgtttta ggttatgtga aagagatgtt tcctcatctc taaggcttac 120
cagaagctct gatttgaaga gaataaatgg attttgcaca aaaccacagg aaagtcccgg 180
agctccatcc cgcacttaca acagagtgcc tttacacaaa cctacggatt ggcagaaaaa 240
gatcctcata tggtcaggtc gcttcaaaaa ggaaggtgaa atcccagaga ctgtctcgtt 300
ggagatgctt gatgctgcaa agaacaagat gcgagtgaag atcagctatc taatgattgc 360
cctgacggtg gtaggatgca tcttcatggt tattgagggc aagaaggctg cccaaagaca 420
cgagacttta acaagcttga acttagaaaa gaaagctcgt ctgaaagagg aagcagctat 480
gaaggccaaa acagagtagc ag 502

<210> 272
<211> 377
<212> DNA
<213> Homo sapiens

<400> 272
ggttctgcgc tggtcggcgg agtagcaagt ggccatgggg agcctcagcg gtctgcgcct 60
ggcagcagga agctgtttta ggttatgtga aagagatgtt tcctcatctc taaggcttac 120
cagaagctct gatttgaaga gaataaatgg attttgcaca aaaccacagg aaagtcccgg 180
agctccatcc cgcacttaca acagagtgcc tttacacaaa cctacggatt ggcagaaaaa 240
gatcctcata tggtcaggtc gcttcaaaaa ggaaggtgaa atcccagaga ctgtctcgtt 300
ggagatgctt gatgctgcaa agaacaagat gcgagtgaag atcagctatc taatgattgc 360
cctgacggtg gtaggaa 377

<210> 273
<211> 552
<212> DNA
<213> Homo sapiens

<400> 273
agctcggaat tcggctcgag tctgctcagc ctggtgaacc cacaggcccg agtttcaccc 60
agtccccact ccacggtgca gctgcggctt atctctcagc ccagcgagat gccagccttc 120
ctgtcccggg ccagcgctct gacatgcaga aggtgaccct gggcctgctt gtgttcctgg 180
caggctttcc tgtcctggac gccaatgacc tagaagataa aaacagtcct ttctactatg 240
actggcacag cctccaggtt ggcgggctca tctgcgctgg ggttctgtgc gccatgggca 300
tcatcatcgt catgagtgca aaatgcaaat gcaagtttgg ccagaagtcc ggtcaccatc 360
caggggagac tccacctctc atcaccccag gctcagccca aagctgatga ggacagacca 420
gctgaaattg ggtggaggac cgttctctgt ccccaggtcc tgtctctgca cagaaacttg 480
aactccagga tggaattctt cctcctctgc tgggactcct ttgcatggca gggcctcatc 540
tcacctctcg ca 552

<210> 274
<211> 186
<212> DNA
<213> Homo sapiens

<400> 274
ctgctcagcc tggtgaacac acagcccgat ttacccagtc cccactccag gtgcagctgc 60
ggcttatctc tcagcccagc gagatgccag ccttcctgtc ccgggccagc gctctgacat 120
gcagaaggtg accctgggcc tgcttgtgtt cctggcaggc tttcctgtcc tggacgccaa 180
tgacct 186

<210> 275
<211> 121
<212> DNA
<213> Homo sapiens

<400> 275
tctgctcagc ctggtgaacc acacaggccc gagtttcacc cagtccccac tccacggtgc 60
agctgcggct tatctctcag cccagcgaga tgccagcctt cctgtcccgg gccagcgctc 120
t 121

<210> 276
<211> 336
<212> DNA
<213> Homo sapiens

<220>
<221> misc_feature
<222> 336
<223> n = A,T,C or G

<400> 276
agggacccgc agctcagcta cagcacagat cagcaccatg aagcttctca cgggcctggt 60
tttctgctcc ttggtcctga gtgtcagcag ccgaagcttc ttttcgttcc ttggcgaggc 120
ttttgatggg gctcgggaca tgtggagagc ctactctgac atgagagaag ccaattacat 180
cggctcagac aaatacttcc atgctcgggg gaactatgat gctgccaaaa ggggacctgg 240
gggtgcctgg gccgcagaag tgatcagcaa tgccagagag aatatccaga gactcacagg 300
ccatggtgcg gaggactcgc tggccgatca ggctgn 336

<210> 277
<211> 460
<212> DNA
<213> Homo sapiens

<400> 277
tgcagacgga ggtcaggtct tcctctttcc tgagactgga tctgttcaaa cagcaaacgc 60
ccacagatgg cccagaggtg gtggtagtca gggtgtgtgg gtgtttttag ggttctttag 120
tgttgtttct ttcacccagg ggtggtggtc ccagccagtt tggtgctgac ggtgagagga 180
aattagaatc tgtttgcaaa ttgtccaacc caccccctca acatgagggg cttccatttt 240
ctgtgttttg taagggaact gtttccttca tgccgccatg ttcctgatat tagttctgat 300
ttctttttaa caaatgttat catgattaag aaaatttcca gcactttaat ggccaattaa 360
ctgagaatgt aagaaaattg atgctgtaca aggcaaataa agctgtttat taaccttgaa 420
aaaaaaaaaa aaaaaaaaaa aaaaaaaaaa ttttttgggg 460

<210> 278
<211> 432
<212> DNA
<213> Homo sapiens

<220>
<221> misc_feature
<222> 46, 151, 350, 362, 383, 403, 417
<223> n = A,T,C or G

<400> 278
ggggttgcag acggaggtca ggtcttcctc tttcctgaga ctgganctgt tcaaacagca 60
aacgcccaca gatggcccag aggtggtggt agtcagggtg tgtgggtgtt tttagggttc 120
tttagtgttg tttctttcac ccaggggtgg ntggtcccag ccagtttggt gctgacggtg 180
agaggaaatt agaatctgtt tgcaaattgt ccaacccacc ccctcaacat gaggggcttc 240
cattttctgt gttttgtaag ggaactgttt ccttcatgcc gccatgttcc tgatattagt 300
tctgatttct ttttaacaaa tgttatcatg attaagaaaa tttccagcan ttaatgggcc 360
anttaactga gaatgtaaga aantgatgct gttacaaggc aantaaagcc gttttantta 420
accctgaaaa aa 432

<210> 279
<211> 467
<212> DNA
<213> Homo sapiens

<400> 279
acgtgacgcg gggccaggcg gccgtacagc agctgcaggc ggagggcctg agcccgcgct 60
tccaccagct ggacatcgac gatctgcaga gcatccgcgc cctgcgcgac ttcctgcgca 120

aggagtacgg gggcctggac gtgctggtca acaacacggg catcgccttc aaggttgctg 180
atcccacacc ctttcatatt caagctgaag tgacgatgaa aacaaatttc tctggtaccc 240
gagatgtgtg cacagaatta ctccctctaa taaaacccca agggagagtg gtgaacgtac 300
ctagcatcat gagcgtcaga gcccttaaaa gctgcagccc agagctgcag cagaagttcc 360
gcagtgagac catcactgag gaggagctgg tggggctcat gaacaagttt gtggaggata 420
caaagaaggg agtgcaccag aaggagggct ggcccagcag cgcatac 467

<210> 280
<211> 626
<212> DNA
<213> Homo sapiens

<400> 280
tacggccggg acgtgacgcg gggccaggcg gccgtacagc agctgcaggc ggagggcctg 60
agcccgcgct tccaccagct ggacatcgac gatctgcaga gcatccgcgc cctgcgcgac 120
ttcctgcgca aggagtacgg gggcctggac gtgctggtca acaacacggg catcgccttc 180
aaggttgctg atcccacacc ctttcatatt caagctgaag tgacgatgaa aacaaatttc 240
tctggtaccc gagatgtgtg cacagaatta ctccctctaa taaaacccca agggagagtg 300
gtgaacgtac ctagcatcat gagcgtcaga gcccttaaaa gctgcagccc agagctgcag 360
cagaagttcc gcagtgagac catcactgag gaggagctgg tggggctcat gaacaagttt 420
gtggaggata caaagaaggg agtgcaccag aaggagggct ggcccagcag cgcatacggg 480
gtgacgaaga ttggcgtcac cgttctgtcc aggatccacg ccaggaaact gagtgagcag 540
aggaaagggg acaagatcct cctgaatgcc tgctgcccag ggtgggtgag aactgacatg 600
gcgggaccca aggccaccaa gagccc 626

<210> 281
<211> 487
<212> DNA
<213> Homo sapiens

<400> 281
tggcctgttc ctcagcgagg gcctgaagct agtggataag tttttggagg atgttaaaaa 60
gttgtaccac tcagaagcct tcactgtcaa cttcggggac accgaagagg ccaagaaaca 120
gatcaacgat tacgtggaga agggtactca agggaaaatt gtggatttgg tcaaggagct 180
tgacagagac acagtttttg ccctggtgaa ttacatcttc tttaaaggca aatgggagag 240
accctttgaa gtcaaggaca ccgaggaaga ggacttccac gtggaccagg cgaccaccgt 300
gaaggtgcct atgatgaagc gtttaggcat gtttaacatc cagcactgta agaagctgtc 360
cagctgggtg ctgctgatga aatacctggg caatgccacc gccatcttct tcctgcctga 420
tgaggggaaa ctacagcacc tggaaaatga actcacccac gatatcatca ccaagttcct 480
ggaaaat 487

<210> 282
<211> 345
<212> DNA
<213> Homo sapiens

<400> 282
tggcctgttc ctcagcgagg gcctgaagct agtggataag tttttggagg atgttaaaaa 60
gttgtaccac tcagaagcct tcactgtcaa cttcggggac accgaagagg ccaagaaaca 120
gatcaacgat tacgtggaga agggtactca agggaaaatt gtggatttgg tcaaggagct 180
tgacagagac acagtttttg ccctggtgaa ttacatcttc tttaaaggca aatgggagag 240
accctttgaa gtcaaggaca ccgaggaaga ggacttccac gtggaccagg cgaccaccgt 300
gaaggtgcct atgatgaagc gtttaggcat gtttaacatc cagca 345

<210> 283
<211> 495
<212> DNA
<213> Homo sapiens

<400> 283
cggccgccct tttttttttt tttttttttt tttttttttt tttttttttt tttttttttt 60
tttttttttc aaaaaaaaat ttttttgggt tttttttttt aaaacttttt tttttttttt 120

ttttgggggg ggccaaattc ccccccaaaa aaaaaaaaaa aggggggggt ttcccccccc 180
cccctttttt tttttggggg ggtttttttt tttggggggg gccccccccc cctttttttt 240
tttttggaaa aaaatccccc ccttgggggg ggtttctttt tcccaaaggg agtttttttt 300
cccccccccc cggggggggg ggggggtttt ttttttttta aaaaaaaaac ccccggaaaa 360
aaaaaaaccc cccccccccc cccccccccc aaaaaaaaaa aaggggggaa aaatgggggc 420
cccccctttt tttttttttt tttttttggg gggggggaaa aaaaaccccc cccccctttt 480
tgggggggtt ttttt 495

<210> 284
<211> 503
<212> DNA
<213> Homo sapiens

<400> 284
attccgttgc tgtcgagcat gaccaagcag ctgggtgact tctggacacg gatggaggag 60
ctccgccacc aagcccggca gcagggggca gaggcagtcc aggcccagca gcttgcggaa 120
ggtgccagcg agcaggcatt gagtgcccaa gagggatttg agagaataaa acaaaagtat 180
gctgagttga aggaccggtt gggtcagagt tccatgctgg gtgagcaggg tgcccggatc 240
cagagtgtga agacagaggc agaggagctg tttggggaga ccatggagat gatggacagg 300
atgaaagaca tggagttgga gctgctgcgg ggcagccagg ccatcatgct gcgctcagcg 360
gacctgacag gactggagaa gcgtgtggag cagatccgtg accacatcaa tgggcgcgtg 420
ctctactatg ccacctgcaa gtgatgctac agcttccagc ccgttgcccc actcatctgc 480
cgcctttgct tttggttggg ggc 503

<210> 285
<211> 581
<212> DNA
<213> Homo sapiens

<400> 285
agtggcactg caggaagctc aggacaccat gcaaggcacc agccgctccc ttcggcttat 60
ccaggacagg gttgctgagg ttcagcaggt actgcggcca gcagaaaagc tggtgacaag 120
catgaccaag cagctgggtg acttctggac acggatggag gagctccgcc accaagcccg 180
gcagcagggg gcagaggcag tccaggccca gcagcttgcg gaaggtgcca gcgagcaggc 240
attgagtgcc caagagggat ttgagagaat aaaacaaaag tatgctgagt tgaaggaccg 300
gttgggtcag agttccatgc tgggtgagca gggtgcccgg atccagagtg tgaagacaga 360
ggcagaggag ctgtttgggg agaccatgga gatgatggac aggatgaaag acatggagtt 420
ggagctgctg cggggcagcc aggccatcat gctgcgctca gcggacctga caggactgga 480
gaagcgtgtg gagcagatcc gtgaccacat caatgggcgc gtgctctact atgccacctg 540
caagtgatgc tacagcttcc agcccgttgc cccactcatc t 581

<210> 286
<211> 598
<212> DNA
<213> Homo sapiens

<400> 286
agtggcactg caggaagctc aggacaccat gcaaggcacc agccgctccc ttcggcttat 60
ccaggacagg gttgctgagg ttcagcaggt actgcggcca gcagaaaagc tggtgacaag 120
catgaccaag cagctgggtg acttctggac acggatggag gagctccgcc accaagcccg 180
gcagcagggg gcagaggcag tccaggccca gcagcttgcg gaaggtgcca gcgagcaggc 240
attgagtgcc caagagggat ttgagagaat aaaacaaaag tatgctgagt tgaaggaccg 300
gttgggtcag agttccatgc tgggtgagca gggtgcccgg atccagagtg tgaagacaga 360
ggcagaggag ctgtttgggg agaccatgga gatgatggac aggatgaaag acatggagtt 420
ggagctgctg cggggcagcc aggccatcat gctgcgctca gcggacctga caggactgga 480
gaagcgtgtg gagcagatcc gtgaccacat caatgggcgc gtgctctact atgccacctg 540
caagtgatgc tacagcttcc agcccgttgc cccactcatc tgccgccttt gcttttgg 598

<210> 287
<211> 316
<212> DNA
<213> Homo sapiens

<400> 287
ctgcccttca cctcgcagtg gacctgcaaa atcctgacct ggtgtcactc ctgttgaagt 60
gtggggctga tgtcaacaga gttacctacc agggctattc tccctaccag ctcacctggg 120
gccgcccaag cacccggata cagcagcagc tgggccagct gacactagaa aaccttcaga 180
tgctgccaga gagtgaggat gaggagagct atgacacaga gtcagagttc acggagttca 240
cagaggacga gctgccctat gatgactgtg tgtttggagg ccagcgtctg acgttatgag 300
cgcaaagggg ctgaaa 316

<210> 288
<211> 275
<212> DNA
<213> Homo sapiens

<400> 288
atgattagga gaagtggtgg ccacagtcga aaaatcccaa ggcccaaacc tgcaccactg 60
actgctgaaa tacagcaaaa gattttgcat ttgccaacat cttgggactg gagaaatgtt 120
catggtatca attttgtcag tcctgttcga aaccaagcat cctgtggcag ctgctactca 180
tttgcttcta tgggtatgct agaagcgaga atccgtatac taaccaacaa ttctcagacc 240
ccaatcctaa gccctcagga ggttgtgtct tgtag 275

<210> 289
<211> 522
<212> DNA
<213> Homo sapiens

<400> 289
cagaagggaa caccagagct ttgctaataa ttagtgtggt caagagccgt ctgagcctaa 60
tgagtcccag ctgcattagg ttaagagact cttccagagc catcgccagg tcgggaatgg 120
cacctctccc taggatacac agcctgcagg tccccaggac ctggatgaca cccgcctcac 180
tgtggcagtg tattgcctgt taattgctgc taattctaat tctgatgatg actcctactc 240
cattgtttac cccaaagcat cagctaggct ggagtgattt gttacaaatg agcaaaagat 300
gagtccttgc ttccctcaga aataaaagga gctcagctgc agcgttgcat tgggcttctt 360
ggcctcccaa ctcttcccac tcccagaatc cagaagtaag ctctgcatgt tccccttcct 420
gggaggaaac cagttgtcag aaggatgtat gatgaccccc tcccctccca tccttcacct 480
cctaagcagt cctggctttt cctcatcact cccctctaca gt 522

<210> 290
<211> 331
<212> DNA
<213> Homo sapiens

<400> 290
aacaccagag ctttgctaat aattagtgtg gtcaagagcc gtctgagcct aatgagtccc 60
agctgcatta ggttaagaga ctcttccaga gccatcgcca ggtcttgaat ggcacctctc 120
cctaggatac acagcctgca ggtccccagg acctggatga cacccgcctc actgtggcag 180
tgtattgcct gttaattgct gctaattcta attctgatga tgactcctac tccattgttt 240
accccaaagc atcagctagg ctggagtgat ttgttacaaa tgagcaaaag atgagtcctt 300
gcttccctca gaaataaaag gagctcagct g 331

<210> 291
<211> 228
<212> DNA
<213> Homo sapiens

<400> 291
gagatgcaaa gcaggattca aaagaacatc tttgcgtttt ctaccggctc cccatcatcg 60
tactagggag gaagaagcgg gtgagaaaca aaacttcttt ccattgtcct gcccgtttct 120
gcggacttgt tctgaggccg aggcacctct aagatactga tggctctgca gaggacccat 180
tcattgcttc tgcttttgct gctgaccctg ctggggctgg ggctggtc 228

<210> 292

<211> 342
<212> DNA
<213> Homo sapiens

<400> 292
ggagctgtcc tgcaccgtgg tggagctgaa gtacacaggc aatgccagcg cactcttcat 60
cctccctgat caagacaaga tggaggaagt ggaagccatg ctgctcccag agaccctgaa 120
gcggtggaga gactctctgg agttcagaga gataggtgag ctctacctgc caaagttttc 180
catctcgagg gactataacc tgaacgacat acttctccag ctgggcattg aggaagcctt 240
caccagcaag gctgacctgt cagggatcac aggggccagg aaccctacag tctcccaggt 300
ggtccataag gctgtgcttg atgtatttga ggagggcaca ga 342

<210> 293
<211> 311
<212> DNA
<213> Homo sapiens

<400> 293
ggagctgtcc tgcaccgtgg tggagctgaa gtacacaggc aatgccagcg cactcttcat 60
cctccctgat caagacaaga tggaggaagt ggaagccatg ctgctcccag agaccctgaa 120
gcggtggaga gactctctgg agttcacaga gataggtgag ctctacctgc caaagttttc 180
catctcgagg gactataacc tgaacgacat acttctccag ctgggcattg aggaagcctt 240
caccagcaag gctgacctgt cagggatcac aggggccagg aacctagcag tctcccaggt 300
ggtccataag g 311

<210> 294
<211> 402
<212> DNA
<213> Homo sapiens

<400> 294
cggctgcgag aagacgacag aagggaagat ggaggaagtg gaagccatgc tgctcccaga 60
gaccctgaag cggtggagag actctctgga gttcagagag ataggtgagc tctacctgcc 120
aaagttttcc atctcgaggg actataacct gaacgacgac ttctccagct gggcattgag 180
gaagccttca ccagcaaggc tgacctgtca gggatcacag gggccaggaa cctagcagtc 240
tcccaggtgg tccataaggc tgtgcttgat gtatttgagg agggcacaga agcatctgct 300
gccacagcag tcaaaatcac cctcctttct gcattagtgg agacaaggac cattgtgcgt 360
ttcaacaggc ccttcctgat gatcattgtg cctacagaca cc 402

<210> 295
<211> 232
<212> DNA
<213> Homo sapiens

<400> 295
ttccatctcg agggactata acctgaacga cgacttctcc agctgggcat tgaggaagcc 60
ttcaccagca aggctgacct gtcagggatc acaggggcca ggaacctagc agtctcccag 120
gtggtccata aggctgtgct tgatgtattt gaggagggca cagaagcatc tgctgccaca 180
gcagtcaaaa tcaccctcct ttctgcatta gtggagacaa ggaccattgt gc 232

<210> 296
<211> 435
<212> DNA
<213> Homo sapiens

<400> 296
tgactctgac ttctgaggaa gaggcccgtt tgaagaagag tgcacatcac tttgggggat 60
ccaaaaggag ctgcaatttt aaagtcttct gatgtcatat catttcactg tctaggctac 120
aacaggattc taggtggagg ttgtgcatgt tgtccttttt atctgatctg cgattaaagc 180
agtaatattt taagatggac tgggaaaaac atcaactcct gaagttagaa ataagaatgg 240
tttgtaaaat ccacagctat atcctgatgc tggatggtat taatcttgtg tagtcttcaa 300
ctggttagtg tgaaatagtt ctgccacctc tgacgcacca ctgccaatgc tgtacgtact 360

gcatttgccc cttgagccag gtggatgttt accgtgtgtt atataactta ctggctcctt 420
cactgaacat gccta 435

<210> 297
<211> 309
<212> DNA
<213> Homo sapiens

<400> 297
atcatttcac tgtctaggct acaacaggat tctaagggga cgttgtgcat gttggccttt 60
gtatctgatc tgtgattaaa gcagtaatat tttaagatgg actgggaaaa acatcaactc 120
ctgaagttag aaataagaat ggtttgtaaa atccacagct gtatgctgaa gctggatggt 180
attaatcttg cgtagtcttc aactggttag gtgaaatagt tctgccacct ctgacgcacc 240
actgccaatg ctgtacgtac tggatttggc ccttgagcca ggtggatgtt caccgggcgt 300
gatataact 309

<210> 298
<211> 342
<212> DNA
<213> Homo sapiens

<220>
<221> misc_feature
<222> 342
<223> n = A,T,C or G

<400> 298
atcatttgac tgtctaggct acaacaggat tctaggtgga ggttgtgcat gttgtccttt 60
ttatctgatc tgtgattaaa gcagtaatat tttaacatgg actgggaaaa acatcaactc 120
ctgaagttag aaataagaat ggtttgtaaa atccacagct atatcctgat gctggatggt 180
attaatcttg tgtagtcttc aactggttag ttgaaatagt tctgccacct ctgacgcacc 240
actgccaatg ctgtacgtac tgcatttgcc ccttgagcca ggtggatgtt taccgtgtgt 300
tatataactt cctggctcct tcactgaaca tgcctagtcc an 342

<210> 299
<211> 266
<212> DNA
<213> Homo sapiens

<400> 299
gggacagaat ggctatctcg gaccttgtga aggtgactct gacttctgag gaagacgccc 60
gcttgaagaa gagagcccat acactttggg ggatccaaaa cgagctgcga ttttcaagtc 120
ttctgatgtc atatcattcc actgtctagg ctacaacagg attctagggg gacgttgtgc 180
atgttggcct ttttatctga tctgtgacta aagcactaat attttaagat ggactgggaa 240
aaacatcaac tcctgaagtt agaaat 266

<210> 300
<211> 383
<212> DNA
<213> Homo sapiens

<400> 300
ggacagaatg gaatctcaga ccttgtgaag gtgactctga cttctgagga agaggcccgt 60
ttgaagaaga gtgcagatac actttggggg atccaaaagg agctgcaatt ttaaagtctt 120
ctgatgtcat atcatttcac tgtctaggct acaacaggat tctaggtgga ggttgtgcat 180
gttgaccttt ttatctgatc tgtgattaaa gcagtaatat tttaagatgg actgggaaaa 240
acatcaactc ctgaagttag aaataagaat ggtttgtaaa atccacagct atatcctgat 300
gctggatggt attaatcttg tgtagtcttc aactggttag tgtgaaatag ttctgccacc 360
tctgacgcac cactgccaat gct 383

<210> 301
<211> 453

<212> DNA
<213> Homo sapiens

<400> 301
aaccgcttct ccgttgaaca acatactaga tggggacaga atggaatctc agaccttgtg 60
aaggtgactc tgacttctga ggaagaggcc cgtttgaaga agagtgcaga tacactttgg 120
gggatccaaa aggagctgca attttaaagt cttctgatgt catatcattt cactgtctag 180
gctacaacag gattctaggt ggaggttgtg catgttgtcc tttttatctg atctgtgatt 240
aaagcagtaa tattttaaga tggactggga aaaacatcaa ctcctgaagt tagaaataag 300
aatggtttgt aaaatccaca gctatatcct gatgctggat ggtattaatc ttgtgtagtc 360
ttcaactggt tagtgtgaaa tagttctgcc acctctgacg caccactgcc aatgctgtac 420
gtactgcatt tgccccttga gccaggtgga tgt 453

<210> 302
<211> 383
<212> DNA
<213> Homo sapiens

<400> 302
ggacagaatg gaatctcaga ccttgtgaag gtgactctga cttctgagga agaggcccgt 60
ttgaagaaga gtgcagatac actttggggg atccaaaagg agctgcaatt ttaaagtctt 120
ctgatgtcat atcatttcac tgtctaggct acaacaggat tctaggtgga ggttgtgcat 180
gttgtccttt ttatctgatc tgtgattaaa gcagtaatat tttaagatgg actgggaaaa 240
acatcaactc ctgaagttag aaataagaat ggtttgtaaa atccacagct atatcctgat 300
gctggatggt attaatcttg tgtagtcttc aactggttag tgtgaaatag ttctgccacc 360
tctgacgcac cactgccaat gct 383

<210> 303
<211> 97
<212> DNA
<213> Homo sapiens

<400> 303
gttgccttgg agatgatcaa agtaactggt ggctatccat ttgaagctta caaaaattgt 60
tttcttaact tagccattcc aattgtagta tttacag 97

<210> 304
<211> 442
<212> DNA
<213> Homo sapiens

<400> 304
gccctagtta ttataccata ttacatcatt actctatgta attatctatg aagctatgta 60
gttatttacc cctgtattaa gtgattttag actgttgtta ttttttgagt tacagcatgt 120
gctttcaaaa tagggagact gtatggttga attaatattt ttttaaataa ctgttaacat 180
gtatagagta ggttgaaagt ttgaaagtat aaaatatact aaaagtatac agacctgtaa 240
taagaaattt atattactat agtcccatag ctgcttttac tatccacaga gaaatgcttg 300
aaaacgtgaa agttgaatag atgcaattaa aatcacggat agttttaggc tgtttatatt 360
atcagatcac cttcttttat ctaggttgcc ttggagatga tcaaagtaac tggtggctat 420
ccatttgaag cttacaaaaa tt 442

<210> 305
<211> 380
<212> DNA
<213> Homo sapiens

<400> 305
gagacgttcg cacacctggg tgccagcgcc ccagaggtcc cgggacagcc cgaggcgccg 60
cgcccgccgc cccgagctcc ccaagccttc gagagcggcg cacactcccg gtctccactc 120
gctcttccaa cacccgctcg ttttggcggc agctcgtgtc ccagagaccg agttgcccca 180
gagaccgaga cgccgccgct gcgaaggacc aatgagagcc ccgctgctac cgccggcgcc 240
ggtggtgctg tcgctcttga tactcggctc aggccattat gctgctggat tggacctcaa 300

tgacacctac tctgggaagc gtgaaccatt ttctggggac cacagggctg atggatttga 360
ggttacctcc agaagggagg 380

<210> 306
<211> 133
<212> DNA
<213> Homo sapiens

<400> 306
ccagtactgc ctcctgtgct cgtgccaaga cacagtgaat ataaccccca gctcagcctc 60
ctggccaagt tccgcagcgc ctccctgcac agtgagccac tcatgccaca caacgccacc 120
tatcctgact ctt 133

<210> 307
<211> 428
<212> DNA
<213> Homo sapiens

<400> 307
tccagtactg cctcctgtgc tcgtgccaag acacagtgaa tataaccccc agctcagcct 60
cctggccaag ttccgcagcg cctccctgca cagtgagcca ctcatgccac acaacgccac 120
ctatcctgac tctttccagc agcctccgtg ctctgcactc cctccctcac ccagccacgc 180
gttctcccag tccccgtgca cggccagcta ccctcactcc ccaggaagtc cttctgagcc 240
agagagtccc tatcaacact cagactttcg accagtttgt tacgaggagc ccccactggt 300
gctcggtcgc ctactatgaa ctgaacaacc gagttgggga gacattccag gcttcctccc 360
gaagtgtgct catagatggg ttcaccgacc cttcaaataa caggaacaga ttctgtcttg 420
gacttctt 428

<210> 308
<211> 497
<212> DNA
<213> Homo sapiens

<400> 308
cggctgcgag aagacgacag aaggggggaa tgtgtctggc ccttcagcag tttctcttgg 60
cagcatcagc tgggctgctt tctttgtgtg tggccccagg tgtcaaaatg acaccagctg 120
tctgtactag acaaggttac caagtgcgga attggttaat actaacagag agatttgctc 180
cattctcttt ggaataacag gacatgctgt atagatacag gcagtaggtt tgctctgtac 240
ccatgtgtac agcctaccca tgcagggact gggattcgag gacttccagg cgcatagggt 300
agaaccaaat gatagggtag gagcatgtgt tctttagggc cttgtaaggc tgtttccttt 360
tgcatctgga actgactata taattgtctt caatgaagac taattcaatt ttgcatatag 420
aggagccaaa gagagatttc agctctgtat ttgtggtatc agtttggaaa aaaaaaatct 480
gatactccat ttgatta 497

<210> 309
<211> 356
<212> DNA
<213> Homo sapiens

<400> 309
gggaatgtgt ctggcccttc agcagtttct cttggcagca tcagctgggc tgctttcttt 60
gtgtgtggcc ccaggtgtca aaatgacacc agctgtctgt actagacaag gttaccaagt 120
gcggaattgg ttaatactaa cagagagatt tgctccattc tctttggaat aacaggacat 180
gctgtataga tacaggcagt aggtttgctc tgtacccatg tgtacagcct acccatgcag 240
ggactgggat tcgaggactt ccaggcgcat agggtagaac caaatgatag ggtaggagca 300
tgtgttcttt aaggccttgt aaggctgttt ccttttgcat ctggaactga ctatat 356

<210> 310
<211> 348
<212> DNA
<213> Homo sapiens

<400> 310
gggaatgtgt ctggcccttc agcagtttct cttggcagca tcagctgggc tgctttcttt 60
gtgtgtggcc ccaggtgtca aaatgacacc agctgtctgt actagacaag gttaccaagt 120
gcggaattgg ttaatactaa cagagagatt tgctccattc tctttggaat aacaggacat 180
gctgtataga tacaggcagt aggtttgctc tgtacccatg tgtacagcct acccatgcag 240
ggactgggat tcgaggactt ccaggcgcat agggtagaac caaatgatag ggtaggagca 300
tgtgttcttt agggccttgt aaggctgttt ccttttgcat ctggaact 348

<210> 311
<211> 337
<212> DNA
<213> Homo sapiens

<400> 311
aagttgtggt ctgacacaca ctgctgtggt tcccctggat ttagtgaaat gccgtatgca 60
ggtggacccc caaaagtaca agggcatatt taacggattc tcagttacac ttaaagagga 120
tggtgttcgt ggtttggcta aaggatgggc tccgactttc cttggctact ccatgcaggg 180
actctgcaag tttggctttt atgaagtctt taaagtcttg tatagcaata tgcttggaga 240
ggagaatact tatctctggc gcacatcact atatttggct gcctctgcca gtgctgaatt 300
ctttgctgac attgccctgg ctcctatgga agctgct 337

<210> 312
<211> 252
<212> DNA
<213> Homo sapiens

<220>
<221> misc_feature
<222> 144
<223> n = A,T,C or G

<400> 312
agcccaagcc ctcagtggaa cctgtcaaga gcatcagcag catggagctg aagaccgagc 60
cctttgatga cttcctgttc ccagtgacac ttcagagagc tggtagttag tagcatgttg 120
agccaggcct gggtctgtgt ctcntttctc tttctcctta gtcttctcat agcattaact 180
aatctattgg gttcattatt ggaattaacc tggtgctgga tattttcaaa ttgtatctag 240
tgcagctgat tt 252

<210> 313
<211> 51
<212> DNA
<213> Homo sapiens

<400> 313
actcccagct gcactggtta cacgtcttcc ttcgtcttca cctaccccga g 51

<210> 314
<211> 348
<212> DNA
<213> Homo sapiens

<400> 314
atggccacag agctggagcc cctgtgcact ccggtggtca cctgtactcc cagctgcact 60
gcttacacgt cttccttcgt cttcacctac cccgaggctg actccttccc cagctgtgca 120
gctgcccacc gcaagggcag cagcagcaat gagccttcct ctgactcgct cagctcaccc 180
acgctgctgg ccctgtgagg gggcagggaa ggggaggcag ccggcaccca caagtgccac 240
tgcccgagct ggtgcattac agagaggaga aacacatctt ccctagaggg ttcctgtaga 300
cctagggagg accttatctg tgcgtgaaac acaccaggct gtgggccc 348

<210> 315
<211> 507
<212> DNA

<213> Homo sapiens

<400> 315
ccggtggtca cctgtactcc cagctgcact gcttacacgt cttccttcgt cttcacctac 60
cccgaggctg actccttccc cagctgtgca gctgcccacc gcaagggcag cagcagcaat 120
gagccttcct ctgactcgct cagctcaccc acgctgctgg ccctgtgagg gggcagggaa 180
ggggaggcag ccggcaccca caagtgccac tgcccgagct ggtgcattac agagaggaga 240
aacacatctt ccctagaggg ttcctgtaga cctagggagg accttatctg tgcgtgaaac 300
acaccaggct gtgggcctca aggacttgaa agcatccatg tgtggactca agtccttacc 360
tcttccggag atgtagcaaa acgcatggag tgtgtattgt tcccagtgac acttcagaga 420
gctggtagtt agtagcatgt tgagccaggc ctgggtctgt gtctcttttc tctttctcct 480
tagtcttctc atagcattaa ctaatct 507

<210> 316
<211> 239
<212> DNA
<213> Homo sapiens

<220>
<221> misc_feature
<222> 223
<223> n = A,T,C or G

<400> 316
agactccaag ccctactggg aggcacggag ggtggcgagg caggctcagc tggaagctca 60
gaaagccacg caggacttcc agagggccac agaggtgctc cgcgccgcca aggagaccat 120
ctccctggcc gagcagcggc tgctggagga tgacaagcgg cagttcgact ccgcctggca 180
ggagatgctg aatcacgcca ctcagagggt catggaggcg ganagaccaa gaccaggag 239

<210> 317
<211> 313
<212> DNA
<213> Homo sapiens

<400> 317
catcagtgat agggatgatt cacaaacaca aagctggtct tttcaaaatg ggaagaaaaa 60
agatgcaatt gatcccttac tattcaagta taaagtgcaa cccactaaaa aagaattaca 120
tgagtctgct attgttaaag caacacaaat cagccggaga aaacacctat tttctcgtga 180
taaactaaag ctttttctga agcaacactg ggaaccacaa gatggagtca ttaaaataaa 240
ggcatcatct ctttcaacgg ataaaatagc cgaacaagat tttttcttat ttcttccctg 300
atgattccac ccc 313

<210> 318
<211> 574
<212> DNA
<213> Homo sapiens

<400> 318
aaataacatc aacagaacag cttcactttg ggccaaacat ttgaaaaact ttttataaaa 60
aattgtttga tatttcttaa tgtctgctct gagccttaaa acacagattg aagaagaaaa 120
gaaagaaaaa acttaaatat ttatttctat gctttgttgc ctctgagaat aatgacaatt 180
tatgaatttg tgtttcaaat tgataaaata tttaggtaca aataacaaga ctaataatat 240
tttcttattt aaaaaaagca tgggaagatt tttatttatc aaaatataga ggaaatgtag 300
acaaaatgga tataaatgaa aattaccatg ttgtaaaacc ttgaaaatca gattctaact 360
ggatttgtat gcaactaagt atttttctga acacctatgc aggtcttatt tacagtagtt 420
actaagggaa cacacaaaga attacacaac gttttcctca agaaaatggt acaaaacaca 480
accgaggagc gtatacagtt gaaaacattt ttgttttgat tggaaggcag attattttat 540
attagtatta aaaatcaaac cctatgtttc tttc 574

<210> 319
<211> 518
<212> DNA

<213> Homo sapiens

<400> 319
gaagggaaat aacatcaaca gaacaacttc actttgggcc aaacatttga aaaacttttt 60
ataaaaaatt gtttgatatt tcttaatgtc tgctctgagc cttaaaacac agattgaaga 120
agaaaagaaa gaaaaaactt aaatatttat ttctatgctt tgttgcctct gagaataatg 180
acaatttatg aatttgtgtt tcaaattgat aaaatattta ggtacaaata acaagactaa 240
taatattttc ttatttaaaa aaagcatggg aagattttta tttatcaaaa tatagaggaa 300
atgtagacaa aatggatata aatgaaaatt accatgttgt aaaaccttga aaatcagatt 360
ctaactggat ttgtatgcaa ctaagtattt ttctgaacac ctatgcaggt cttatttaca 420
gtagttacta agggaacaca caaagaatta cacaacgttt tcctcaagaa aatggtacaa 480
aacacaaccg aggagcgtat acagttgaaa acattttt 518

<210> 320
<211> 353
<212> DNA
<213> Homo sapiens

<400> 320
aaataacatc aacagaacaa cttcactttg ggccaaacat ttaaaaaact ttttataaaa 60
aaatgtttga tatttcttaa tgtctgctct gagccttaca acacagattg aagaagaaaa 120
gaaagaacaa acttagatat ttatttctat gctttgttgc ctctgagaat aatgacaatt 180
tatgaatttg agtttcaaat tgataaaata tttaggtact aataacaaga ctaataatat 240
tttcttattt ataaaaagca tgggaagatt tttatttatc aaaatataca ggaagtgtag 300
acaaaatgga tataaatgaa aattaccatg ttgtaaaacc ttgaaaatca gag 353

<210> 321
<211> 401
<212> DNA
<213> Homo sapiens

<400> 321
gacctgcaca cagagactcc ctcctgggct cctggcacca tggccccctg aagagctggc 60
cctggtcacc ctcctcctgg gggcttctct gcagcacatc cacgcagctc gagggaccaa 120
tgtgggccgg gagtgctgcc tggagtactt caagggagcc attcccctta gaaagctgaa 180
gacgtggtac cagacatctg aggactgctc caggcatgcc atcgtttttg taactgtgca 240
gggcagggcc atctgttcgg accccaacaa caagagagtg aagaatgcag ttaaatacct 300
gcaaagcctt gagaggtctt gaagcctcct caccccagac tcctgactgt ctcccgggac 360
tacctgggac ctccaccggt ggtgttcacc gcccccaccc t 401

<210> 322
<211> 547
<212> DNA
<213> Homo sapiens

<400> 322
gacctgcaca cagagactcc ctcctgggct cctggcacca tggccccact gaagatgctg 60
gccctggtca ccctcctcct gggggcttct ctgcagcaca tccacgcagc tcgagggacc 120
aatgtgggcc gggagtgctg cctggagtac ttcaagggag ccattcccct tagaaagctg 180
aagacgtggt accagacatc tgaggactgc tccagggatg ccatcgtttt tgtaactgtg 240
cagggcaggg ccatctgttc ggaccccaac aacaagagag tgaagaatgc agttaaatac 300
ctgcaaagcc ttgagaggtc ttgaagcctc ctcaccccag actcctgact gtctcccggg 360
actacctggg acctccaccg ttggtgttca ccgcccccac cctgagcgcc tgggtccagg 420
ggaggccttc cagggacgaa gaagagccac agtgagggag atcccatccc cttgtctgaa 480
ctggagccat gggcacaaag ggcccagatt aaagtcttta tcctcaaaaa aaaaaaaaaa 540
aaaaaaa 547

<210> 323
<211> 283
<212> DNA
<213> Homo sapiens

<400> 323
ctgagcagag ggacctgcac acagagactc cctcctgggc tcctggcacc atggccccac 60
tgaagatgct ggccctggtc accctcctcc tgggggcttc tctgcagcac atccacgcag 120
ctcgagggac caatgtgggc cgggagtgct gcctggagta cttcaaggga gccattcccc 180
ttagaaagct gaagacgtgg taccagacat ctgaggactg ctccagggat gccatcgttt 240
ttgtaactgt gcagggcagg gccatctgtt cggaccccaa caa 283

<210> 324
<211> 160
<212> DNA
<213> Homo sapiens

<400> 324
gcggtgacga cggggaccat tttaccatca ccacccaccc tgagagcaac cagggcatcc 60
tgacaaccag gaagggtttg gattttgagg ccaaaaacca gcacaccctg tacgttgaag 120
tgaccaacga ggcccctttt gtgctgaagc tcccaacctc 160

<210> 325
<211> 300
<212> DNA
<213> Homo sapiens

<400> 325
tttttttttg gggccaattc tttaatttaa ctaaattagg aacgcagctt ttacagaaca 60
ataaacacaa gggacggggc caccccagga tctaacagct tttcagggac ctatgttgca 120
agctcaaaag taatccacta acgaaccaag tcaaactcca gtttttaata aaaaggggct 180
gggggaggtt gtcaaacccc ttccaatata aatccccaat ccgagggcca ccaaatgaaa 240
aagcaccaaa aatggaagga aaactttcaa aaattctgca aaaaatatgc cccctttttt 300

<210> 326
<211> 394
<212> DNA
<213> Homo sapiens

<400> 326
gtctattctt ttattttact aaattaggaa cgcagcattt acagaacaat aaacacaagt 60
gacgtggcca ccccaggatc taacagctct tcagtgagct atgttgcaag ctcagaagta 120
atccactaac gaaccaagtc agactccagt tcttcatcaa aaggtgctgg tggaggttgt 180
cagacgcctt ccaatataga tccccaatcc gatggccagc aaatgagaga gcagcagaga 240
tggaaggaaa acttccagaa attctgcaga gaatatgccc cctttcttca tgacgctcgt 300
gttcccccat gctgaaggtg gccgtgcgct tccggtgttt aaagaagaac ccttgggggg 360
aatatttccc ggccatttga ccaatcccat tcca 394

<210> 327
<211> 524
<212> DNA
<213> Homo sapiens

<400> 327
gtctattctt ttattttact aaattaggaa cgcagcattt acagaacaaa taaacacaag 60
tgacgtggcc accccaggat ctaacagctc ttcagtgagc tatgttgcaa gctcagaagt 120
aatccactaa cgaaccaagt cagactccag ttcttcatca aaaggtgctg gtggaggttg 180
tcagacgcct tccaatatag atccccaatc cgatggccag caaatgagag agcagcagag 240
atggaaggaa aactttcaga aattctgcag agaatatgcc ccctttcttc atgacgctcg 300
tgttcctcat gctgaggtgg ccgtgcgctt ccggtgttta aagaagaacc cttgggggga 360
atatttccgg ccgacttgac caatcccata tccatctgat ttttcttcca gaagctttca 420
cttccttcct ccttcaatat cactccctca actgtgactg ttttcccccc aatgctatgg 480
tttctgttca aaaccccgtt ggttctgttg ggtcgctact ccgt 524

<210> 328
<211> 55

<212> DNA
<213> Homo sapiens

<400> 328
ggccgccctt tttttttttt ttttttcggg ggcgtttttt gatttttaaa attgg     55

<210> 329
<211> 463
<212> DNA
<213> Homo sapiens

<400> 329
tcactatagg gaaagctggt acgcctgcag gtaccggtcc ggaattcccg ggtcgaccca 60
cgcgtccgcc gcccccgaga cctgtgaaga aaaccatctt gtgaggggct gcctggactg 120
gtctggcagg ttgggcctgg atggggaggc tctagcatct ctcataggtg caacctgaga 180
gtgggggagc taagccatga ggtaggggca ggcaagagag aggattcaga cgctctggga 240
gccagttcct agtcctcaac tccagccacc tgccccagct cgacggcact gggccagttc 300
cccctctgct ctgcagctcg gtttcctttt ctagaatgga aatagtgagg gccaatgccc 360
agggttggag ggaggagggc gttcatagaa gaacacacat gcgggcacct tcatcgtgtg 420
tggcccactg tcagaactta ataaaagtca actcatttgc tgg                   463

<210> 330
<211> 274
<212> DNA
<213> Homo sapiens

<220>
<221> misc_feature
<222> 144, 218, 268
<223> n = A,T,C or G

<400> 330
ccgcccccga gaccatgtga agaaaaccat cttgtgaggg gctgcctgga ctggtctggc 60
aggttgggcc tggatgggga ggctctagca tctctcatag gtgcaacctg agagtggggg 120
agctaagcca tgaggtaggg gcangcaaga gagaggattc agacgctctg ggagccagtt 180
cctagtcctc aactccagcc acctgcccca gctcgacngc actgggccag ttccccctct 240
gctctgcagt cggtttcctt ttctagantg gaaa                             274

<210> 331
<211> 232
<212> DNA
<213> Homo sapiens

<400> 331
cggctgtgag aatacgacag aagggtccgg ctgcgagaag acgacagaag ggggatctca 60
gcggggagcc acgtctcttg cactgtggtc tctgcatgga ccccagggct gtggggactt 120
gggggacagt aatcaagtaa tccccttttc cagaatgcat taacccactc ccctgacctc 180
acgctggggc aggtccccaa gtgtgcaagc tcagtattca tgatggtggg gg         232

<210> 332
<211> 321
<212> DNA
<213> Homo sapiens

<400> 332
gttgtgttga gatccagtgc agttgtgatt tctgtggatc ccagcttggt tccaggaatt 60
ttgtgtgatt ggtttaaatc cagttttcaa tcttcgacag ctgggctgga acgtgaactc 120
agtagctgaa cctgtctgac ccggtcacgt tcttggatcc tcagaactct ttgctcttgt 180
cggggtgggg gtgggaactc tcgtgaggag cgccagctgt gtaaatgcca cgactccgta 240
attcttattc ggtgggacct tgcttccctc tgggagctgg ctcgttttgt tggtgtctaa 300
cctttcgccg aatcgttaaa g                                           321

<210> 333
<211> 344
<212> DNA
<213> Homo sapiens

<220>
<221> misc_feature
<222> 265, 267, 272, 337
<223> n = A,T,C or G

<400> 333
gtcctatttc tcattttgtt gataatttct gcatttaatg gtctgtgctt taaatggtaa 60
cgctacggcc ccaggtcact gcgaggcact taccatgtag atacgggctc aaaagtcacc 120
tctcagagac ctacgtcatc cactcaggaa ttcgcgcctc tcatacttgc ctgtctcatt 180
ttatcttcct tctagcagct gtctgaaatt ggttcgtctg ttttcttgtt tatggtattc 240
tcaagccctt gacagaccgg ctagngnggt tntcccgtgc atcttcagcc tggcacatta 300
tggacactta aatactacgt attgatctaa tattcanggg ttaa 344

<210> 334
<211> 405
<212> DNA
<213> Homo sapiens

<400> 334
ggcacgaggg atgaagggtg ctgctcattt tcattagatg tatgtgaagg cacagtgaaa 60
atggaaatgt tcttggagct acttcctcaa aatctatcct tagtcacctc agtgcaacag 120
ctgggagggg gccgtgttaa gatttttttt gctacaaaga ggaggtggca atggtagatc 180
cacccttatg cttctcagtt tagcataacc tcttatggat tttcatcaaa ttcagcgtgt 240
tggtcactgg aaagagcctt ttccttctcc ttttcttact ctcccctcat ggggttcccc 300
tcttaaagga gaggagcttt taatttacac ttaccacctc atttgctttt ttggaggcca 360
tgccatataa gcgggactac cgagttaatc tcctttttac aaaag 405

<210> 335
<211> 227
<212> DNA
<213> Homo sapiens

<400> 335
ggatgaacta ttcagatgct atcgtttggc taaaagaaca tgatgtaaag aaagaagatg 60
gaactttcta tgaatttgga gaagatatcc cagaagctcc tgagagactg atgacagaca 120
ccattaatga accaatcttg ctgtgtcgat ttcctgtgga gatcaagtcc ttctacatgc 180
agcgatgtcc tgaggattcc cgtcttactg aatctgtcga cgtgttg 227

<210> 336
<211> 521
<212> DNA
<213> Homo sapiens

<400> 336
tcgaattcgg atgaactatt cagatgctat cgtttggcta aaagaacatg atgtaaagaa 60
agaagatgga actttctatg aatttggaga agatatccca gaagctcctg agagactgat 120
gacagacacc attaatgaac caatcttgct gtgtcgattt cctgtggaga tcaagtcctt 180
ctacatgcag cgatgtcctg aggattcccg tcttactgaa tctgtcgacg tgttgatgcc 240
caatgttggt gagattgtgg gaggctcaat gcgtatcttt gatagtgaag aaatactggc 300
aggttataaa agggaaggga ttgaccccac tccctattac tggtatacgg atcagagaaa 360
atacggtaca tgtccccatg gaggatatgg cttgggcttg gaacgattct taacgtggat 420
tctgaatagg tatcacatcc gagacgtgtg cttataccct cgatttgtcc agcgttgcac 480
gccataacca ttttctccag aagcgtggag gaaagattat g 521

<210> 337
<211> 325
<212> DNA

<213> Homo sapiens

<400> 337
ggactttccc gatcgccagg caggagtttc tctcggtgac tactatcgct gtcatgtctg 60
gtcgtggcaa gcaaggaggc aaggcccgcg ccaaggccaa gtcgcgctcg tcccgcgcgg 120
gccttcagtt cccggtaggg cgagtgcatc gcttgctgcg caaaggcaac tacgcggagc 180
gagtgggggc cggcgcgccc gtctacatgg ctgcgttcct cgagtatctg accgctgaga 240
tcctggagct ggcgggcaac gcggctcggg acaacaagaa gacgcgcatc atccctcgtc 300
acctccagct ggccatccgc aacga 325

<210> 338
<211> 401
<212> DNA
<213> Homo sapiens

<220>
<221> misc_feature
<222> 264
<223> n = A,T,C or G

<400> 338
cgttgctgtc ggttttagga aacctggcat ggtgctttca ggtctggggc ttttagagcc 60
ccccgtgtgg cttacaaatt ctacagcata cagagcaggc cacgctcagg cccggcatgc 120
gggccaccaa gttctggaaa ccacgtggtg tccctgcgaa tggggcgatc aagtccagag 180
ccggggcact ttcagagttt gaaggtaact gagagcagat ggtcctccat ttcaactcca 240
gaagtggggc tctgggaggg atgntctaac cctccctggc atgtcacaac caggctctgg 300
ctggaggatc cctccatccg gctcctgtca tcccctacac tttggcctag caagaggtgg 360
aataaccact tgtgtgctca ttactgttgg gaggaacaaa g 401

<210> 339
<211> 460
<212> DNA
<213> Homo sapiens

<400> 339
catgcgggcc accaagttct ggaaaccacg tggtgtccct gcgaatgggg cgatcaagtc 60
cagagccggg gcactttcag agtttgaagg taactgagag cagatggtcc tccatttcaa 120
ctccagaagt ggggctctgg gagggatgtt ctagccctcc ctggcatgtc agaaccaggc 180
tctgcctgga ggatccctcc atccggctcc tgtcatcccc tacactttgg ccaagcaaga 240
agtggtagaa ccacttggct gctccttcct tctggaggac acacagtctc agtccagatg 300
ccttcctgtc tttctggccc tttctggacc agatcctact cttcctttct aaatctgaga 360
tctccctcca gggaatccgc ctgcagagga cagagctggc tgtcttcccc cacccctaac 420
ctggcttatt cccaactgct ctgcccactg tgaaaccact 460

<210> 340
<211> 496
<212> DNA
<213> Homo sapiens

<400> 340
tttttttttt tttttttttt tttttgggat tcttaaatat agatgtattt ttttcatctc 60
atctccggac acactccaat cacacccctc ctgccctccc ctctcaactg caaaccaagc 120
ggtgcagaca cagcacagca cacatgaggg gccctccctt tcaccaaagc tgaaggcagg 180
gcacagtttg gggatggaag agcctcgagg taaatgtggg ggttctagaa cccagtgacc 240
tcagttctgg atcatgggaa agggatcagt atgcagtaac gtggtaaggt tccagatcta 300
gaagccagga cctagaacct agtggtttca cagtgggcag agcagttggg aataagccag 360
gttaggggtg ggggaagaca gccagctctg tcctctgcag gcggattccc tggagggaga 420
tctcagattt agaaaggaag agtaggatct ggtccagaaa gggccagaaa gacaggaagg 480
catctggact gagact 496

<210> 341
<211> 283

<212> DNA
<213> Homo sapiens

<400> 341
tttttttttt tttttttttt ttttttttag gatttgaata catttattgt gacaagaatg 60
ctgttataaa tattcataag caaaggccat ctttttatct aggaattgtc aaagagaaga 120
ttccaaattg gaaggataca tcttttgtaa aatctgccac caattcctgc tttgagaata 180
agcacctatt gtaaaatttc tactaacatt ataaatggtc acagcacatg ccacttgata 240
caatccaaac tttgaaatgt ttgacttctc agtgggctgt ccc 283

<210> 342
<211> 335
<212> DNA
<213> Homo sapiens

<400> 342
tgtcgggcag caggcgcagc ccagcctcga aatgcagaac gacgccggcg agttcgtgga 60
cctgtacgtg ccgcggaaat gctccgctag caatcgcatc atcggtgcca aggaccacgc 120
atccatccag atgaacgtgg ccgaggttga caaggtcaca ggcaggttta atggccagtt 180
taaaacttat gctatctgcg gggccattcg taggatgggt gagtcagatg attccattct 240
ccgattggcc aaggccgatg gcatcgtctc aaagaacttt tgactggaga gaatcacaga 300
tgtggaatat ttgtcataaa taaataatga aaacc 335

<210> 343
<211> 75
<212> DNA
<213> Homo sapiens

<400> 343
gggtagagtt cttaaatcga gatctggagg tagatggacg ctttgtaacc ctccagatct 60
gggacactgc agggc 75

<210> 344
<211> 611
<212> DNA
<213> Homo sapiens

<400> 344
gccggggggc agcggcgggc gcgagcggca gctgtcaggc caccgaggtc caagccgcac 60
ttgctgcccc attgaggacg aggaggcagc aggagcagtg acggtgactc taaggagccg 120
gattcccggc acgcagagct gacctgcctg gcacccgcgg ccctctcctg tttccttccc 180
attgtgttgg caccctaaaa agaaagaata aaacaacaac aggaaaaaaa ggaaaatatt 240
taaattgtga caaaaaccca ctgggttctc ttggttacaa actccttccc ttctggtgct 300
acaaaaatga gtgggaaatc cctgctctta aaggtcattc tcttgggtga tggtggagtt 360
gggaaaagtt cgcttatgaa ccgttacgta accaacaaat ttgactccca ggcttttcac 420
accatagggg tagagttctt aaatcgagat ctggaggtag atggacgctt tgtaaccctc 480
cagatctggg acactgcagg gcaggaacgt ttcaagagcc ttaggacacc cttctacagg 540
ggagcagact gctgcctctt gaccttcagc gtggatgatc ggcagagctt cgagaatctt 600
ggtaactggc a 611

<210> 345
<211> 441
<212> DNA
<213> Homo sapiens

<400> 345
ggcctttgca agcctcaccg gcgatgcaag gatagtcatc aacagggccc gggtggagtg 60
ccagagccac cggctgactg tggaggaccc ggtcactgtg gagtacatca cccgctacat 120
cgccagtctg aagcagcgtt atacgcatag cactgggcgc aggcgtttgg catctctgcc 180
ctcatcgtgg gtttctactt tgatggcact cctaggctct atcagactga cccctctgtc 240
acataccatg cctggaaggc caatgccata cgccggggtg ccaactcagt gcgtgagttc 300
ctggagaaga actatactga cgaagccatt gtaacatatg atctgaccat taagctggtg 360

atcaacgcac tcctggaagt ggttcactca ggtggcaaaa acattgaact tgctgtcatg 420
aggcgagatc aatccctcaa g 441

<210> 346
<211> 323
<212> DNA
<213> Homo sapiens

<400> 346
ggcctttgca ggcctcaccg ccgatgcaag gatagtcatc aacagggccc gggtggagtg 60
ccagagccac cggctgactg tggaggaccc ggtcactgtg gagtacatca cccgctacat 120
cgccagtctg aagcagcgtt atacgcacag caatgggcgc aggcgtttgg catctctgcc 180
ctcatcgtgg gtttcgactt tgatggcact cctaggctct atcagactga cccctcgggc 240
acataccatg cctggaaggc caatgccata tgccggggtg ccaagtcagt gcgtgagttc 300
ctggagaaga actatactga cga 323

<210> 347
<211> 567
<212> DNA
<213> Homo sapiens

<400> 347
ccagcggcct cttccccttc ctggtgctgc ttgccctggg aactctggca ccttgggctg 60
tggaaggctc tggaaagtcc ttcaaagctg gagtctgtcc tcctaagaaa tctgcccagt 120
gccttagata caagaaacct gagtgccaga gtgactggca gtgtccaggg aagaagaaat 180
gttgtcctga cacttgtggc atcaaatgcc tggatcctgt tgacacccca aacccaacaa 240
ggaggaagcc tgggaagtgc ccagtgactt atggccaatg tttgatgctt aaccccccca 300
atttctgtga gatggatggc cagtgcaagc gtgacttgaa gtgttgcatg ggcatgtgtg 360
ggaaatcctg cgtttcccct gtgaaagctt gattcctgcc atatggagga ggctctggag 420
tcctgctctg tgtggtccag gtcctttcca ccctgagact tggctccacc actgatatcc 480
tcctttgggg aaaggcttgg cacacagcag gctttcaaga agtgccagtt gatcaatgaa 540
taaataaacg agcctatttc tctttgc 567

<210> 348
<211> 314
<212> DNA
<213> Homo sapiens

<220>
<221> misc_feature
<222> 48
<223> n = A,T,C or G

<400> 348
atgaagtcca gcggcctctt ccccttcctg gtgctgcttg ccctgggnac tctggcacct 60
tgggctgtgg aaggctctgg aaagtccttc aaagctggag tctgtcctcc taagaaatct 120
gcccagtgcc ttagatacaa gaaacctgag tgccagagtg actggcagtg tccagggaag 180
aagagatgtt gtcctgacac ttgtggcatc aaatgcctgg atcctgttga caccccaaac 240
ccaacaagga ggaagcctgg gaagtgccca gtgacttatg gccaatgttt gatgcttaac 300
ccccccaatt tctg 314

<210> 349
<211> 611
<212> DNA
<213> Homo sapiens

<400> 349
ggctctgctc tgcagcacac ccgtgggtga cccctcaccc cagaagcagc agtggcagct 60
tgggaaatgt gaggaaggga aggagggaga gacgggaggg aggagagaga ggagaaggga 120
ggcaggggag gggcagcaga accaaggcaa atatttcagc tgggctatac ccctctcccc 180
atccctgtta tagaagctta gagagccagc cagcaatgga accttctggt tcctgcgcca 240
atcgccacca gtatcaattg tgtgagcttg ggtgcgagtg cacgcgtgcg tgagtacgga 300

gagtatatat agatctctat ctcttagcaa aggtgaatgc cagatgtaaa tggcgcctct 360
gggcaaagga ggcttgtatt ttgcacattt tataaaaact tgagagaatg agatttctgc 420
ttgtatattt ctaaaaagag gaaggagccc aaaccatcct ctccttacca ctcccatccc 480
tgtgagccct accttacccc tctgccccta gccaaggagt gtgaatttat agatctaact 540
ttcataggca aaacaaaagc ttcgagctgt tgcgtgtgtg agtctgttgt gtggatgtgc 600
gtgtgtggtc c 611

<210> 350
<211> 370
<212> DNA
<213> Homo sapiens

<400> 350
tggctggatg ggcttggact gtggtcctga aagcagcaag aagtatgctg aggctgtcac 60
tcgggctaag cagattgtgt ggaatggtcc tgtgggggta tttgaatggg aagcttttgc 120
ccggggaacc aaagctctca tggatgaggt ggtgaaagcc acttctaggg gctgcatcac 180
catcataggt ggtggagaca ctgccacttg ctgtgccaaa tggaacacgg aggataaagt 240
cagccatgtg agcactgggg gtggtgccag tttggagctc ctggaaggta aagtccttcc 300
tggggtggat gctctcagca atatttagta ctttcctgcc ttttagttcc tgtgcacagc 360
ccctaagtca 370

<210> 351
<211> 177
<212> DNA
<213> Homo sapiens

<400> 351
gggctgcatc accatcatag gtggtggaga cactgccact tgctgtgcca aatggaacac 60
ggaggataaa gtcagccatg tgagcactgg gggtggtgcc agtttggagc tcctggaagg 120
gaaagtcctt cctggggtgg atgctctcag caatatttag tactttcctg cctttta 177

<210> 352
<211> 204
<212> DNA
<213> Homo sapiens

<220>
<221> misc_feature
<222> 53, 55, 76, 86, 137
<223> n = A,T,C or G

<400> 352
atggctttta ccttccttaa ggtgctcaac aacatggaga ttgggcactt tcncnggttg 60
atgaagaagg aagccnagat ttgtcnaaga cctaatgtcc aaaagctgag aagaatggtg 120
tgaagattac cttgccntgt tgacttgtca ctgctgacaa gtttgatgag aatgcccaag 180
actggcccag ccactggtgg cttc 204

<210> 353
<211> 489
<212> DNA
<213> Homo sapiens

<400> 353
cttttacctt ccttaaggtg ctcaggacat ggagattggc acttctctgt ttgatgaaga 60
gggagccaag attgtcaaag acctaatgtc caaagctgag aagaatggtg tgaagattac 120
cttgcctgtt gactttgtca ctgctgacaa gtttgatgag aatgccaaga ctggccaagc 180
cactgtggct tctggcatac ctgctggctg gatgggcttg gactgtggtc ctgaaagcag 240
caagaagtat gctgaggctg tcactcgggc taagcagatt gtgtggaatg gtcctgtggg 300
ggtatttgaa tgggaagctt ttgcccgggg aaccaaagct ctcatggatg aggtggtgaa 360
agccacttct aggggctgca tcaccatcat aggtggtgga gacactgcca cttgctgtgc 420
caaatggaac acggaggata aagtcagcca tgtgagcact gggggtggtg ccagtttgga 480
gctcctgga 489

<210> 354
<211> 885
<212> DNA
<213> Homo sapiens

<400> 354
tttttttttt tcacggtttc aatggacact tttattgttt acttaatgga tcatcaattt 60
tgtctcacta cctacaaatg gaatttcatc ttgtttccat gctgagtagt gaaacagtga 120
caaagctaat cataataacc tacatcaaaa gagaactaag ctaacactgc tcactttctt 180
tttaacaggc aaaatataaa tatatgcact ctaaaatgca caatggttta gtcactaaaa 240
aattcaaatg ggatcttgaa gaatgtatgc aaatccaggg tgcagtgaaa atgagctgag 300
atgctgtgca actgtttaag ggttcctggc actgcatctc ttggccacta gctgaatctt 360
gacatggaag gttttagcta atgcccaggg gaaatgcaaa aaatgctaat ttgacttagg 420
gcctgtgcac aggaactaaa aggcaggaaa gtactaaata ttgctgagag catccacccc 480
aggaaggact ttaccttcca ggagctccaa actggcacca cccccagtgc tcacatggct 540
gactttatcc tccgtgttcc atttggcaca gcaagtggca gtgtctccac cacctatgat 600
ggtgatgcag ccccctaaaa gtggctttca ccacctcatc catgagagct ttggttcccc 660
gggcaaaagc ttcccattca aataccccca caggaccatt ccacacaatc tgcttaaccc 720
gagtgacagc ctcagcatac ttcttgctgg tttcaggacc acagtccaag ccccatccca 780
ccagcaggta tgcaagaagg cccagtgggc ttgccagtct tggcatttct catcaacttg 840
tcagcagtga caaagtcaac cgggaaggaa tcttcacacc atctt 885

<210> 355
<211> 434
<212> DNA
<213> Homo sapiens

<400> 355
cggctgcgag aagacgacag aaggggggag tggttgctat accttgactt catttatatg 60
aatttccact ttattaaata atagaaaaga aaatcccggt gcttgcagta gagtgatagg 120
acattctatg cttacagaaa atatagccat gattgaaatc aaatagtaaa ggctgttctg 180
gctttttatc ttcttagctc atcttaaata agcagtacac ttggatgcag tgcgtctgaa 240
gtgctaatca gttgtaacaa tagcacaaat cgaacttagg atttgtttct tctcttctgt 300
gtttcgattt ttgatcaatt ctttaatttt ggaagcctat aatacagttt tctattcttg 360
gagataaaaa ttaaatggat cactgatatt ttagtcattc tgcttctcat ctaaatattt 420
ccatattctg tatt 434

<210> 356
<211> 318
<212> DNA
<213> Homo sapiens

<400> 356
gggagtgggt gctatacctt gacttcattt atatgaattt ccactttatt aaataataga 60
aaagaaaatc ccggtgcttg cagtagagtg ataggacatt ctatgcttac agaaaatata 120
gccatgattg aaatcaaata gtaaaggctg ttctggcttt ttatcttctt agctcatctt 180
aaataagcag tacacttgga tgcagtgcgt ctgaagtgct aatcagttgt aacaatagca 240
caaatcgaac ttaggatttg cttcttctct tctgtgttgc gatttttgat caattcttta 300
attttggaag cctataat 318

<210> 357
<211> 231
<212> DNA
<213> Homo sapiens

<400> 357
cggctgcgag aagacaacag aagggggctc ccgctcggga tctcgctccg gatctcgctc 60
cgggtcccgc agtgggtccc ggagaggaag ctttgacgcc acaaggaatt cttcctactc 120
ttattcctac tcatttagca gtagttctat tgggcactat tagtcagttg ggagtgggtg 180
ctataccttg acttcattta tatgaatttc cactttatta aataatagaa a 231

<210> 358
<211> 446
<212> DNA
<213> Homo sapiens

<400> 358
atttgctgta tgccgagaat ggaaaaattg gaccacctaa actggatatc agaaaggagg 60
agaagcaaat catgattgac atatttcacc cttcagtttt tgtaaatgga gacgagcagg 120
aagtcgatta tgatcccgaa actacctgtt acattagggt gtacaatgtg tatgtgagaa 180
tgaacggaag tgagatccag tataaaatac tcacgcagaa ggaagatgat tgtgacgaga 240
ttcagtgcca gttagcgatt ccagtatcct cactgaattc tcagtactgt gtttcagcag 300
aaggagtctt acatgtgtgg ggtgttacaa ctgaaaagtc aaaagaagtt tgtattacca 360
ttttcaatag cagtataaaa ggttctcttt ggattccagt tgttgctgct ttactactct 420
ttctagtgct tagcctggta ttcatc 446

<210> 359
<211> 209
<212> DNA
<213> Homo sapiens

<220>
<221> misc_feature
<222> 19, 185, 193
<223> n = A,T,C or G

<400> 359
gagaatttgc tgtatgccng agatggaaaa attggaccac ctaaactgga tatcagaaag 60
gaggagaagc aaatcatgat tgacatattt cacccttcaa gtttttgtaa atggagacga 120
gcaggaagtc gattatgatc ccgaaactac ctgttacatt agggtgtaca atgtgtatgt 180
gagantgaac ggnagtgaga tccagtata 209

<210> 360
<211> 521
<212> DNA
<213> Homo sapiens

<400> 360
tgctgtcggt gactactgaa gaaatattcc tgacgtggtc ccgggcagcc atctgactcc 60
aatagagaga gagagttctt cacctttaag tagtaaccag tctgaacctg gcagcatcgc 120
tttaaactcg tatcactcca gaaattgttc tgagagtgat cactccagaa atggttttga 180
tactgattcc agctgtctgg aatcacatag ctccttatct gactcagaat ttcccccaaa 240
taataaaggt gaaataaaaa cagaaggaca agagctcata accgtaataa aagcccccac 300
ctcctttggt tatgataaac cacatgtgct agtggatcta cttgtggatg atagcggtaa 360
agagtccttg attggttata gaccaacaga agattccaaa gaattttcat gagatcagct 420
aagttgcacc aactttgaag tctgattttc ctggacagtt ttctgcttta atttcatgaa 480
aagattatga tctcagaaat tgtatcttag ttggtatcaa c 521

<210> 361
<211> 522
<212> DNA
<213> Homo sapiens

<400> 361
tggccctcga ggccaagaat tcggcactag gggagaggag cttgaatttc tgacacacat 60
aacatgtaaa aagtatttgg catttcataa ggatttgggg tggggtaaac gcaaggttag 120
tctgttttaa aaaatgtttt cattaacgag cacataactg gtggttccta atgggaatac 180
ttgacccagg cagaaactag aaaagtagca agtaggaaac ttccatttct ctcccctaaa 240
caacccctta aggcactgtg agctggagac aggagaggtg ttgcccaacc tttgttcata 300
tactcggtga cgatgtagat gggctcctca gacaccactg catagagctg gaccagcttg 360
tcgtgcttca gcttcttcat gatctgcgct tcctcaagga atgattcggg ggacattgtg 420
cctggtttaa gagtctttat ggctactttt gtgtttccat tccaggtacc tacaaacatc 480
ccagaatatg aagtcaaacc aaagatcttc ttttgatgga aa 522

<210> 362
<211> 421
<212> DNA
<213> Homo sapiens

<220>
<221> misc_feature
<222> 12, 331, 372
<223> n = A,T,C or G

<400> 362
ttaatgagtt anaaatctta atatagccat cttagccata accacaaata aactcatttt 60
ttctgttaaa atacttgaca gagtccttgc aattgaatgt ctttgttcaa caaaaactgt 120
attaagtgtt ttaaatttaa aatctaatct tatgcaaata gctggtggtc aaaacctttt 180
tccatcaaaa gaagatcttt ggtttgactt catattctgg gatgtttgta ggtacctgga 240
atggaaacac aaaagtagcc ataaagactc ttaaaccagg cacaatgtcc cccgaatcat 300
tccttgagga agcgcagatc atgaagaagc ngaagcacga caagctggtc cagctctatg 360
gcagtggtgt cngaggagcc catctacatc gtcaccgagt atatgaacaa aggttgggca 420
a 421

<210> 363
<211> 503
<212> DNA
<213> Homo sapiens

<400> 363
cagaaggggt ttccgaatgt tttagttagc cttttggtgg agccgccagc tgacaggaca 60
tcttacaaga gaatttgcac atctctggaa gcttagcaat cttattgcac actgttcgct 120
ggaagctttt tgaagagcac attctcctca gtgagctcat gaggttttca tttttattct 180
tccttccaac gtggtgctat ctctgaaacg agcgttagag tgccgcctta gacggaggca 240
ggagtttcgt tagaaagcgg acgctgttct aaaaaaggtc tcctgcagat ctgtctgggc 300
tgtgatgacg aatattatga aatgtgcctt ttctgaagag attgtgttag ctccaaagct 360
tttcctgtcg cagtgtttca gttctttatt ttcccttgtg gatatgctgt gtgaaccgtc 420
gtgtgagtgt ggtatgcctg atcacagatg gattttgtta taagcatcaa tgtgacactt 480
gcaggacact acaacgtggg aca 503

<210> 364
<211> 365
<212> DNA
<213> Homo sapiens

<400> 364
ggccgccctt tttttttttt ttgggggcga aaaaattttt ttttaaaaaa aaaaaaactt 60
cccccctggg gaaaaaaaaa ggttttttaa aaaaaaaacc aaacaaaatt ttcccgggcc 120
ctttaggggt tttaaatttt cccccgggtt gaaccccttt taaaaaaaaa ggaatttttt 180
tggggggaaa taatggggga aaaaccaaaa aaaaaagggg gttttttttt taaaaccctt 240
ttttttttaa aaaaccttcc cccaggggaa aaattcccaa aaccttttaa aaaaaaaggg 300
ccgaaatttt taatccaaag gggaaaaacc ccccccccaa caaaaaaccc ccaaagggga 360
aaaag 365

<210> 365
<211> 680
<212> DNA
<213> Homo sapiens

<220>
<221> misc_feature
<222> 172, 173, 176, 186, 199, 200, 591, 625, 659, 670
<223> n = A,T,C or G

<400> 365

aggacacaga caaggaactt gctgaaaggc caaccatttc aggatcagtc aaaggcagca 60
agcagataga ctcaaggtgt gtgaaagatg ttatacacca ggagctgcca cttcatgtcc 120
caaccagact gtgtctgtct gtgtctgcat gtaagagtga gggagggaag gnnggnacta 180
caaganagtc ggagatgann cagcacacac acaattcccc agcccacgtg atgcttgtgt 240
tgaccagatg ttcctgagtc tggagcaagc acccaggcca gaataacaga gctttcttag 300
ttggtgaaga cttaaacatc tgcctgaggt caggaggcaa tttgcctgcc ttgtacaaaa 360
gctcaggtga aagactgaga tgaatgtctt tcctctccct gcctcccacc agacttcctc 420
ctggaaaacg ctttggtaga tttggccagg agctttcttt tatgtaattg gataaataca 480
cacaccatac actatccaca gatatagcca agtagatttg ggtagaggat actatttcca 540
gaatagtgtt tagctcacct agggggatat gttgtatcac atttgcatat nccacatggg 600
gacataagct aattttttac agacncgatt ctgtcatgct gttaatagcc atggttaanc 660
ccccattggn ggggccggtg 680

<210> 366
<211> 570
<212> DNA
<213> Homo sapiens

<400> 366
taagctcggg attcggctcg agcggctcga gtcaagagaa aacacaagaa ggacatcagc 60
cagaacaagc gagccgtgag gcggctgcgc accgcctgcg agagggccaa gaggaccctg 120
tcgtccagca cccaggccag cctggagatc gactccctgt ttgagggcat cgacttctac 180
acgtccatca ccagggcgag gttcgaggag ctgtgctccg acctgttccg aagcaccctg 240
gagcccgtgg agaaggctct gcgcgacgcc aagctggaca aggcccagat tcacgacctg 300
gtcctggtcg ggggctccac ccgcatcccc aaggtgcaga agctgctgca ggacttcttc 360
aacgggcgcg acctgaacaa gagcatcaac cccgacgagg ctgtggccta cggggcggcg 420
gtgcaggcgg ccatcctgat gggggacaag tccgagaacg tgcaggacct gctgctgctg 480
gacgtggctc ccctgtcgct ggggctggag acggccggag gcgtgatgac tgccctgatc 540
aagcgcaact ccaccatccc caccaagcag 570

<210> 367
<211> 454
<212> DNA
<213> Homo sapiens

<400> 367
   gccgcccttt tttttttttt tttttttttt tttttttttg tttttttttt tttttcaaaa 60
   aaaaaaaatc tttttagaaa aaaaaacccc cccccaacaa aaaatggggg ggggggggga 120
   ttttccctcc cgggggaagg agaaaaagcc gcagtaataa aaaggggggg aaccaaaaaa 180
   tttttttttt tttttaaaaa aggttttttt gggggccccc ccccccaaaa aaaaaaaagg 240
   tcccccccct ttttttcccc cctttttggg ggggaaaaaa aaaaaagggg ggggaaaaaa 300
   acagaaaatt ttccccaaaa atttaaaaaa aaaagggggg ggggggggaa aaaaaaggtt 360
   tttttacccc cctggggggg aaaaaaaaaa aatttggggc caccaaaaag gggggggggc 420
   cccccaaaaa agggggtttt ttttaaaaaa aaaa 454

<210> 368
<211> 651
<212> DNA
<213> Homo sapiens

<400> 368
taagctcggg attcggctcg agtggtcttc gtctactccg ggtctttcag gaggccaaaa 60
ggcagctcca gaagattgac aaatctgagg gccgcttcca tgtccagaac cttagccagg 120
tggagcagga tgggcggacg gggcatggac tccgcagatc ttccaagttc tgcttgaagg 180
agcacaaagc cctcaagacg ttaggcatca tcatgggcac tttcaccctc tgctggctgc 240
ccttcttcat cgttaacatt gtgcatgtga tccaggataa cctcatccgt aaggaagttt 300
acatcctcct aaattggata ggctatgtca attctggttt caatcccctt atctactgcc 360
ggagcccaga tttcaggatt gccttccagg agcttctgtg cctgcgcagg tcttctttga 420
aggcctatgg gaatggctac tccagcaacg gcaacacagg ggagcagagt ggatatcacg 480
tggaacagga gaaagaaaat aaactgctgt gtgaagacct cccaggcacg gaagactttg 540
tgggccatca aggtactgtg cctagcgata acattgattc acaagggagg aattgtagta 600
caaatgactc actgctgtaa agcagttttt ctacttttaa agaccccccc c 651

<210> 369
<211> 280
<212> DNA
<213> Homo sapiens

<220>
<221> misc_feature
<222> 112
<223> n = A,T,C or G

<400> 369
tggtcttcgt ctactccagg gtctttcagg aggccaaaag gcagctccag aagattgaca 60
aatctgaggg ccgcttccat gtccagaacc ttagccaggt ggagcaggat gngcggacgg 120
ggcatggact ccgcagatct tccaagttct gcttgaagga gcacaaagcc ctcaagacgt 180
taggcatcat catgggcact ttcaccctct gctggctgcc cttcttcatc gttaacattg 240
tgcatgtgat ccaggataac ctcatccgta agaagtttac 280

<210> 370
<211> 418
<212> DNA
<213> Homo sapiens

<400> 370
ggccgccctt tttttttttt ttttttcccg ggcttttttg ggaaaaaccc ccctttccca 60
taaaaaaatt tttttggggg tttcccaatt tttttttcca atttcaaata atttttttcc 120
aaaaaaaacc caaacccttg gggccttttt tttttttttt aaagggcctt tttacttttc 180
cccaaggagg ccttggggaa ataaaaaaaa cccggttggg gggccccaaa aaaggggtgg 240
gcccccttga atcccccatt ggtttggggg taaaaaaggc ccccccatgg gcccccttcc 300
cccggggggg ggaacccccc cccaagaccc ccccggggga aaccgggccc aaaaaaaaaa 360
ccctttaaaa ttttaaaaaa cgggcccccc cctaaaaaaa ctttttttta aaaagggg 418

<210> 371
<211> 292
<212> DNA
<213> Homo sapiens

<400> 371
ttagggtata agttgctgta aaatttgtgt aaatttgtat ccacacaaat tcagtctctg 60
aatacacagt attcagagtc tctgatacac agtaattgtg acaatagggc taaatgttta 120
aagaaatcaa aagaatctat tagattttag aaaaacattt aaacttttta aaatacttat 180
taaaaaattt gtataagcca cttgtcttga aaactgtgca actttttaaa gtaaattatt 240
aagcagactg gaaaagtgat gtattttcat agtgacctgt gtttcactta at 292

<210> 372
<211> 415
<212> DNA
<213> Homo sapiens

<400> 372
tccttattta tttaacttca cccgagttcc tctgggtttc taagcagtta tggtgatgac 60
ttagcgtcaa gacatttgct gaactcagca cattcgggac caatatatag tgggtacatc 120
aagtccatct gacaaaatgg ggcagaagag aaaggactca gtgtgtgatc cggtttcttt 180
ttgctcgccc ctgttttttg tagaatctct tcatgcttga catacctacc agtattattc 240
ccgacgacac atatacatat gagaatatac cttatttatt tttgtgtagg tgtctgcctt 300
cacaaatgtc attgtctact cctagaagaa ccaaatacct caatttttgt ttttgagtac 360
tgtactatcc tgtaaatata tcttaagcag gtttgttttc agcactgatg gaaaa 415

<210> 373
<211> 326
<212> DNA
<213> Homo sapiens

<400> 373
tccttattta tttaacttca cccgagttcc tctgggtttc taagcagtta tggtgatgac 60
ttagcgtcaa gacatttgct gaactcagca cattcgggac caatatatag tgggtacatc 120
aagtccatct gacaaaatgg ggcagaagag aaaggactca gtgtgtgatc cggtttcttt 180
ttgctcgccc ctgttttttg tagaatctct tcatgcttga catacctacc agtattattc 240
ccgacgacac atatacatat gagaatatac cttatttatt tttgtgtagg ggtctgcctt 300
cacaaatgtc attgtctact cctaca 326

<210> 374
<211> 324
<212> DNA
<213> Homo sapiens

<400> 374
tccttattta tttaacttca cccgagttcc tctgggtttc taagcagtta tggtgatgac 60
ttagcgtcaa gacatttgct gaactcagca cattcgggac caatatatag tgggtacatc 120
aagttcatct gacaaaatgg ggcagaagag aaaggactca gtgtgtgatc cggtttcttt 180
ttgctcgccc ctgttttttg tagaatcttt tcatgcttga catacctacc agtattattc 240
ccgacgacac atatacatat gagaatatac cttatttatt tttgagtagg tgtctgcctt 300
cacaaatggc attggctact ccag 324

<210> 375
<211> 466
<212> DNA
<213> Homo sapiens

<400> 375
taactctggg aggggctcga gagggctggt ccttatttat ttaacttcac ccgagttcct 60
ctgggtttct aagcagttat ggtgatgact tagcgtcaag acatttgctg aactcagcac 120
attcgggacc aatatatagt gggtacatca agtccatctg acaaaatggg gcagaagaga 180
aaggactcag tgtgtgatcc ggtttctttt tgctcgcccc tgttttttgt agaatctctt 240
catgcttgac atacctacca gtattattcc cgacgacaca tatacatatg agaatatacc 300
ttatttattt ttgtgtaggt gtctgccttc acaaatgtca ttgtctactc ctagaagaac 360
caaatacctc aatttttgtt tttgagtact gtactatcct gtaaatatat cttaagcagg 420
tttgttttca gcactgatgg aaaataccag tgttgggttt tttttt 466

<210> 376
<211> 324
<212> DNA
<213> Homo sapiens

<400> 376
tccttattta tttaacttca cccgagttcc tctgggtttc taagcagtta tggtgatgac 60
ttagcgtcaa gacatttgct gaactcagca cattcgggac caatatatag tgggtacatc 120
aagttcatct gacaaaatgg ggcagaagag aaaggactca gtgtgtgatc cggtttcttt 180
ttgctcgccc ctgttttttg tagaatcttt tcatgcttga catacctacc agtattattc 240
ccgacgacac atatacatat gagaatatac cttatttatt tttgagtagg tgtctgcctt 300
cacaaatggc attggctact ccag 324

<210> 377
<211> 326
<212> DNA
<213> Homo sapiens

<400> 377
tccttattta tttaacttca cccgagttcc tctgggtttc taagcagtta tggtgatgac 60
ttagcgtcaa gacatttgct gaactcagca cattcgggac caatatatag tgggtacatc 120
aagtccatct gacaaaatgg ggcagaagag aaaggactca gtgtgtgatc cggtttcttt 180
ttgctcgccc ctgttttttg tagaatctct tcatgcttga catacctacc agtattattc 240
ccgacgacac atatacatat gagaatatac cttatttatt tttgtgtagg ggtctgcctt 300
cacaaatgtc attgtctact cctaca 326

<210> 378
<211> 494
<212> DNA
<213> Homo sapiens

<400> 378
atgccccgca tagatgcgga cctcaagctc gacttcaagg atgtcctgct ccgacctaag 60
cggagcagcc tcaagagccg agccgaggtg gatcttgaac gcaccttcac gtttcgaaat 120
tcaaagcaga cctactcagg gattcccatc atcgtggcca acatggacac tgtgggcacg 180
tttgagatgg cagccgtgat gtcacagcac tccatgttta cagcaattca taagcattac 240
tccctggatg actggaagct ctttgccaca aatcacccag aatgcctgca gaatgtagcc 300
gtgagttcag gcagtgggca gaatgatctg gaaaagatga ccagcatcct ggaagctgtg 360
ccacaggtta agtttatttg cctggatgtg gccaatgggt attcaaaaca ttttgtggaa 420
ttcgtgaaac ttgtccgtgc caaatttcct gaacacacca ttatggcagg gaacgtggtg 480
acaggagaaa tggt 494

<210> 379
<211> 243
<212> DNA
<213> Homo sapiens

<400> 379
gccgctgcac catgccccgc atagatgcgg acctcaagct cgacttcaag gatgtcctgc 60
tccgacctaa gcggacagcc tcaagagccg agccgaggtg gatcttgaac gcaccttcac 120
gtttcgaaat tcaaagcaga cctactcagg gattcccatc atcgtggcca acatggacac 180
tgtgggcacg tttgagatgg cagccgtgat gtcacagcac tccatgttta cagcaattca 240
taa 243

<210> 380
<211> 804
<212> DNA
<213> Homo sapiens

<400> 380
gcaaatgttt gattaattct gctcatatgc acatctgaaa gcatgagaca cactccacag 60
acagcacgca ctggagctgg tggggcagat gggcactcgc cgattaggta ttaatgtcaa 120
taatacgtgc ataaagtgct gataaaataa cttaagtgtt acaaaaacag acagtccacg 180
gtggctgcag gcacatgcag gcgggactgg gtcagacact ccagggctgc acatgttcca 240
gctggcctga gtccgacacg tcatagctgg ccttgtactt ggccaggatt ttcatgaggg 300
gccgtagctt gagccaccac tgttctttgg gaatcctgtg ctcaaaatcc gtttgcttct 360
tcagctctgc cacaggtttg aaaaataacg tttcttttgc ttattcccag cacacaaatg 420
gaatcatcgg tggtaaattt ttttcctctg ccccgggcct ccttgagttt tgcagtgatc 480
cactccatag ctctggcaga gattttggtt ccaaagtttc tatcaaatgg agagggtgcc 540
ccaccctgct gcatgtgacc cagcacgttc ttcctgcagt caaacacgcc tttgccctct 600
tctgaataca gctggtaaat gaagtcggtg gtgtagtttt cactgcagct ctcatttctg 660
agcacaaggc ctctctggat ggtggtcttc attttctccg tcaggtgctc cacgttggac 720
tgcagatcct gatgtcgaag ggctcttcga aatgtatgcg gcatcagtcc ggccgcagcc 780
ccccatgttg gcaggtagca cagt 804

<210> 381
<211> 624
<212> DNA
<213> Homo sapiens

<220>
<221> misc_feature
<222> 514
<223> n = A,T,C or G

<400> 381
tggagttgta ggcaaatgtt taattaattc tgctcatatg cacatctgaa agcatgagac 60

acactccaca gacagcacgc actggggctg gtggggcaga tgggcactcg ccgattaggt 120
attaatgtca ataatacgtg cataaagtgc tgataaaata acttaagtgt tacaaaaaca 180
gacagtccac ggtggctgca ggcacatgca ggcgggactg ggtcagacac tccagggctg 240
cacatgttcc agctggcctg agtccgacac gtcatagctg gccttgtact tggccaggat 300
tttcatgagg ggccgtagct tgagccacca ctgttctttg ggaatcctgt gctcaaaatc 360
cgtttgcttc ttcagctctg ccacaggttg aaaaataacg tttcttttgc ttattcccag 420
cacacaaatg gaatcatcgg tggtaaattt ttttcctctg ccccgggcct ccttgagttt 480
tgcagtgatc cactccatag ctctggcaga gatnttggtt ccaaagtttc tatcaaatgg 540
agaggtgccc caccctgctg atgtgacccc acacgttctt cctgagtcaa acacgccttt 600
gccctcttct gaatacaagc tggt 624

<210> 382
<211> 507
<212> DNA
<213> Homo sapiens

<220>
<221> misc_feature
<222> 301, 460, 498
<223> n = A,T,C or G

<400> 382
   ttttttggag ttgtaggaaa tgtttaattc tgctcatatg cacatctgaa agcatgagac 60
   acactccaca agacagcacg cactggggct ggtggggcag atgggcactc gcgattaggt 120
   attaatgtta ataatacgtg cataaagtgc tgataaaata acttaagtgt tacaaaaaca 180
   gacagtccac ggtggctgca ggcacatgca ggcgggactg ggtcagacac tccagggctg 240
   cacatgttcc agctggcctg agtcccgaca cgtcatagct ggccttgtac ttggccaggg 300
   nttttcatga ggggccctag ctttgagcca ccacttgttc tttggggaat cctgtgcttc 360
   aaaatcccgt tttgcttctt tcagctcttc ccacaggttt gaaaaataac gttttctttt 420
   tgcttatttc ccagcacaca aatgggattc atcggtgggn aatttttttc ctctgccccg 480
   gggcttcttg agtttttnca gtgattc 507

<210> 383
<211> 224
<212> DNA
<213> Homo sapiens

<220>
<221> misc_feature
<222> 198, 219
<223> n = A,T,C or G

<400> 383
atcagatccc aaagaccaat tgcaacgtag ctgtcatcaa cgtgggggca cccgcggctg 60
ggatgaacgc ggccgtacgc tcagctgtgc gcgtgggcat tgccgacggc acaggatgct 120
cgccatctat gatggtttga cggcttcgca agggccagat caaagaaatc ggctggacag 180
atgtcggggg ctggaccngc caaggaggct ccattcttng gaca 224

<210> 384
<211> 507
<212> DNA
<213> Homo sapiens

<220>
<221> misc_feature
<222> 301, 460, 498
<223> n = A,T,C or G

<400> 384
ttttttggag ttgtaggaaa tgtttaattc tgctcatatg cacatctgaa agcatgagac 60
acactccaca agacagcacg cactggggct ggtggggcag atgggcactc gcgattaggt 120
attaatgtta ataatacgtg cataaagtgc tgataaaata acttaagtgt tacaaaaaca 180

gacagtccac ggtggctgca ggcacatgca ggcgggactg ggtcagacac tccagggctg 240
cacatgttcc agctggcctg agtcccgaca cgtcatagct ggccttgtac ttggccaggg 300
nttttcatga ggggccctag ctttgagcca ccacttgttc tttggggaat cctgtgcttc 360
aaaatcccgt tttgcttctt tcagctcttc ccacaggttt gaaaaataac gttttctttt 420
tgcttatttc ccagcacaca aatgggattc atcggtgggn aatttttttc ctctgccccg 480
gggcttcttg agtttttnca gtgattc 507

<210> 385
<211> 224
<212> DNA
<213> Homo sapiens

<220>
<221> misc_feature
<222> 198, 219
<223> n = A,T,C or G

<400> 385
atcagatccc aaagaccaat tgcaacgtag ctgtcatcaa cgtgggggca cccgcggctg 60
ggatgaacgc ggccgtacgc tcagctgtgc gcgtgggcat tgccgacggc acaggatgct 120
cgccatctat gatggtttga cggcttcgca agggccagat caaagaaatc ggctggacag 180
atgtcggggg ctggaccngc caaggaggct ccattcttng gaca 224

<210> 386
<211> 232
<212> DNA
<213> Homo sapiens

<400> 386
acgacagaag ggtacggctg cgagaagacg acagatgggt acggctgtga gaagacgact 60
gatgggaaca gctaaggact gctaaacccc actctgcatc aactgaacgc aaatcagcca 120
ctttaattaa gctaagccct tactagacca atgggactta aacccacaaa cacttagtta 180
acagctaagc accctaatca actggcttca atgtacttct cccgccgtcg gg 232

<210> 387
<211> 339
<212> DNA
<213> Homo sapiens

<400> 387
   tactggtttt ggagaacttg tctacaacca gggattgatt ttaaagatgt ctttttttat 60
   tttacttttt tttaagcacc aaattttgtt gttttttttt ttttctccct tccccacaaa 120
   tcccttttaa aatatttttg ttaaccccct ttccaacggg ccgaggaaac ttaaaacccc 180
   tttttcctcg gcctggttcc tctttaattt ttaatttttc cccatcagtt taaaggtttt 240
   ggcatacttg gcatcttttt tcaaagggaa aacttttttt gccattcttt ggacttcccc 300
   ttttttaaag gaaatggggg ggccaaaagg ggatttcaa 339

<210> 388
<211> 456
<212> DNA
<213> Homo sapiens

<400> 388
tttttttttt tttttttttt tttaaccatc aaattcacag ctatttttcg cttttagtgt 60
gctcacagaa aattagaaca ccttaagcag gagtttaata gcattttttg taagcaaagt 120
tacattccat ctctaagtca aattggtcaa agcttctcca gtatttacaa aacatgatag 180
acaagatgct acacaaaacc attgcatctg aagattttgt tttcctttat tctcaaagac 240
gactggaaaa gaaagcatta tctgctgtaa tcaaaaacat accacagtat aaacagttac 300
cattccactt atcacagctt ggttgagttt agaattagtg ttttaaaaag tccaagatga 360
ctgcagtttt acaaaaatgg gcagggtgga aagttgcaaa cttcatgtgc ttctggatat 420
caagatttgt ttttatacaa tagtcacagt taaaaa 456

<210> 389
<211> 490
<212> DNA
<213> Homo sapiens

<400> 389
ttacattgaa tactacatat gtcgagggaa tgcagaaaga gttaaggaag gcaggttgtc 60
ctgctatgga ggccactctt cgttttccat gtactgcatg ctgtttgtgg cactttatct 120
tcaagccagg atgaagggag actgggcaag actcttaccc cccacactgc aatttggtct 180
tgttgccgta tccatttatg tgggcctttc tcgagttgct gattataaac accactggag 240
cgatgtgttg actggactca ttcagggagc tctggttgca atattagttg ctgtatatgt 300
atcggatttc ttcaaagaaa gaacttcttt taaagaaaga aaagaggagg actctcatac 360
aactctgcat gaaacaccaa caactgggaa tcactatccg agcaatcacc agccttgaaa 420
ggcagcaggg tgcccaggtg aagctggcct gttttctaaa ggaaaatgat tgccacaagg 480
caagaggatg 490

<210> 390
<211> 334
<212> DNA
<213> Homo sapiens

<400> 390
gaactcggtg gtggccactg cgcagaccag acttcgctcg tactcgtgcg cctcgcttcg 60
cttttcctcc gcaaccatgt ctgacaaacc cgatatggct gagatcgaga aattcgataa 120
gtcgaaactg aagaagacag agacgcaaga gaaaaatcca ctgccttcca aagaaacgat 180
tgaacaggag aagcaagcag gcgaatcgta atgaggcgtg cgccgccaat atgcactgta 240
cattccacaa gcattgcctt cttattttac ttcttttagc tgtttaactt tgtaagatgc 300
aaagaggttg gatcaagatt aaatgactgt gctg 334

<210> 391
<211> 377
<212> DNA
<213> Homo sapiens

<220>
<221> misc_feature
<222> 349
<223> n = A,T,C or G

<400> 391
gaactcggtg gtggccactg cgcagaccag acttcgctcg tactcgtgcg cctcgcttcg 60
cttttcctcc gcaaccatgt ctgacaaacc cgatatggct gaggtcgaga aattcgataa 120
gtcgaaactg aagaagacag agacgcaaga gaaaaatcca ctgccttcca aagaaacgat 180
tgaacaggag aagcaagcag gcgaatcgta atgaggcgtg cgccgccaat atgcactgta 240
cattccacaa gcattgcctt cttattttac ttcttttagc tgtttaactt tgtaagacgc 300
atagagggtg gatcaagttt aaatgactgt gctgcccctt tcacatcana gaactactga 360
caacgaaggc cgcgcct 377

<210> 392
<211> 555
<212> DNA
<213> Homo sapiens

<400> 392
ctcggtggtg gccactgcgc agaccagact tcgctcgtac tcgtgcgcct cgctttgctt 60
ttcctccgca accatgtctg acaaacccga tatggctgag atcgagaaat tcgataagtc 120
gaaactgaag aagacagaga cgcaagagaa aaatccactg ccttccaaag aaacgattga 180
acaggagaag caagcaggcg aatcgtaatg aggcgtgcgc cgccaatatg cactgtacat 240
tccacaagca ttgccttctt attttacttc ttttagctgt ttaactttgt aagatgcaaa 300
gaggttggat caagtttaaa tgactgtgct gcccctttca catcaaagaa ctactgacaa 360
cgaaggccgc gcctgccttt cccatctgtc tatctatctg gctggcaggg aaggaaagaa 420
cttgcatgtt ggtgaaggaa gaagtggggt ggaagaagtg gggtgggacg acagtgaaat 480

ctagagtaaa accaagctgg cccaaggtgt cctgcaggct gtaatgcagt ttaatcagag 540
tgccattttt ttttt 555

<210> 393
<211> 300
<212> DNA
<213> Homo sapiens

<400> 393
gctcaattgg actatgttga cctctatctt attcattctc caatgtctct aaagccaggt 60
gaggaacttt caccaacaga tgaaaatgga aaagtaatat ttgacatagt ggatctctgt 120
accacctggg aggccatgga gaagtgtaag gatgcatgat tggccaagtc cattggggtg 180
tcaaacttca accgcaggca gctggagatg atcctcaaca agccaggact caagtacaag 240
cctggctgca accaggtaga aagtcattcg tatttcaacc ggagtaaatt gctagaatcg 300

<210> 394
<211> 344
<212> DNA
<213> Homo sapiens

<400> 394
acagaagggt acggctgcga gaagacgaca gaagggtacg gctgcgagaa gacgacagaa 60
gggtacggct gcgagaagac gacagaaggg taaaacactg aactgacaat taacagccca 120
atatctacaa tcaaccgaca agtcattatt accctcactg tcaacccaac acaggcatgc 180
tcataaggaa aggttaaaaa aagtaaaagg aactcggcaa atcttacccc gcctgtttac 240
caaaaacatc acctgtagca tcaccagtat tagaggcacc gcctgcccag tgacacatgt 300
ttaacggccg cggtacccta accgtgcaaa ggtagcataa tcac 344

<210> 395
<211> 507
<212> DNA
<213> Homo sapiens

<400> 395
tgctcggtcc ttccgaggaa gctaaggctg cgttggggtg aggccctcac ttcatccggc 60
gactagcacc gcgtccggca gcgccagccc tacactcgcc cgcgccatgg cctctgtctc 120
cgagctcgcc tgcatctact cggccctcat tctgcacgac gatgaggtga cagtcacgga 180
ggataagatc aatgccctca ttaaagcagc cggtgtaaat gttgagcctt tttggcctgg 240
cttgtttgca aaggccctgg ccaacgtcaa cattgggagc ctcatctgca atgtaggggc 300
cggtggacct gctccagcag ctggtgctgc accagcagga ggtcctgccc cctccactgc 360
tgctgctcca gttgaggaga agaaagtgga agcaaagaaa gaagaatccg aggagtctga 420
tgatgacatg ggctttggtc tttttgacta aacctctttt ataacatgtt caataaaaag 480
ctgaacttta aaaaaaaaaa aaaaaaa 507

<210> 396
<211> 488
<212> DNA
<213> Homo sapiens

<400> 396
gaggccctca cttcatccgg cgactagcac cgcgtccggc agcgccagcc ctacactcgc 60
ccgcgccatg gcctctgtct ccgagctcgc ctgcatctac tcggccctca ttctgcacga 120
cgatgaggtg acagtcacgg aggataagat caatgccctc attaaagcag ccggtgtaaa 180
tgttgagcct ttttggcctg gcttgtttgc aaaggccctg gccaacgtca acattgggag 240
cctcatctgc aatgtagggg ccggaggacc tgctccagca gctggtgctg caccagcagg 300
aggtcctgcc ccctgcactg ctgctgctcc agttgaggag aagaaagtgg aagcatagaa 360
agaagaatcc gacgagtctg atgatgacat gggctatggt ctttttgact aaacctcttt 420
tataacatgt tcaataaaaa gctgaacttt aaaaagaaaa aaaaaaaact cgagcctcta 480
gaactata 488

<210> 397

<211> 180
<212> DNA
<213> Homo sapiens

<400> 397
ctgcgttggg gtgaggccct cacttcatcc ggcgactagc accgcgtccg gcagcgccag 60
ccctacactc gcccgcgcca tggcctctgt ctccgagctc gcctgcatct actcggccct 120
cattctgcac gacgatgagg tgacagtcac ggaggataag atcaatgccc tcattaaagc 180

<210> 398
<211> 491
<212> DNA
<213> Homo sapiens

<220>
<221> misc_feature
<222> 12, 154, 255, 348, 368, 402, 409, 450, 471
<223> n = A,T,C or G

<400> 398
tttttttttt tntttcactg ttcaaggttt attgggggtt ttagttggta taacacttgg 60
atagttggtt gcattgtttg tatgtagatc tttttacatt atatggtaat gtacactact 120
gatatagttc acaaaataag atcctttgga aganttatac acaagacatg atattggatt 180
tatacactgg atcccaggga tgtgactcac tgggaaaaaa tgttggacta ggcatgttca 240
gtgaaggagc caggnagtta tataacacac ggtaaacatc cacctggctc aaggggcaaa 300
tgcagtacgt acagcattgg cagtggtgcg tcagaggtgg cagaactntt tcacactaac 360
cagttganga ctacacaaga ttaataccat ccagcatcag gntatagcnt gtggatttta 420
caaaccattt cttatttcta actttcaggn gttgatgttt ttcccagtcc ntcttaaaat 480
ttttactgct t 491

<210> 399
<211> 235
<212> DNA
<213> Homo sapiens

<400> 399
tgatttctgt ggatcccagc ttggttccag gaattttgtg tgattggctt aaatccagtt 60
ttcaatcttc gacagctggg ctggaacgtg aactcagtag ctgaacctgt ctgacccggt 120
cacgttcttg gatcctcaga actctttgct cttgtcgggg tgggggtggg aactcacgtg 180
gggagcggtg gctgagaaaa tgtaaggatt ctggaataca tattccatgg gactt 235

<210> 400
<211> 465
<212> DNA
<213> Homo sapiens

<400> 400
tacggctgcg agaagacgac agaagggtac ggctgcgaga agacgacaga agggtacggc 60
tgcgagaaga cgacagaagg gtacggctgc gagaagacga cagaagggtg atttctgtgg 120
atcccagctt ggttccagga attttgtgtg attggcttaa atccagtttt caatcttcga 180
cagctgggct ggaacgtgaa ctcagtagct gaacctgtct gacccggtca cgttcttgga 240
tcctcagaac tctttgctct tgtcggggtg ggggtgggaa ctcacgtggg gagcggtggc 300
tgagaaaatg taaggattct ggaatacata ttccatggga ctttccttcc ctctcctgct 360
tcctcttttc ctgctcccta acctttcgcc gaatggggca gcaccactga cgtttctggg 420
cggccagtgc ggctgccagg ttcctgtact actgccttgt acttt 465

<210> 401
<211> 243
<212> DNA
<213> Homo sapiens

<400> 401
tgatttctgt ggatcccagc ttggttccag gaattttgtg tgattggctt aaatccagtt 60
ttcaatcttc gacagctggg ctggaacgtg aactcagtag ctgaacctgt ctgacccggt 120
cacgttcttg gatcctcaca actctttgct cttgtcgggg tgggggtggg aactcacgtg 180
gggagcggtg gctgagaaaa tgtaaggatt ctggaataca tattccatgg gactttcctt 240
ccc 243

<210> 402
<211> 506
<212> DNA
<213> Homo sapiens

<400> 402
ttctagcatc ctcttaacgt gcagcaaaag caggcgacaa aatctcctgg ctttacagac 60
aaaaatattt cagcaaacgt tgggcatcat ggtttttgaa ggctttagtt ctgctttctg 120
cctctcctcc acagccccaa cctcccaccc ctgatacatg agccagtgat tattcttgtt 180
cagggagaag atcatttaga tttgttttgc attccttaga atggagggca acattccaca 240
gctgccctgg ctgtgatgag tgtccttgca ggggccggag taggagcact ggggtggggg 300
cggaattggg gttactcgat gtaagggatt ccttgttgtt gtgttgagat ccagtgcagt 360
tgtgatttct gtggatccca gcttggttcc aggaattttg tgtgattggc ttaaatccag 420
ttttcaatct tcgacagctg ggctggaacg tgaactcagt agctgaacct gtctgacccg 480
gtcacgttct tggatcctca gaactc 506

<210> 403
<211> 390
<212> DNA
<213> Homo sapiens

<400> 403
gtagtcgcct ctctttcagc agttacccag ggtttttgga gtctctggat gatttttaca 60
ttcttagcag tggattgata ttgctgcaga ccacaaacag tgtgtttaat aaaaccctgc 120
taaagcaggt aatacccgag actctcctgt cctggcaaag agtccgtgtg gccaatatga 180
tggcagatag tggcaagagg tgggcagaca tcttttcaaa atacaactct ggcacctata 240
acaatcaata catggttctg gacctgaaga aagtaaagct gaaccacagt cttgacaaag 300
gcactctgta cattgtggag caaattccta catatgtaga atattctgaa caaactgatg 360
ttctacggaa aggatattgg ccctcctaca 390

<210> 404
<211> 372
<212> DNA
<213> Homo sapiens

<400> 404
aggagattca gaagcacaac cacagcaaga gcacctggct gatcctgcac cacaaggtgt 60
acgatttgac caaatttctg gaagagcatc ctggtgggga agaagtttta agggaacaag 120
ctggaggtga cgctactgag aactttgagg atgtcgggca ctctacaaat gccagggaaa 180
tgtccaaaac attcatcatt ggggagctcc atccagatga cagaccaaag ttaaacaagc 240
ctccggaaac tcttatcact actattgatt ctagttccag ttggtggacc aactgggtga 300
tccctgccat ctctgcagtg gccgtcgcct tgatgtatcg cctatacatg gcagaggact 360
gaacacctcc tc 372

<210> 405
<211> 619
<212> DNA
<213> Homo sapiens

<400> 405
tcccgggtgg agctggctga gtcgcgcgct ctgctccacc cgacggggct gtgtgtgctg 60
ggcctggctc gcggcgaacc gagatggcag agcagtcgga cgaggccgtg aagtactaca 120
ccctagagga gattcagaag cacaaccaca gcaagagcac ctggctgatc ctgcaccaca 180
aggtgtacga tttgaccaaa tttctggaag agcatcctgg tggggaagaa gttttaaggg 240
aacaagctgg aggtgacgct actgagaact ttgaggatgt cgggcactct acaaatgcca 300

gggaaatgtc caaaacattc atcattgggg agctccatcc agatgacaga ccaaagttaa 360
acaagcctcc ggaaactctt atcactacta ttgattctag ttccagttgg tggaccaact 420
gggtgatccc tgccatctct gcagtggccg tcgccttgat gtatcgccta tacatggcag 480
aggactgaac acctcctcag aagtcagcgc aggaagagcc tgctttggac acgggagaaa 540
agaagccatt gctaactact tcaactgaca gaaaccttca cttgaaaaca atgattttaa 600
tatatctctt tctttttct 619

<210> 406
<211> 499
<212> DNA
<213> Homo sapiens

<400> 406
taagctcgga attcggctcg agggctccag ctgagctcct gcttctactg aggacatacc 60
tcccagatga ggtggggccc ccaaccccat tccctgagcc tggagcagag ccccctctca 120
ctgtgggctt gctcaaagcc ctgctggagc agactggggc tcaaggatgg ctgtcggccc 180
cagttctaag cccatatgag gacatcctat gggaccccag cactccaccc ccgactccac 240
ctcgggacct atgactaccc ttcaggcatc agaacactca gggcctggag gcttgcttgg 300
gactggaggc ttgcttggac agttcctctg tgtcactgac acaggaaatc atttctagga 360
cacagtgatc agggaagggt gcctgggact tggagggtcc catgtatgga cctgtgtatg 420
caatactgtt ctgtcatctg gagctatttt taagatgtgt gtgttaaata tatacatagt 480
ttaatatata aaaaaaaaa 499

<210> 407
<211> 229
<212> DNA
<213> Homo sapiens

<400> 407
ggctccagct gagctcctgc ttctactgag gacatacctc ccagatgagg tggggccccc 60
aaccccattc cctgagcctg gagcagagcc ccctctcact gtgggcttgc tcaaagccct 120
gctggagcag actggggctc aaggatggct gtcggcccca gttctaagcc catatgagga 180
catcctatgg gaccccagca ctccaccccc gactccacct cgggaccta 229

<210> 408
<211> 467
<212> DNA
<213> Homo sapiens

<400> 408
ggaagttctg cgctggtcgg cggagtatca agtggccatg gggagcctca gcggtctgcg 60
cctggcagca ggaagctgtt ttaggttatg tgaaagagat gttggcctca tctctaaggc 120
ttaccagaag ctctgatttg aagagaataa atggattttg cacaaaacca caggaaagtc 180
ccggagctcc atcccgcact tacaacagag tgcctttaca caaacctacg gattggcaga 240
aaaagatcct catatggtca ggtcgcttca aaaaggaaga tgaaatccca gagactgtct 300
cgttggagat gcttgatgct gcaaagaaca agatgcgagt gaagatcagc tatctaatga 360
ttgccctgac ggtggtagga tgcatcttca tggttattga gggcaagaag gctgcccaaa 420
gacacgagac tttaacaagc ttgaacttat aaaagaaagc tcgtctg 467

<210> 409
<211> 338
<212> DNA
<213> Homo sapiens

<400> 409
ggaagttctg cgctggtcgg cggagtagca agtggccatg gggagcctca gcggtctgcg 60
cctggcagca ggaagctgtt ttaggttatg tgaaagagat gtttcctcat ctctaaggct 120
taccagaagc tctgatttga agagaataaa tggattttgc acaaaaccac aggaaagtcc 180
cggagctcca tcccgcactt acaacagagt gcctttacac aaacctacgg attggcagaa 240
aaagatcctc atatggtcag gtcgcttcaa aaaggaagat gaaatcccag agactgtctc 300
gttggagatg cttgatgctg cagagatcaa gatgcgag 338

<210> 410
<211> 601
<212> DNA
<213> Homo sapiens

<400> 410
tttgcacgat gccttccaca tcccacggcg ctgctgctgg gggcagattg gcctggggag 60
gcagcacttg ctctccagct catctgggtt gcttttcccc gcagtggata tcacaggcta 120
aagggggggg cagtccccac catatttgag tctttctcca agttgcgccg gacaaccaag 180
accaaaggac acagttaccc acctggcccc tctgaagtca gccggctcag acgatgcagg 240
aagcgctgct ccgagggccg agggcccaca actccatttt ctccacctcc acctgctgat 300
gtcacctgct ttcctgtgga agaggcctca gcacctgcca ctttgccggc ctccccagct 360
gggaggctgg agcctggcct tagcagcccc ttttcagacc tactgggccc cttgggtgcc 420
caggcagatg aagcaggctg cagcgcccag ccttcaccag agcggcagcc ctcccctctc 480
gaaccacggc cagtctcccc ctcagcgtat atgctgcgcc tgcccccacc cgccggagcc 540
tacatccaga atgaacacag ctaccaggtg ggcagcgcct tactctggaa gcggcgagcc 600
g 601

<210> 411
<211> 52
<212> DNA
<213> Homo sapiens

<400> 411
gccccttggg tgcccaggca gatgaagcag gctgcagcgc ccagccttca cc 52

<210> 412
<211> 525
<212> DNA
<213> Homo sapiens

<400> 412
cgtttcggtt tctagggttg ttacgaagct gcaggagcga gatggaggtg gacgcaccgg 60
gtgttgatgg tcgagatggt ctccgggagc ggcgaggctt tagcgaggga gggaggcaga 120
acttcgatgt gaggcctcag tctggggcaa atgggcttcc caaacactcc tactggttgg 180
acctctggct tttcatcctt ttcgatgtgg tggtgtttct ctttgtgtat tttttgccat 240
gacttgttcg ctgatatcta aattaagaag ttggttcttg agtgaattct gaaaatggct 300
acaaacttct tgaataaaga agacaggact ctcaatagaa gaatttcaca tctccaaggg 360
acccttcctt tcattttaca ctttgttact aatttgcaga actctattaa ttgggtagga 420
tttcacccat tcctagctaa gttcttaaaa ttaaaccctt tggttcgtgt ttaaaaactt 480
tcaaacatct gatggcttta caggggctga atataaaagc atttg 525

<210> 413
<211> 604
<212> DNA
<213> Homo sapiens

<220>
<221> misc_feature
<222> 12, 14, 18, 20, 24, 27, 29, 31, 33, 35, 54, 594, 595
<223> n = A,T,C or G

<400> 413
ttcgaaccca tncntttncn atcnganana ngntnctagt tcttctgaag accncatcga 60
ttcgtttcgg tttctagggt tgttacgaag ctgcaggagc gagatggagg tggacgcacc 120
gggtgttgat ggtcgagatg gtctccggga gcggcgaggc tttagcgagg gagggaggca 180
gaacttcgat gtgaggcctc agtctggggc aaatgggctt cccaaacact cctactggtt 240
ggacctctgg cttttcatcc ttttcgatgt ggtggtgttt ctctttgtgt attttttgcc 300
atgacttgtt cgctgatatc taaattaaga agttggttct tgagtgaatt ctgaaaatgg 360
ctacaaactt cttgaataaa gaagacagga ctctcaatag aagaatttca catctccaag 420
ggacccttcc tttcatttta cactttgtta ctaatttgca gaactctatt aattgggtag 480
gatttcaccc attcctagct aagttcttaa aattaaaccc tttggttcgt gtttaaaaac 540

tttcaaacat ctgatggctt tacaggggct gaatataaaa gcatttgtac ttannaaaaa 600
aaaa 604

<210> 414
<211> 285
<212> DNA
<213> Homo sapiens

<400> 414
ctctaacgtg ggcaacagag accctgtctc aaaaagaaaa tattcctgtt agccctaaag 60
gctttacatg aggaatggta gaagtggtct tttgtttaaa ttagttgcat tcagcatata 120
tgaattgtct taaatatttt ggggatactc ccccgccttt taaacagggc ataagatctg 180
gtaaactctc tgtatatctt cctacctttc aaaatcgttc ttagggttag tcaagtctgg 240
aatataattg ctgactataa agttagcaat tatgctttaa ggtga 285

<210> 415
<211> 241
<212> DNA
<213> Homo sapiens

<400> 415
atttacactt gatggctaat aaagatggac agctaatgac agaattattt aatcgattag 60
aaagtcagca tcatttccag atagaaaagg ctctagttga gaaacttcag caggattttg 120
tagctgactg gtgctctgag ggagagtgcc tagcagctat taactccacc tataatactt 180
cagggtatat tttggatcca cacactgctg ttgcaaaagt ggttgcagat agggtgcaag 240
a 241

<210> 416
<211> 315
<212> DNA
<213> Homo sapiens

<400> 416
cggcttctgg aagagggggt gttgcggcag atccctgtag tgggcttcgt gctgaattgg 60
ttttctccgg tccaggcttc acagtaggga agaactttta acttgacagc aggctctctg 120
gagtccacag aacccatata tgtctacaaa gcacaaggtg caggagtcac gctgcctcca 180
acgccctcgg gcagtcgcac caagcagagg cttccaggcc agaagccttt taaaaggtcc 240
ctgcgaggtt cagatgcttt gagtgagacc agctcagtca gtcatattga agacttagaa 300
aaggtggagc gccta 315

<210> 417
<211> 164
<212> DNA
<213> Homo sapiens

<400> 417
tggatccccc gggctgcagg aattcgaatt ctgtgtgtgt gtgtgtgtat gaatggtata 60
tttattacat tatttagaaa gagaatgagt gtgttatgag gataatgtta tatacagtct 120
aagtggatgt ttctgtttgg cacagaatgt aggatttctg aaac 164

<210> 418
<211> 206
<212> DNA
<213> Homo sapiens

<400> 418
tatatttatt acattatttt gaaagagaat tagtgtgtta tgtggataat gttatataca 60
gccaaagtgg atgtttctgt ttggcaagga aggtaggatt tctgaaactc aggccttaac 120
caataggttg gaagacaaga ccaattgaag agttaggaaa tgtgagtttt tgttacttct 180
gttattccag tcttggtttc attgtc 206

<210> 419

<211> 238
<212> DNA
<213> Homo sapiens

<220>
<221> misc_feature
<222> 159, 227
<223> n = A,T,C or G

<400> 419
agcagtgtac ataatattcc agtaggaaac tgcttccaag tttaagcatg agctccccaa 60
actggagaaa acatattttg ctattctgag acaacaatca gaatacagac tttggattcc 120
aggtcacagt ttgcttttta gacaaggtaa agcaaagana gccacattgt gccatcttca 180
gctccagtgg ctttagcagt gactgtttga cataaaacat gtaaganttg cttgttgg 238

<210> 420
<211> 504
<212> DNA
<213> Homo sapiens

<400> 420
cggcgtgctt gctgctggag ggtgatggcc ctgcaaggct gtgggctccg acctcaccgg 60
gagtcgacag cgagaggttc gccgaagagc gaggttctgg gcgagcgctg aacgccggcc 120
ccaagcaccc cgggtcttta cacagtccgc gtccacagac tctgacgaag acgtggatct 180
gctctcgctt tagctgctcg cggtcctcca gatcatgtcc gcgactcctg cgactccgcg 240
cggaaaaaaa agtttgccag gcgtggactc aatgaccttt ccaagctgtg cgcctcgctg 300
cctggaccgg gtctgagcgc ggctgcccag gttgaccttt ctgcgggagg gctttctcta 360
cgtgctgttg tctcactggg tttttgtcgg agccccacgc cctccggcct ctgattcctg 420
gaagaaaggg ttggtcccct cagcaccccc agcatcccgg aaaatgggga gcaaggctct 480
gccagcgccc atcccgctcc accc 504

<210> 421
<211> 814
<212> DNA
<213> Homo sapiens

<220>
<221> misc_feature
<222> 38, 93, 94, 95, 422, 440, 467, 474, 508, 519, 529, 535, 554,
557, 561, 565, 584, 594, 604, 619, 641, 655, 674, 679, 690,
695, 702, 704, 706, 712, 716, 724, 734, 737, 740, 743, 780,
781, 808, 813
<223> n = A,T,C or G

<400> 421
   cggggacgga gctcggcgtg cttgctgctg gagggttntg gccctgcaag gctgtgggct 60
   ccgacctcac cgggagtcga cagcgagagg ttnnncgaag agcgaggttc tgggcgagcg 120
   ctgaacgccg gccccaagca ccccgggtct ttacacagtc cgcgtccaca gactctgacg 180
   aagacgtgga tctgctctcg ctttagctgc tcgcggtcct ccagatcatg tccgcgactc 240
   ctgcgactcc gcgcggaaaa aaaagtttgc caggcgtgga ctcaatgacc tttccaagct 300
   gtgcgcctcg ctgcctggac cgggtctgag cgcggctgcc caggttgacc ttttctgcgg 360
   aagggctttc tctacgtgct gttgctcatg ggtttttgtc ggagccccaa cgcccttccg 420
   gncttttgat tcctggaaan aaaaggggtt ggttcccctt caagcanccc caancattcc 480
   ccgggaaaaa atgggggagc caaaggcntt ttggccaang gccccaatnc ccggnttcaa 540
   cccgttgggt tggnaanttt naccnaaatt aacttccttt cctncaaggc ccgnggaaaa 600
   aacnttttcc cgggccacng gggggaaccc aaccttgcaa nggggccttg taccnggtct 660
   tcaaacggcg ggtnccaana acccttgccn ccatngaaac cnantnggaa cncctngggg 720
   gttnttcccc aatnggngcn ccnaaaaaac aaccccggtt ccaaccattt aagggaaaan 780
   nggcgggggg gccccaaggg cccttttngg acnt 814

<210> 422
<211> 375

<212> DNA
<213> Homo sapiens

<400> 422
ctgacgaaga cgtggatctg ctctcgcttt agctgctcgc ggtcctccag atcatgtccg 60
cgactcctgc gactccgcgc ggaaaaaaaa gtttgccagg cgtggactca atgacctttc 120
caagctgtgc gcctcgctgc ctggaccggg tctgagcgcg gctgcccagg ttgacctttc 180
tgcgggaggg ctttctctac gtgctgttgt ctcactgggt ttttgtcgga gccccacgcc 240
ctccggcctc tgattcctgg aagaaagggt tggtcccctc agcaccccca gcatcccgga 300
aaatggggag caaggctctg cagcgcccat cccgctccac cgtcgctgca gctcccaatt 360
actcttctgc aggcg 375

<210> 423
<211> 405
<212> DNA
<213> Homo sapiens

<400> 423
ggggacggag ctcggcgtgc ttgctgctgg agggtgatgg ccctgcaagg ctgtgggctc 60
cgacctcacc gggagtcgac agcgagaggt tcgccgaaga gcgaggttct gggcgagcgc 120
tgaacgccgg ccccaagcac cccgggtctt tacacagtcc gcgtccacag actctgacga 180
agacgtggat ctgctctcgc tttagctgct cgcggtcctc cagatcatgt ccgcgactcc 240
tgcgactccg cgcggaaaaa aaagtttgcc aggcgtggac tcaatgacct ttccaagctg 300
tgcgcctcgc tgcctggacc gggtctgagc gcggctgccc aggttgacct ttctgcggga 360
gggctttctc tacgtgctgt tgtctcactg ggtttttgtc ggacc 405

<210> 424
<211> 139
<212> DNA
<213> Homo sapiens

<400> 424
ctcgtgttca gctgtcagaa taacagccaa taaaaactac aggagcaaaa cctctcagga 60
aggtgcttta aaaaagatgc atgaggaaga acaccatcaa caaatgtcca tcttacaact 120
gcaactgata caaatgaat 139

<210> 425
<211> 273
<212> DNA
<213> Homo sapiens

<400> 425
ttctggctgg gaagcgcgat tgtggcttta aaccaccatc atggtctagc aaagaggcaa 60
agaccaagac caccaagaag cgccctcagc gtgcaacatc caatgtgttt gccatgtttg 120
accagtcaca gattcaggag ttcaaagagg ccttcaacat gattgatcag aacagagatg 180
gcttcatcga caaggaagat ttgcatgata tgcttgcttc tctaggaaag aatcccactg 240
atgcatacct tgatgccatg atgaatgagg ccc 273

<210> 426
<211> 56
<212> DNA
<213> Homo sapiens

<400> 426
gggaaccgcc attctgcctg ggaaccgcca ttctggccgg gaaccgccat tatgac 56

<210> 427
<211> 365
<212> DNA
<213> Homo sapiens

<400> 427

ggcgcattct tacctgtcgg ggtgcggcga gtgtctcacc tctctgcact tccaaggact 60
cttgtcatct gccttaggcg ggaaatgctg ttgctggatt gcaaccccga ggtggatggt 120
ctgaagcatt tgctggagac aggggcctcg gtcaacgcac ccccggatcc ctgcaagcag 180
tcgcctgtcc acttagccgc aggaagcggc cttgcttgct ttcttctctg gcagctgcaa 240
acgggcgctg acctcaacca gcaggatgtt ttaggagaag ctccactaca caaggcagca 300
aaagttggaa gcctggagtg cctaagcctg cttgtagcca gtgatgccca aattgattta 360
tgtag 365

<210> 428
<211> 119
<212> DNA
<213> Homo sapiens

<400> 428
gagcggtggc tgagaaatgt aaggattctg gaatacatat tccatgggac tttccttccc 60
tctcctgctt cctcttttcc tgctccctaa cctttcgccg aatggggcag caccactga 119

<210> 429
<211> 421
<212> DNA
<213> Homo sapiens

<220>
<221> misc_feature
<222> 130, 185, 246, 256, 336, 361, 385, 412
<223> n = A,T,C or G

<400> 429
tttttttttt tttttttgga aataagtcaa agcattgttt atttatgaca tatttacata 60
tttacaaaac tgattttact caatacatca tcctgcgtaa tatcataaaa tgaacaccat 120
atcctgggan taaaaatcca tatttcttaa taatttatgt atagcccaac ttttagaaca 180
tagantatta tcaatttggc ttcccaaact acaaagtcct gtttataatt ttttctagcc 240
aaggancaga gtaggntcaa caggcatatt aaagtaattt agttaaccct gaggtaatta 300
ctaacttggc ataattttgg aatggggtat atatancaca ctttccatct ggcacttagg 360
ntacttatta ctattcacac taccnttttg gtatttatcc acctcaattt tncaacttcc 420
t 421

<210> 430
<211> 481
<212> DNA
<213> Homo sapiens

<400> 430
gggtagccgc ttttcgtcga ctcttaccgg ttggctgggc cagctgcgcc gcggctcaca 60
gctgacgatg gggaccccca gcaagcagga catcttgacc atcttcaagc gcctccgctc 120
ggtgcccact aacaaggtgt gttttgattg tggtgccaaa aaatcccagc tgggcaagca 180
taacctatgg agtgttcctt tgcattgatt gctcagggtc ccaccggtca cttggtgttc 240
acttgagttt tattcgatct acagagttgg attccagctg gtcatggttt cagttgcgat 300
gcatgcaagt cggaggaaac gctagtgcat cttccttttt tcatcaacat gggtgttcca 360
ccaatgacac caatgccaag tacaacagtc gtgctgctca gctctatagg gagaaaatca 420
aatcgctcgc ctctcaagca acacggaagc atggcactga tctgtggctt gatagttgtg 480
t 481

<210> 431
<211> 136
<212> DNA
<213> Homo sapiens

<400> 431
ggggtaagtt tagaaatacg gctgggcatg tccagccctg accacggcca gctctggagg 60
gctgtccttt ggctgtaccc acttggaaga gaaagaaaaa gaaaaaaaaa aaaaaaaaaa 120
aaaatttttt tttttt 136

<210> 432
<211> 578
<212> DNA
<213> Homo sapiens

<400> 432
aaacaacaaa caccagaaaa attacctata ccaatgatag caaaaaacct tatgtgtgaa 60
ctcgatgaag actgtgaaaa gaatagtaag agggactact taagttctag ttttctatgt 120
tctgatgatg atagagcttc taaaaatatt tctatgaact ctgattcatc ttttcctgga 180
atttctataa tggaaagtcc attagaaagt cagcccttag attcagatag aagcattaaa 240
gaatcctctt ttgaagaatc aaatattgaa gatccactta ttgtaacacc agattgccaa 300
gaaaagacct caccaaaagg tgtcgagaac cctgctgtac aagagagtaa ccaaaaaatg 360
ttaggtcctc ctttggaggt gctgaaaacg ttagcctcta aaagaaatgc tgttgctttt 420
cgaagtttta acagtcatat taatgcatcc aataactcag aaccatccag aatgaacatg 480
acttctttag atgccaatgg atatttcgtg tgcctacagt ggttcatatc ccatggctat 540
aacccctact caaaaaagaa gatcctgtat gccacatc 578

<210> 433
<211> 229
<212> DNA
<213> Homo sapiens

<220>
<221> misc_feature
<222> 35, 37
<223> n = A,T,C or G

<400> 433
gcctaggtgc ccaggctatg atgagtctgc ttttnangga ggtagggaat gacatcttcc 60
ttggacccaa agcttaaaag taatgtatgc tttgctgacc actgtttgtt aggccttaaa 120
caacattcac tgtggtggta tcaggcacac tgctatgtgc atcaattatt tttttgcttt 180
ccaaacagaa tctctggggc acaagtttta cactcaagct aagtataac 229

<210> 434
<211> 503
<212> DNA
<213> Homo sapiens

<400> 434
tggtacgcct gcaggtaccg gtccggaatt cccgggtcga cccacgcgtc cggcgtcatg 60
gagctgacct ggttcccatc tactcctttg gagagaatga agtgtacaag caggtgatct 120
tcgaggaggg ctcctggggc cgatgggtcc agaagaagtt ccagaaatac attggtttcg 180
ccccatgcat cttccatggt cgaggcctct tctcctccga cacctggggg ctggtgccct 240
actccaagcc catcaccact gttgtgggag agcccatcac catccccaag ctggagcacc 300
caacccagca agacatcgac ctgtaccaca ccatgtacat ggaggccctg gtgaagctct 360
tcgacaagca caagaccaag ttcggcctcc cggagactga ggtcctggag gtgaactgag 420
ccagccttcg gggccaattc cctggaggaa ccagctgcaa atcacttttt tgctctgtaa 480
atttggaagt gtcatgggtg tct 503

<210> 435
<211> 248
<212> DNA
<213> Homo sapiens

<400> 435
gcgtcatgga gctgacctgg ttcccatcta ctcctttgga gagaatgaag tgtacaagca 60
ggtgatcttc gaggagggct cctggggccg atgggtccag aagaagttcc agaaatacat 120
tggtttcgcc ccatgcatct tccatggtcg aggcctcttc tcctccgaca cctgggggct 180
ggtgcctact ccaagcccat caccactgtt gtgggagagc ccatcaccat ccccaagctg 240
gagcacca 248

<210> 436
<211> 457
<212> DNA
<213> Homo sapiens

<400> 436
atcttgtctc ttttcatcgt gatggtgtga tgctgacgag aatatcttat gctttcttca 60
gcctgttgca atctgagcca atgattttct ttgcactgat cctttctact ctggagagaa 120
gctcttttga cacagatcct gccccgttta atagactcca gctgctggca ctgccttctg 180
agttctttca cttccgaatt cttatcgtcc tgcagcccca ccacagtcaa tgactaagtt 240
cctctggact ttcacatgga tcgtaataga caacttcatc ctgtttttct taccagaccc 300
taaaatgtgc ctccaagaca gtcgtgggaa cagtatggag ccagcagcag aagccactca 360
cgaaccaatg gaggagaaca actcagaaac agacccaagt caatctaagg tttaactttt 420
ataagtcttt caagagagtc caactgtgta gtaagca 457

<210> 437
<211> 589
<212> DNA
<213> Homo sapiens

<400> 437
gcttccaggt ctccttccag catccacaca agtacctgct ccactacctg gtttccctcc 60
agaactggct gaaccgccac agctggcagc ggacccctgt tgccgtcacc gcctgggccc 120
tgctgcggga cagctaccat ggggcgctgt gcctccgctt ccaggcccag cacatcgccg 180
tggcggtgct ctacctggcc ctgcaggtct acggagttga ggtgcccgcc gaggtcgagg 240
ctgagaagcc gtggtggcag gtgtttaatg acgaccttac caagccaatc attgataata 300
ttgtgtctga tctcattcag atttatacca tggacacaga gatcccctaa gg[?]cctggcc 360
caggcctgcc caaagagaag cccaggatgg tcggctgcct ggggacattg tcaccacgtc 420
gccatgacgg ctggtcccca caggaccagc tgggaggact ggttgtgctg ctggagaagg 480
gctggagaag gcaatggcat gctgccgctt tgccagtccc taaaagtcgc ggtgcaggtg 540
atggtgggag ccgcgcctcc agcgggcagg ccgggagtgt actgtgtgc 589

<210> 438
<211> 241
<212> DNA
<213> Homo sapiens

<400> 438
cgcttccagg tctccttcca gcatccacac aagtacctgc tccactacct ggtttccctc 60
cagaactggc tgaaccgcca cagctggcag cggacccctg ttgccgtcac cgcctgggcc 120
ctgctgcggg acagctacca tggggcgctg tgcctccgct tccaggccca gcacatcgcc 180
gtggcggtgc tctacctggc cctgcaggtc tacggagttg aggtgcccgc cgaggtcgag 240
g 241

<210> 439
<211> 221
<212> DNA
<213> Homo sapiens

<400> 439
ttcagctctg caaacactgt cacatccttt cctggaaggg cactgaccat ccgtgcactg 60
ccaataaccc agagagctgc tccgtttcac tttcacccca ggactttatc aacttgttca 120
agttctgaat cccagcacat gacaacactt cagaagggtc cccctgctga ctggagagct 180
gggaatatgg catttggaca cttcatttgt aaatagtgta c 221

<210> 440
<211> 228
<212> DNA
<213> Homo sapiens

<220>
<221> misc_feature

<222> 191
<223> n = A,T,C or G

<400> 440
gagctttctt aataaccgta cttctcaaaa tcagagtttt actgtttcaa taaatgttca 60
ccctagattg taagtttttt gttgttgagc cctagatttt tttctactag tgtaaatctg 120
tattccctcc aagtatggtg ataaggggac tgagtcttat ttacatttgt acaatcacta 180
ctttacctgt ngtatttgca gtaagtcttt tgagccctat taaacctg 228

<210> 441
<211> 531
<212> DNA
<213> Homo sapiens

<400> 441
tttcttaata accgtacttc tcaaaatcag agttttactg tttcaataaa tgttcaccct 60
agattgtaag ttttttgttg ttgagcccta gatttttttc tactagtgta aatctgtatt 120
ccctccaagt atggtgataa ggggactgag tcttatttac atttgtacaa tcactacttt 180
acctgttgta tttgcagtaa gtcttttgag ccctattaaa cctgtcaatt ttcttgtcct 240
gtcagaaaac tgagattttg gctcaaaaat ggatgttatt aacaaagggg aacaatatag 300
atgtcttagt acaaagaaaa tgaaatgtaa gaggagattg tctggagttc aggggataga 360
gtgtcaagtc ttaaatggtt acatcttttt gctaagtgtt actcagaata tagttacaaa 420
tatggtactt aaatatctag ctgaaatttg tttgtcccat gagcttctca catgagtcta 480
ctgggcaatt ttatgtgagt tttggtcaaa attggtaatc tcttttatct t 531

<210> 442
<211> 147
<212> DNA
<213> Homo sapiens

<220>
<221> misc_feature
<222> 112
<223> n = A,T,C or G

<400> 442
aacttgttac ccaataacaa tttaatgtta aatttggctt tcttctgtgt cccagcctct 60
taaattaata gatgggcctt tccattatca ttatgaccgg acattgtaaa gnacttaagg 120
taacacccag ttttctatta cttgccc 147

<210> 443
<211> 518
<212> DNA
<213> Homo sapiens

<400> 443
acctgaagaa tattagaaga aattgtgcac cctccacaaa acatacaaag tttaaaagtt 60
tggatctttt tctcagcagg tatcagttgt aaataatgaa ttaggggcca aaatgcaaaa 120
cgaaaaatga atcatctaca tgtagttagt aatttctagt ttgaactgta attgaatatt 180
gtggcttcat atgtattatt ttatattgta cttttttcat tattgatggt ttggacttta 240
ataagagaaa ttccatagtt tttaatatcc cagaagtgag acaatttgaa cagtgtattc 300
tggaaaacaa cacactaact gaacagaagt gaatgcttat atatattatg atagccttaa 360
acctttttcc tctaatgcct taactgtcaa ataattataa ccttttaaag cataggacta 420
tagtcagcat gctagactga gaggtaaaca ctgatgcaat tagaacaggt actgatgctg 480
tcagtgttta acactatgtt tagctgtgtt tatgctat 518

<210> 444
<211> 76
<212> DNA
<213> Homo sapiens

<400> 444

gctgctcatg agcagcatgg acgacctgat acgccactgt aacgggaagc tgggcagcta 60
caaaatcaat ggccgg 76

<210> 445
<211> 308
<212> DNA
<213> Homo sapiens

<400> 445
gagcattatg agcattatgt cagaatagaa tagaattggg gttcgatctt aacaggccag 60
aaatgcctgg gtttttttgg tttgtttttg tttttgtttt tttatcaaat cctgcctgac 120
tgtctgcttg ttttgcctac catcgtgaca tctccatggc tgtaccacct tgtcgggtag 180
cttatcagac tgatgttgac tgttgaatct catggcaaca ccagtcgatg ggctgtctga 240
cattttggta tctttcatct gaccatccat atccaatgtt ctcatttaaa cattacccag 300
catcattg 308

<210> 446
<211> 530
<212> DNA
<213> Homo sapiens

<400> 446
tgtgttaatg ttttctagca tgtactctgg tttcaacaga cacaaattta tatgttaacc 60
cagttttctt gccgttctgt aagtgtttta ttcttagtgt gatttttttc cattgggatg 120
tttttgattg aacttgttca ttttgttttg cttgggagga aaataaacaa ttttactttt 180
ttcctttagg agcattatga gcattatgtc agaatagaat agaattgggg ttcgatctta 240
acaggccaga aatgcctggg tttttttggt ttgtttttgt ttttgttttt ttatcaaatc 300
ctgcctgact gtctgcttgt tttgcctacc atcgtgacat ctccatggct gtaccacctt 360
gtcgggtagc ttatcagact gatgttgact gttgaatctc atggcaacac cagtcgatgg 420
gctgtctgac attttggtat ctttcatctg accatccata tccaatgttc tcatttaaac 480
attacccagc atcattgttt ataatcagaa actctggtcc ttctgtctgg 530

<210> 447
<211> 104
<212> DNA
<213> Homo sapiens

<400> 447
ggacgtgcct ggaaccacct cgtccacgtc cacgtccacc tgggggcctc gggaggctag 60
gcccctcctc aaaggcccac cagcccggcg ctcatgctga gccc 104

<210> 448
<211> 417
<212> DNA
<213> Homo sapiens

<400> 448
tatctttcat ctgaccatcc atatccaatg ttctcattta aacattaccc agcatcattg 60
tttataatca gaaactctgg tccttctgtc tggtggcact taaagtcttt tgtgccataa 120
tgcagcagta tggagggagg attttatgga gaaatgggga tagtcttcat gaccacaaat 180
aaataaagga aaactaagct gcactgtggg ttttgaaaag gttattatac ttcttaacaa 240
ttcttttttt cagggacttt tctagctgta tgactgttac ttgaccttct ttgaaaagca 300
ttcccaaaat gctctatttt agatagttta acattaacca acataatttt ttttagatcg 360
agtcagcata aatttctaag tcagcctcta gtcgtggttc atctctttca cctgcat 417

<210> 449
<211> 630
<212> DNA
<213> Homo sapiens

<400> 449
tttttttttt tttttttttt ttggaatcgc aagaattccc aggccctctt tttatttaca 60

gtgataccaa accatccact tgcaaattct ttggtctccc atcagctgga attaagtagg 120
tactgtgtat ctttgagatc atgtatttgt ctccactttg gtggatacaa gaaaggaagg 180
cacgaacagc tgaaaaagaa gggtatcaca ccgctccagc tggaatccag caggaacctc 240
tgagcatgcc acagctgaac acttaaaaga ggaaagaagg acagctgctc ttcatttatt 300
ttgaaagcaa attcatttga aagtgcataa atggtcatca taagtcaaac gtatcaatta 360
gaccttcaac ctaggaaaca aaattttttt ttctatttaa taatacacca cactgaaatt 420
atttgccaat gaatcccaaa gatttggtac aaatagtaca attcgtattt gctttcctct 480
ttcctttctt cagacaaaca ccaaataaaa tgcaggtgaa agagatgaac cacgactaga 540
ggctgactta gaaatttatg ctgactcgat ctaaaaaaaa ttatgttggt taatgttaaa 600
ctatctaaaa tagagcattt tgggaatgct 630

<210> 450
<211> 596
<212> DNA
<213> Homo sapiens

<400> 450
tttttttttt tttttttttt tttggggtaa aagttatatc ttattgccat gctacaaaat 60
gtatgaagtt ggcactgata gggagaaata gagaacaaag ggtgggaagg gatagaggga 120
aaattatgtt gttacatata caacaaggtt ttattttaat taacagtggt tacgttttgc 180
caatattaaa aatgcaaacc aaaatttaaa atgctgatct gaaacagcat taagatacaa 240
tgtatgcata gtacagtatc acttatgtct ttttattaga gaaatatgga atgtttataa 300
aagaaattaa ccatgggggt aaaattcata tttcatatac aatttggcaa tggtagtccc 360
actgttggac aattttttat aaaagaaaaa attaaaaatc taataagcta cctttataca 420
aagttgctat atttatgcct ttacgtagga aaaaaacatt tataatgcaa attaggacat 480
acaatagtct tacaatacta tacaatgtaa tgaaaataaa acataacaca aagtttgtcc 540
tttataaaat gtatattttg cattactaat gcaaatgtgg cacactggtg actact 596

<210> 451
<211> 559
<212> DNA
<213> Homo sapiens

<400> 451
tggcgggttg ctttccaaaa tggcgcgggt gctgaaggct gcagccgcga atgccgtagg 60
gcttttttcc agacttcaag ctcccattcc aacagtaaga gcttcttcca catcacagcc 120
cttggatcaa gtgacaggtt ctgtgtggaa cctgggtcga ctcaaccatg tagccatagc 180
agtgccagat ttggaaaagg ctgcagcatt ttataagaat attctggggg cccaggtaag 240
tgaagcggtc cctcttcctg aacatggagt atctgttgtt tttgtcaacc tgggaaatac 300
caagatggaa ctgcttcatc cattgggacg tgacagtcca attgcaggtt ttctgcagaa 360
aaacaaggct ggaggaatgc atcacatctg catcgaggtg gataatatta atgcagctgt 420
gatggatttg aaaaaaaaag aagatccgca gtctaagtga aggggtcaaa ataggagcac 480
atggaaaacc agtgattttt ctccatccta aagactgtgg tggagtcctt gtggaactgg 540
agcaagcttg acttatatt 559

<210> 452
<211> 638
<212> DNA
<213> Homo sapiens

<400> 452
tggcgggttg cgttccaaat ggcgcgggtg ctgaaggctg cagccgcgaa tgccgtaggg 60
cttttttcca gacttcaagc tcccattcca acagtaagag cttcttccac atcacagccc 120
ttggatcaag tgacaggttc tgtgtggaac ctgggtcgac tcaaccatgt agccatagca 180
gtgccagatt tggaaaaggc tgcagcattt tataagaata ttctgggggc ccaggtaagt 240
gaagcggtcc ctcttcctga acatggagta tctgttgttt ttgtcaacct gggaaatacc 300
aagatggaac tgcttcatcc attgggacgt gacagtccaa ttgcaggttt tctgcagaaa 360
aacaaggctg gaggaatgca tcacatctgc atcgaggtgg ataatattaa tgcagctgtg 420
atggatttga aaaaaaaaga agatccgcag tctaagtgaa ggggtcaaaa taggagcaca 480
tggaaaacca gtgatttttc tccatcctaa agactgtggt ggagtccttg tggaactgga 540
gcaagcttga cttatatttg caagcaacta aattaattga cctgaaaaag cctatcaaat 600
actatcaaaa tgtactatga cattgagtcc ttcactgc 638

<210> 453
<211> 57
<212> DNA
<213> Homo sapiens

<400> 453
gactacattt ggggatgatg cattccttta agattgaatg attctgccct tgggcag    57

<210> 454
<211> 538
<212> DNA
<213> Homo sapiens

<400> 454
gccgggctgc taattctgtt taattgttcc tgggctaaaa agaattagaa ggaagctgtc 60
tgtttcccac tgcggttatg tttcagtaaa ttagacgtac tttctgatga atactaatta 120
gccactgagc atttgcaccc actgtctttg ctggttgtgt gcagaacagc tgccaagttg 180
cccaagaccc tcgctatccc atccccctct cttgctttcc acttttgggc ttcctttgcc 240
tagattagaa gagatttcag ttccgagaaa gtaaaaggtg atccaaggaa gtaatcaccg 300
agtgtctcat ggtttttcct tgttgacaaa attcaaaact cacacatgtg tagtctaatg 360
atagcgctag gatttaaaga aagtgtttta gtgctgtgct tatttaggac tacatttggg 420
gatgatgcat tcctttaaga ttgaatgatt ctgcccttgg gcagagctcc caattaggga 480
ggattaggta agctttttgt ggcgatgggt aataccattc ttttcctcat tgtgcctg   538

<210> 455
<211> 548
<212> DNA
<213> Homo sapiens

<400> 455
   tgaatcagta ggaatgtggg gaagggagtg aggggagacc ccctccttga ctcagcagtg 60
   gtgacggtcg gtgtgtcctg cagacctgaa gccaagatca agggggcttg agcaccagga 120
   gcccccgcag ttgctgaatg accagcggag ggcaggtgcc agcctgtggc aaaataggaa 180
   agaaaaggac aggatgggga cttcaccatt tttttcagcc ttaaattgtt ccttaaacct 240
   tcatgtcctt ttctctaatg tgtgttcttg tttggtaaaa taaaaaagtt tgtaaccctg 300
   agttctctaa agatatacat tcttttttac tggtttgtga agtcagaagg atgagagctg 360
   ctatttcttg gaaccgtgca ataaatatta gcatattcag tctcggttct gcctagagga 420
   cctatttgct tttctttatc tcgtaaccca taactcacag gacattaacc agggtgtcca 480
   agaacagtct gggaaagttt tgataattac ttcagcattg ctgtgtgatg ggagacattg 540
   ttttaaaa                                                          548

<210> 456
<211> 354
<212> DNA
<213> Homo sapiens

<400> 456
tcagtgggag tgaatcagta ggaatgtggg gaagggagtg aggggagacc ccctccttga 60
ctcagcagtg gtgacggtcg gtgtgtcctg cagacctgaa gccaagatca agggggcttg 120
agcaccagga gcccccgcag ttgctgaatg accagcggag ggcaggtgcc agcctgtggc 180
aaaataggaa agaaaaggac aggatgggga cttcaccatt tttttcagcc ttaaattgtt 240
ccttaaacct tcatgtcctt ttctctaatg tgtgttcttg tttggtaaaa taaaaaagtt 300
tgtaaccctg agttctctaa agatatacat tcttttttac tggtttgtga agtc      354

<210> 457
<211> 570
<212> DNA
<213> Homo sapiens

<400> 457
cttttatagg attcatttaa aggtgaataa aataatgaat gtgaaactca tattagagct 60

taacatatag tagtaatgat ttataaaata tttgcctccc ttagaccaga gcagctacta 120
aatttgattt taataataag ataaacaaat taataagatc acaaagttgt tatgtaataa 180
cataaacagc tgtgttaaaa ttagtagtga cccatatcaa agaaacacaa ttacaaagag 240
attaagaagg ataatattta aagtgtagct ttactcagtc ttttgtgtga aggtattctt 300
agggataaaa caatgtattt ggaagctgct ggaagaatat ggtgcaaaga atatttttaa 360
atgcttgtga atgttctgta accacaaaca tagatacata acagatcaaa gacatatttt 420
agactgccat gtggacttaa atcatgggag gcggaagagt ggctccccaa agaggactat 480
atcgtaatac cagaacttgt gaatatatta ctttaagtgg caaaagggac tttacagatg 540
tgattaaaat taaggacctt gaaatggggg 570

<210> 458
<211> 540
<212> DNA
<213> Homo sapiens

<400> 458
aactagactt cttttatagg attcatttaa aggtgaataa aataatgaat gtgaaactca 60
tattagagct taacatatag tagtaatgat ttataaaata tttgcctccc ttagaccaga 120
gcagctacta aatttgattt taataataag ataaacaaat taataagatc acaaagttgt 180
tatgtaataa cataaacagc tgtgttaaaa ttagtagtga cccatatcaa agaaacacaa 240
ttacaaagag attaagaagg ataatattta aagtgtagct ttactcagtc ttttgtgtga 300
aggtattctt agggataaaa caatgtattt ggaagctgct ggaagaatat ggtgcaaaga 360
atatttttaa atgcttgtga atgttctgta accacaaaca tagatacata acagatcaaa 420
gacatatttg agactggcat gtggacttaa atcatgggag gcggaagagt ggctccccaa 480
agaggactat atcgtagtac cagaacttgt gaatatatta ctttaagtgg caaaagggac 540

<210> 459
<211> 622
<212> DNA
<213> Homo sapiens

<400> 459
acttaagatt ttttcaatgt aagaaaaatg caatgaaata atagctgcaa atacccacta 60
ctaacaattg cttggccttc ttatatagac ctcccgaggt tctcatcttt tacatttcag 120
gagtagaatc agttaaaaac taatctttat atgtaaggga tgagagagag aaagaggagg 180
gtatgtgtat gcacacatgt gtgtgtgtgt ggtgggtagt aattttaatt caatgattta 240
ctagagttcg atgtcgtttg ctgataaatg aagcaggagg aagagccagg tttggagggg 300
acgagagaat gagttccatt tgtctcatat agaagttgaa gtaactgagt gatgatgggt 360
agagatgtcc ctcaggggta gccacagtat tttatttact ttttattcac cacatgcagc 420
aaggagcttt gttctccaaa atgctgtcaa ttatttttct aaattacagg tttgattgct 480
tcactgtatt ttcatgtctc attactacct ttacgcttaa aaccagaaac tgtgccacag 540
cgttaaagat tctgctaact tttaaaatac agaactctgg agatgccata attagattgc 600
agatttatga gtcttctgga ta 622

<210> 460
<211> 378
<212> DNA
<213> Homo sapiens

<400> 460
acaatgggtt tgttctctgc cttataaatt gggggattct agaggagtct gcttttctcc 60
caagaaggac ctcttctttt cttgcttttc atatgctctc cttgagatat cttgggtatt 120
ctcatggctt taaatagcac ttatatccag aagactcata aatctgcaat ctaattatgg 180
catctccaga gttctgtatt ttaaaagtta gcagaatctt taacgctgtg gcaaagtttc 240
tggttttaag cgtaaaggta gtaatgagac atgaaaatac cgtgaagcca tcaaacctgt 300
aatttaaaaa aataattgac agcattttgg agaacaaagc tccttgctgc atgtggggaa 360
taaaaagtaa ataaaata 378

<210> 461
<211> 396
<212> DNA

<213> Homo sapiens

<400> 461
ccttctgctc tacgagaact atgggcagtc ggaaacggga ctaatttgtg ccacctactg 60
gggaatgaag atcaagccgg gtttcatggg gaaggccact ccaccctacg acgtccaggt 120
cattgatgac aagggcagca tcctgccacc taacacagaa ggaaacattg gcatcagaat 180
caaacctgtc aggcctgtga gcctcttcat gtgctatgag ggtgacccag agaagacagc 240
taaagtggaa tgtggggact tctacaacac tggggacaga ggaaagatgg atgaagaggg 300
ctacatttgt ttcctgggga ggagtgatga catcattaat gcctctgggt atcgcatcgg 360
gcctgcagag gttgaaagtg ctttggtgga gcaccc 396

<210> 462
<211> 529
<212> DNA
<213> Homo sapiens

<400> 462
tttttttttt tttttttttt ttttttcggt agaaatgggg ttttaccatg ttgcccaggc 60
tagtctcgaa ctcctgggct taagcaatcc acacacctcg cttccaaaaa agctggggtt 120
acaggtgtga gccatcacac ccagcctaat atacaatctc aaatattttg ttttaaatca 180
ttacttactg aactataaag taaaactaat ttttagacag cattttaata catattttac 240
tttttaaagg ttataaagaa aacactaaca atatggaaaa tgcatattta aagaaaattg 300
aaatcaaata taatcttatg gctcaaaatc attagtgtta atattttgat acctaccttc 360
cccatctttt gcctacgaat actgggttaa gagtttttaa atagttttgt ccttgctttg 420
taattttcgt atgttctcac aaaagagaag ctgaggaagc atttggctat tgggaaaatt 480
aattaataga tgttaactta ccaagatata ctataataga ttagacagc 529

<210> 463
<211> 485
<212> DNA
<213> Homo sapiens

<400> 463
tttaaagtaa atgactcatg ttgaggaaag aggttattac ctaaatctgg actgcggcct 60
aaggaaattc ccttaacctc tattctggtt tcctatttca aaatggttgt gtaggaggct 120
aatggaagtt agttggttgc tatgatccaa aaactctatg ggtgaaaatt taaagtacag 180
atttcttatt taatcgttaa acagctttag ttgtgagttc tatgtcctgg tataatggat 240
cctgattatt aatgcattaa atatgcattc agtgaattca aatgttgcta attattcttt 300
taccaatcaa agaaaactca aagcatggga ttaagagggg ttggccaaaa gtatttggac 360
caggttgcat accaggacca tgaagaaatt gagaacagag cctacatctt ttatactatg 420
gctcaaagca agggctgttg gaatgtgctg cttctccaaa gtaggactta tgaaaaaatg 480
agggt 485

<210> 464
<211> 576
<212> DNA
<213> Homo sapiens

<400> 464
tatcagcatc tgtagaggag aaagcagaat aagcactggg gtatttgata gacttgagaa 60
taagagaacc ccaaagttgt caataggtat ttgctagaaa gttcagtggg tcagggtggg 120
aatagcagct gaaattggca gggattttga ctattcaaat aatgggtgag tagaagggat 180
ctgtggaata gccattatga cctcttgaaa ccaggcaact agggggtccc ttctagaatg 240
atgctgcgta cctaagaaat tcagtaggga gtggagtcaa aatgatcaga aaagatagag 300
atagttgtgg caaaagatga tctaagagtg tgtgtgtatg tgtgtgagtg agagagagaa 360
atctcaagaa atagtggcta tggtgttgaa cactacatga aagcaaccta aaacagctgt 420
gtgaagttag aaaaggtact ctggaccata ttgccctgta aaagctcagg aaaactaatt 480
ttgcataaac ataagcaaca ggaaattatt gctgtcaaat ctcattcaga gttattgtac 540
aaaaaaagag acaagaatcc ctatagacaa tgaaag 576

<210> 465
<211> 459

<212> DNA
<213> Homo sapiens

<400> 465
   ttatctaacg tttctaacag gggtgttaat gatattagca gcaagagcta tgagaaataa 60
   ctttagacat tatttcattg aaccttccca actgaaatta ttttatgatg ttataacatg 120
   gatagtaact caagtagcaa taagttacac agttgtgcca tttgtgcttc tttctataaa 180
   accatcactc acgttttaca gctcctggta ttattgcctg cacattcttg gtatcttagt 240
   attatcgttg ttgccagtga aaaaaactca aagaaggaag aatacacatg aaaacattca 300
   gctctcacaa tccaaaaagt ttgatgaagg agaaaattct ttgggacaga acagtttttc 360
   tacaacaaac aatgtttgca atcagaatca agaaatagcc tcgagacatt catcactaaa 420
   gcagtgatcg ggaaggctct gagggctgtt ttttttttt 459

<210> 466
<211> 250
<212> DNA
<213> Homo sapiens

<400> 466
tatacccagg atattatcta acgtgtctaa caggggtgtt aatgatatta gcagcaagag 60
ctatgagaaa taactttaga cattatttca ttgaaccttc ccaactgaaa ttattttatg 120
atgttataac atggatagta actcaagtag caataagtta cacagttgtg ccatttgtgc 180
ttctttctat aaaaccatca ctcacgtttt acagctcctg gtattattgc ctgcacattc 240
ttggtatctt 250

<210> 467
<211> 509
<212> DNA
<213> Homo sapiens

<400> 467
atactttatc tattttcggg caacttgctt ccctcatgaa ccatggacat ctcaatgtgc 60
cattacacac aggagttata tgttaggtat tgttgtccca ttttacagaa gagaatccgc 120
aaggttcaca gagtgaatca taggcataaa gtccttcagg tggtaaatgg caaggctggt 180
gttccaacca gtcttctctg gctccaggga ctggctcctt cagactacat ttcaccagct 240
gcctccagga acagaagacg ggaattcacc tttcatgcga catataccag aaacgtggac 300
ctcagccacc ctgggtccta tttgatcccc agggccttca tttggccctc gaataaaaac 360
cttatttttt tatctcctta cctttcccag aattcatagt aggacttggc tggtgaaagg 420
ctggttgctg agaaggctac agtgtggcta ggctgcagtt ccctgttatt acattgcccc 480
aggtattaat attgtatatt taggcagct 509

<210> 468
<211> 554
<212> DNA
<213> Homo sapiens

<400> 468
ggatttcaaa tctgagatga tactttatct attttcgggc aacttgcttc cctcatgaac 60
catggacatc tcaatgtgcc attacacaca ggagttatat gttaggtatt gttgtcccat 120
tttacagaag agaatccgca aggttcacag agtgaatcat aggcataaag tccttcaggt 180
ggtaaatggc aaggctggtg ttccaaccag tcttctctgg ctccagggac tggctccttc 240
agactacatt tcaccagctg cctccaggaa cagaagacgg gaattcacct ttcatgcgac 300
atataccaga aacgtggacc tcagccaccc tgggtcctat ttgatcccca gggccttcat 360
ttggccctcg aataaaaacc ttattttttt atctccttac ctttcccaga attcatagta 420
ggacttggct ggtgaaaggc tggttgctga gaaggctaca gtgtggctag gctgcagttc 480
cctgttatta cattgcccca ggtattaata ttgtatattt aggcagctgt tctcatccgt 540
gcctggcagt gaaa 554

<210> 469
<211> 537
<212> DNA
<213> Homo sapiens

<400> 469
attctgaccc cattgtgcac cttagtcatg gcaaactttc cagttgctcc ttgccaaaac 60
tcaagaataa aagggcccaa gctagagagg ctgtcctcac aagcatcagc tgctgggggc 120
ttccactcat tttcctctga aacaacagag aaagagacca tctctcattc gcagagcagc 180
ccaaggcctt ctgaggagac tgtgagtctc ctctaagtca tttctctctg ctttgtagca 240
gtggagctac caagggtgag atgagcaggt tgagaggcct ctgaagcctg ctgggcacaa 300
tgctctgtga taagtttcag ctccactgga gcttatcatc caccagcaat cgacttcatg 360
gctgctgctc agaggcccta ggtgctgcgc tgctcactgc cctcacgtct ctgggacttc 420
cacacataaa gccatctctt tccattgcac tatggcactt gtagggagga tcccacactt 480
agggcccaaa atgagaccat ttgagtcaaa tttctaattg tctttcaaat tttatta 537

<210> 470
<211> 492
<212> DNA
<213> Homo sapiens

<400> 470
attctgaccc cattgtgcac cttagtcatg gcaaactttc cagttgctcc ttgccaaaac 60
tcaagaataa aagggcccaa gctagagagg ctgtcctcac aagcatcagc tgctgggggc 120
ttccactcat tttcctctga aacaacagag aaagagacca tctctcattc gcagagcagc 180
ccaaggcctt ctgaggagac tgtgagtctc ctctaagtca tttctctctg ctttgtagca 240
gtggagctac caagggtgag atgagcaggt tgagaggcct ctgaagcctg ctgggcacaa 300
tgctctgtga taagtttcag ctccactgga gcttatcatc caccagcaat cgacttcatg 360
gctgctgctc agaggcccta ggtgctgcgc tgctcactgc cctcacgtct ctgggacttc 420
cacacataaa gccatctctt tccattgcac tatggcactt gtagggagga tcccacactt 480
agggcccaaa tg 492

<210> 471
<211> 509
<212> DNA
<213> Homo sapiens

<400> 471
aagacattca aattagccac cactggagta gatgacctaa aagttcttac aactctcaat 60
tatacccagt gatgtctcga ttagcactta ttataaaaat taaaatttat aattcaacat 120
ttataccatc cagaaaaagt taaaatatat taatagccta tttctcttca ataaagcgta 180
tatataactc tatttgttaa tgtttctatt ctccatgaca ttctgtttat agataagccc 240
tatgctattt ctagtcaagt gctaatctct tgaatgaagc tgaattaggt agtcaactac 300
tagatgtatc ctgaaaagca agtaatgtgt atatttcatt tattttatac ataagagcta 360
cagactgttg tcacaatctt ttcaagggct attaaattca ttattttaac taacattttt 420
gaacatctgt cttatgttgt taattgagga catttctgaa tgtataacaa cataagaata 480
atagttgtta aacttcaaag agatgacag 509

<210> 472
<211> 649
<212> DNA
<213> Homo sapiens

<400> 472
caaattagcc accactggag tagatgacct aaaagttctt acaactctca attataccca 60
gtgatgtctc gattagcact tattataaaa attaaaattt ataattcaac atttatacca 120
tccagaaaaa gttaaaatat attaatagcc tatttctctt caataaagcg tatatataac 180
tctatttgtt aatgtttcta ttctccatga cattctgttt atagataagc cctatgctat 240
ttctagtcaa gtgctaatct cttgaatgaa gctgaattag gtagtcaact actagatgta 300
tcctgaaaag caagtaatgt gtatatttca tttattttat acataagagc tacagactgt 360
tgtcacaatc ttttcaaggg ctattaaatt cattatttta actaacattt ttgaacatct 420
gtcttatgtt gttaattgag gacatttctg aatgtataac aacataagaa taatagtttt 480
taaacttcaa agagatgaca ggttaatgag taaaggagaa atatgaaata tcacagaatt 540
ccttgacact aaatgatgtt ttgcaaatac tgaacagaat gatgtttgta aactttccac 600
tggttttcaa gagtcccaaa acattaggaa aatgtacatc acctaactt 649

<210> 473
<211> 494
<212> DNA
<213> Homo sapiens

<400> 473
atatcagaag taaaacaatt tttcttgttg actgctttgg taaaaaacag tttgatggat 60
agttttacat ttcactggac tagataaaaa atggtgctaa tatttatgta gcttgatgct 120
atagttgctt tggtatcaaa cttaatacct aacccatata agatccttat tatataattt 180
tgtgatcagt aaaatgatat tttaaagagt gatcttaaaa atatgacctg gtcattgcac 240
aacgtttgca tttgaaatga atttttgtac tatagggtgg atatggagtt attcagtgca 300
agtgtgtgct taatatcaaa ccctatgcaa ggagctatgt ctagattttt ggtccgaatt 360
tgcctccttc aagcctacta gtgtgagatg gaaaaaaatc gattgctctt ttaatattat 420
ttccattttg aaattctcga cacttgaatg aaggcagtag aagcctcttt ttggatttct 480
cttctaataa caaa 494

<210> 474
<211> 630
<212> DNA
<213> Homo sapiens

<400> 474
aaaacatttt tcttgttgac tgctttggta aaaaacagtt tgatggatag ttttacattt 60
cactggacta gataaaaaat ggtgctaata tttatgtagc ttgatgctat agttgctttg 120
gtatcaaact taatacctaa cccatataag atccttatta tataattttg tgatcagtaa 180
aatgatattt taaagagtga tcttaaaaat atgacctggt cattgcacaa cgtttgcatt 240
tgaaatgaat ttttgtacta tagggtggat atggagttat tcagtgcaag tgtgtgctta 300
atatcaaacc ctatgcaagg agctatgtct agatttttgg tccgaatttg cctccttcaa 360
gcctactagt gtgagatgga aaaaaatcga ttgctctttt aatattattt ccattttgaa 420
attctcgaca cttgaatgaa ggcagtagag gcctcttttt ggatttctct tctaataaca 480
aaactttatt tagggaaggt ttccctgtgc tatcgtaagt ttgttttgag cactgcattc 540
actttaaaat tctggaggaa caaaggctgg gcacataatc acaaagccca ggccacacaa 600
taattccggg gttgtatttt ctaagaacta 630

<210> 475
<211> 156
<212> DNA
<213> Homo sapiens

<400> 475
gggggagata aggcaaagag gcacttttgg atttctccat ctgagcagct ctgtgattca 60
ttatctgttc tagaaagcag cacacgcagt tccagcaaaa aaaaaaaaaa aaaaaaattt 120
tttttttttt cccccctttt tttttttttt ttcccc 156

<210> 476
<211> 579
<212> DNA
<213> Homo sapiens

<400> 476
attccgttgc tgtcggcggc cgggtcccga tgagcctcct gttgcctccg ctggcgctgc 60
tgctgcttct cgcggcgctt gtggccccag ccacagccgc cactgcctac cggccggact 120
ggaaccgtct gagcggccta acccgcgccc gggtagagac ctgcggggga tgacagctga 180
accgcctaaa ggaggtgaag gctttcgtca cgcaggacat tccattctat cacaacctgg 240
tgatgaaaca cctccctggg gccgaccctg agctcgtgct gctgggccgc cgctacgagg 300
aactagagcg catcccactc agtgaaatga cccgcgaaga gatcaatgcg ctagtgcagg 360
agctcggctt ctaccgcaag gcggcgcccg acgcgcaggt gccccccgag tacgtgtggg 420
cgcccgcgaa gcccccagag gaaacttcgg accacgctga cctgtaggtc cgggggcgcg 480
gcggagctgg gacctacctg cctgagtcct ggagacagaa tgaagcgctc agcatcccgg 540
gaatacttct cttgctgaga gccgatgccc gtccccggg 579

<210> 477

<211> 472
<212> DNA
<213> Homo sapiens

<400> 477
ggcttagcgg ataacaattt cacacaggag ctagcagaca ccacaagata ccaacagagc 60
ttctgaaaca gatacccata gcattggaga gaaaaacagc tcacagtctg aggaagatga 120
tattgaaaga aggaaagaag ttgaaagcat cttgaagaaa aactcagatt ggatatggga 180
ttggtcaagt cggccggaaa atattccccc caaggagttc ctctaaacac ccgaagcgca 240
cggccaccct cagcatgagg aacacgagcg tcatgaagaa agggggcata ttctctgcag 300
aatttctgaa agatttcctt ccatctctgc tgctctctca tttgctggcc atcggattgg 360
ggatctatat tggaaggcgt gtgacaacct ccaccagcac cttttgatga agaactggag 420
tctgacttgg ttcgttagtg gattacttct gagcttgcaa catagctcac tg 472

<210> 478
<211> 355
<212> DNA
<213> Homo sapiens

<400> 478
tctacactta aagctttgga gcaattccca tcgaccagag ttggtccgac cagccttgga 60
aaggtcactg aaaaatcttc aattggacta tggtgacctc tatcttatac attttccatt 120
gtctgcaaag ccaggtgagg aagtgatccc aaaagatgac aatggaaaaa tactatttga 180
cacagtggat ctctgtgcca catgggaggc catggagaag tgtaaagatg cacgattggc 240
caagtccatc ggggtgtcca acttcaacca caggctgctg gagatgatcc tcaacaagcc 300
agggctcaag tacaagcctg tctgcaacca ggtggaatgt catccttact tcaac 355

<210> 479
<211> 510
<212> DNA
<213> Homo sapiens

<400> 479
   aagactactg aatctgctac caaaacagtg aatcagtgag tcgatgttct attttttgtt 60
   ttgtttcctc ccctatctgt attcccaaaa attactttgg ggctaattta acaagaactt 120
   taaattgtgt tttaattgta aaaatggcag ggggtggaat tattactcta tacattcaac 180
   agagactgaa tagatatgaa agctgatttt ttttaattac catgcttcac aatgttaagt 240
   tatatgggga gcaacagcaa acaggtgcta atttgttttg gatatagtat aagcagtgtc 300
   tgtgttttga aagaatagaa cacagtttgt agtgccactg ttgttttggg ggggcttttt 360
   tcttttcgga aatcttaaac cttaagatac taaggacgtt gttttggttg tactttggaa 420
   ttcttagtca caaaatatat tttgtttaca aaaatttctg taaaacaggt tataacagtg 480
   tttaaagtct cagtttcttg cttggggaac 510

<210> 480
<211> 371
<212> DNA
<213> Homo sapiens

<400> 480
   ttccgttgct gtcggaattg aggaagagct gggggatgaa gctcgctttg ccggacataa 60
   cttccgtaat cccagtgtgc tgtgattcct ctgcttgcct ggagacgtgg aacctctgtc 120
   tcatcctcct ggaaccttgc tgtcctgatc tgtgatagtt caccccctga gatcccctga 180
   gccccagggt gcccagaact tccctgattg acctgctccg ctgctccttg gcttacctga 240
   cctcttgctg tctctgctcg ccctcctttc tgtgccctac tcattggggt tccgcacttt 300
   ccacttcttc ctttctcttt ctctcttccc tcaaaaacta gaaatgtgaa tgaggattat 360
   tataaaaggg g 371

<210> 481
<211> 543
<212> DNA
<213> Homo sapiens

<400> 481
aattccgttg ctgtcggtgt ctggaggcca tcctccagaa ctctcctgac gccaaaatct 60
tctgcctggt gcacaaaatg gatctggttc aggaggatca gcgtgacctg atttttaaag 120
agcgagagga agacctgagg cgtctgtctc gcccgctgga gtgtgcttgt tttcgaacgt 180
ccatctggga tgagacgctc tacaaagcct ggtccagcat cgtctaccag ctgattccca 240
acgttcagca gctggagatg aacctcagga attttgccca aatcattgag gccgatgaag 300
ttctgctgtt cgaaagagct acattcttgg ttatttccca ctaccagtgc aaagagcagc 360
gcgacgtcca ccggtttgag aagatcagca acatcatcaa acagttcaag ctgagctgca 420
gtaaattggc cgcttccttc cagagcatgg aagttaggaa ttccaacttc gctgctttca 480
tcgacatctt cacctcaaat acgtacgtga tggtggtcat gtcagatccg tcgatccctt 540
ctg 543

<210> 482
<211> 415
<212> DNA
<213> Homo sapiens

<400> 482
ggcttactca ctatagggct tttttttttt tcgggtctat tctttaattt tactaaatta 60
ggaacgcagc ttttacagaa caaataaccc caggggacgg ggccccccca ggatctaaca 120
gcttttcagg gagctatgtt gcaagctcaa aagtaatcca ctaacgaacc aagtcaaact 180
ccagttttta ataaaaaggg gctgggggag gttgtcaaac cccttccaat ataaatcccc 240
aatccgatgg ccaccaaatg aaaaagcacc agggatggaa ggaaaacttt caaaaattct 300
gcaaaaaata tgcccccttt tttaatgacc ctcgggttcc taatgctaag gggggccgcc 360
cccttcgggg gttaaaaaag gaactccttg gggggaatat tttccggccg acttg 415

<210> 483
<211> 240
<212> DNA
<213> Homo sapiens

<400> 483
   tttttttttt taaagtcatg gaggccatgg ggttggcttg aaaccacctt tggggggtcc 60
   aatcccttcc ttttttgcct aaattttatg tatacgggtt cttcaaatgc gtggtagggg 120
   ggggggcatc catatagtcc ctccaggttt atggagggtt cttctactat taggactttt 180
   cgcttcaaaa caaaggcttt tcaaatcatg aaaattttta attttcctgc tgttaaaaaa 240

<210> 484
<211> 293
<212> DNA
<213> Homo sapiens

<400> 484
tttttttttt aataaatctc ctaaggggat ggctactttt tctatctaaa taataatata 60
tagacctatt cgatcagaga tacaggggac taacaatcac aatcctgtga tcgacatccg 120
aacataagtc actatctatc agaataaaca atgatccaac gaataataga ggagtaaggg 180
gacatgtcca aagcatcagg tatcgtcatg atcgaaaacc actgtcaagc aagacacaaa 240
caaacaaaac agctttacac acaagtcagc agtccaagcg ttcatgtccc aag 293

<210> 485
<211> 221
<212> DNA
<213> Homo sapiens

<400> 485
tttttttttt tcaaggacaa ctttaatggt taacttaagg gatcatcaat tttgcctcac 60
tacctacaaa gggaatttca tcttgtcccc atgctgagta gggaaacagg gacaaagtta 120
atcataatac cctacatcaa aaaaaaacta agctaacact gctaactttt tttttaacag 180
gcaaaatata aatatatgcc ctctaaaatg cccaagggtt t 221

<210> 486

<211> 563
<212> DNA
<213> Homo sapiens

<400> 486
ttccgttgct gtcgcctccg ctctgctctt cgtggaacac gaccgtggtg cccggccctt 60
gggagccttg gggccagctg gcctgctgct ctccagtcaa gtagcgaagc tcctaccacc 120
cagacaccca aacagccgtg gccccagagg tcctggccaa atatgggggc ctgcctaggt 180
tggtggaaca gtgctcctta tgtaaactga gccctttgtt taaaaaacaa ttccaaatgt 240
gaaactagaa tgagagggaa gagataacat ggcatgcagc acacacggct gctccagttc 300
atggcctccc aggggtgctg gggatgcatc caaagtggtt gtctgagaca gagttggaaa 360
ccctcaccaa ctggcctctt caccttccac attatcccgc tgccaccggc tgccctgtct 420
cactgcagat tcaggaccag cttgggctgc gtgcgttctg ccttgccagt cagccgagga 480
tgtagttgtt gctgccgtcg tcccaccacc tcagggacca gagggctagg ttggcactgc 540
ggccctcacc aggtcctggg ctc 563

<210> 487
<211> 271
<212> DNA
<213> Homo sapiens

<400> 487
ctcatatggt caggtcgctt caaaaaggaa gatgaaatcc cagagactgt ctcgttggag 60
atgcttgatg ctgcaaagaa caagatgcga gtgaagatca gctatctaat gattgccctg 120
acggtggtag gatgcatctt catggttatt gagggcaaga aggctgccca aagacacgag 180
actttaacaa gcttgaactt agaaaagaaa gctcgtctga aagaggaagc agctatgaag 240
gccaaaacag agtagcagag gtatccgtgt t 271

<210> 488
<211> 342
<212> DNA
<213> Homo sapiens

<400> 488
ggcttgtaat acgactcact atagggcttt ttttttttcg aattaaaaaa attccgttag 60
ccttttctcc atctcctcta attctggtag catctttgga cccctaacac ttggcatctg 120
ctacttcaga caaacaaacc ctatgtaaat gacaaagaag gggcctccca accttctccc 180
tgtgttacta tttcaaaagc actactcggg gcacaggggt acaaatttct tatggccact 240
agcatctttt ttcaattttc aaaggaatca tcaaacatct gggtcaatta tacttaaatt 300
acagaagccc ggaattttag gcaacaggcc cctcatttta cc 342

<210> 489
<211> 326
<212> DNA
<213> Homo sapiens

<400> 489
tttttttttt aaaaagtcat ggaggccatg gggttggctt gaaaccagct ttgggggtt 60
cgattccttc cttttttgtc taaattttat gtatacgggt tcttcaaatg tgtggtaggg 120
tggggggcat ccatatagtc actccaggtt tatggagggt tcttctacta ttaggacttt 180
tcgcttcgaa gcgaaggctt ctcaaatcat gaaaattatt aatattactg ctgttagaaa 240
aatgaatgag cctaccgatg ataggatgtt tcatgtggtg tatgcatcgg ggtagtccga 300
gtaacgtcgg ggcattccgg ataggc 326

<210> 490
<211> 55
<212> DNA
<213> Homo sapiens

<400> 490
tttttttttt tttttttttg agaaaccggg gggggttttt tttttaaaat tgggg 55

<210> 491
<211> 558
<212> DNA
<213> Homo sapiens

<400> 491
cgccgcgtcc ccttctcgct cctgcggggc cccagctggg accccttccg cgactggtac 60
ccgcatagcc gcctcttcga ccaggccttc gggctgcccc ggctgccgga ggagtggtcg 120
cagtggttag gcggcagcag ctggccaggc tacgtgcgcc ccctgccccc cgccgccatc 180
gagagccccg cagtggccgc gcccgcctac agccgcgcgc tcagccggca actcagcagc 240
ggggtcttcg gagatccggc acactgcgga ccgctggcgc gtgtccctgg atgtcaacca 300
cttcgccccg gacgagctga cggtcaagac caaggatggc gtggtggaga tcaccggcaa 360
gcacgaggag cggcaggacg agcatggcta catctcccgg tgcttcacgc ggaaatacac 420
gctgcccccc ggtgtggacc ccacccaagt ttcctcctcc ctgtcccctg agggcacact 480
gaccgtggag gcccccatgc ccaagctagc cacgcagtcc aacgagatca ccatcccagt 540
caccttcgag tcgcgggc 558

<210> 492
<211> 370
<212> DNA
<213> Homo sapiens

<400> 492
ggctagcgga taacaatttc acacaggatg gattggtcag agtgaattga atattgtaag 60
tcagccactg ggacccgagg atttctggga ccccgcagtt gggaggagga agtagtccag 120
ccttccaggt ggcgtgagag gcaatgactc gttacctgcc gcccatcacc ttggaggcct 180
tccctggcct tgagtagaaa agtcggggat cggggcaaga gaggctgagt acggatggga 240
aactattgtg cacaagtctt tccagaggag tttcttaatg agatatttgt atttatttcc 300
agaccaataa atttgtaact ttgcgaaaaa aaaaaagccc tatagtgagt cgtattacaa 360
gccgaattcc 370

<210> 493
<211> 560
<212> DNA
<213> Homo sapiens

<400> 493
cagccagcat gaccgagcgc cgcgtcccct tctcgctcct gcggggcccc agctgggacc 60
ccttccgcga ctggtacccg catagccgcc tcttcgacca ggccttcggg ctgccccggc 120
tgccggagga gtggtcgcag tggttaggcg gcagcagctg gccaggctac gtgcgccccc 180
tgccccccgc cgccatcgag agccccgcag tggccgcgcc cgcctacagc cgcgcgctca 240
gccggcaact cagcagcggg gtctcggaga tccggcacac tgcggaccgc tggcgcgtgt 300
ccctggatgt caaccacttc gccccggacg agctgacggt caagaccaag gatggcgtgg 360
tggagatcac cggcaagcac gaggagcggc aggacgagca tggctacatc tcccggtgct 420
tcacgcggaa atacacgctg ccccccggtg tggaccccac ccaagtttcc tcctccctgt 480
cccctgaggg cacactgacc gtggaggccc ccatgcccaa gctagccacg cagtccaacg 540
agatcaccat cccagtcacc 560

<210> 494
<211> 443
<212> DNA
<213> Homo sapiens

<400> 494
ggcttgtaat acgactcact atagggcttt tttttttgca agtgctgtgg gaagaaagtt 60
agatttacgc cgatgaatat gatagtgaaa tggattttgg cgtaggtttg gtctagggtg 120
tagcctgaga ataggggaaa tcagtgaatg aagcctccta tgatggcaaa tacagctcct 180
attgatagga catagtggaa gtgagctaca acgtagtacg tgtcgtgtag tacgatgtct 240
agtgatgagt ttgctaatac aatgccagtc aggccaccta cggtgaaaag aaagatgaat 300
cctagggctc aaagcactgc agcagatcat ttcatattgc ctccgtggag tgtggcgagt 360
cagctaaata ctttgacgcc ggtggggata gcgatgatta tggtagcatc atcctgtgtg 420
aaattgttat ccgctaagcc gaa 443

<210> 495
<211> 249
<212> DNA
<213> Homo sapiens

<400> 495
tttttttttt cgaaggattt ggcaaagatt tgtttttttt tccatttcca gttttttaaa 60
gtaaacacag atttgcttaa aataaagctg attttaaaag cccacaaaag ttgaacacaa 120
aggagaggat taaattcccc aatgcagagt gataaaaagg aaaagatcct gagtaggtgc 180
cttcagcaaa aaactgatca tccagggtga tcacctaata atcggagact taattcctta 240
taatgcaaa 249

<210> 496
<211> 434
<212> DNA
<213> Homo sapiens

<400> 496
tttccgtatc tgcttcgggc ttccacctca tttttttcgc tttgcccatt ctgtttcagc 60
cagtcgccaa gaatcatgaa agtcgccagt ggcagcaccg ccaccgccgc cgcgggcccc 120
agctgcgcgc tgaaggccgg caagacagcg agcggtgcgg gcgaggtggt gcgctgtctg 180
tctgagcaga gcgtggccat ctcgcgctgc gccgggggcg ccggggcgcg cctgcctgcc 240
ctgctggacg agcagcaggt aaacgtgctg ctctacgaca tgaacggctg ttactcacgc 300
ctcaaggagc tggtgcccac cctgccccag aaccgcaagg tgagcaaggt ggagattctc 360
cagcacgtca tcgactacat cagggacctt cagttggagc tgaactcgga atccgaagtt 420
ggaacccccg gggg 434

<210> 497
<211> 368
<212> DNA
<213> Homo sapiens

<400> 497
tttttttttg cttatggagg gttcctctac tattaggact tttcgcttcg aagcgaaggc 60
ttctcaaatc atgaaaatta ttaatattac tgctgttaga gaagtgaatg accctacaga 120
tgataggatg tttcatgtgg tgtatgcatc ggggtagtcc gagtaacgtc ggggcattcc 180
ggataggccg aaaaagtgtt gtgggaaaaa agttagattt accccgatga atatgatagt 240
gaaatggatt ttggcgtagg tttggtctag ggtgtaccct gagaataggg gaaatcagtg 300
aatgaagcct cctatgatgg caaatacagc tcctattgat aggacatagt ggaagtgagc 360
tacaacgt 368

<210> 498
<211> 482
<212> DNA
<213> Homo sapiens

<400> 498
ccagccttcc tgtcccgggc cagcgctctg acatgcagaa ggtgaccctg ggcctgcttg 60
tgttcctggc aggctttcct gtcctggacg ccaatgacct agaagataaa aacagtcctt 120
tctactatga ctggcacagc ctccaggttg gcgggctcat ctgcgctggg gttctgtgcg 180
ccatgggcat catcatcgtc atgagtgcaa aatgcaaatg caagtttggc cagaagtccg 240
gtcaccatcc aggggagact ccacctctca tcaccccagg ctcagcccaa agctgatgag 300
gacagaccag ctgaaattgg gtggaggacc gttctctgtc cccaggtcct gtctctgcac 360
agaaacttga actccaggat ggaattcttc ctcctctgct gggactcctt tgcatggcag 420
ggcctcatct cacctctcgc aagagggtct ctttgttcaa ttttttttta tctaaaatga 480
tt 482

<210> 499
<211> 489
<212> DNA
<213> Homo sapiens

<400> 499
tggcgagcag tttcccactt gccaaagatc ccttttaacc aacactagcc cttgttttta 60
acacacgctc cagcccttca tcagcctggg cagtcttacc aaaatgttta aagtgatctc 120
agaggggccc atggattaac gccctcatcc caaggtccgt cccatgacat aacactccac 180
acccgcccca gccaacttca tgggtcactt tttctggaaa ataatgatct gtacagacag 240
gacagaatga aactcctgcg gctctttggc ctgaaagttg ggaatggttg ggggagagaa 300
gggcagcagc ttattggtgg tcttttcacc attggcagaa acagtgagag ctgtgtggtg 360
cagaaatcca gaaatgaggt gtagggaatt ttgcctgcct tcctgcagac ctgagctggc 420
tttggaatga ggttaaagtg tcagggacgt tgcctgagcc caaatgtgta gtgtggtctg 480
ggcaggcag 489

<210> 500
<211> 25
<212> DNA
<213> Artificial Sequence

<220>
<223> Oligonucleotide primer

<400> 500
ggaatcaccg ctttgccatc ttcaa 25

<210> 501
<211> 25
<212> DNA
<213> Artificial Sequence

<220>
<223> Oligonucleotide primer

<400> 501
aacttctacc gtttcgccac taagg 25

<210> 502
<211> 23
<212> DNA
<213> Artificial Sequence

<220>
<223> Oligonucleotide primer

<400> 502
gaccgtgtac tgcgtgtcgt gcg 23

<210> 503
<211> 23
<212> DNA
<213> Artificial Sequence

<220>
<223> Oligonucleotide primer

<400> 503
gcgtgctgtg cgtcatgtgc cag 23

<210> 504
<211> 24
<212> DNA
<213> Artificial Sequence

<220>

<223> Oligonucleotide primer

<400> 504
gccgtcttca ggcaacaact ccca                                              24

<210> 505
<211> 24
<212> DNA
<213> Artificial Sequence

<220>
<223> Oligonucleotide primer

<400> 505
tgctggacga ggctgtcatc ttgc                                              24

<210> 506
<211> 25
<212> DNA
<213> Artificial Sequence

<220>
<223> Oligonucleotide primer

<400> 506
acagggagaa aactggttgt cctgg                                             25

<210> 507
<211> 23
<212> DNA
<213> Artificial Sequence

<220>
<223> Oligonucleotide primer

<400> 507
aaggcagaac ccatccactc caa                                               23

<210> 508
<211> 25
<212> DNA
<213> Artificial Sequence

<220>
<223> Oligonucleotide primer

<400> 508
gctgctggat tcgtttggca taact                                             25

<210> 509
<211> 25
<212> DNA
<213> Artificial Sequence

<220>
<223> Oligonucleotide primer

<400> 509
tcaatacggt ttgcttaggt cgtcg                                             25

<210> 510
<211> 24

<212> DNA
<213> Artificial Sequence

<220>
<223> Oligonucleotide primer

<400> 510
tctcctctga gttcaaccgc tgct                                              24

<210> 511
<211> 24
<212> DNA
<213> Artificial Sequence

<220>
<223> Oligonucleotide primer

<400> 511
tcgtcgccaa cttgagtctc ctct                                              24

<210> 512
<211> 406
<212> PRT
<213> Homo sapiens

<400> 512
Met Ala Glu Asn Gly Lys Asn Cys Asp Gln Arg Arg Val Ala Met Asn
1               5                   10                  15
Lys Glu His His Asn Gly Asn Phe Thr Asp Pro Ser Ser Val Asn Glu
            20                  25                  30
Lys Lys Arg Arg Glu Arg Glu Glu Arg Gln Asn Ile Val Leu Trp Arg
        35                  40                  45
Gln Pro Leu Ile Thr Leu Gln Tyr Phe Ser Leu Glu Ile Leu Val Ile
    50                  55                  60
Leu Lys Glu Trp Thr Ser Lys Leu Trp His Arg Gln Ser Ile Val Val
65                  70                  75                  80
Ser Phe Leu Leu Leu Leu Ala Val Leu Ile Ala Thr Tyr Tyr Val Glu
                85                  90                  95
Gly Val His Gln Gln Tyr Val Gln Arg Ile Glu Lys Gln Phe Leu Leu
            100                 105                 110
Tyr Ala Tyr Trp Ile Gly Leu Gly Ile Leu Ser Ser Val Gly Leu Gly
        115                 120                 125
Thr Gly Leu His Thr Phe Leu Leu Tyr Leu Gly Pro His Ile Ala Ser
    130                 135                 140
Val Thr Leu Ala Ala Tyr Glu Cys Asn Ser Val Asn Phe Pro Glu Pro
145                 150                 155                 160
Pro Tyr Pro Asp Gln Ile Ile Cys Pro Asp Glu Glu Gly Thr Glu Gly
                165                 170                 175
Thr Ile Ser Leu Trp Ser Ile Ile Ser Lys Val Arg Ile Glu Ala Cys
            180                 185                 190
Met Trp Gly Ile Gly Thr Ala Ile Gly Glu Leu Pro Pro Tyr Phe Met
        195                 200                 205
Ala Arg Ala Ala Arg Leu Ser Gly Ala Glu Pro Asp Asp Glu Glu Tyr
    210                 215                 220
Gln Glu Phe Glu Glu Met Leu Glu His Ala Glu Ser Ala Gln Asp Phe
225                 230                 235                 240
Ala Ser Arg Ala Lys Leu Ala Val Gln Lys Leu Val Gln Lys Val Gly
                245                 250                 255
Phe Phe Gly Ile Leu Ala Cys Ala Ser Ile Pro Asn Pro Leu Phe Asp
            260                 265                 270
Leu Ala Gly Ile Thr Cys Gly His Phe Leu Val Pro Phe Trp Thr Phe
        275                 280                 285

Phe Gly Ala Thr Leu Ile Gly Lys Ala Ile Ile Lys Met His Ile Gln
    290                 295                 300
Lys Ile Phe Val Ile Ile Thr Phe Ser Lys His Ile Val Glu Gln Met
305                 310                 315                 320
Val Ala Phe Ile Gly Ala Val Pro Gly Ile Gly Pro Ser Leu Gln Lys
                325                 330                 335
Pro Phe Gln Glu Tyr Leu Glu Ala Gln Arg Gln Lys Leu His His Lys
            340                 345                 350
Ser Glu Met Gly Thr Pro Gln Gly Glu Asn Trp Leu Ser Trp Met Phe
        355                 360                 365
Glu Lys Leu Val Val Val Met Val Cys Tyr Phe Ile Leu Ser Ile Ile
    370                 375                 380
Asn Ser Met Ala Gln Ser Tyr Ala Lys Arg Ile Gln Gln Arg Leu Asn
385                 390                 395                 400
Ser Glu Glu Lys Thr Lys
                405

<210> 513
<211> 1221
<212> DNA
<213> Homo sapiens

<400> 513
atggcagaga atggaaaaaa ttgtgaccag agacgtgtag caatgaacaa ggaacatcat 60
aatggaaatt tcacagaccc ctcttcagtg aatgaaaaga agaggaggga gcgggaagaa 120
aggcagaata ttgtcctgtg gagacagccg ctcattacct tgcagtattt ttctctggaa 180
atccttgtaa tcttgaagga atggacctca aaattatggc atcgtcaaag cattgtggtg 240
tcttttttac tgctgcttgc tgtgcttata gctacgtatt atgttgaagg agtgcatcaa 300
cagtatgtgc aacgtataga gaaacagttt cttttgtatg cctactggat aggcttagga 360
attttgtctt ctgttgggct tggaacaggg ctgcacacct ttctgcttta tctgggtcca 420
catatagcct cagttacatt agctgcttat gaatgcaatt cagttaattt tcccgaacca 480
ccctatcctg atcagattat ttgtccagat gaagagggca ctgaaggaac catttctttg 540
tggagtatca tctcaaaagt taggattgaa gcctgcatgt ggggtatcgg tacagcaatc 600
ggagagctgc ctccatattt catggccaga gcagctcgcc tctcaggtgc tgaaccagat 660
gatgaagagt atcaggaatt tgaagagatg ctggaacatg cagagtctgc acaagacttt 720
gcctcccggg ccaaactggc agttcaaaaa ctagtacaga aagttggatt ttttggaatt 780
ttggcctgtg cttcaattcc aaatccttta tttgatctgg ctggaataac gtgtggacac 840
tttctggtac ctttttggac cttctttggt gcaaccctaa ttggaaaagc aataataaaa 900
atgcatatcc agaaaatttt tgttataata acattcagca agcacatagt ggagcaaatg 960
gtggctttca ttggtgctgt ccccggcata ggtccatctc tgcagaagcc atttcaggag 1020
tacctggagg ctcaacggca gaagcttcac cacaaaagcg aaatgggcac accacaggga 1080
gaaaactggt tgtcctggat gtttgaaaag ttggtcgttg tcatggtgtg ttacttcatc 1140
ctatctatca ttaactccat ggcacaaagt tatgccaaac gaatccagca gcggttgaac 1200
tcagaggaga aaactaaata a                                           1221

<210> 514
<211> 338
<212> DNA
<213> Homo sapiens

<400> 514
gtgctgtccc cggcataggt ccatctctgc agaagccatt tcaggagtac ctggaggctc 60
aacggcagaa gcttcaccac aaaagcgaaa tgggcacacc acagggagaa aactgcttgt 120
cctggatgtt tgaaaagtcg gtcgatgtca tggtgtgtta cttcatccta tctatcatta 180
actccatggc acaaagttat gccaaacgaa tccagcagcg gttgaactca gaggagaaaa 240
ctaaataagt agagaaagtt ttaaactgca gaaattggag tggatgggtt ctgccttata 300
ttgggaggac tccaagccgg gaaggaaaat tccctttt                         338

<210> 515
<211> 186
<212> DNA

<213> Homo sapiens

<400> 515
tgtgttaatg ttttctagca tgtactctgg tttcaacaga cacaaattta tatgttaacc 60
cagttttctt gccgttctgt aagtgtttta ttcttagtgt gatttttttc cattgggatg 120
tttttgattg aacttgttca ttttgttttg cttgggagga aaataaacaa ttttactttt 180
ttcctt 186

<210> 516
<211> 118
<212> DNA
<213> Homo sapiens

<400> 516
acagggagaa aactggttgt cctggatgtt tgaaaagttg gtcgttgtca tggtgtgtta 60
cttcatccta tctatcatta actccatggc acaaagttat gccaaacgaa tccagcag 118